(19) Europäisches Patentamt / European Patent Office / Office européen des brevets

(11) **EP 4 342 884 A1**

(12) **EUROPEAN PATENT APPLICATION**
published in accordance with Art. 153(4) EPC


(43) Date of publication:
**27.03.2024 Bulletin 2024/13**

(21) Application number: **22804044.0**

(22) Date of filing: **19.05.2022**

(51) International Patent Classification (IPC):
***C07D 223/16*** (2006.01) ***C07D 403/12*** (2006.01)
***A61K 31/55*** (2006.01) ***A61P 35/00*** (2006.01)
***A61P 31/12*** (2006.01)

(52) Cooperative Patent Classification (CPC):
**A61K 31/55; A61K 38/07; A61K 38/08; A61K 47/68; A61P 31/12; A61P 35/00; C07D 223/16; C07D 401/12; C07D 403/12; C07D 471/04; C07D 519/00; C07K 5/02; C07K 7/02**

(86) International application number:
**PCT/CN2022/093953**

(87) International publication number:
**WO 2022/242724 (24.11.2022 Gazette 2022/47)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **19.05.2021 CN 202110547613**
**21.07.2021 CN 202110825530**
**11.05.2022 CN 202210515784**



(71) Applicant: **Shanghai de Novo Pharmatech Co., Ltd.**
**Shanghai 201203 (CN)**

(72) Inventors:
- **TONG, Zhaolong**
**Shanghai 201203 (CN)**
- **ZHANG, Man**
**Shanghai 201203 (CN)**
- **YANG, Heping**
**Shanghai 201203 (CN)**
- **LIU, Xiaohuan**
**Shanghai 201203 (CN)**
- **WANG, Longsheng**
**Shanghai 201203 (CN)**
- **REN, Jicheng**
**Shanghai 201203 (CN)**
- **GAO, Daxin**
**Shanghai 201203 (CN)**

(74) Representative: **Henderson, Helen Lee**
**Withers & Rogers LLP**
**2 London Bridge**
**London SE1 9RA (GB)**


(54) **NITROGEN-CONTAINING COMPOUND, CONJUGATE CONTAINING SAID COMPOUND, AND APPLICATION THEREOF**


(57) The present application relates to a nitrogen-containing compound, a conjugate containing said compound, and an application thereof. Specifically, the present application provides a compound represented by formula (I), a solvate thereof, a pharmaceutically acceptable salt thereof, or a solvate of a pharmaceutically acceptable salt thereof. The nitrogen-containing compound has a good regulating effect on TLR8 and can effectively treat, alleviate, an/or prevent various diseases caused by immunosuppression, such as a cancer or a viral infection.


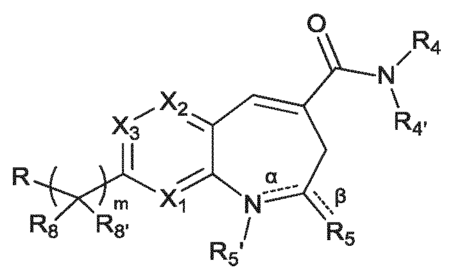

(I)

EP 4 342 884 A1

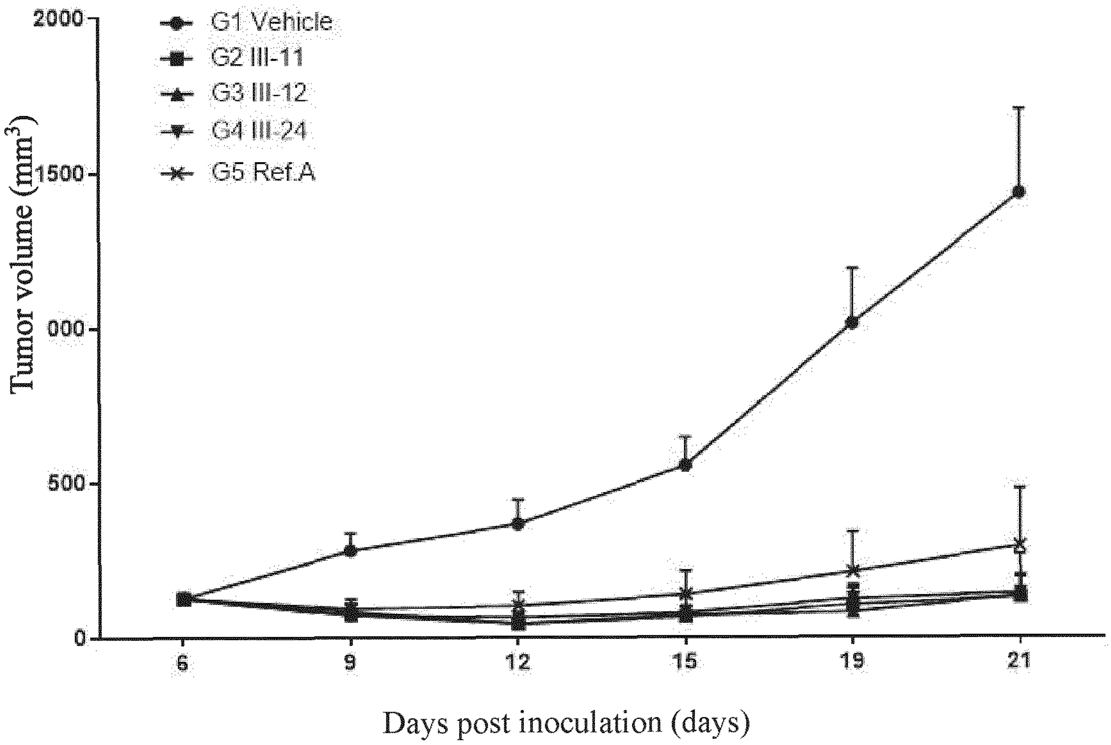
G1 Vehicle
G2 III-11
G3 III-12
G4 III-24
G5 Ref.A
Tumor volume (mm3)
2000
1500
000
500
0
6
9
12
15
19
21
Days post inoculation (days)

Figure 1

## Description

The present application claims the priorities of Chinese patent application 202110547613.7 filed on May 19, 2021, Chinese patent application 202110825530.X filed on July 21, 2021, and Chinese patent application 202210515784.6 filed on May 11, 2022. The contents of the above Chinese patent applications are incorporated herein by reference in their entireties.

TECHNICAL FIELD

The present disclosure relates to a nitrogen-containing compound, a conjugate containing the same, and a use thereof.

BACKGROUND

Toll-like receptors family (TLRs) is an important family of proteins for recognizing pathogen-associated molecular patterns, and it can sense and initiate innate immune responses and promote the development of adaptive immune responses. TLRs are mainly expressed in immune cells (such as myeloid dendritic cells (mDC), plasmacytoid dendritic cells (pDC), monocytes, and B cells (Kawai and Akira, 2010)) and lung. In humans, more than 10 TLRs are deemed to have significant functions. TLR1/2/4/5 and 6 are located in the cell membranes, and their primary function is to recognize extracellular macromolecular ligands from bacteria and fungi. In contrast, TLR3/7/8/9 are located in intracellular endosomal membranes, and their primary function is to recognize exogenous nucleic acids from intracellular pathogenic cells. Although most TLRs function through specific signaling pathways (primarily through MyD88-dependent pathways), different TLRs can coordinate various downstream molecules. The addition of specific TLRs leads to the activation of different cell populations (Schreibelt, et al., 2010) and the production of different patterns of cytokines and other inflammatory mediators (Ghosh, et al., 2006), thereby eliciting different immune responses. For example, after binding with a ligand, TLR8 dimerizes and undergoes conformational changes that lead to the recruitment of the adapter protein MyD88. MyD88 then recruits interleukin-1 receptor-associated kinase, resulting in the activation of downstream signaling pathways, including mitogen-activated protein kinases and the transcription factor NF-$\kappa$B.

TLRs located in endosomes, primarily TLR7/8/9, have been considered as highly appealing new targets for anticancer immunotherapy (Kanzler, et al., 2007; Kreig 2008; Smits, et al., 2008; Hennessy, et al., 2010; Kaczanowska, et al., 2013; Beesu, et al., 2016). For example, TLR7 activation of pDCs leads to a response against viral infections, inducing high levels of interferon $\alpha$ and promoting adaptive T cell responses against endogenous viral antigens (Liu, et al., 2009). Compared with TLR7/9, TLR8 is more widely expressed on different subtypes of immune cells. Regulatory T cells (Tregs) possess potent immune response suppression capabilities, representing a major hurdle for effective cancer immunotherapy. TLR8 signaling pathway has been demonstrated to be a necessary and sufficient condition for reversing the suppressive function of Treg cells, leading to potent tumor suppression. TLR8 selective agonists effectively activate various immune cells, including mDCs and monocytes (Gorden, et al., 2005), and can promote the generation of adaptive immune responses against cancer cells (Krug, et al., 2003; Schnurr, et al., 2005). Activated mDCs phagocytose apoptotic and necrotic tumor cells, and subsequently, compared to pDCs, more effectively cross-present tumor-associated antigens to CD8+ CTLs (Berard, et al., 2000; Dalgaard, et al., 2005). In addition, the activation of mDCs leads to the release of TNF$\alpha$ and interleukin-12 (IL-12), which can stimulate the activation of T cells and NK cells. Activation of NK cells is the main mechanism of antibody-mediated cytotoxicity (ADCC). Consequently, enhancing the killing of tumor cells through ADCC may present significant therapeutic opportunities for TLR8 selective inhibitors (Lu, et al., 2011). Some monoclonal antibody therapies are widely used in the treatment of cancer patients, such as rituximab and trastuzumab, which can exert therapeutic efficacy through ADCC (Ferris, et al., 2010). Indeed, the addition of TLR8 agonists to mAb therapy methods can enhance ADCC, thus increasing the therapeutic efficacy of mAb treatments (Ferris, et al., 2015). In addition, recent studies have also shown that TLR8 agonists can directly exert anti-tumor effects without relying on its immunomodulatory function (Ignatz-Hoover, et al., 2015). Therefore, TLR8 agonists can not only function as a monotherapy, but also enhance the efficacy of various chemotherapy and targeted anti-cancer drugs by enhancing host's immune responses.

Among the TLRs family members that recognize the nucleic acid of pathogenic microorganisms, TLR7 and TLR8 have high homology and can recognize some artificially synthesized small molecules with antiviral effects, such as Imidazoquinolines small molecule compounds (ligands for TLR7 and TLR8). Research on Imidazoquinolines in a guinea pig genital herpes model infected with HSV found that this compound has a small effect on viral replication *in vitro,* but has a strong effect *in vivo.* This indicates that this class of compounds promote the generation of proinflammatory and regulatory cytokines by immune cells, leading to an antiviral response (Int Immunopharmacol 2002; 2:443-451). More importantly, TLR7 and TLR8 can recognize viral ssRNA. Studies have shown that ssRNA viruses are natural ligands for TLR7 and TLR8, such as human immunodeficiency virus type I (HIV), influenza virus, Sendai virus, dengue

fever virus, Newcastle disease virus (NDV), vesicular stomatitis virus (VSV), hepatitis B virus (HBV), and hepatitis C virus (HCV). TLR8 can recognize antiviral compounds, ssRNA viruses, artificially synthesized oligonucleotides, etc. It induces Th1 cytokine secretion, suppresses Th2 cytokine secretion, and inhibits Treg proliferation through the MyD88-dependent signaling pathway, which mediates antiviral immunity and exerts antiviral and anti-allergic effects.

Therefore, TLR8 is currently an extremely attractive therapeutic target. Despite extensive research on TLRs, there are still significant opportunities to further broaden their application and advantages. The compounds and applications described in the present disclosure will contribute to the development of TLR8 agonists, meeting unmet clinical needs.

CONTENT OF THE PRESENT INVENTION

The technical problem to be solved by the present disclosure is that the structure of existing TLR8 agonists is relatively single. To this end, the present disclosure provides a compound containing nitrogen, a conjugate containing the same, and a use thereof. The nitrogen-containing compound exhibits excellent modulatory effects on TLR8 and can effectively treat, alleviate, and/or prevent various diseases related to immune suppression, such as cancer or viral infections.

The present disclosure provides a compound of formula I, a solvate thereof, a pharmaceutically acceptable salt thereof, or a solvate of the pharmaceutically acceptable salt thereof;

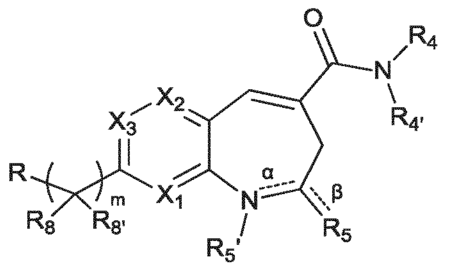

**(I)**

wherein

α and β are independently a single bond or a double bond; at least one selected from the group of α and β is a single bond;

m is 0 or 1;

$X_1$ is N or $CR_2$, $X_2$ is N or $CR_3$, and $X_3$ is N or $CR_1$;

R is $-C(O)-L_1-R_7$, $-C(O)-NR_9-L_1-R_7$, $-C(S)-NR_9-L_1-R_7$, $-NR_9-L_1-R_7$, $-NR_9-C(O)-L_1-R_7$, $-NR_9-C(O)-NR_9-L_1-R_7$, $-O-L_1-R_7$, $-S(O)_2-NR_9-L_1-R_7$, $-CH=CH-L_1-R_7$, or $-S(O)_2-L_1-R_7$;

$R_1$, $R_2$, and $R_3$ are each independently hydrogen, deuterium, halogen, hydroxyl, amino, cyano, alkyl, haloalkyl, $-L_2-OR_a$, or $-L_2-NR_aR_b$;

$R_4$ and $R_{4'}$ are each independently hydrogen, alkyl, alkenyl, alkynyl, cycloalkyl, heterocycloalkyl, aryl, heteroaryl, cycloalkylalkyl, heterocycloalkylalkyl, arylalkyl, or heteroarylalkyl; the $R_4$ or $R_{4'}$ is unsubstituted or optionally substituted at any position by one or more than one substituent selected from halogen, cyano, $-L_2-OR_a$, $-L_2-OC(O)R_a$, $-L_2-NR_aR_b$, $-L_2-NR_aC(O)OR_b$, $-L_2-NR_aC(O)NR_aR_b$, $-L_2-NR_bC(NR_b)NR_aR_b$, and $-L_2-C(O)OR_b$; or, $R_4$ and $R_{4'}$ together with the N atom to which they are attached form a 3- to 8-membered heterocycloalkyl group; the 3- to 8-membered heterocycloalkyl group is unsubstituted or further substituted at any position by 1 to 3 substituents selected from halogen, cyano, $-L_2-OR_a$, $-L_2-OC(O)R_a$, $-L_2-NR_aR_b$, $-L_2-NR_aC(O)OR_b$, $-L_2-NR_aC(O)NR_aR_b$, $-L_2-NR_bC(NR_b)NR_aR_b$, and $-L_2-C(O)OR_b$;

$R_5$ is =O, $=NR_a$, $-OR_a$, or $-NR_aR_b$;

$R_{5'}$ is absent, or $R_{5'}$ is $-R_c$, $-L_2-OR_a$, $-L_2-NR_aR_b$, $-L_2-NR_aC(O)OR_b$, $-L_2-NR_aC(O)NR_aR_b$, $-L_2-NR_bC(NR_b)NR_aR_b$, or $-L_2-C(O)OR_b$;

$R_7$ is phenyl, 5- to 6-membered heteroaryl, 3- to 8-membered heterocycloalkyl, or an 8- to 12-membered fused ring group; the $R_7$ is unsubstituted or optionally substituted at any position by one or more than one substituent selected from $-L_3-W$, $-R_c$, halogen, cyano, nitro, alkyl, alkenyl, alkynyl, alkoxy, haloalkyl, haloalkoxy, and alkylamino;

$R_8$ and $R_{8'}$ are each independently hydrogen, halogen, or alkyl, and the $R_8$ or $R_{8'}$ is unsubstituted or optionally substituted at any position by one or more than one substituent selected from $-L_3-W$, halogen, cyano, nitro, alkyl, alkenyl, alkynyl, alkoxy, haloalkyl, haloalkoxy, and alkylamino; $R_8$ and $R_{8'}$ are each independent substituents, or, $R_8$ and $R_{8'}$ together with the carbon atom to which they are attached form an oxo, thio, $C_{1-6}$ alkylene, $C_{3-10}$ cycloalkyl,

or 3- to 10-membered heterocycloalkyl group; the $C_{1-6}$ alkylene, $C_{3-10}$ cycloalkyl, or 3- to 10-membered heterocycloalkyl group is unsubstituted or optionally substituted at any position by one or more than one substituent selected from deuterium, halogen, hydroxyl, amino, cyano, oxo, alkyl, haloalkyl, $-L_2-OR_a$, and $-L_2-NR_aR_b$;

$R_9$ is hydrogen, alkyl, haloalkyl, $-L_2-OR_a$, or $-L_2-NR_aR_b$;

W is Cyl, $-SR_d$, $-OR_d$, $-OC(O)R_e$, $-OC(O)NR_eR_{e'}$, $-C(O)OR_e$, $-C(O)R_e$, $-C(O)NR_eR_{e'}$, $-C(O)NR_eS(O)_2R_e$, $-NR_dR_e$, $-NR_dC(O)R_e$, $-N(R_d)C(O)OR_e$, $-N(R_d)C(O)NR_eR_{e'}$, $-NR_dS(O)_2R_e$, $-NR_dS(O)_2NR_eR_{e'}$, $-S(O)_{1-2}R_e$, $-S(O)_2NR_eR_{e'}$, $-S(O)(=NR_d)R_e$, $-S(O)_2N(R_e)C(O)R_{e'}$, $-P(O)(OR_e)_2$, $-P(O)(OR_e)R_{e'}$, $-OP(O)(OR_e)_2$, or $-B(OR_e)_2$;

$Cy^1$ is cycloalkyl, heterocycloalkyl, aryl, or heteroaryl; the $Cy^1$ is unsubstituted or optionally substituted at any position by one or more than one substituent selected from halogen, alkyl, haloalkyl, haloalkoxy, alkenyl, alkynyl, cyano, $-R_c$, $-L_4-SR_d$, $-L_4-OC(O)R_e$, $-L_4-C(O)OR_e$, $-L_4-C(O)R_e$, $-L_4-C(O)NR_eR_{e'}$, $-L_4-NR_dC(O)R_e$, $-L_4-NR_dS(O)_2R_e$, $-L_4-S(O)_{1-2}R_e$, $-L_4-S(O)_2NR_eR_{e'}$, $-L_4-OR_d$, and $-L_4-NR_eR_{e'}$;

$L_1$, $L_2$, $L_3$, and $L_4$ are each independently a linkage bond, $C_{1-6}$ alkylene, $C_{2-6}$ alkenylene, or $C_{2-6}$ alkynylene; the $L_1$, $L_2$, $L_3$, or $L_4$ is unsubstituted or optionally substituted at any position by one or more than one substituent selected from oxo, hydroxyl, amino, halogen, cyano, alkyl, haloalkyl, alkoxy, and haloalkoxy;

$R_a$, $R_b$, $R_d$, $R_e$, and $R_{e'}$ are each independently $-R_c$, amino, $C_{1-6}$ alkyl, $C_{2-6}$ alkenyl, $C_{2-6}$ alkynyl, $C_{3-10}$ cycloalkyl, 3- to 10-membered heterocycloalkyl, $C_{6-10}$ aryl, 5- to 10-membered heteroaryl, $C_{3-10}$ cycloalkyl-$C_{1-6}$ alkyl, 3- to 10-membered heterocycloalkyl-$C_{1-6}$ alkyl, phenyl-$C_{1-6}$ alkyl, or 5- to-10 membered heteroaryl-$C_{1-6}$ alkyl; the $R_a$, $R_b$, $R_d$, $R_e$, or $R_{e'}$ is unsubstituted or optionally substituted at any position by 1 to 3 substituents selected from $-OR_f$, $-OC(O)-L_4-R_f$, $-NR_fR_{f'}$, halogen, cyano, nitro, $C_{1-6}$ alkyl, halo-$C_{1-6}$ alkyl, and halo-$C_{1-6}$ alkoxy;

$R_f$ and $R_{f'}$ are each independently $-R_c$, $-NHR_c$, or $C_{1-6}$ alkyl;

each $R_c$ is independently hydrogen; and

the compound of formula I satisfies 1, 2, 3, or 4 of the following conditions:

(1) m is 1;

(2) R is $-C(S)-NR_9-L_1-R_7$, $-NR_9-L_1-R_7$, $-NR_9-C(O)-L_1-R_7$, $-NR_9-C(O)-NR_9-L_1-R_7$, $-O-L_1-R_7$, $-S(O)_2-NR_9-L_1-R_7$, $-CH=CH-L_1-R_7$, or $-S(O)_2-L_1-R_7$;

(3) at least one selected from the group of $X_1$, $X_2$, and $X_3$ is N;

(4) at least one selected from the group of $R_4$ and $R_{4'}$ is substituted at any position by one or more than one substituent selected from halogen, cyano, $-L_2-OR_a$, $-L_2-OC(O)R_a$, $-L_2-NR_aR_b$, $-L_2-NR_aC(O)OR_b$, $-L_2-NR_aC(O)NR_aR_b$, $-L_2-NR_bC(NR_b)NR_aR_b$, and $-L_2-C(O)OR_b$.

In some embodiments, in the compound of formula I, the solvate thereof, the pharmaceutically acceptable salt thereof, or the solvate of the pharmaceutically acceptable salt thereof, some of the groups are defined as follows, and the rest of the groups are defined as described in any other embodiment (hereinafter referred to as "in some embodiments"): m can be 1.

In some embodiments, m can be 1, and $R_8$ and $R_{8'}$ are each independently unsubstituted alkyl; or, $R_8$ and $R_{8'}$ together with the carbon atom to which they are attached form an oxo or unsubstituted $C_{3-10}$ cycloalkyl group.

In some embodiments, R can be $-C(S)-NR_9-L_1-R_7$, $-NR_9-L_1-R_7$, $-NR_9-C(O)-L_1-R_7$, $-NR_9-C(O)-NR_9-L_1-R_7$, $-O-L_1-R_7$, $-S(O)_2-NR_9-L_1-R_7$, $-CH=CH-L_1-R_7$, or $-S(O)_2-L_1-R_7$.

In some embodiments, R can be $-C(S)-NR_9-L_1-R_7$ or $-NR_9-L_1-R_7$.

In some embodiments, R can be $-NR_9-L_1-R_7$.

In some embodiments, at least one selected from the group of $X_1$, $X_2$ and $X_3$ can be N.

In some embodiments, $X_1$ can be N.

In some embodiments, $X_1$ can be N; $X_2$ and $X_3$ are CH.

In some embodiments, at least one selected from the group of $R_4$ and $R_{4'}$ is substituted at any position by one or more than one substituent selected from halogen, cyano, $-L_2-OR_a$, $-L_2-OC(O)R_a$, $-L_2-NR_aR_b$, $-L_2-NR_aC(O)OR_b$, $-L_2-NR_aC(O)NR_aR_b$, $-L_2-NR_bC(NR_b)NR_aR_b$, and $-L_2-C(O)OR_b$.

In some embodiments, at least one selected from the group of $R_4$ and $R_{4'}$ is substituted at any position by one or more than one substituent selected from $-L_2-OR_a$ and $-L_2-NR_aR_b$.

In some embodiments, $R_4$ and $R_{4'}$ are each independently alkyl; at least one selected from the group of $R_4$ and $R_{4'}$ is substituted at any position by one or more than one substituent selected from $-L_2-OR_a$ and $-L_2-NR_aR_b$.

In some embodiments, $R_4$ and $R_{4'}$ are each independently alkyl; at least one selected from the group of $R_4$ and $R_{4'}$ is substituted at any position by one substituent selected from hydroxyl and amino.

In some embodiments, $R_4$ and $R_{4'}$ are each independently alkyl; at least one selected from the group of $R_4$ and $R_{4'}$ is substituted at any position by one hydroxyl.

In some embodiments, R is $-C(O)-NR_9-L_1-R_7$, $-C(S)-NR_9-L_1-R_7$, or $-NR_9-L_1-R_7$; $R_9$ is hydrogen, $L_1$ is a linkage bond; $R_7$ is an unsubstituted 8- to 12-membered fused ring group, or 5- to 6-membered heteroaryl substituted by one $-L_3-W$; $L_3$ is $C_{1-6}$ alkylene, and W is $-NR_dR_e$; $R_d$ and $R_e$ are $-R_c$, and $R_c$ is hydrogen.

In some embodiments, m is 0 or 1; $R_8$ and $R_{8'}$ are each independently unsubstituted alkyl; or, $R_8$ and $R_{8'}$ together with the carbon atom to which they are attached form an oxo or unsubstituted $C_{3-10}$ cycloalkyl group.

In some embodiments, $X_1$ is N or $CR_2$, $X_2$ is N or $CR_3$, and $X_3$ is N or $CR_1$; $R_1$, $R_2$, and $R_3$ are each independently hydrogen, deuterium, halogen, or cyano.

In some embodiments, $\alpha$ is a double bond, and $R_{5'}$ is absent; $\beta$ is a single bond, and $R_5$ is $-NR_aR_b$; $R_a$ and $R_b$ are $-R_c$, and $R_c$ is hydrogen.

In some embodiments, $R_4$ and $R_{4'}$ are each independently alkyl; the $R_4$ and $R_{4'}$ are each independently unsubstituted or optionally substituted at any position by one or more than one substituent selected from $-L_2-OR_a$ and $-L_2-NR_aR_b$; $L_2$ is a linkage bond; $R_a$ and $R_b$ are $-R_c$, and $R_c$ is hydrogen.

In some embodiments,

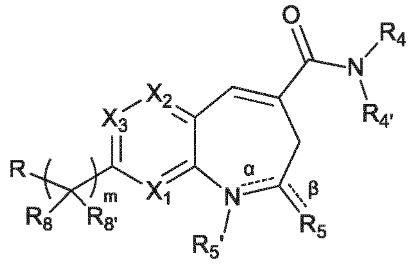

(I)

R is $-C(O)-NR_9-L_1-R_7$, $-C(S)-NR_9-L_1-R_7$, or $-NR_9-L_1-R_7$; $R_9$ is hydrogen, $L_1$ is a linkage bond; $R_7$ is an unsubstituted 8- to 12-membered fused ring group, or 5- to 6-membered heteroaryl substituted by one $-L_3-W$; $L_3$ is $C_{1-6}$ alkylene, W is $-NR_dR_e$;

m is 0 or 1; $R_8$ and $R_{8'}$ are each independently unsubstituted alkyl; or, $R_8$ and $R_{8'}$ together with the carbon atom to which they are attached form an oxo or unsubstituted $C_{3-10}$ cycloalkyl group;

$X_1$ is N or $CR_2$, $X_2$ is N or $CR_3$, and $X_3$ is N or $CR_1$; $R_1$, $R_2$, and $R_3$ are each independently hydrogen, deuterium, halogen, or cyano;

$\alpha$ is a double bond, and $R_{5'}$ is absent; $\beta$ is a single bond, and $R_5$ is $-NR_aR_b$;

$R_4$ and $R_{4'}$ are each independently alkyl; the $R_4$ and $R_{4'}$ are each independently unsubstituted or optionally substituted at any position by one or more than one substituent selected from $-L_2-OR_a$ and $-L_2-NR_aR_b$; $L_2$ is a linkage bond;

$R_a$, $R_b$, $R_d$, and $R_e$ are $-R_c$, and $R_c$ is hydrogen;

the compound of formula I satisfies 1, 2, 3, or 4 of the following conditions:

(1) m is 1;

(2) R is $-C(S)-NR_9-L_1-R_7$ or $-NR_9-L_1-R_7$;

(3) at least one selected from the group of $X_1$, $X_2$ and $X_3$ is N;

(4) at least one selected from the group of $R_4$ and $R_{4'}$ is substituted at any position by one or more than one substituent selected from $-L_2-OR_a$ and $-L_2-NR_aR_b$.

In some embodiments, in $R_7$, the 8- to 12-membered fused ring group can be ring A-fused ring B, the ring A is 5- to 6-membered heteroaryl, and the ring B is 5- to 6-membered heterocycloalkene; in the 5- to 6-membered heteroaryl, the heteroatom is selected from one or more than one of N, O, and S, and the number of heteroatoms is 1, 2, or 3; in the 5- to 6-membered heterocycloalkene, the heteroatom is selected from one or more than one of N, O, and S, and the number of heteroatoms is 1, 2, or 3.

In some embodiments, in $R_7$, the 8- to 12-membered fused ring group can be ring A-fused ring B, the ring A is 5- to 6-membered heteroaryl, and the ring B is 5- to 6-membered heterocycloalkene, which is attached to the $L_1$ through the ring A; in the 5- to 6-membered heteroaryl, the heteroatom is N, and the number of heteroatoms is 1 or 2; in the 5- to 6-membered heterocycloalkene, the heteroatom is N, and the number of heteroatoms is 1 or 2.

In some embodiments, in $R_7$, the 8- to 12-membered fused ring group can be

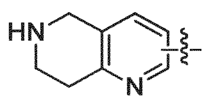

,

and also can be

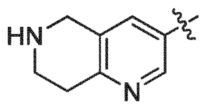

In some embodiments, in $R_7$, in the 5- to 6-membered heteroaryl, the heteroatom can be selected from one or more than one of N, O, and S, and the number of heteroatoms can be 1, 2, or 3.

In some embodiments, in $R_7$, in the 5- to 6-membered heteroaryl, the heteroatom can be N, and the number of heteroatoms can be 1 or 2.

In some embodiments, in $R_7$, the 5- to 6-membered heteroaryl can be pyridyl, and also can be pyridin-3-yl.

In some embodiments, in $L_3$, $C_{1-6}$ alkylene can be $C_{1-3}$ alkylene, and also can be $-CH_2-$, $-CH_2CH_2-$, or $-CH_2CH_2CH_2-$.

In some embodiments, in $R_7$, the 5- to 6-membered heteroaryl substituted by one $-L_3$-W can be

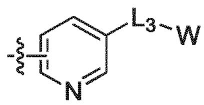,

and also can be

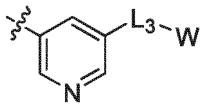,

and further can be

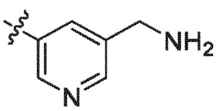.

In some embodiments, in $R_8$ and $R_{8'}$, the alkyl can be $C_{1-6}$ alkyl, and also can be methyl, ethyl, *n*-propyl, isopropyl, *n*-butyl, isobutyl, *sec*-butyl, or *tert*-butyl.

In some embodiments, the $C_{3-10}$ cycloalkyl group formed by $R_8$ and $R_{8'}$ together with the carbon atom to which they are attached can be a saturated monocyclic group.

In some embodiments, the $C_{3-10}$ cycloalkyl group formed by $R_8$ and $R_{8'}$ together with the carbon atom to which they are attached can be a $C_{3-6}$ cycloalkyl group, and can also be cyclopropyl, cyclobutyl, cyclopentyl, or cyclohexyl.

In some embodiments, in $R_1$, $R_2$, and $R_3$, the halogen can be fluorine, chlorine, bromine, or iodine, and also can be fluorine.

In some embodiments, in $R_4$ and $R_{4'}$, the alkyl can be $C_{1-6}$ alkyl, and also can be methyl, ethyl, *n*-propyl, isopropyl, *n*-butyl, isobutyl, *sec*-butyl, or *tert*-butyl.

In some embodiments, $R_4$ is $-CH_2CH_2OH$ or $-CH_2CH_2CH_3$.

In some embodiments, $R_{4'}$ is $-CH_2CH_2OH$ or $-CH_2CH_2CH_3$.

In some embodiments, $R_4$ is $-CH_2CH_2OH$ or $-CH_2CH_2CH_3$; $R_{4'}$ is $-CH_2CH_2OH$ or $- CH_2CH_2CH_3$.

In some embodiments,

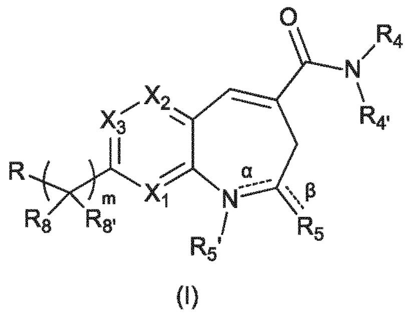

(I)

wherein

$\alpha$ and $\beta$ are independently a single bond or a double bond; at least one selected from the group of $\alpha$ and $\beta$ is a single bond;
m is 0 or 1;
$X_1$ is N or $CR_2$, $X_2$ is N or $CR_3$, $X_3$ is N or $CR_1$; when $X_1$ is $CR_2$, $X_2$ is $CR_3$, and $X_3$ is $CR_1$, m is 1;
R is $-C(O)-L_1-R_7$, $-C(O)-NR_9-L_1-R_7$, $-C(S)-NR_9-L_1-R_7$, $-NR_9-L_1-R_7$, $-NR_9-C(O)-L_1-R_7$, $-NR_9-C(O)-NR_9-L_1-R_7$, $-O-L_1-R_7$, $-S(O)_2-NR_9-L_1-R_7$, $-CH=CH-L_1-R_7$, or $-S(O)_2-L_1-R_7$;
$R_1$, $R_2$, and $R_3$ are each independently hydrogen, deuterium, halogen, hydroxyl, amino, cyano, alkyl, haloalkyl, $-L_2-OR_a$, or $-L_2-NR_aR_b$,
$R_4$ and $R_{4'}$ are each independently hydrogen, alkyl, alkenyl, alkynyl, cycloalkyl, heterocycloalkyl, aryl, heteroaryl, cycloalkylalkyl, heterocycloalkylalkyl, arylalkyl, or heteroarylalkyl; the $R_4$ or $R_{4'}$ is unsubstituted or optionally substituted at any position by one or more than one substituent selected from halogen, cyano, $-L_2-OR_a$, $-L_2-OC(O)R_a$, $-L_2-NR_aR_b$, $-L_2-NR_aC(O)OR_b$, $-L_2-NR_aC(O)NR_aR_b$, $-L_2-NR_bC(NR_b)NR_aR_b$, and $-L_2-C(O)OR_b$; or, $R_4$ and $R_{4'}$ together with the N atom to which they are attached form a 3- to 8-membered heterocycloalkyl group; the 3- to 8-membered heterocycloalkyl group is unsubstituted or further substituted at any position by 1 to 3 substituents selected from halogen, cyano, $-L_2-OR_a$, $-L_2-OC(O)R_a$, $-L_2-NR_aR_b$, $-L_2-NR_aC(O)OR_b$, $-L_2-NR_aC(O)NR_aR_b$, $-L_2-NR_bC(NR_b)NR_aR_b$, and $-L_2-C(O)OR_b$;
$R_5$ is =O, $=NR_a$, $-OR_a$, or $-NR_aR_b$;
$R_{5'}$ is absent, or $R_{5'}$ is $-R_c$, $-L_2-OR_a$, $-L_2-NR_aR_b$, $-L_2-NR_aC(O)OR_b$, $-L_2-NR_aC(O)NR_aR_b$, $-L_2-NR_bC(NR_b)NR_aR_b$, or $-L_2-C(O)OR_b$;
$R_7$ is phenyl, 5- to 6-membered heteroaryl, 3- to 8-membered heterocycloalkyl, or an 8- to 12-membered fused ring group; the $R_7$ is unsubstituted or optionally substituted at any position by one or more than one substituent selected from $-L_3-W$, $-R_c$, halogen, cyano, nitro, alkyl, alkenyl, alkynyl, alkoxy, haloalkyl, haloalkoxy, and alkylamino;
$R_8$ and $R_{8'}$ are each independently hydrogen, halogen, or alkyl, and the $R_8$ or $R_{8'}$ is unsubstituted or optionally substituted at any position by one or more than one substituent selected from $-L_3-W$, halogen, cyano, nitro, alkyl, alkenyl, alkynyl, alkoxy, haloalkyl, haloalkoxy, and alkylamino; $R_8$ and $R_{8'}$ are each independent substituents, or, $R_8$ and $R_{8'}$ together with the carbon atom to which they are attached form an oxo, thio, $C_{1-6}$ alkylene, $C_{3-10}$ cycloalkyl, or 3- to 10-membered heterocycloalkyl group; the $C_{1-6}$ alkylene, $C_{3-10}$ cycloalkyl, or 3- to 10-membered heterocycloalkyl group is unsubstituted or optionally substituted at any position by one or more than one substituent selected from deuterium, halogen, hydroxyl, amino, cyano, oxo, alkyl, haloalkyl, $-L_2-OR_a$, and $-L_2-NR_aR_b$;
$R_9$ is hydrogen, alkyl, haloalkyl, $-L_2-OR_a$, or $-L_2-NR_aR_b$;
W is Cyl, $-SR_d$, $-OR_d$, $-OC(O)R_e$, $-OC(O)NR_eR_{e'}$, $-C(O)OR_e$, $-C(O)R_e$, $-C(O)NR_eR_{e'}$, $-C(O)NR_eS(O)_2R_e$, $-NR_dR_e$, $-NR_dC(O)R_e$, $-N(R_d)C(O)OR_e$, $-N(R_d)C(O)NR_eR_{e'}$, $-NR_dS(O)_2R_e$, $-NR_dS(O)_2NR_eR_{e'}$, $-S(O)_{1-2}R_e$, $-S(O)_2NR_eR_{e'}$, $-S(O)(=NR_d)R_e$, $-S(O)_2N(R_e)C(O)R_{e'}$, $-P(O)(OR_e)_2$, $-P(O)(OR_e)R_{e'}$, $-OP(O)(OR_e)_2$, or $-B(OR_e)_2$;
$Cy^1$ is cycloalkyl, heterocycloalkyl, aryl, or heteroaryl; the $Cy^1$ is unsubstituted or optionally substituted at any position by one or more than one substituent selected from halogen, alkyl, haloalkyl, haloalkoxy, alkenyl, alkynyl, cyano, $-R_c$, $-L_4-SR_d$, $-L_4-OC(O)R_e$, $-L_4-C(O)OR_e$, $-L_4-C(O)R_e$, $-L_4-C(O)NR_eR_{e'}$, $-L_4-NR_dC(O)R_e$, $-L_4-NR_dS(O)_2R_e$, $-L_4-S(O)_{1-2}R_e$, $-L_4-S(O)_2NR_eR_{e'}$, $-L_4-OR_d$, and $-L_4-NR_eR_{e'}$;
$L_1$, $L_2$, $L_3$, and $L_4$ are each independently a linkage bond, $C_{1-6}$ alkylene, $C_{2-6}$ alkenylene, or $C_{2-6}$ alkynylene; the $L_1$, $L_2$, $L_3$, or $L_4$ is unsubstituted or optionally substituted at any position by one or more than one substituent selected from oxo, hydroxyl, amino, halogen, cyano, alkyl, haloalkyl, alkoxy, and haloalkoxy;
$R_a$, $R_b$, $R_d$, $R_e$, and $R_{e'}$ are each independently $-R_c$, amino, $C_{1-6}$ alkyl, $C_{2-6}$ alkenyl, $C_{2-6}$ alkynyl, $C_{3-10}$ cycloalkyl, 3- to 10-membered heterocycloalkyl, $C_{6-10}$ aryl, 5- to 10-membered heteroaryl, $C_{3-10}$ cycloalkyl-$C_{1-6}$ alkyl, 3- to 10-membered heterocycloalkyl-$C_{1-6}$ alkyl, phenyl-$C_{1-6}$ alkyl, or 5- to-10 membered heteroaryl-$C_{1-6}$ alkyl; the $R_a$, $R_b$, $R_d$, $R_e$, or $R_{e'}$ is unsubstituted or optionally substituted at any position by 1 to 3 substituents selected from $-OR_f$, $-OC(O)-L_4-R_f$, $-NR_fR_{f'}$, halogen, cyano, nitro, $C_{1-6}$ alkyl, halo-$C_{1-6}$ alkyl, and halo-$C_{1-6}$ alkoxy;
$R_f$ and $R_{f'}$ are each independently $-R_c$, $-NHR_c$, or $C_{1-6}$ alkyl;
each $R_c$ is independently hydrogen.

In some embodiments,

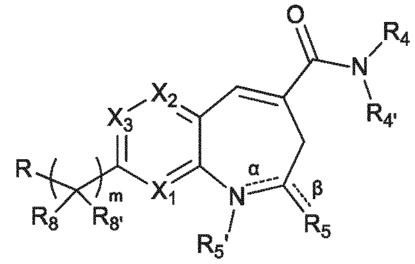

(I)

wherein

$\alpha$ and $\beta$ are independently a single bond or a double bond; at least one selected from the group of $\alpha$ and $\beta$ is a single bond;
m is 0 or 1;
$X_1$ is N or $CR_2$, $X_2$ is N or $CR_3$, $X_3$ is N or $CR_1$; when $X_1$ is $CR_2$, $X_2$ is $CR_3$, and $X_3$ is $CR_1$, m is 1;
R is $-C(O)-L_1-R_7$, $-C(O)-NR_9-L_1-R_7$, $-C(S)-NR_9-L_1-R_7$, $-NR_9-L_1-R_7$, $-NR_9-C(O)-L_1-R_7$, $-NR_9-C(O)-NR_9-L_1-R_7$, $-O-L_1-R_7$, $-S(O)_2-NR_9-L_1-R_7$, $-CH=CH-L_1-R_7$, or $-S(O)_2-L_1-R_7$;
$R_1$, $R_2$, and $R_3$ are each independently hydrogen, deuterium, halogen, hydroxyl, amino, cyano, alkyl, haloalkyl, $-L_2-OR_a$, or $-L_2-NR_aR_b$;
$R_4$ and $R_{4'}$ are each independently hydrogen, alkyl, alkenyl, alkynyl, cycloalkyl, heterocycloalkyl, aryl, heteroaryl, cycloalkylalkyl, heterocycloalkylalkyl, arylalkyl, or heteroarylalkyl; the $R_4$ or $R_{4'}$ is unsubstituted or optionally substituted at any position by one or more than one substituent selected from halogen, cyano, $-L_2-OR_a$, $-L_2-NR_aR_b$, $-L_2-NR_aC(O)OR_b$, $-L_2-NR_aC(O)NR_aR_b$, $-L_2-NR_bC(NR_b)NR_aR_b$, and $-L_2-C(O)OR_b$; or, $R_4$ and $R_{4'}$ together with the N atom to which they are attached form a 3- to 8-membered heterocycloalkyl group; the 3- to 8-membered heterocycloalkyl group is unsubstituted or further substituted at any position by 1 to 3 substituents selected from halogen, cyano, $-L_2-OR_a$, $-L_2-NR_aR_b$, $-L_2-NR_aC(O)OR_b$, $-L_2-NR_aC(O)NR_aR_b$, $-L_2-NR_bC(NR_b)NR_aR_b$, and $-L_2-C(O)OR_b$;
$R_5$ is =O, $=NR_a$, $-OR_a$, or $-NR_aR_b$;
$R_{5'}$ is absent, or $R_{5'}$ is $-R_c$, $-L_2-OR_a$, $-L_2-NR_aR_b$, $-L_2-NR_aC(O)OR_b$, $-L_2-NR_aC(O)NR_aR_b$, $-L_2-NR_bC(NR_b)NR_aR_b$, or $-L_2-C(O)OR_b$;
$R_7$ is phenyl, 5- to 6-membered heteroaryl, 3- to 8-membered heterocycloalkyl, or an 8- to 12-membered fused ring group; the $R_7$ is unsubstituted or optionally substituted at any position by one or more than one substituent selected from $-L_3-W$, $-R_c$, halogen, cyano, nitro, alkyl, alkenyl, alkynyl, alkoxy, haloalkyl, haloalkoxy, and alkylamino;
$R_8$ and $R_{8'}$ are each independently hydrogen, halogen, or alkyl, and the $R_8$ or $R_{8'}$ is unsubstituted or optionally substituted at any position by one or more than one substituent selected from $-L_3-W$, halogen, cyano, nitro, alkyl, alkenyl, alkynyl, alkoxy, haloalkyl, haloalkoxy, and alkylamino; $R_8$ and $R_{8'}$ are each independent substituents, or, $R_8$ and $R_{8'}$ together with the carbon atom to which they are attached form an oxo, thio, $C_{1-6}$ alkylene, $C_{3-10}$ cycloalkyl, or 3- to 10-membered heterocycloalkyl group; the $C_{1-6}$ alkylene, $C_{3-10}$ cycloalkyl, or 3- to 10-membered heterocycloalkyl group is unsubstituted or optionally substituted at any position by one or more than one substituent selected from deuterium, halogen, hydroxyl, amino, cyano, oxo, alkyl, haloalkyl, $-L_2-OR_a$, and $-L_2-NR_aR_b$;
$R_9$ is hydrogen, alkyl, haloalkyl, $-L_2-OR_a$, or $-L_2-NR_aR_b$;
W is $Cy^1$, $-SR_d$, $-OR_d$, $-OC(O)R_e$, $-OC(O)NR_eR_{e'}$, $-C(O)OR_e$, $-C(O)R_e$, $-C(O)NR_eR_{e'}$, $-C(O)NR_eS(O)_2R_e$, $-NR_dR_e$, $-NR_dC(O)R_e$, $-N(R_d)C(O)OR_e$, $-N(R_d)C(O)NR_eR_{e'}$, $-NR_dS(O)_2R_e$, $-NR_dS(O)_2NR_eR_{e'}$, $-S(O)_{1-2}R_e$, $-S(O)_2NR_eR_{e'}$, $-S(O)(=NR_d)R_e$, $-S(O)_2N(R_e)C(O)R_{e'}$, $-P(O)(OR_e)_2$, $-P(O)(OR_e)R_{e'}$, $-OP(O)(OR_e)_2$, or $-B(OR_e)_2$;
$Cy^1$ is cycloalkyl, heterocycloalkyl, aryl, or heteroaryl; the $Cy^1$ is unsubstituted or optionally substituted at any position by one or more than one substituent selected from halogen, alkyl, haloalkyl, haloalkoxy, alkenyl, alkynyl, cyano, $-R_c$, $-L_4-SR_d$, $-L_4-OC(O)R_e$, $-L_4-C(O)OR_e$, $-L_4-C(O)R_e$, $-L_4-C(O)NR_eR_{e'}$, $-L_4-NR_dC(O)R_e$, $-L_4-NR_dS(O)_2R_e$, $-L_4-S(O)_{1-2}R_e$, $-L_4-S(O)_2NR_eR_{e'}$, $-L_4-OR_d$, and $-L_4-NR_eR_{e'}$;
$L_1$, $L_2$, $L_3$, and $L_4$ are each independently a linkage bond, $C_{1-6}$ alkylene, $C_{2-6}$ alkenylene, or $C_{2-6}$ alkynylene; the $L_1$, $L_2$, $L_3$, or $L_4$ is unsubstituted or optionally substituted at any position by one or more than one substituent selected from oxo, hydroxyl, amino, halogen, cyano, alkyl, haloalkyl, alkoxy, and haloalkoxy;
$R_a$, $R_b$, $R_d$, $R_e$, and $R_{e'}$ are each independently $-R_c$, amino, $C_{1-6}$ alkyl, $C_{2-6}$ alkenyl, $C_{2-6}$ alkynyl, $C_{3-10}$ cycloalkyl, 3- to 10-membered heterocycloalkyl, $C_{6-10}$ aryl, 5- to 10-membered heteroaryl, $C_{3-10}$ cycloalkyl-$C_{1-6}$ alkyl, 3- to 10-membered heterocycloalkyl-$C_{1-6}$ alkyl, phenyl-$C_{1-6}$ alkyl, or 5- to-10 membered heteroaryl-$C_{1-6}$ alkyl; the $R_a$, $R_b$, $R_d$, $R_e$, or $R_{e'}$ is unsubstituted or optionally substituted at any position by 1 to 3 substituents selected from $-OR_f$, $-NR_fR_{f'}$, halogen, cyano, nitro, $C_{1-6}$ alkyl, halo-$C_{1-6}$ alkyl, and halo-$C_{1-6}$ alkoxy;
$R_f$ and $R_{f'}$ are each independently $-R_c$, $-NHR_c$, or $C_{1-6}$ alkyl;

each $R_c$ is independently hydrogen.

In some embodiments, $X_3$ is $CR_1$.

In some embodiments, $X_1$ is N or $CR_2$; $X_2$ is $CR_3$.

In some embodiments, $X_1$ is $CR_2$; $X_2$ is N.

In some embodiments, $R_a$, $R_b$, $R_d$, $R_e$, and $R_{e'}$ are each independently $-R_c$, amino, $C_{1-6}$ alkyl, $C_{2-6}$ alkenyl, $C_{2-6}$ alkynyl, $C_{3-10}$ cycloalkyl, 3- to 10-membered heterocycloalkyl, $C_{6-10}$ aryl, 5- to 10-membered heteroaryl, $C_{3-10}$ cycloalkyl-$C_{1-6}$ alkyl, 3- to 10-membered heterocycloalkyl-$C_{1-6}$ alkyl, phenyl-$C_{1-6}$ alkyl, or 5- to-10 membered heteroaryl-$C_{1-6}$ alkyl; the $R_a$, $R_b$, $R_d$, $R_e$, or $R_{e'}$ is unsubstituted or optionally substituted at any position by 1 to 3 substituents selected from $-OR_f$, $-OC(O)-(CH_2)_{1-5}-R_f$, $-NR_fR_{f'}$, halogen, cyano, $C_{1-6}$ alkyl, halo-$C_{1-6}$ alkyl, and halo-$C_{1-6}$ alkoxy.

In some embodiments, R is $-C(O)-NR_9-L_1-R_7$.

In some embodiments, $R_1$, $R_2$, and $R_3$ are each independently hydrogen, deuterium, halogen, hydroxyl, amino, cyano, $C_{1-6}$ alkyl, halo-$C_{1-6}$ alkyl, $-L_2-OR_a$, or $-L_2-NR_aR_b$.

In some embodiments, $R_1$, $R_2$, and $R_3$ are each independently H, F, Cl, Br, $-CH_3$, $-OCH_3$, $-CF_3$, $-CH_2F$, $-CHF_2$, $-OCF_3$, -CN, or $-(CH_2)_{0-5}-NH_2$.

In some embodiments, $R_1$ is H; $R_2$ is H.

In some embodiments, $R_4$ and $R_{4'}$ are each independently hydrogen, $C_{1-6}$ alkyl, $C_{2-6}$ alkenyl, $C_{2-6}$ alkynyl, $C_{3-8}$ cycloalkyl, 3- to 8-membered heterocycloalkyl, $C_{6-10}$ aryl, 5- to 10-membered heteroaryl, $C_{3-8}$ cycloalkyl-$C_{1-6}$ alkyl, 3- to 8-membered heterocycloalkyl-$C_{1-6}$ alkyl, $C_{6-10}$ aryl-$C_{1-6}$ alkyl, or 5- to 10-membered heteroaryl-$C_{1-6}$ alkyl; the $R_4$ or $R_{4'}$ is unsubstituted or optionally substituted at any position by 1 to 3 substituents selected from halogen, cyano, $-L_2-OR_a$, $-L_2-OC(O)R_a$, $-L_2-NR_aR_b$, $-L_2-NR_aC(O)OR_b$, $-L_2-NR_aC(O)NR_aR_b$, $-L_2-NR_bC(NR_b)NR_aR_b$, and $-L_2-C(O)OR_b$; or, $R_4$ and $R_{4'}$ together with the N atom to which they are attached form a 3- to 8-membered heterocycloalkyl group; the 3- to 8-membered heterocycloalkyl group is unsubstituted or further substituted at any position by 1 to 3 substituents selected from halogen, cyano, $-L_2-OR_a$, $-L_2-NR_aR_b$, $-L_2-NR_aC(O)OR_b$, $-L_2-NR_aC(O)NR_aR_b$, $-L_2-NR_bC(NR_b)NR_aR_b$, and $-L_2-C(O)OR_b$.

In some embodiments, $R_4$ and $R_{4'}$ are each independently $C_{1-6}$ alkyl; the $R_4$ or $R_{4'}$ is unsubstituted or optionally substituted at any position by 1 to 3 substituents selected from $-OR_a$, $-NR_aR_b$, $-OC(O)R_a$, $-NR_aC(O)OR_b$, $-NR_aC(O)NR_aR_b$, $-NR_bC(NR_b)NR_aR_b$, and $-C(O)OR_b$.

In some embodiments, $R_4$ is $C_{1-6}$ alkyl; the $R_4$ is unsubstituted or optionally substituted at any position by one $-OR_a$, $-OC(O)R_a$, $-NR_aR_b$, $-NR_aC(O)OR_b$, $-NR_aC(O)NR_aR_b$, $-NR_bC(NR_b)NR_aR_b$, or $-C(O)OR_b$; $R_{4'}$ is $C_{1-6}$ alkyl; the $R_{4'}$ is unsubstituted or optionally substituted at any position by 1 to 3 substituents selected from halogen, $-OR_a$, and $-NR_aR_b$.

In some embodiments, $R_4$ and $R_{4'}$ are each independently $C_{1-6}$ alkyl; the $R_4$ or $R_{4'}$ is unsubstituted or optionally substituted at any position by one substituent selected from halogen, $-OR_a$, and $-NR_aR_b$.

In some embodiments, $R_4$ is $-CH_2CH_2OH$ or $-CH_2CH_2CH_3$; $R_{4'}$ is $-CH_2CH_2OH$, $-CH_2CH_2CH_3$, $-CH_2CH_2CF_3$, or $-CH_2CH_2CHF_2$.

In some embodiments, $\alpha$ is a double bond, $\beta$ is a single bond, $R_{5'}$ is absent, and $R_5$ is $-NR_aR_b$.

In some embodiments, $\alpha$ is a double bond, $\beta$ is a single bond, $R_{5'}$ is absent, and $R_5$ is $-NH_2$.

In some embodiments, $\alpha$ is a single bond, $\beta$ is a double bond, $R_{5'}$ is H, and $R_5$ is =O.

In some embodiments, $R_8$ and $R_{8'}$ are each independently hydrogen, halogen, or $C_{1-6}$ alkyl; the $R_8$ or $R_{8'}$ is unsubstituted or optionally substituted at any position by 1 to 3 substituents selected from $-L_3-W$, halogen, cyano, nitro, $C_{1-6}$ alkyl, $C_{2-6}$ alkenyl, $C_{2-6}$ alkynyl, $C_{1-6}$ alkoxy, halo-$C_{1-6}$ alkyl, halo-$C_{1-6}$ alkoxy, and $C_{1-6}$ alkylamino.

In some embodiments, $R_8$ and $R_{8'}$ are each independently $C_{1-6}$ alkyl, and the $C_{1-6}$ alkyl is preferably methyl.

In some embodiments, $R_8$ and $R_{8'}$ together with the carbon atom to which they are attached form an oxo, thio, $C_{3-6}$ cycloalkyl, or 3- to 6-membered heterocycloalkyl group; the $C_{3-6}$ cycloalkyl or 3- to 6-membered heterocycloalkyl group is unsubstituted or optionally substituted at any position by 1 to 3 substituents selected from deuterium, halogen, hydroxyl, amino, cyano, oxo, $C_{1-6}$ alkyl, halo-$C_{1-6}$ alkyl, $-L_2-OR_a$, and $-L_2-NR_aR_b$.

In some embodiments, $R_8$ and $R_{8'}$ together with the carbon atom to which they are attached form an oxo, thio, cyclopropyl, cyclobutyl, azetidinyl, or oxetanyl group; the cyclopropyl, cyclobutyl, azetidinyl, or oxetanyl group is unsubstituted or optionally substituted at any position by 1 to 3 substituents selected from deuterium, halogen, hydroxyl, amino, cyano, oxo, $C_{1-6}$ alkyl, halo-$C_{1-6}$ alkyl, $-L_2-OR_a$, and $-L_2-NR_aR_b$.

In some embodiments, $R_8$ and $R_{8'}$ together with the carbon atom to which they are attached form an oxo, thio, cyclopropyl, or cyclobutyl group.

In some embodiments, $R_9$ is hydrogen, $C_{1-6}$ alkyl, halo-$C_{1-6}$ alkyl, $-L_2-OR_a$, or $-L_2-NR_aR_b$.

In some embodiments, $R_9$ is hydrogen.

In some embodiments, $Cy^1$ is $C_{3-8}$ cycloalkyl, 3- to 8-membered heterocycloalkyl, $C_{6-10}$ aryl, or 5- to 10-membered heteroaryl; the $Cy^1$ is unsubstituted or optionally substituted at any position by 1 to 3 substituents selected from halogen, cyano, $C_{1-6}$ alkyl, halo-$C_{1-6}$ alkyl, halo-$C_{1-6}$ alkoxy, $C_{2-6}$ alkenyl, $C_{2-6}$ alkynyl, $-L_4-SR_d$, $-L_4-OC(O)R_e$, $-L_4-C(O)OR_e$, $-L_4-C(O)R_e$, $-L_4-C(O)NR_eR_{e'}$, $-L_4-NR_dC(O)R_e$, $-L_4-NR_dS(O)_2R_e$, $-L_4-S(O)_{1-2}R_e$, $-L_4-S(O)_2NR_eR_{e'}$,

$-L_4-OR_d$, or $-L_4-NR_eR_{e'}$.

In some embodiments, $L_1$ is a linkage bond or $C_{1-6}$ alkylene; the $L_1$ is unsubstituted or optionally substituted at any position by 1 to 3 substituents selected from oxo, halogen, hydroxyl, amino, cyano, $C_{1-6}$ alkyl, halo-$C_{1-6}$ alkyl, $C_{1-6}$ alkoxy, or halo-$C_{1-6}$ alkoxy.

In some embodiments, $L_1$ is a linkage bond or $-CH_2-$.

In some embodiments, $L_2$ is a linkage bond or $C_{1-6}$ alkylene; the $L_2$ is unsubstituted or optionally substituted at any position by 1 to 3 substituents selected from oxo, halogen, hydroxyl, amino, cyano, $C_{1-6}$ alkyl, halo-$C_{1-6}$ alkyl, $C_{1-6}$ alkoxy, or halo-$C_{1-6}$ alkoxy.

In some embodiments, $L_3$ is a linkage bond or $C_{1-6}$ alkylene; the $L_3$ is unsubstituted or optionally substituted at any position by 1 to 3 substituents selected from oxo, halogen, hydroxyl, amino, cyano, $C_{1-6}$ alkyl, halo-$C_{1-6}$ alkyl, $C_{1-6}$ alkoxy, or halo-$C_{1-6}$ alkoxy.

In some embodiments, $L_3$ is a linkage bond, $-CH_2-$, $-CH_2CH_2-$, $-CH_2CH_2CH_2-$, $-CH_2CH_2C(CH_3)_2-$, $-CH_2CH_2CH_2CH_2-$, or $-CH_2CH(CH_3)CH_2-$.

In some embodiments, $L_3$ is $-CH_2-$.

In some embodiments, $L_4$ is a linkage bond or $C_{1-6}$ alkylene; the $L_4$ is unsubstituted or optionally substituted at any position by one or more than one substituent selected from oxo, halogen, hydroxyl, amino, cyano, $C_{1-6}$ alkyl, halo-$C_{1-6}$ alkyl, $C_{1-6}$ alkoxy, or halo-$C_{1-6}$ alkoxy.

In some embodiments, the moiety $-L_3-W$ is

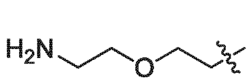, 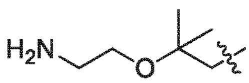,

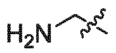, 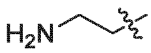, 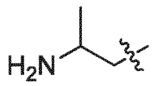, 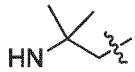, 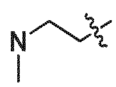, 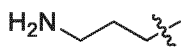,

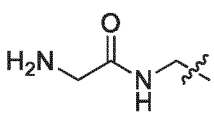, 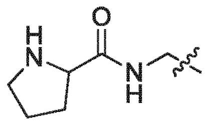, 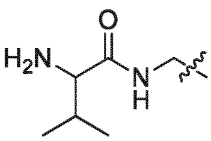, 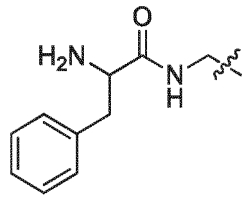, 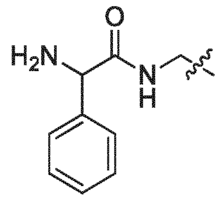,

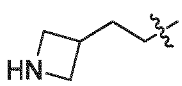, 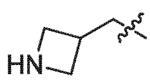, 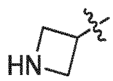, 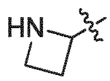, 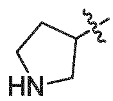, 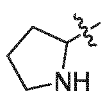, 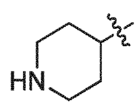, 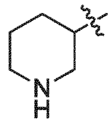,

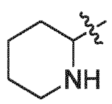, 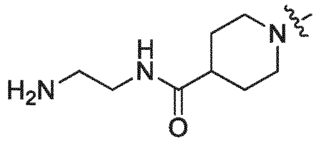, 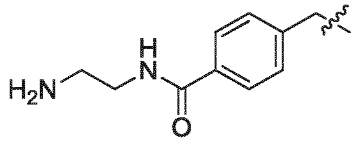, 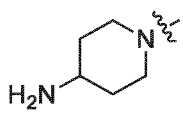,

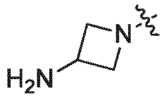, 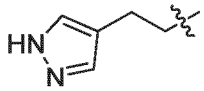, 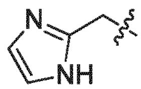, 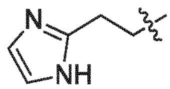, 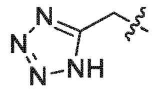, 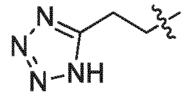,

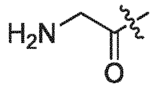, or 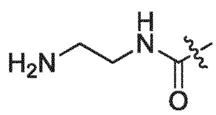.

In some embodiments, the moiety $-L_3$-W is

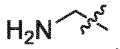.

In some embodiments, $R_7$ is phenyl, 5- to 6-membered heteroaryl, 3- to 8-membered heterocycloalkyl, or an 8- to 12-membered fused ring group; the $R_7$ is unsubstituted or optionally substituted at any position by 1 to 3 substituents selected from $-L_3$-W, halogen, cyano, nitro, $C_{1-6}$ alkyl, $C_{2-6}$ alkenyl, $C_{2-6}$ alkynyl, $C_{1-6}$ alkoxy, halo-$C_{1-6}$ alkyl, halo-$C_{1-6}$ alkoxy, and $C_{1-6}$ alkylamino.

In some embodiments, $R_7$ is an 8- to 12-membered fused ring group; the $R_7$ is unsubstituted or optionally substituted at any position by 1 to 3 substituents selected from $-L_3$-W, halogen, cyano, nitro, $C_{1-6}$ alkyl, $C_{2-6}$ alkenyl, $C_{2-6}$ alkynyl, $C_{1-6}$ alkoxy, halo-$C_{1-6}$ alkyl, halo-$C_{1-6}$ alkoxy, and $C_{1-6}$ alkylamino.

In some embodiments, $R_7$ is

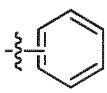, 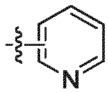, 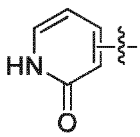, 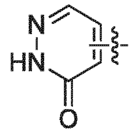, 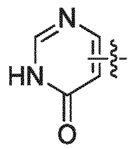,

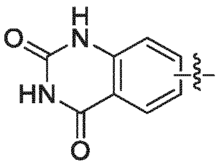, 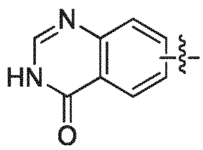, 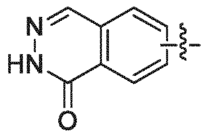, 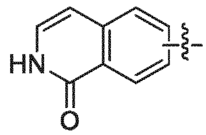, 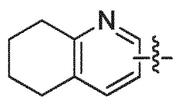,

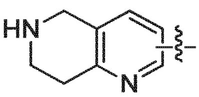, 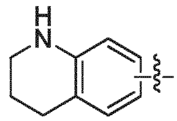, 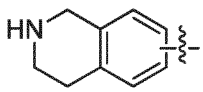, 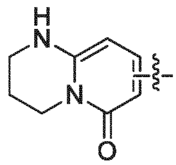, 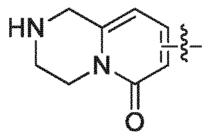,

or

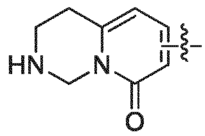;

the $R_7$ is unsubstituted or optionally substituted at any position by 1 to 3 substituents selected from $-L_3$-W, halogen, cyano, nitro, $C_{1-6}$ alkyl, $C_{2-6}$ alkenyl, $C_{2-6}$ alkynyl, $C_{1-6}$ alkoxy, halo-$C_{1-6}$ alkyl, halo-$C_{1-6}$ alkoxy, and $C_{1-6}$ alkylamino.

In some embodiments, $R_7$ is

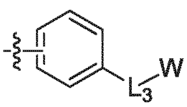, 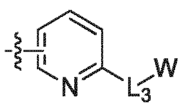, 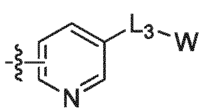,

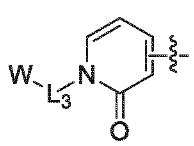, 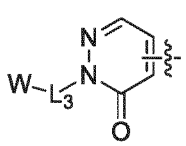, 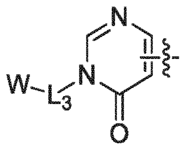, 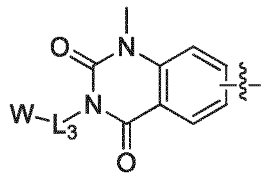, 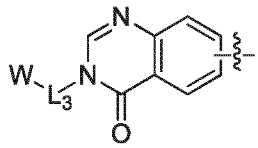,

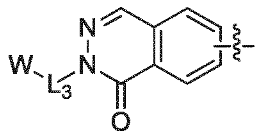, 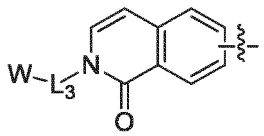, 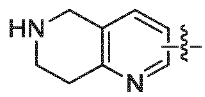, 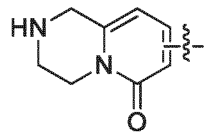,

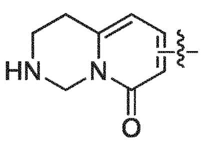, 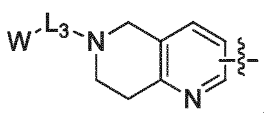, 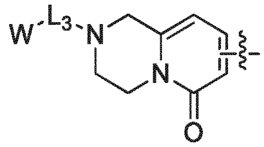, or 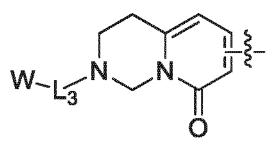.

In some embodiments, $R_7$ is

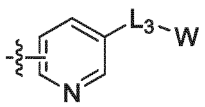 or 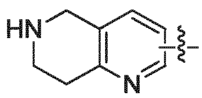.

In some embodiments, $X_1$ is N or $CR_2$, $X_2$ is N or $CR_3$, and $X_3$ is N or $CR_1$; when $X_1$ is $CR_2$, $X_2$ is $CR_3$, and $X_3$ is $CR_1$, m is 1;

In some embodiments, m is 0 or 1;
$X_1$ is N or $CR_2$, $X_2$ is N or $CR_3$, and $X_3$ is N or $CR_1$; when $X_1$ is $CR_2$, $X_2$ is $CR_3$, and $X_3$ is $CR_1$, m is 1;
R is -C(O)-$L_1$-$R_7$, -C(O)-$NR_9$-$L_1$-$R_7$, or -C(S)-$NR_9$-$L_1$-$R_7$.

In some embodiments, m is 1;

$X_1$ is N or $CR_2$, $X_2$ is N or $CR_3$, and $X_3$ is N or $CR_1$;
R is -C(O)-$L_1$-$R_7$, -C(O)-$NR_9$-$L_1$-$R_7$, or -C(S)-$NR_9$-$L_1$-$R_7$.

In some embodiments, the compound of formula I is a compound of formula IA,

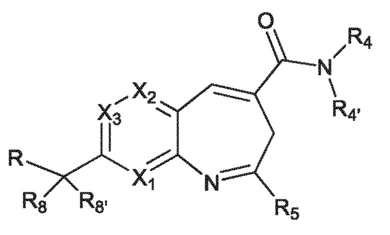

(IA)

wherein R is -C(O)-$L_1$-$R_7$, -C(O)-$NR_9$-$L_1$-$R_7$, or -C(S)-$NR_9$-$L_1$-$R_7$;
$X_1$, $X_2$, $X_3$, R, $R_4$, $R_{4'}$, $R_5$, $R_8$, and $R_{8'}$ are defined as above.

In some embodiments, in IA, $L_1$ is a linkage bond.
In some embodiments, in IA, $R_8$ and $R_{8'}$ together with the carbon atom to which they are attached form a $C_{3-6}$ cycloalkyl or 3- to 6-membered heterocycloalkyl group; the $C_{3-6}$ cycloalkyl or 3- to 6-membered heterocycloalkyl group

is unsubstituted or optionally substituted at any position by 1 to 3 substituents selected from deuterium, halogen, hydroxyl, amino, cyano, oxo, $C_{1-6}$ alkyl, halo-$C_{1-6}$ alkyl, $-L_2-OR_a$, and $-L_2-NR_aR_b$.

In some embodiments, in IA, Rs and $R_{8'}$ together with the carbon atom to which they are attached form the following groups:

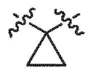, 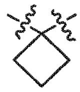, 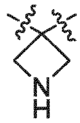, or 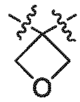.

In some embodiments, in IA, $X_1$ is CH, $X_2$ is $CR_3$, and $X_3$ is CH; m is 1; $R_3$ is H or halogen.

In some embodiments, $R_5$ is $-NH_2$.

In some embodiments, the compound of formula I is a compound of formula IB,

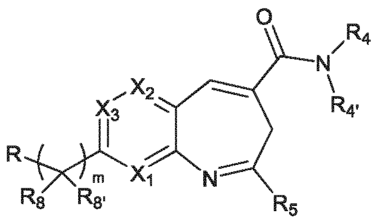

(IB)

wherein R is $-C(O)-L_1-R_7$, $-C(O)-NR_9-L_1-R_7$, or $-C(S)-NR_9-L_1-R_7$;
$R_4$ is $C_{1-6}$ alkyl; the $R_4$ is optionally substituted at any position by one $-OR_a$;
$X_1$, $X_2$, $X_3$, R, $R_{4'}$, $R_5$, $R_8$, $R_{8'}$, $R_a$, and m are defined as above.

In some embodiments, in IB, $R_a$ is H.

In some embodiments, in IB, $X_1$ is N or $CR_2$, $X_2$ is N or $CR_3$, and $X_3$ is N or $CR_1$; m is 1; $L_1$ is a linkage bond.

In some embodiments, in IB, $R_8$ and $R_{8'}$ together with the carbon atom to which they are attached form a $C_{3-6}$ cycloalkyl or 3- to 6-membered heterocycloalkyl group; the $C_{3-6}$ cycloalkyl or 3- to 6-membered heterocycloalkyl group is unsubstituted or optionally substituted at any position by 1 to 3 substituents selected from deuterium, halogen, hydroxyl, amino, cyano, oxo, $C_{1-6}$ alkyl, halo-$C_{1-6}$ alkyl, $-L_2-OR_a$, and $-L_2-NR_aR_b$.

In some embodiments, in IB, $R_8$ and $R_{8'}$ together with the carbon atom to which they are attached form the following groups:

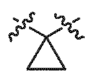, 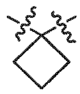, 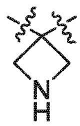, or 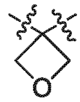.

In some embodiments, $R_5$ is $-NH_2$.

In some embodiments, the compound of formula I is any one of the structures in Table 1:

Table 1:

| | |
|---|---|
| 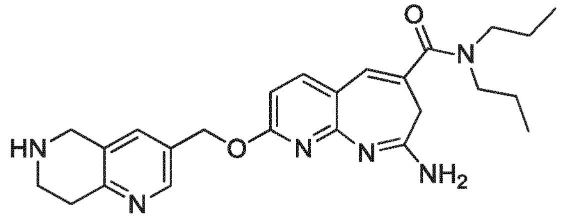 | 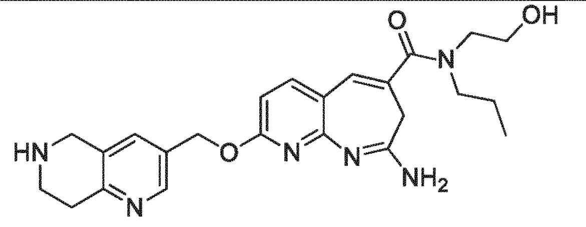 |
| 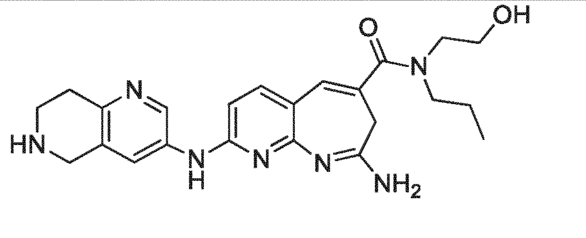 | 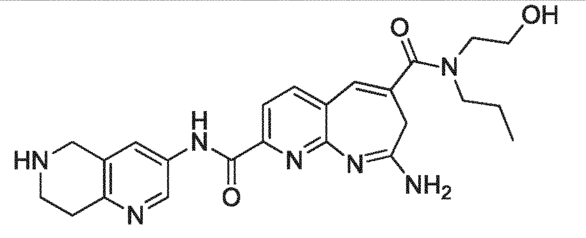 |
| 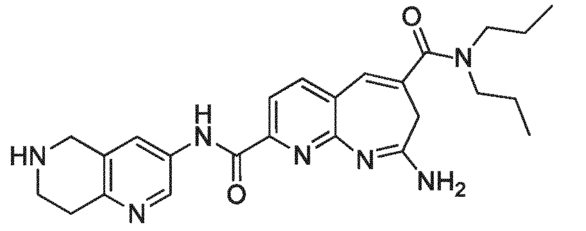 | 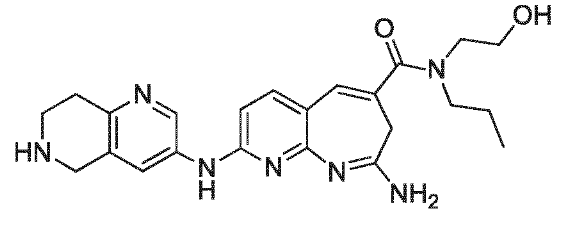 |
| 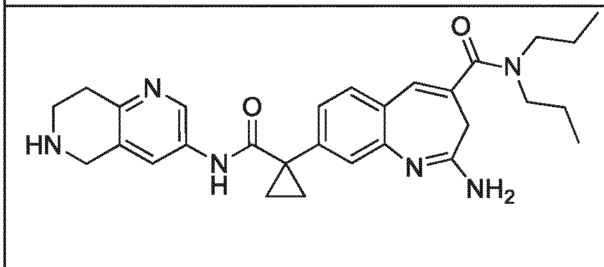 | 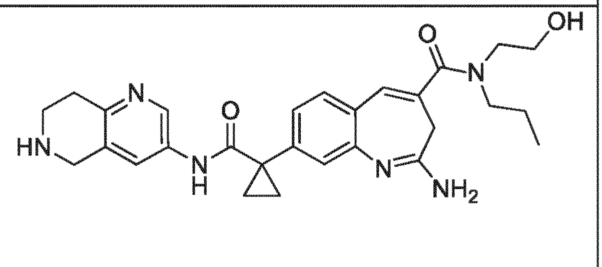 |
| 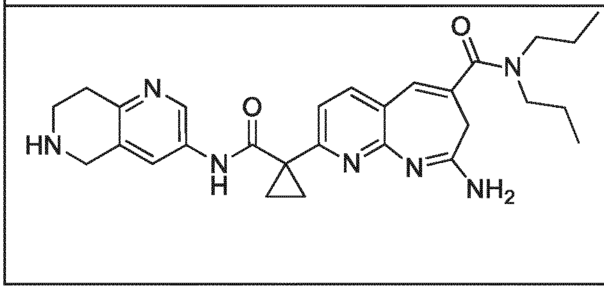 | 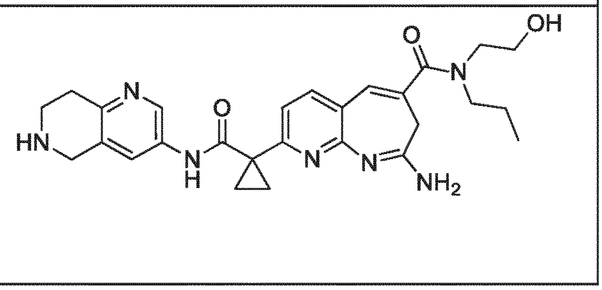 |

| | |
|---|---|
| 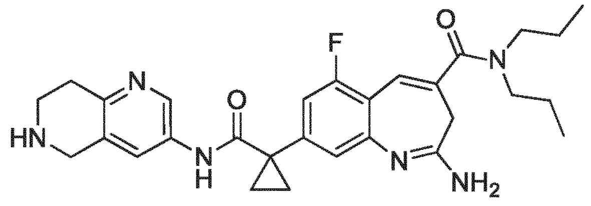 | 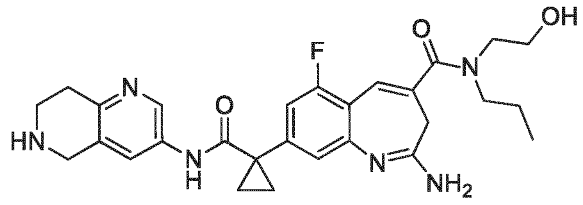 |
| 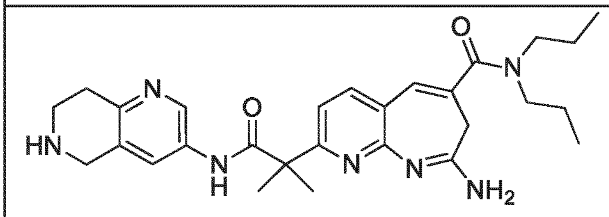 | 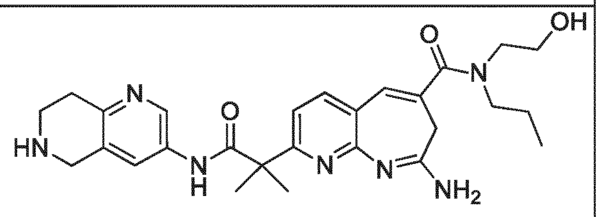 |
| 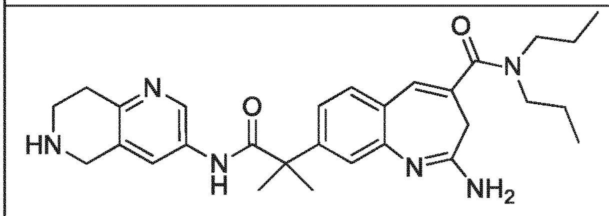 | 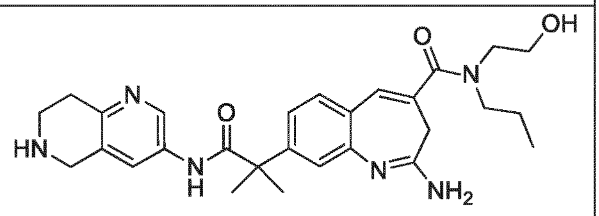 |
| 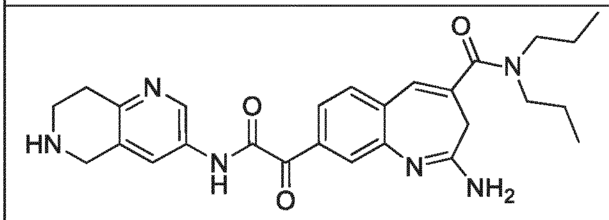 | 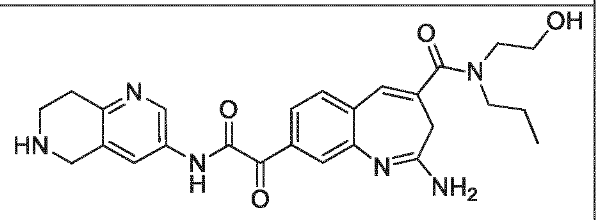 |
| 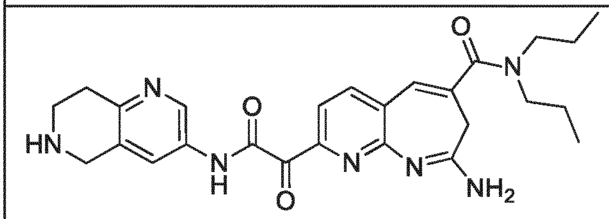 | 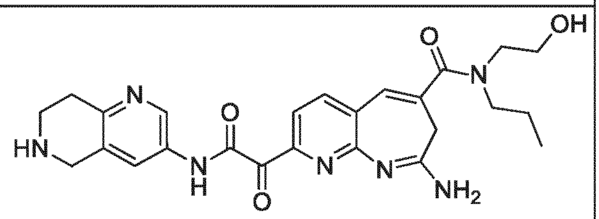 |
| 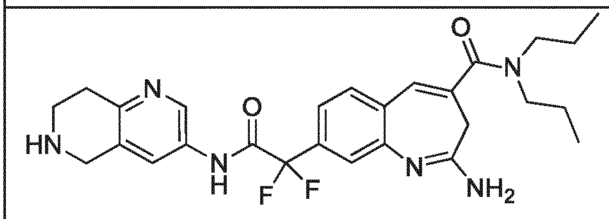 | 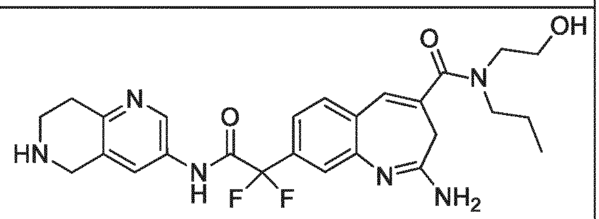 |

| | |
|---|---|
| 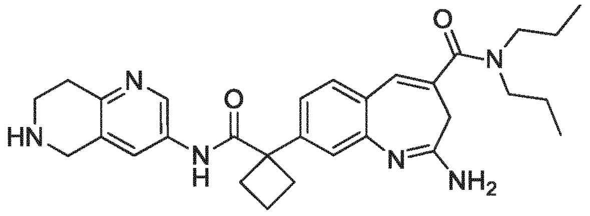 | 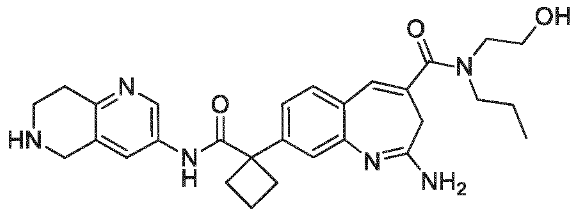 |
| 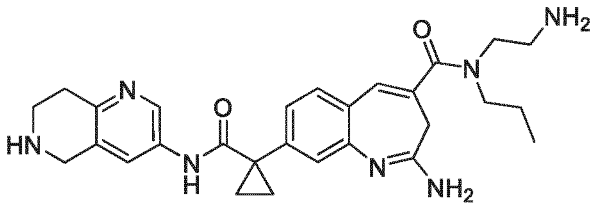 | 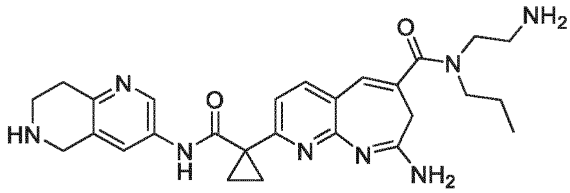 |
| 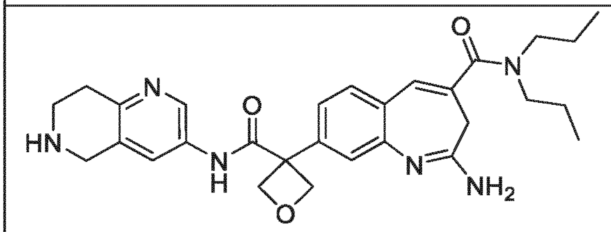 | 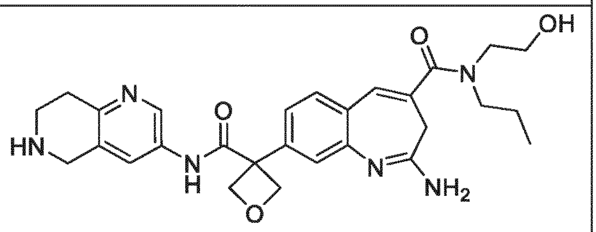 |
| 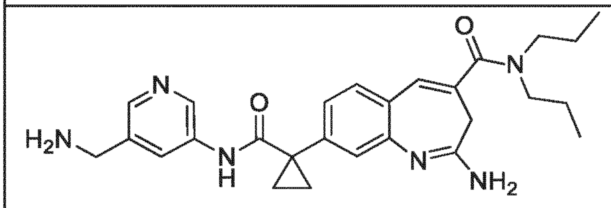 | 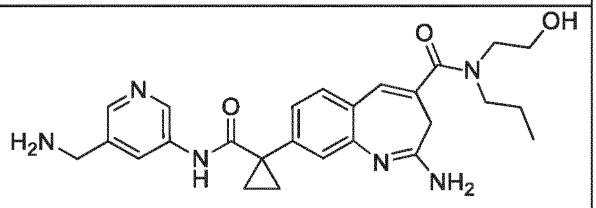 |
| 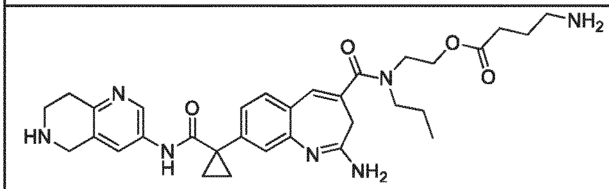 | 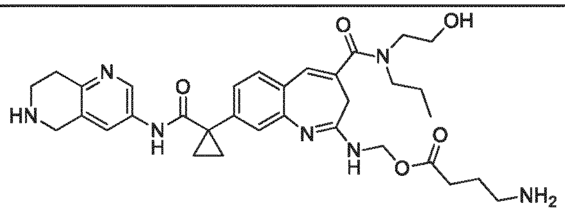 |
| 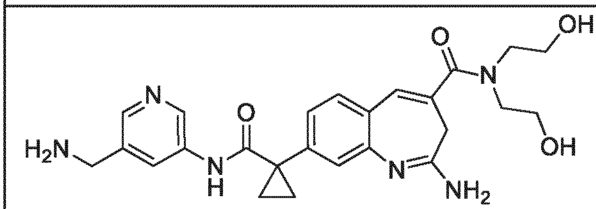 | 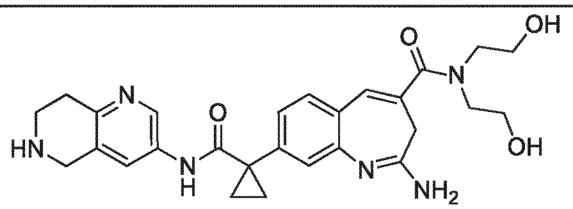 |

| | |
|---|---|
| 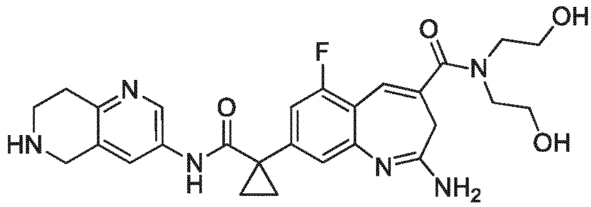 | 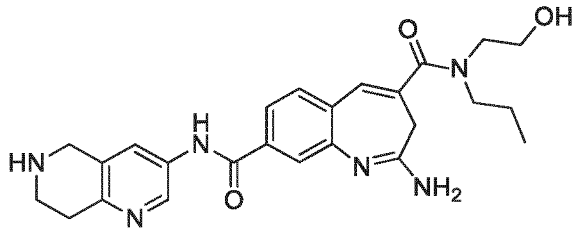 |
| 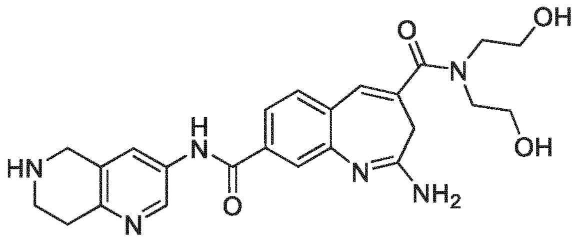 | |

, and the pharmaceutically acceptable salt thereof.

The present disclosure also provides any one of the following nitrogen-containing compounds:

| | |
|---|---|
| 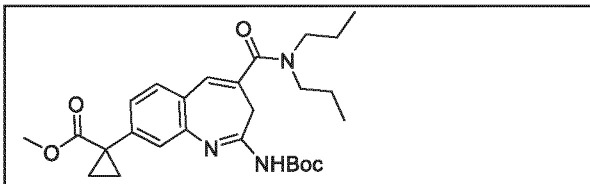 | 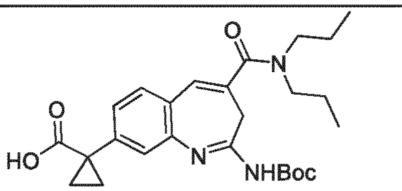 |
| 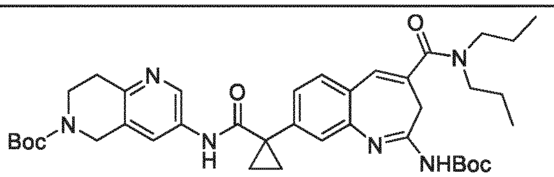 | 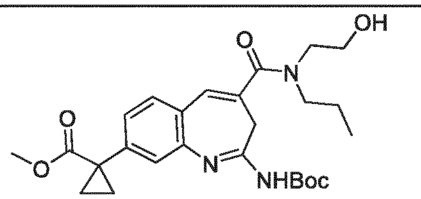 |
| 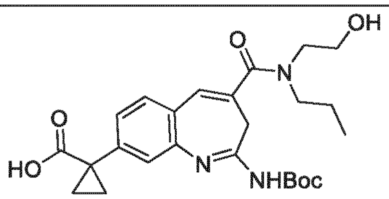 | 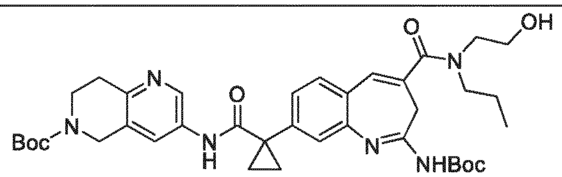 |
| 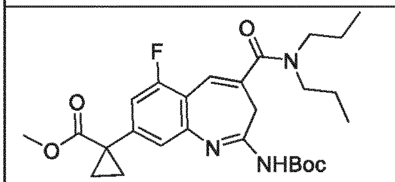 | 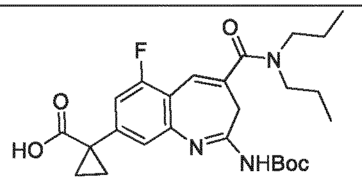 |
| 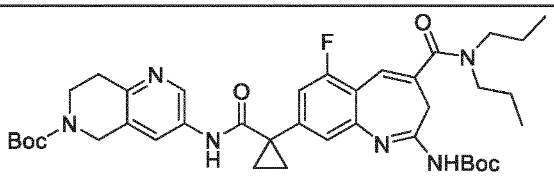 | 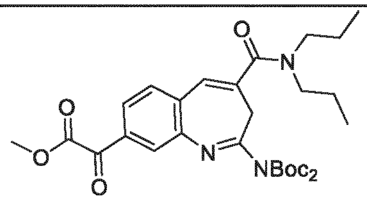 |

| | |
|---|---|
| 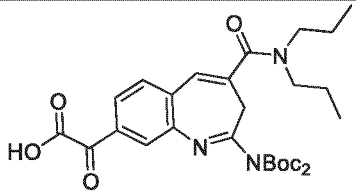 | 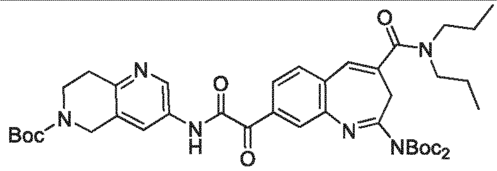 |
| 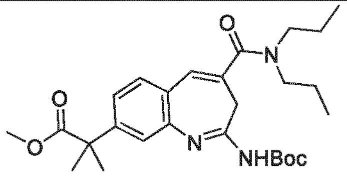 | 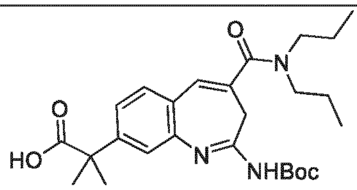 |
| 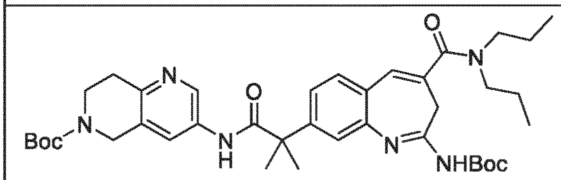 | 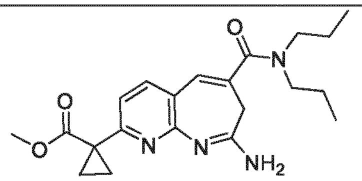 |
| 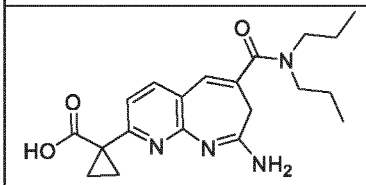 | 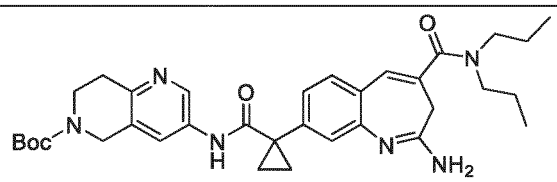 |
| 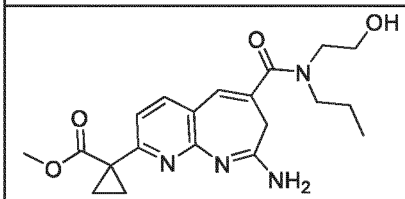 | 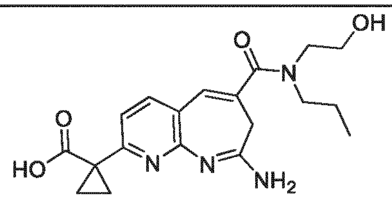 |
| 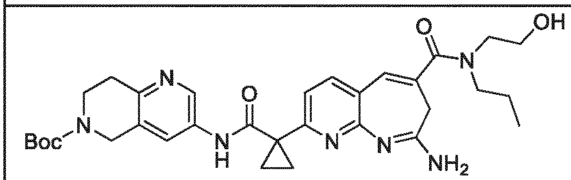 | 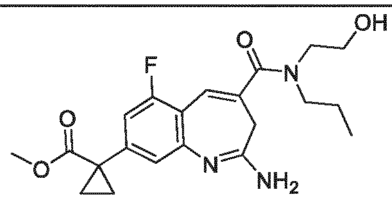 |
| 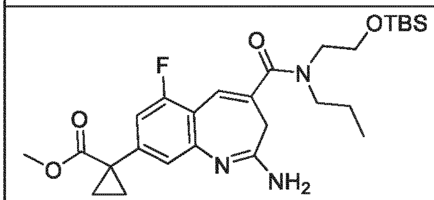 | 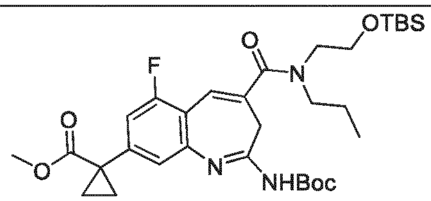 |
| 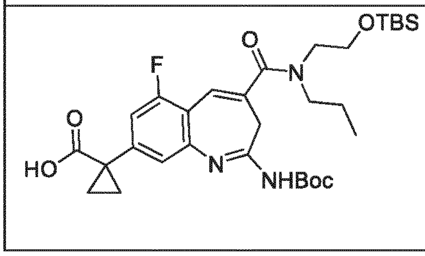 | 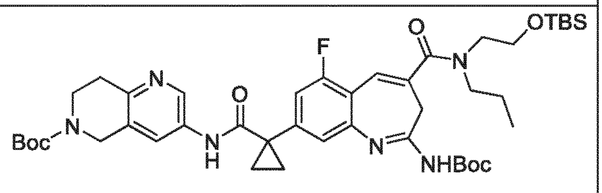 |

| | |
|---|---|
| 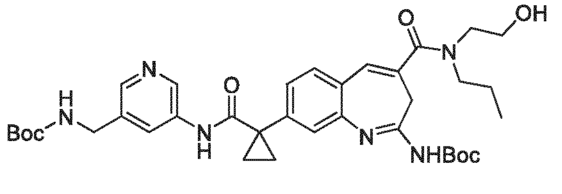 | 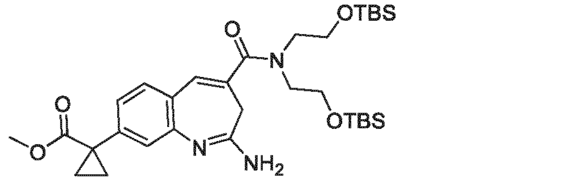 |
| 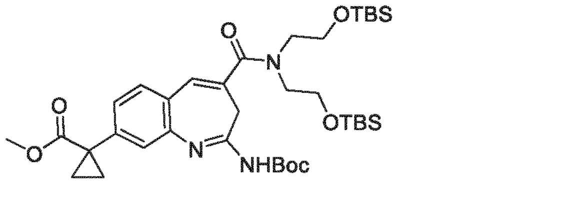 | 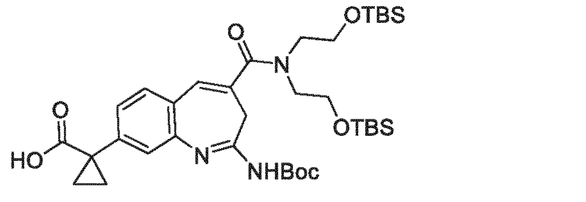 |
| 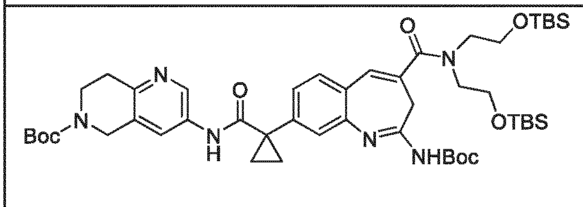 | 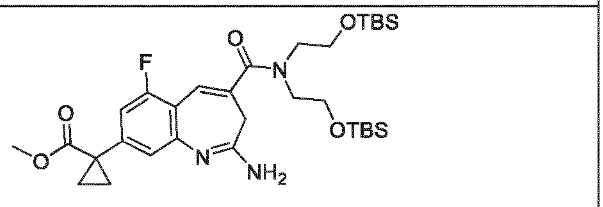 |
| 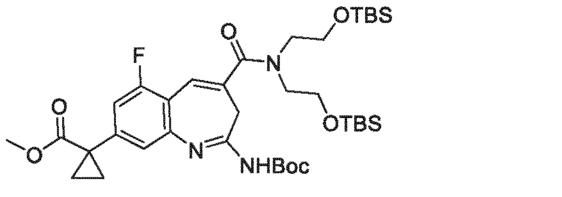 | 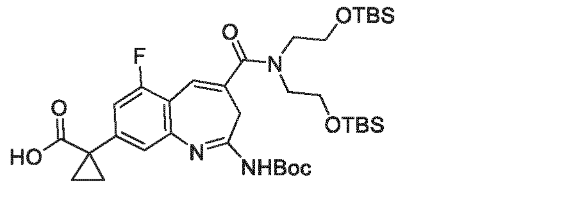 |
| 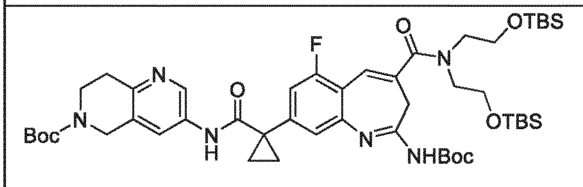 | 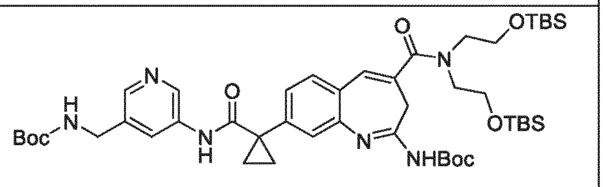 |
| 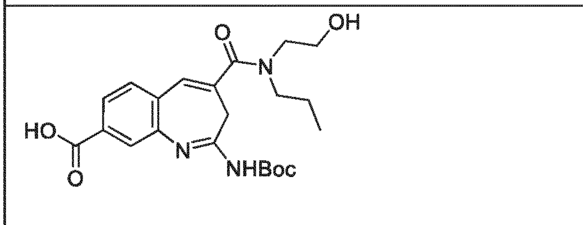 | 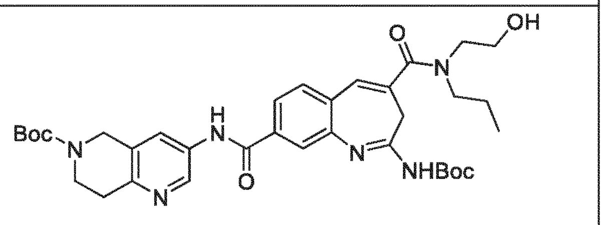 |
| 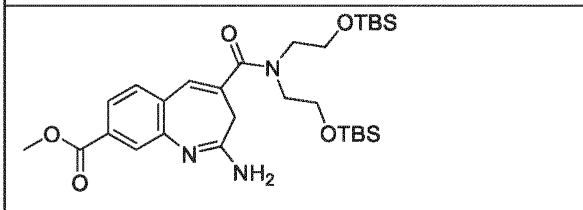 | 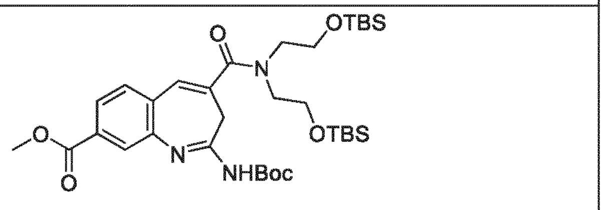 |
| 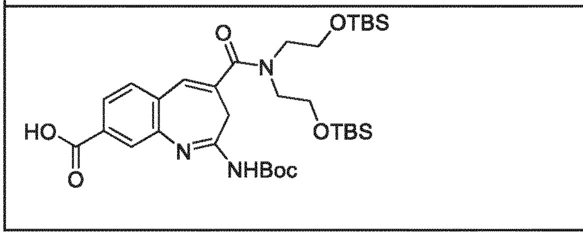 | 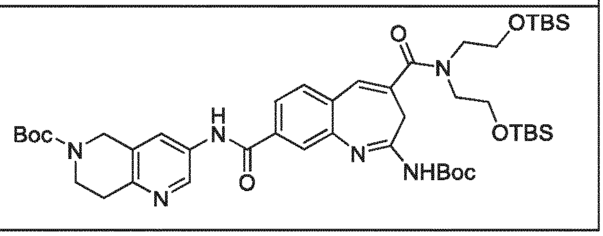 |

The present disclosure also provides a compound of formula II', a solvate thereof, a pharmaceutically acceptable salt thereof, or a solvate of the pharmaceutically acceptable salt thereof,

**D-LinkerX** (II') .

D is a group formed by losing one hydrogen atom in the compound of formula I;
linker X is linker 2.

In some embodiments, in the compound of formula II', the solvate thereof, the pharmaceutically acceptable salt thereof, or the solvate of the pharmaceutically acceptable salt thereof, some of the groups are defined as follows, and the rest of the groups are defined as described in any other embodiment (hereinafter referred to as "in some embodiments"):
the D can be a group formed by losing one hydrogen atom from $R_7$ of the compound of formula I.

In some embodiments, the D can be a group formed by losing one hydrogen atom from the secondary or primary amine of $R_7$ of the compound of formula I, i.e., attached to the linker X via an N atom.

In some embodiments, the D can be attached to the linker X via the a-end of

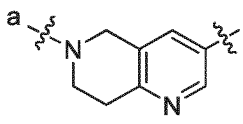 or 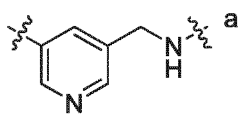 .

In the compound of formula II', the solvate thereof, the pharmaceutically acceptable salt thereof, or the solvate of the pharmaceutically acceptable salt thereof, the linker 2 is a monovalent group attached to the D via one site.

In the compound of formula II', the solvate thereof, the pharmaceutically acceptable salt thereof, or the solvate of the pharmaceutically acceptable salt thereof, the linker 2 is a conventional linker unit in the field of ISAC.

In some embodiments, the linker X can be a degradable linker or a non-degradable linker.

In some embodiments, the linker X can be a linker that can be degraded by lysosomal enzymes.

In some embodiments, the linker X can be

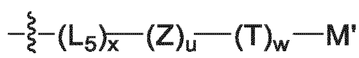 ;

x is 1;

u is 1, 2, 3, 4, 5, or 6;

w is 1;

$L_5$ is

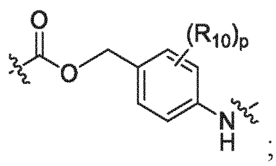 ;

p is 1; $R_{10}$ is hydrogen; the carbonyl end of the $L_5$ is attached to the D;

each Z is independently

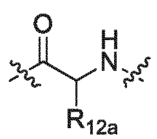 ;

each $R_{12a}$ is independently hydrogen, methyl, ethyl, n-propyl, isopropyl,

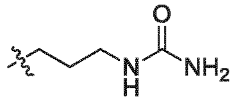, or 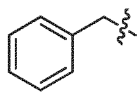;

the carbonyl end of the

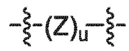

is attached to the amino end of the $L_5$;

T is

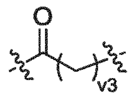;

v3 is 1, 2, 3, 4, 5, or 6; the carbonyl end of T is attached to the amino end of

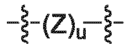;

M'is

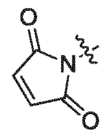,

the amino end of the M' is attached to the non-carbonyl end of the T.

In some embodiments, the compound of formula II' can be

$$D-(L_5)_x-(Z)_u-(T)_w-M'$$

(II);

D is a group formed by losing one hydrogen atom in the compound of formula I;

x is 0, 1, 2, or 3;

u is 0, 1, 2, 3, 4, 5, or 6;

w is 0, 1, 2, 3, 4, 5, or 6;

each $L_5$ is independently

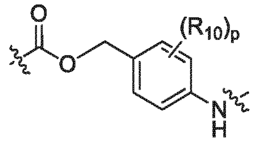, 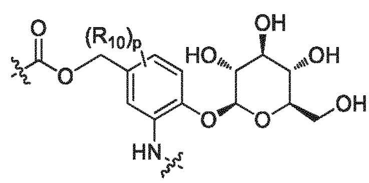,

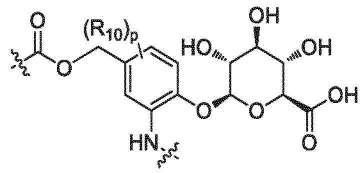, 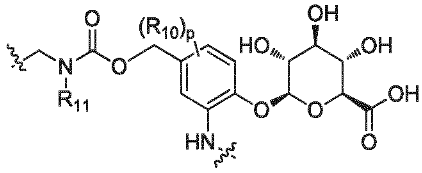, or 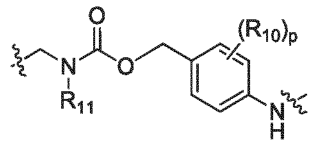;

P is 1, 2, or 3; each $R_{10}$ is independently hydrogen, halogen, $C_{1-6}$ alkyl, halo-$C_{1-6}$ alkyl, nitro, cyano, $C_{1-6}$ alkoxy, $C_{1-6}$ alkylamino, $-OC(O)R_{11}$, or $-C(O)N(R_{11})_2$; $R_{11}$ is hydrogen, $C_{1-6}$ alkyl, or $C_{1-6}$ alkylamino-$C_{1-6}$ alkyl;

each Z is independently

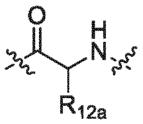, 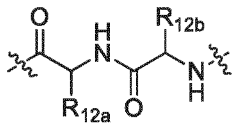, 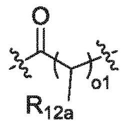,

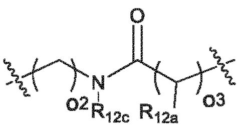, 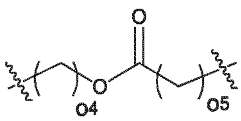, 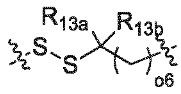,

and $-(CH_2CH_2O)_{o7}-$; o1, o2, o3, o4, o5, oG, o7 are each independently 0, 1, 2, 3, 4, 5, 6, 7, or 8; $R_{12a}$, $R_{12b}$, and $R_{12c}$ are each independently hydrogen, methyl, ethyl, n-propyl, isopropyl, $-SO_3H$,

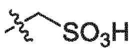, 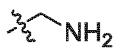,

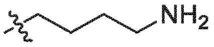, 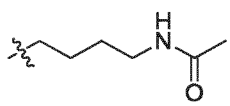, 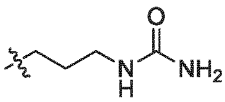, 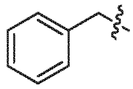, 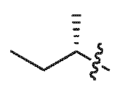, 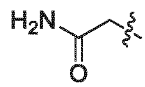,

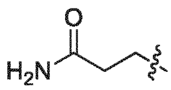, or 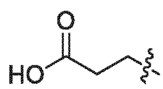;

$R_{13a}$ and $R_{13b}$ are each independently hydrogen or methyl;

each T is independently $-(CH_2)_{v1}-$, $-(CH_2CH_2O)_{v2}-$,

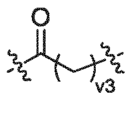, 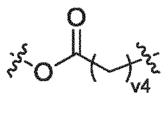,

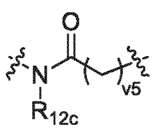, 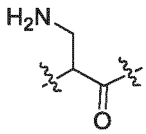, 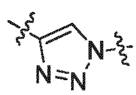, or 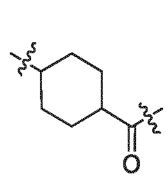;

v1, v2, v3, v4, and v5 are each independently 1, 2, 3, 4, 5, or 6;

M' is

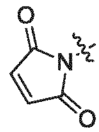, 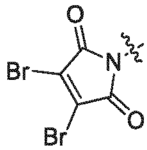, 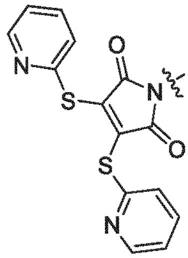, or 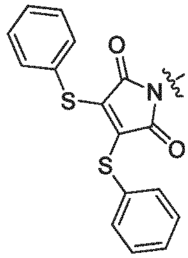.

In some embodiments, the D is a group formed by losing one hydrogen atom from $R_7$ of the compound of formula I;

linker X is

$$-(L_5)_x-(Z)_u-(T)_w-M';$$

x is 1;

u is 1, 2, 3, 4, 5, or 6;

w is 1;

$L_5$ is

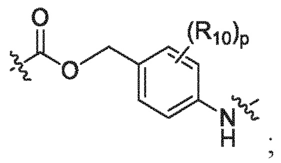;

p is 1; $R_{10}$ is hydrogen; the carbonyl end of the $L_5$ is attached to the D;

each Z is independently

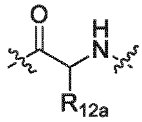;

each $R_{12a}$ is independently hydrogen, methyl, ethyl, n-propyl, isopropyl,

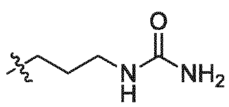, or 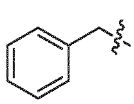;

the carbonyl end of the

$$-(Z)_u-$$

is attached to the amino end of Ls;

T is

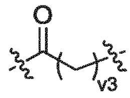;

v3 is 1, 2, 3, 4, 5, or 6; the carbonyl end of T is attached to the amino end of

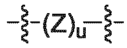;

M'is

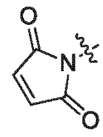,

the amino end of the M' is attached to the non-carbonyl end of the T.

In some embodiments, the $L_5$ can be

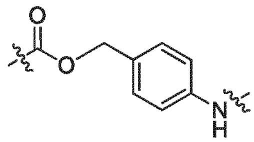.

In some embodiments, the carbonyl end of $L_5$ can be attached to the D.
In some embodiments, the Z can be an amino acid.
In some embodiments, the

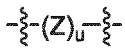

can be a peptide chain.
In some embodiments, the

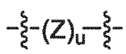

can be a dipeptide, tripeptide, or tetrapeptide linker unit.
In some embodiments, the carbonyl end of

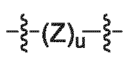

can be attached to the amino end of $L_5$.
In some embodiments, the

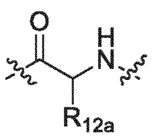

can be

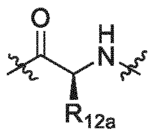 or 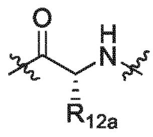 ,

and also can be

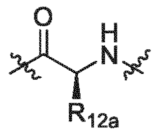 .

In some embodiments, the carbonyl end of T can be attached to the amino end of

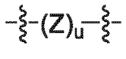 .

In some embodiments, the amino end of M' can be attached to the non-carbonyl end of T.

In some embodiments, the compound of formula II' can be

$$D-(L_5)_x-(Z)_u-(T)_w-M'$$

(II) ;

wherein D is the compound of formula I,

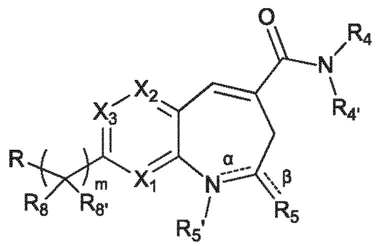

(I) ;

$\alpha$ and $\beta$ are independently a single bond or a double bond; at least one selected from the group of $\alpha$ and $\beta$ is a single bond;

m is 0 or 1;

$X_1$ is N or $CR_2$, $X_2$ is N or $CR_3$, and $X_3$ is N or $CR_1$; when $X_1$ is $CR_2$, $X_2$ is $CR_3$, and $X_3$ is $CR_1$, m is 1;

R is $-C(O)-L_1-R_7$, $-C(O)-NR_9-L_1-R_7$, $-C(S)-NR_9-L_1-R_7$, $-NR_9-L_1-R_7$, $-NR_9-C(O)-L_1-R_7$, $-NR_9-C(O)-NR_9-L_1-R_7$, $-O-L_1-R_7$, $-S(O)_2-NR_9-L_1-R_7$, $-CH=CH-L_1-R_7$, or $-S(O)_2-L_1-R_7$;

$R_1$, $R_2$, and $R_3$ are each independently hydrogen, deuterium, halogen, hydroxyl, amino, cyano, alkyl, haloalkyl, $-L_2-OR_a$, or $-L_2-NR_aR_b$;

$R_4$ and $R_{4'}$ are each independently hydrogen, alkyl, alkenyl, alkynyl, cycloalkyl, heterocycloalkyl, aryl, heteroaryl, cycloalkylalkyl, heterocycloalkylalkyl, arylalkyl, or heteroarylalkyl; the $R_4$ or $R_{4'}$ is unsubstituted or optionally substituted at any position by one or more than one substituent selected from halogen, cyano, $-L_2-OR_a$, $-L_2-OC(O)R_a$, $-L_2-NR_aR_b$, $-L_2-NR_aC(O)OR_b$, $-L_2-NR_aC(O)NR_aR_b$, $-L_2-NR_bC(NR_b)NR_aR_b$, and $-L_2-C(O)OR_b$; or, $R_4$ and $R_{4'}$ together with the N atom to which they are attached form a 3- to 8-membered heterocycloalkyl group; the 3- to 8-membered heterocycloalkyl group is unsubstituted or further substituted at any position by 1 to 3 substituents selected from halogen, cyano, $-L_2-OR_a$, $-L_2-OC(O)R_a$, $-L_2-NR_aR_b$, $-L_2-NR_aC(O)OR_b$, $-L_2-NR_aC(O)NR_aR_b$, $-L_2-NR_bC(NR_b)NR_aR_b$, and $-L_2-C(O)OR_b$;

$R_5$ is =O, $=NR_a$, $-OR_a$, or $-NR_aR_b$;

$R_{5'}$ is absent, or $R_{5'}$ is $-R_c$, $-L_2-OR_a$, $-L_2-NR_aR_b$, $-L_2-NR_aC(O)OR_b$, $-L_2-NR_aC(O)NR_aR_b$, $-L_2-NR_bC(NR_b)NR_aR_b$, or $-L_2-C(O)OR_b$;

$R_7$ is phenyl, 5- to 6-membered heteroaryl, 3- to 8-membered heterocycloalkyl, or an 8- to 12-membered fused ring group; the $R_7$ is unsubstituted or optionally substituted at any position by one or more than one substituent selected from $-L_3-W$, $-R_c$, halogen, cyano, nitro, alkyl, alkenyl, alkynyl, alkoxy, haloalkyl, haloalkoxy, and alkylamino;

$R_8$ and $R_{8'}$ are each independently hydrogen, halogen, or alkyl, and the $R_8$ or $R_{8'}$ is unsubstituted or optionally substituted at any position by one or more than one substituent selected from $-L_3-W$, halogen, cyano, nitro, alkyl, alkenyl, alkynyl, alkoxy, haloalkyl, haloalkoxy, and alkylamino; $R_8$ and $R_{8'}$ are each independent substituents, or, $R_8$ and $R_{8'}$ together with the carbon atom to which they are attached form an oxo, thio, $C_{1-6}$ alkylene, $C_{3-10}$ cycloalkyl, or 3- to 10-membered heterocycloalkyl group; the $C_{1-6}$ alkylene, $C_{3-10}$ cycloalkyl, or 3- to 10-membered heterocycloalkyl group is unsubstituted or optionally substituted at any position by one or more than one substituent selected from deuterium, halogen, hydroxyl, amino, cyano, oxo, alkyl, haloalkyl, $-L_2-OR_a$, and $-L_2-NR_aR_b$;

$R_9$ is hydrogen, alkyl, haloalkyl, $-L_2-OR_a$, or $-L_2-NR_aR_b$;

W is Cyl, $-SR_d$, $-OR_d$, $-OC(O)R_e$, $-OC(O)NR_eR_{e'}$, $-C(O)OR_e$, $-C(O)R_e$, $-C(O)NR_eR_{e'}$, $-C(O)NR_eS(O)_2R_e$, $-NR_dR_e$, $-NR_dC(O)R_e$, $-N(R_d)C(O)OR_e$, $-N(R_d)C(O)NR_eR_{e'}$, $-NR_dS(O)_2R_e$, $-NR_dS(O)_2NR_eR_{e'}$, $-S(O)_{1-2}R_e$, $-S(O)_2NR_eR_{e'}$, $-S(O)(=NR_d)R_e$, $-S(O)_2N(R_e)C(O)R_{e'}$, $-P(O)(OR_e)_2$, $-P(O)(OR_e)R_{e'}$, $-OP(O)(OR_e)_2$, or $-B(OR_e)_2$;

$Cy^1$ is cycloalkyl, heterocycloalkyl, aryl, or heteroaryl; the $Cy^1$ is unsubstituted or optionally substituted at any position by one or more than one substituent selected from halogen, alkyl, haloalkyl, haloalkoxy, alkenyl, alkynyl, cyano, $-R_c$, $-L_4-SR_d$, $-L_4-OC(O)R_e$, $-L_4-C(O)OR_e$, $-L_4-C(O)R_e$, $-L_4-C(O)NR_eR_{e'}$, $-L_4-NR_dC(O)R_e$, $-L_4-NR_dS(O)_2R_e$, $-L_4-S(O)_{1-2}R_e$, $-L_4-S(O)_2NR_eR_{e'}$, $-L_4-OR_d$, and $-L_4-NR_eR_{e'}$;

$L_1$, $L_2$, $L_3$, and $L_4$ are each independently a linkage bond, $C_{1-6}$ alkylene, $C_{2-6}$ alkenylene, or $C_{2-6}$ alkynylene; the $L_1$, $L_2$, $L_3$, or $L_4$ is unsubstituted or optionally substituted at any position by one or more than one substituent selected from oxo, hydroxyl, amino, halogen, cyano, alkyl, haloalkyl, alkoxy, and haloalkoxy;

$R_a$, $R_b$, $R_d$, $R_e$, and $R_{e'}$ are each independently $-R_c$, amino, $C_{1-6}$ alkyl, $C_{2-6}$ alkenyl, $C_{2-6}$ alkynyl, $C_{3-10}$ cycloalkyl, 3- to 10-membered heterocycloalkyl, $C_{6-10}$ aryl, 5- to 10-membered heteroaryl, $C_{3-10}$ cycloalkyl-$C_{1-6}$ alkyl, 3- to 10-membered heterocycloalkyl-$C_{1-6}$ alkyl, phenyl-$C_{1-6}$ alkyl, or 5- to-10 membered heteroaryl-$C_{1-6}$ alkyl; the $R_a$, $R_b$, $R_d$, $R_e$, or $R_{e'}$ is unsubstituted or optionally substituted at any position by 1 to 3 substituents selected from $-OR_f$, $-OC(O)-L_4-R_f$, $-NR_fR_{f'}$, halogen, cyano, nitro, $C_{1-6}$ alkyl, halo-$C_{1-6}$ alkyl, and halo-$C_{1-6}$ alkoxy;

$R_f$ and $R_{f'}$ are each independently $-R_c$, $-NHR_c$, or $C_{1-6}$ alkyl;

each $R_c$ is independently hydrogen or a linkage bond; at least one $R_c$ in D is a linkage bond;

x is 0, 1, 2, or 3;

u is 0, 1, 2, 3, 4, 5, or 6;

w is 0, 1, 2, 3, 4, 5, or 6;

each $L_5$ is independently

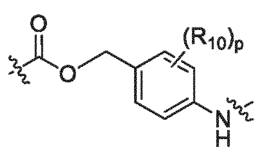,
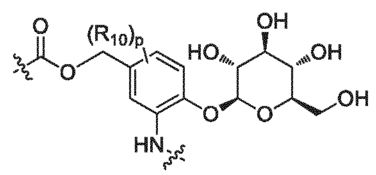,

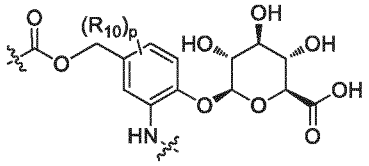,
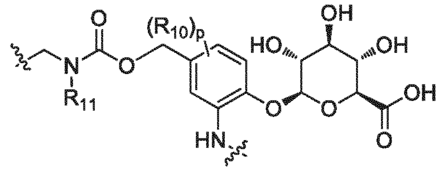, or
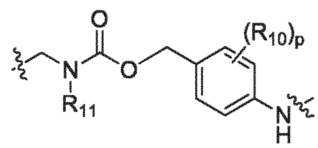;

p is 1, 2, or 3; each $R_{10}$ is independently hydrogen, halogen, $C_{1-6}$ alkyl, halo-$C_{1-6}$ alkyl, nitro, cyano, $C_{1-6}$ alkoxy, $C_{1-6}$ alkylamino, $-OC(O)R_{11}$, or $-C(O)N(R_{11})_2$; $R_{11}$ is hydrogen, $C_{1-6}$ alkyl, or $C_{1-6}$ alkylamino-$C_{1-6}$ alkyl;

each Z is independently

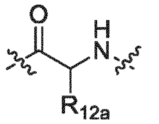,
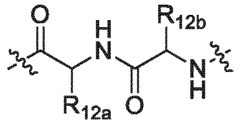,
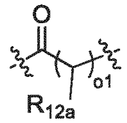,

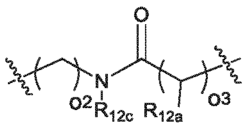, 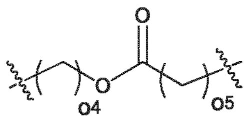, 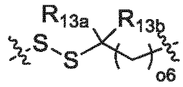,

and $-(CH_2CH_2O)_{o7}-$; o1, o2, o3, o4, o5, o6, o7 are each independently 0, 1, 2, 3, 4, 5, 6, 7, or 8; $R_{12a}$, $R_{12b}$, and $R_{12c}$ are each independently hydrogen, methyl, ethyl, *n*-propyl, isopropyl, $-SO_3H$,

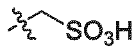, 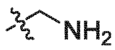,

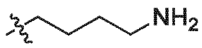, 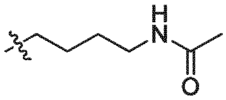, 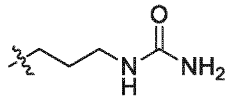, 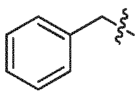, 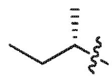, 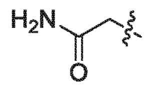,

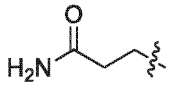, or 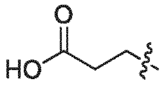;

$R_{13a}$ and $R_{13b}$ are each independently hydrogen or methyl;
each T is independently $-(CH_2)_{v1}-$, $-(CH_2CH_2O)_{v2}-$,

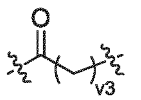, 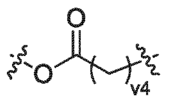,

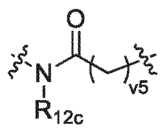, 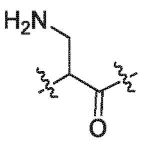, 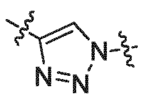, or 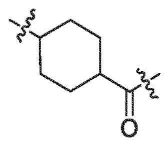;

v1, v2, v3, v4, and v5 are each independently 1, 2, 3, 4, 5, or 6;

M' is 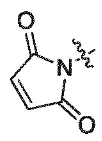, 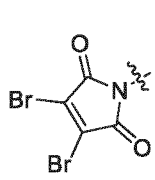, 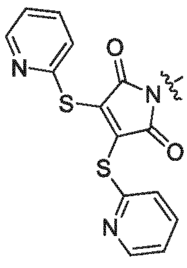, or 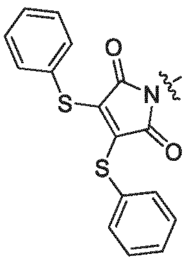.

In some embodiments, the compound of formula II' can be

$$D-(L_5)_x-(Z)_u-(T)_w-M'$$

(II) ;

wherein D is the compound of formula I,

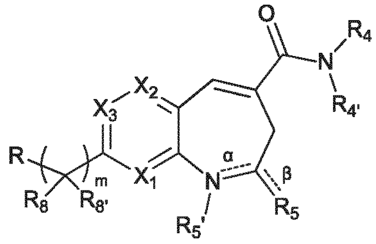

(I) ;

$\alpha$ and $\beta$ are independently a single bond or a double bond; at least one selected from the group of $\alpha$ and $\beta$ is a single bond;
m is 0 or 1;
$X_1$ is N or $CR_2$, $X_2$ is N or $CR_3$, and $X_3$ is N or $CR_1$; when $X_1$ is $CR_2$, $X_2$ is $CR_3$, and $X_3$ is $CR_1$, m is 1;
R is $-C(O)-L_1-R_7$, $-C(O)-NR_9-L_1-R_7$, $-C(S)-NR_9-L_1-R_7$, $-NR_9-L_1-R_7$, $-NR_9-C(O)-L_1-R_7$, $-NR_9-C(O)-NR_9-L_1-R_7$, $-O-L_1-R_7$, $-S(O)_2-NR_9-L_1-R_7$, $-CH=CH-L_1-R_7$, or $-S(O)_2-L_1-R_7$;
$R_1$, $R_2$, and $R_3$ are each independently hydrogen, deuterium, halogen, hydroxyl, amino, cyano, alkyl, haloalkyl, $-L_2-OR_a$, or $-L_2-NR_aR_b$;
$R_4$ and $R_{4'}$ are each independently hydrogen, alkyl, alkenyl, alkynyl, cycloalkyl, heterocycloalkyl, aryl, heteroaryl, cycloalkylalkyl, heterocycloalkylalkyl, arylalkyl, or heteroarylalkyl; the $R_4$ or $R_{4'}$ is unsubstituted or optionally substituted at any position by one or more than one substituent selected from halogen, cyano, $-L_2-OR_a$, $-L_2-NR_aR_b$, $-L_2-NR_aC(O)OR_b$, $-L_2-NR_aC(O)NR_aR_b$, $-L_2-NR_bC(NR_b)NR_aR_b$, and $-L_2-C(O)OR_b$; or, $R_4$ and $R_{4'}$ together with the N atom to which they are attached form a 3- to 8-membered heterocycloalkyl group; the 3- to 8-membered heterocycloalkyl group is unsubstituted or further substituted at any position by 1 to 3 substituents selected from halogen, cyano, $-L_2-OR_a$, $-L_2-NR_aR_b$, $-L_2-NR_aC(O)OR_b$, $-L_2-NR_aC(O)NR_aR_b$, $-L_2-NR_bC(NR_b)NR_aR_b$, and $-L_2-C(O)OR_b$;
$R_5$ is =O, $=NR_a$, $-OR_a$, or $-NR_aR_b$;
$R_{5'}$ is absent, or $R_{5'}$ is $-R_c$, $-L_2-OR_a$, $-L_2-NR_aR_b$, $-L_2-NR_aC(O)OR_b$, $-L_2$-NRaC(O)NRaRb, $-L_2-NR_bC(NR_b)NR_aR_b$, or $-L_2-C(O)OR_b$;
$R_7$ is phenyl, 5- to 6-membered heteroaryl, 3- to 8-membered heterocycloalkyl, or an 8- to 12-membered fused ring group; the $R_7$ is unsubstituted or optionally substituted at any position by one or more than one substituent selected from $-L_3-W$, $-R_c$, halogen, cyano, nitro, alkyl, alkenyl, alkynyl, alkoxy, haloalkyl, haloalkoxy, and alkylamino;
$R_8$ and $R_{8'}$ are each independently hydrogen, halogen, or alkyl, and the $R_8$ or $R_{8'}$ is unsubstituted or optionally substituted at any position by one or more than one substituent selected from $-L_3-W$, halogen, cyano, nitro, alkyl, alkenyl, alkynyl, alkoxy, haloalkyl, haloalkoxy, and alkylamino; $R_8$ and $R_{8'}$ are each independent substituents, or, $R_8$ and $R_{8'}$ together with the carbon atom to which they are attached form an oxo, thio, $C_{1-6}$ alkylene, $C_{3-10}$ cycloalkyl, or 3- to 10-membered heterocycloalkyl group; the $C_{1-6}$ alkylene, $C_{3-10}$ cycloalkyl, or 3- to 10-membered heterocycloalkyl group is unsubstituted or optionally substituted at any position by one or more than one substituent selected from deuterium, halogen, hydroxyl, amino, cyano, oxo, alkyl, haloalkyl, $-L_2-OR_a$, and $-L_2-NR_aR_b$;
$R_9$ is hydrogen, alkyl, haloalkyl, $-L_2-OR_a$, or $-L_2-NR_aR_b$;
W is Cyl, $-SR_d$, $-OR_d$, $-OC(O)R_e$, $-OC(O)NR_eR_{e'}$, $-C(O)OR_e$, $-C(O)R_e$, $-C(O)NR_eR_{e'}$, $-C(O)NR_eS(O)_2R_e$, $-NR_dR_e$, $-NR_dC(O)R_e$, $-N(R_d)C(O)OR_e$, $-N(R_d)C(O)NR_eR_{e'}$, $-NR_dS(O)_2R_e$, $-NR_dS(O)_2NR_eR_{e'}$, $-S(O)_{1-2}R_e$, $-S(O)_2NR_eR_{e'}$, $-S(O)(=NR_d)R_e$, $-S(O)_2N(R_e)C(O)R_{e'}$, $-P(O)(OR_e)_2$, $-P(O)(OR_e)R_{e'}$, $-OP(O)(OR_e)_2$, or $-B(OR_e)_2$;
$Cy^1$ is cycloalkyl, heterocycloalkyl, aryl, or heteroaryl; the $Cy^1$ is unsubstituted or optionally substituted at any position by one or more than one substituent selected from halogen, alkyl, haloalkyl, haloalkoxy, alkenyl, alkynyl, cyano, $-R_c$, $-L_4-SR_d$, $-L_4-OC(O)R_e$, $-L_4-C(O)OR_e$, $-L_4-C(O)R_e$, $-L_4-C(O)NR_eR_{e'}$, $-L_4-NR_dC(O)R_e$, $-L_4-NR_dS(O)_2R_e$, $-L_4-S(O)_{1-2}R_e$, $-L_4-S(O)_2NR_eR_{e'}$, $-L_4-OR_d$, and $-L_4-NR_eR_{e'}$;
$L_1$, $L_2$, $L_3$, and $L_4$ are each independently a linkage bond, $C_{1-6}$ alkylene, $C_{2-6}$ alkenylene, or $C_{2-6}$ alkynylene; the $L_1$, $L_2$, $L_3$, or $L_4$ is unsubstituted or optionally substituted at any position by one or more than one substituent selected from oxo, hydroxyl, amino, halogen, cyano, alkyl, haloalkyl, alkoxy, and haloalkoxy;
$R_a$, $R_b$, $R_d$, $R_e$, and $R_{e'}$ are each independently $-R_c$, amino, $C_{1-6}$ alkyl, $C_{2-6}$ alkenyl, $C_{2-6}$ alkynyl, $C_{3-10}$ cycloalkyl, 3- to 10-membered heterocycloalkyl, $C_{6-10}$ aryl, 5- to 10-membered heteroaryl, $C_{3-10}$ cycloalkyl-$C_{1-6}$ alkyl, 3- to 10-membered heterocycloalkyl-$C_{1-6}$ alkyl, phenyl-$C_{1-6}$ alkyl, or 5- to-10 membered heteroaryl-$C_{1-6}$ alkyl; the $R_a$, $R_b$, $R_d$, $R_e$, or $R_{e'}$ is unsubstituted or optionally substituted at any position by 1 to 3 substituents selected from $-OR_f$, $-NR_fR_{f'}$, halogen, cyano, nitro, $C_{1-6}$ alkyl, halo-$C_{1-6}$ alkyl, and halo-$C_{1-6}$ alkoxy;
$R_f$ and $R_{f'}$ are each independently $-R_c$, $-NHR_c$, or $C_{1-6}$ alkyl;
each $R_c$ is independently hydrogen or a linkage bond; at least one $R_c$ in D is a linkage bond;
x is 0, 1, 2, or 3;

u is 0, 1, 2, 3, 4, 5, or 6;
w is 0, 1, 2, 3, 4, 5, or 6;
each $L_5$ is independently

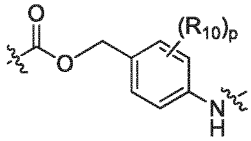, 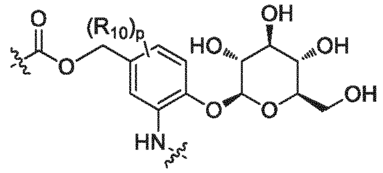,

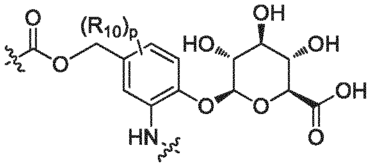, 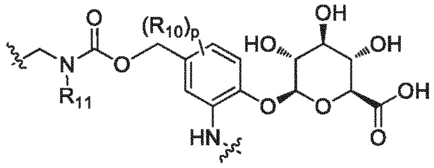, or 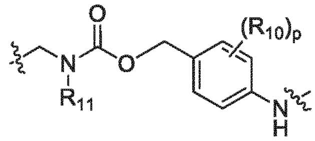;

p is 1, 2, or 3; each $R_{10}$ is independently hydrogen, halogen, $C_{1-6}$ alkyl, halo-$C_{1-6}$ alkyl, nitro, cyano, $C_{1-6}$ alkoxy, $C_{1-6}$ alkylamino, $-OC(O)R_{11}$, or $-C(O)N(R_{11})_2$; $R_{11}$ is hydrogen, $C_{1-6}$ alkyl, or $C_{1-6}$ alkylamino-$C_{1-6}$ alkyl;
each Z is independently

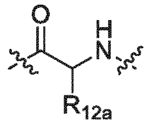, 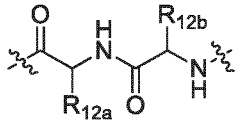, 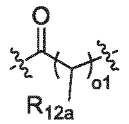,

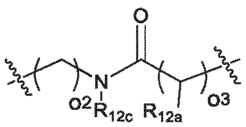, 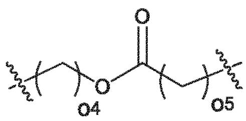, 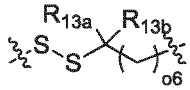,

and $-(CH_2CH_2O)_{o7}-$; o1, o2, o3, o4, o5, o6, o7 are each independently 0, 1, 2, 3, 4, 5, 6, 7, or 8; $R_{12a}$, $R_{12b}$, and $R_{12c}$ are each independently hydrogen, methyl, ethyl, n-propyl, isopropyl, $-SO_3H$,

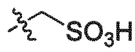, 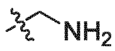,

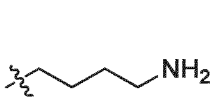, 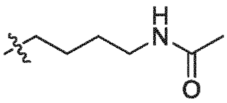, 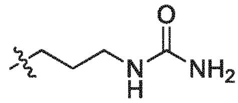, 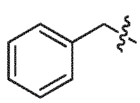, 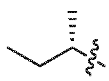, 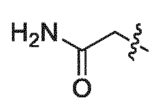,

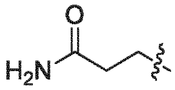, or 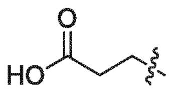;

$R_{13a}$ and $R_{13b}$ are each independently hydrogen or methyl;
each T is independently $-(CH_2)_{v1}-$, $-(CH_2CH_2O)_{v2}-$,

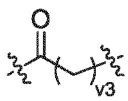, 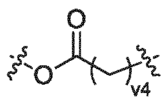,

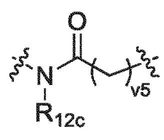, 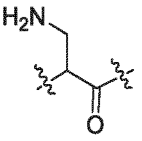, 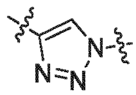, or 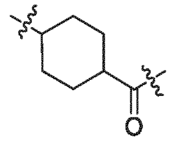;

v1, v2, v3, v4, and v5 are each independently 1, 2, 3, 4, 5, or 6;
M'is

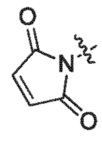, 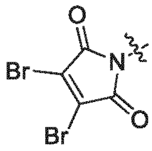, 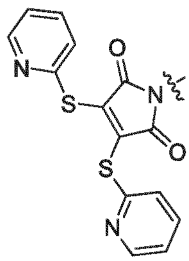, or 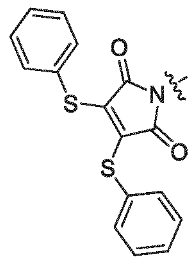.

All embodiments of formula II described below and combinations of variables are included in the scope of the structural formula of formula II of the present disclosure.

In some embodiments, M' is

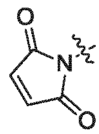.

In some embodiments, x is 0 or 1.

In some embodiments, x is 0 or 1, $-L_5-$ is

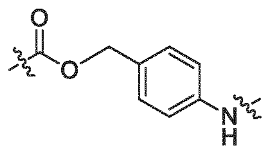;

$-(Z)_u-$ is

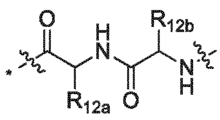, 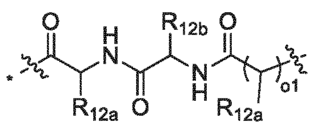,

or

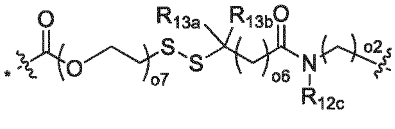;

o1, o2, o6, o7, $R_{12a}$, $R_{12b}$, $R_{12c}$, $R_{13a}$, and $R_{13b}$ are defined as above; the marked site * is the site attached to Ls;

$-(T)_w-$ is $-(CH_2CH_2O)_{v2}-*$,

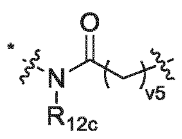 , 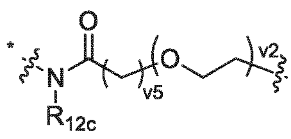 , or 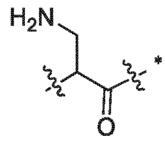 ;

v1, v2, v5, and $R_{12c}$ are defined as above; the marked site * is the site attached to Z.

In some embodiments, x is 1, $-L_5-$ is

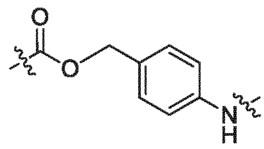 ;

$-(Z)_u-$ is

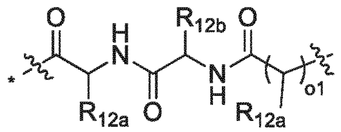 ;

o1, $R_{12a}$, and $R_{12b}$ are defined as above; the marked site * is the site attached to $L_5$;

$-(T)_w-$ is a linkage bond, $-(CH_2CH_2O)_{v2}-$*,

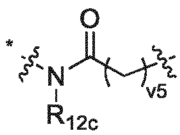 , or 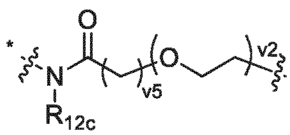 ;

v1, v2, v5, and $R_{12c}$ are defined as above; the marked site * is the site attached to Z.

In some embodiments, $-(Z)_u-$ is

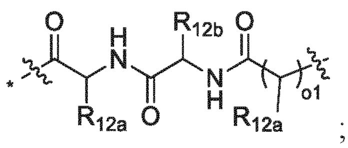 ;

o1, $R_{12a}$, and $R_{12b}$ are defined as above; the marked site * is the site attached to $L_5$.

In some embodiments, $-(Z)_u-$ is

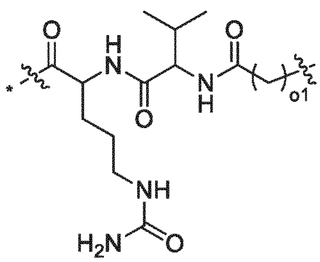 , 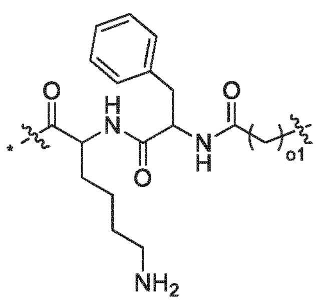 ,

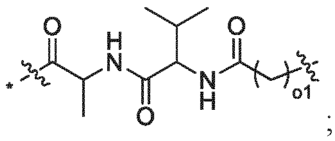

;

o1 is defined as above; the marked site * is the site attached to $L_5$.

In some embodiments, $-(Z)_u-$ is

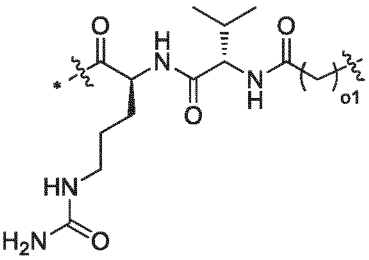

or

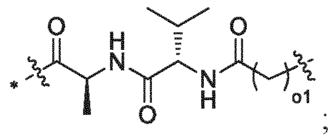

;

o1 is defined as above; the marked site * is the site attached to $L_5$.

In some embodiments, in D, $X_3$ is $CR_1$; $X_1$ is N; $X_2$ is $CR_3$.

In some embodiments, in D, $X_3$ is $CR_1$; $X_1$ is $CR_2$; $X_2$ is $CR_3$.

In some embodiments, in D, $R_a$, $R_b$, $R_d$, $R_e$, and $R_{e'}$ are each independently $-R_c$, amino, $C_{1-6}$ alkyl, $C_{2-6}$ alkenyl, $C_{2-6}$ alkynyl, $C_{3-10}$ cycloalkyl, 3- to 10-membered heterocycloalkyl, $C_{6-10}$ aryl, 5- to 10-membered heteroaryl, $C_{3-10}$ cycloalkyl-$C_{1-6}$ alkyl, 3- to 10-membered heterocycloalkyl-$C_{1-6}$ alkyl, phenyl-$C_{1-6}$ alkyl, or 5- to-10 membered heteroaryl-$C_{1-6}$ alkyl; the $R_a$, $R_b$, $R_d$, $R_e$, or $R_{e'}$ is unsubstituted or optionally substituted at any position by 1 to 3 substituents selected from $-OR_f$, $-OC(O)-(CH_2)_{1-5}-R_f$, $-NR_fR_{f'}$, halogen, cyano, $C_{1-6}$ alkyl, halo-$C_{1-6}$ alkyl, and halo-$C_{1-6}$ alkoxy.

In some embodiments, in D, R is $-C(O)-NR_9-L_1-R_7$.

In some embodiments, in D, $R_1$, $R_2$, and $R_3$ are each independently hydrogen, deuterium, halogen, hydroxyl, amino, cyano, $C_{1-6}$ alkyl, halo-$C_{1-6}$ alkyl, $-L_2-OR_a$, or $-L_2-NR_aR_b$.

In some embodiments, in D, $R_1$, $R_2$, and $R_3$ are each independently preferably H, F, Cl, Br, $-CH_3$, $-OCH_3$, $-CF_3$, $-CH_2F$, $-CHF_2$, $-OCF_3$, -CN, or $-(CH2)_{0-5}-NH_2$.

In some embodiments, in D, $R_1$ is H; $R_2$ is H.

In some embodiments, in D, $R_4$ and $R_{4'}$ are each independently hydrogen, $C_{1-6}$ alkyl, $C_{2-6}$ alkenyl, $C_{2-6}$ alkynyl, $C_{3-8}$ cycloalkyl, 3- to 8-membered heterocycloalkyl, $C_{6-10}$ aryl, 5- to 10-membered heteroaryl, cycloalkyl-$C_{1-6}$ alkyl, 3- to 8-membered heterocycloalkyl-$C_{1-6}$ alkyl, $C_{6-10}$ aryl-$C_{1-6}$ alkyl, or 5- to 10-membered heteroaryl-$C_{1-6}$ alkyl; the $R_4$ or $R_{4'}$ is unsubstituted or optionally substituted at any position by 1 to 3 substituents selected from halogen, cyano, $-L_2-OR_a$, $-L_2-OC(O)R_a$, $-L_2-NR_aR_b$, $-L_2-NR_aC(O)OR_b$, $-L_2-NR_aC(O)NR_aR_b$, $-L_2-NR_bC(NR_b)NR_aR_b$, and $-L_2-C(O)OR_b$; or, $R_4$ and $R_{4'}$ together with the N atom to which they are attached form a 3- to 8-membered heterocycloalkyl group; the 3- to 8-membered heterocycloalkyl group is unsubstituted or further substituted at any position by 1 to 3 substituents selected from halogen, cyano, $-L_2-OR_a$, $-L_2-NR_aR_b$, $-L_2-NR_aC(O)OR_b$, $-L_2-NR_aC(O)NR_aR_b$, $-L_2-NR_bC(NR_b)NR_aR_b$, and $-L_2-C(O)OR_b$.

In some embodiments, in D, $R_4$ and $R_{4'}$ are each independently $C_{1-6}$ alkyl; the $R_4$ or $R_{4'}$ is unsubstituted or optionally substituted at any position by 1 to 3 substituents selected from $-OR_a$, $-OC(O)R_a$, $-NR_aR_b$, $-NR_aC(O)OR_b$, $-NR_aC(O)NR_aR_b$, $-NR_bC(NR_b)NR_aR_b$, and $-C(O)OR_b$.

In some embodiments, in D, $R_4$ is $C_{1-6}$ alkyl; the $R_4$ is unsubstituted or optionally substituted at any position by one $-OR_a$, $-OC(O)R_a$, $-NR_aR_b$, $-NR_aC(O)OR_b$, $-NR_aC(O)NR_aR_b$, $-NR_bC(NR_b)NR_aR_b$, or $-C(O)OR_b$; $R_{4'}$ is $C_{1-6}$ alkyl; the $R_{4'}$ is unsubstituted or optionally substituted at any position by 1 to 3 substituents selected from halogen, $-OR_a$, and $-NR_aR_b$.

In some embodiments, in D, $R_4$ and $R_{4'}$ are each independently $C_{1-6}$ alkyl; the $R_4$ or $R_{4'}$ is unsubstituted or

optionally substituted at any position by one substituent selected from halogen, $-OR_a$, and $-NR_aR_b$.

In some embodiments, in D, $R_4$ is $-CH_2CH_2OH$ or $-CH_2CH_2CH_3$; $R_{4'}$ is $-CH_2CH_2OH$, $-CH_2CH_2CH_3$, $-CH_2CH_2CF_3$, or $-CH_2CH_2CHF_2$.

In some embodiments, in D, $\alpha$ is a double bond, $\beta$ is a single bond, $R_{5'}$ is absent, and $R_5$ is $-NR_aR_b$.

In some embodiments, in D, $\alpha$ is a double bond, $\beta$ is a single bond, $R_{5'}$ is absent, and $R_5$ is $-NH_2$.

In some embodiments, in D, $\alpha$ is a single bond, $\beta$ is a double bond, $R_{5'}$ is H, and $R_5$ is =O.

In some embodiments, in D, Rs and $R_{8'}$ are each independently hydrogen, halogen, or $C_{1-6}$ alkyl; the $R_8$ or $R_{8'}$ is unsubstituted or optionally substituted at any position by 1 to 3 substituents selected from $-L_3$-W, halogen, cyano, nitro, $C_{1-6}$ alkyl, $C_{2-6}$ alkenyl, $C_{2-6}$ alkynyl, $C_{1-6}$ alkoxy, halo-$C_{1-6}$ alkyl, halo-$C_{1-6}$ alkoxy, and $C_{1-6}$ alkylamino.

In some embodiments, in D, $R_8$ and $R_{8'}$ are each independently $C_{1-6}$ alkyl, and the $C_{1-6}$ alkyl is preferably methyl.

In some embodiments, in D, $R_8$ and $R_{8'}$ together with the carbon atom to which they are attached form an oxo, thio, $C_{3-6}$ cycloalkyl, or 3- to 6-membered heterocycloalkyl group; the $C_{3-6}$ cycloalkyl or 3- to 6-membered heterocycloalkyl group is unsubstituted or optionally substituted at any position by 1 to 3 substituents selected from deuterium, halogen, hydroxyl, amino, cyano, oxo, $C_{1-6}$ alkyl, halo-$C_{1-6}$ alkyl, $-L_2-OR_a$, and $-L_2-NR_aR_b$.

In some embodiments, in D, $R_8$ and $R_{8'}$ together with the carbon atom to which they are attached form an oxo, thio, cyclopropyl, cyclobutyl, azetidinyl, or oxetanyl group; the cyclopropyl, cyclobutyl, azetidinyl, or oxetanyl group is unsubstituted or optionally substituted at any position by 1 to 3 substituents selected from deuterium, halogen, hydroxyl, amino, cyano, oxo, $C_{1-6}$ alkyl, halo-$C_{1-6}$ alkyl, $-L_2-OR_a$, and $-L_2-NR_aR_b$.

In some embodiments, in D, $R_8$ and $R_{8'}$ together with the carbon atom to which they are attached form an oxo, thio, cyclopropyl, or cyclobutyl group.

In some embodiments, in D, $R_9$ is hydrogen, $C_{1-6}$ alkyl, halo-$C_{1-6}$ alkyl, $-L_2-OR_a$, or - $L_2-NR_aR_b$.

In some embodiments, in D, $R_9$ is hydrogen.

In some embodiments, in D, $Cy^1$ is $C_{3-8}$ cycloalkyl, 3- to 8-membered heterocycloalkyl, $C_{6-10}$ aryl, or 5- to 10-membered heteroaryl; the $Cy^1$ is unsubstituted or optionally substituted at any position by 1 to 3 substituents selected from halogen, cyano, $C_{1-6}$ alkyl, halo-$C_{1-6}$ alkyl, halo-$C_{1-6}$ alkoxy, $C_{2-6}$ alkenyl, $C_{2-6}$ alkynyl, $-L_4-SR_d$, $-L_4-OC(O)R_e$, $-L_4-C(O)OR_e$, $-L_4-C(O)R_e$, $-L_4-C(O)NR_eR_{e'}$, $-L_4-NR_dC(O)R_e$, $-L_4-NR_dS(O)_2R_e$, $-L_4-S(O)_{1-2}R_e$, $-L_4-S(O)_2NR_eR_{e'}$, $-L_4-OR_d$, or $-L_4-NR_eR_{e'}$;

In some embodiments, in D, $L_1$ is a linkage bond or $C_{1-6}$ alkylene; the $L_1$ is unsubstituted or optionally substituted at any position by 1 to 3 substituents selected from oxo, halogen, hydroxyl, amino, cyano, $C_{1-6}$ alkyl, halo-$C_{1-6}$ alkyl, $C_{1-6}$ alkoxy, or halo-$C_{1-6}$ alkoxy.

In some embodiments, in D, $L_1$ is a linkage bond or $-CH_2-$.

In some embodiments, in D, $L_2$ is a linkage bond or $C_{1-6}$ alkylene; the $L_2$ is unsubstituted or optionally substituted at any position by 1 to 3 substituents selected from oxo, halogen, hydroxyl, amino, cyano, $C_{1-6}$ alkyl, halo-$C_{1-6}$ alkyl, $C_{1-6}$ alkoxy, or halo-$C_{1-6}$ alkoxy.

In some embodiments, in D, $L_3$ is a linkage bond or $C_{1-6}$ alkylene; the $L_3$ is unsubstituted or optionally substituted at any position by 1 to 3 substituents selected from oxo, halogen, hydroxyl, amino, cyano, $C_{1-6}$ alkyl, halo-$C_{1-6}$ alkyl, $C_{1-6}$ alkoxy, or halo-$C_{1-6}$ alkoxy.

In some embodiments, in D, $L_3$ is a linkage bond, $-CH_2-$, $-CH_2CH_2-$, $-CH_2CH_2CH_2-$, - $CH_2CH_2C(CH_3)_2-$, $-CH_2CH_2CH_2CH_2-$, or $-CH_2CH(CH_3)CH_2-$.

In some embodiments, in D, $L_3$ is $-CH_2-$.

In some embodiments, in D, $L_4$ is a linkage bond or $C_{1-6}$ alkylene; the $L_4$ is unsubstituted or optionally substituted at any position by 1 to 3 substituents selected from oxo, halogen, hydroxyl, amino, cyano, $C_{1-6}$ alkyl, halo-$C_{1-6}$ alkyl, $C_{1-6}$ alkoxy, or halo-$C_{1-6}$ alkoxy.

In some embodiments, in D, $R_7$ is phenyl, 5- to 6-membered heteroaryl, 3- to 8-membered heterocycloalkyl, or an 8- to 12-membered fused ring group; the $R_7$ is unsubstituted or optionally substituted at any position by one or more than one substituent selected from $-L_3$-W, $-R_c$, halogen, cyano, nitro, $C_{1-6}$ alkyl, $C_{2-6}$ alkenyl, $C_{2-6}$ alkynyl, $C_{1-6}$ alkoxy, halo-$C_{1-6}$ alkyl, halo-$C_{1-6}$ alkoxy, and $C_{1-6}$ alkylamino.

In some embodiments, in D, $R_7$ is an 8- to 12-membered fused ring group; the $R_7$ is unsubstituted or optionally substituted at any position by one or more than one substituent selected from $-L_3$-W, $-R_c$, halogen, cyano, nitro, $C_{1-6}$ alkyl, $C_{2-6}$ alkenyl, $C_{2-6}$ alkynyl, $C_{1-6}$ alkoxy, halo-$C_{1-6}$ alkyl, halo-$C_{1-6}$ alkoxy, and $C_{1-6}$ alkylamino.

In some embodiments, in D, $R_7$ is

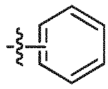, 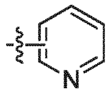, 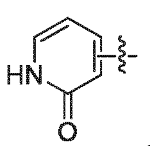, 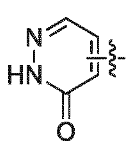,

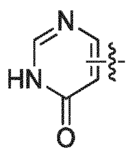, 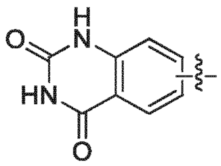, 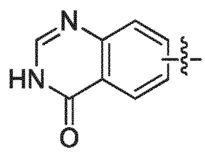, 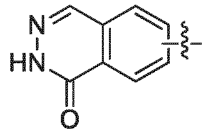, 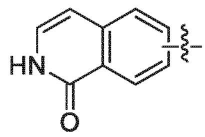,

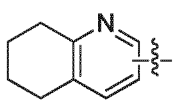, 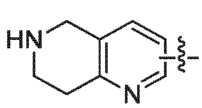, 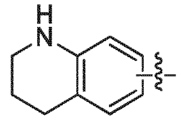, 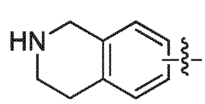, 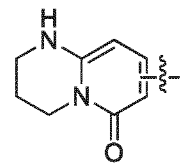,

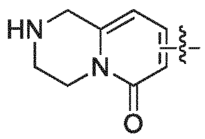, or 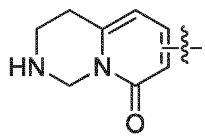;

the $R_7$ is unsubstituted or optionally substituted at any position by 1 to 3 substituents selected from $-L_3$-W, $-R_c$, halogen, cyano, nitro, $C_{1-6}$ alkyl, $C_{2-6}$ alkenyl, $C_{2-6}$ alkynyl, $C_{1-6}$ alkoxy, halo-$C_{1-6}$ alkyl, halo-$C_{1-6}$ alkoxy, and $C_{1-6}$ alkylamino.

In some embodiments, in D, $R_7$ is

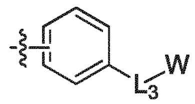, 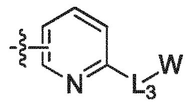, 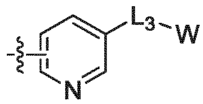,

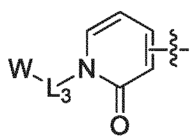, 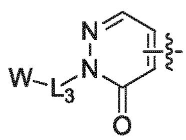, 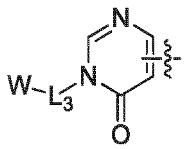, 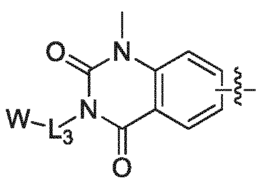, 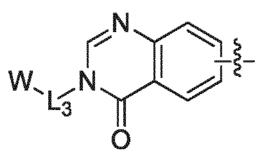,

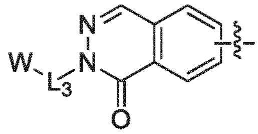, 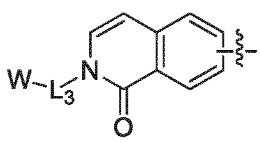, 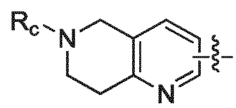, 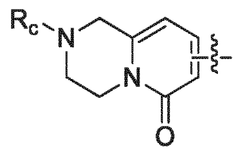,

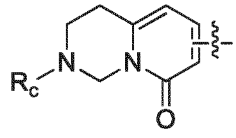, 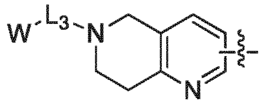, 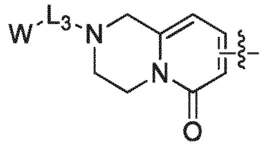, or 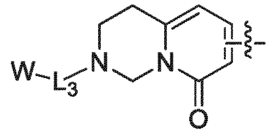.

In some embodiments, in D, $R_7$ is

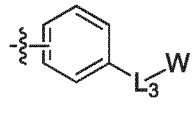 or 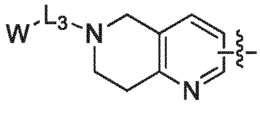.

In some embodiments, in D, $X_1$ is N or $CR_2$, $X_2$ is N or $CR_3$, and $X_3$ is N or $CR_1$; when $X_1$ is $CR_2$, $X_2$ is $CR_3$, and $X_3$ is $CR_1$, m is 1;

In some embodiments, in D, m is 0 or 1;
$X_1$ is N or $CR_2$, $X_2$ is N or $CR_3$, and $X_3$ is N or $CR_1$; when $X_1$ is $CR_2$, $X_2$ is $CR_3$, and $X_3$ is $CR_1$, m is 1;
R is $-C(O)-L_1-R_7$, $-C(O)-NR_9-L_1-R_7$, or $-C(S)-NR_9-L_1-R_7$.

In some embodiments, in D, m is 1;

$X_1$ is N or $CR_2$, $X_2$ is N or $CR_3$, and $X_3$ is N or $CR_1$;
R is $-C(O)-L_1-R_7$, $-C(O)-NR_9-L_1-R_7$, or $-C(S)-NR_9-L_1-R_7$.

In some embodiments, D is a compound of formula IA:

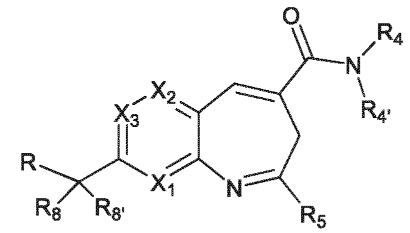

(IA) ;

R is $-C(O)-L_1-R_7$, $-C(O)-NR_9-L_1-R_7$, or $-C(S)-NR_9-L_1-R_7$;
$X_1$, $X_2$, $X_3$, R, $R_4$, $R_{4'}$, $R_5$, $R_8$, and $R_{8'}$ are defined as above.

In some embodiments, D is a compound of formula IB:

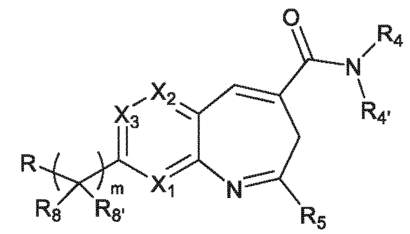

(IB) ;

R is $-C(O)-L_1-R_7$, $-C(O)-NR_9-L_1-R_7$, or $-C(S)-NR_9-L_1-R_7$;
$R_4$ is $C_{1-6}$ alkyl; the $R_4$ is optionally substituted at any position by one $-OR_a$;
$X_1$, $X_2$, $X_3$, R, $R_{4'}$, $R_5$, $R_8$, $R_{8'}$, $R_a$, and m are defined as above.

In some embodiments, in D, in IA, $L_1$ is a linkage bond.

In some embodiments, in D, in IA, $R_8$ and $R_{8'}$ together with the carbon atom to which they are attached form a $C_{3-6}$ cycloalkyl or 3- to 6-membered heterocycloalkyl group; the $C_{3-6}$ cycloalkyl or 3- to 6-membered heterocycloalkyl group is unsubstituted or optionally substituted at any position by 1 to 3 substituents selected from deuterium, halogen, hydroxyl, amino, cyano, oxo, $C_{1-6}$ alkyl, halo-$C_{1-6}$ alkyl, $-L_2-OR_a$, and $-L_2-NR_aR_b$.

In some embodiments, in D, in IA, $R_8$ and $R_{8'}$ together with the carbon atom to which they are attached form the following groups:

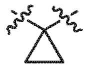 , 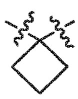 , 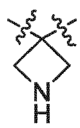 , or 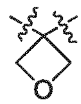 .

In some embodiments, in D, in IA, $X_1$ is CH, $X_2$ is $CR_3$, and $X_3$ is CH; m is 1; $R_3$ is H or halogen.

In some embodiments, in D, in IA, $R_5$ is $-NH_2$.

In some embodiments, in D, in IB, $R_a$ is H.

In some embodiments, in D, in IB, $X_1$ is N or $CR_2$, $X_2$ is N or $CR_3$, and $X_3$ is N or $CR_1$; m is 1; $L_1$ is a linkage bond.

In some embodiments, in D, in IB, $R_8$ and $R_{8'}$ together with the carbon atom to which they are attached form a $C_{3-6}$ cycloalkyl or 3- to 6-membered heterocycloalkyl group; the $C_{3-6}$ cycloalkyl or 3- to 6-membered heterocycloalkyl group is unsubstituted or optionally substituted at any position by 1 to 3 substituents selected from deuterium, halogen, hydroxyl, amino, cyano, oxo, $C_{1-6}$ alkyl, halo-$C_{1-6}$ alkyl, $-L_2-OR_a$, and $-L_2-NR_aR_b$.

In some embodiments, in D, in IB, $R_8$ and $R_{8'}$ together with the carbon atom to which they are attached form the following groups:

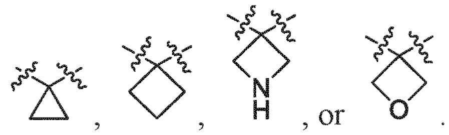

In some embodiments, in D, in IB, $R_5$ is $-NH_2$.

In some embodiments, D is the compound shown in Table 1 and is covalently attached to $L_5$.

In some embodiments, D is the compound shown in Table 1 and is covalently attached to $L_5$ via a nitrogen atom in the molecule.

In some embodiments, the compound of formula II' has any one of the following structures:

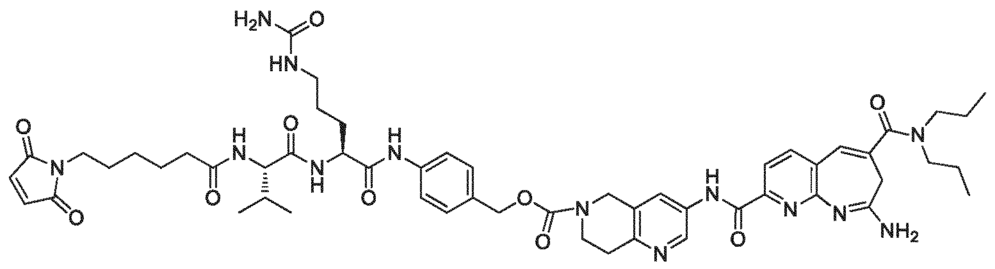

,

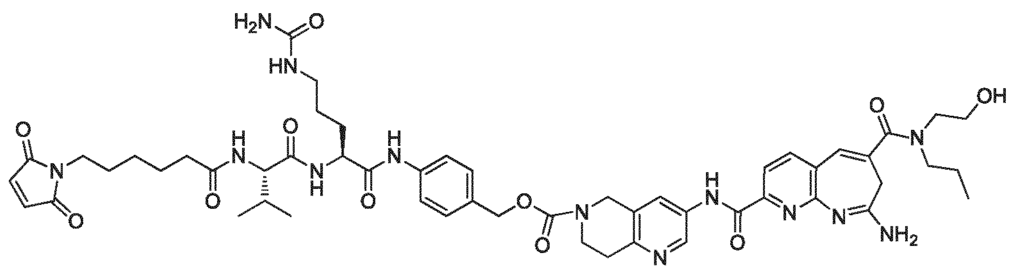

,

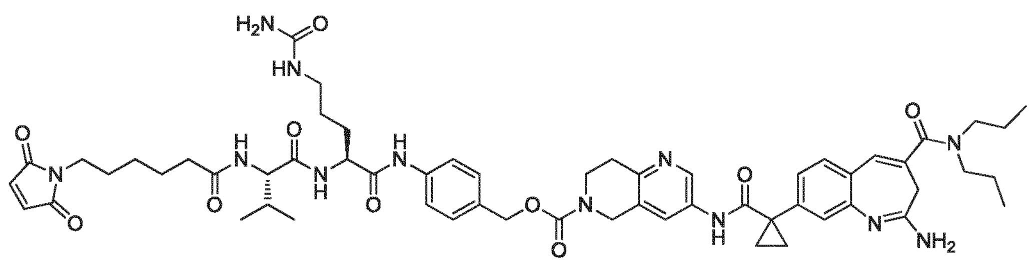

,

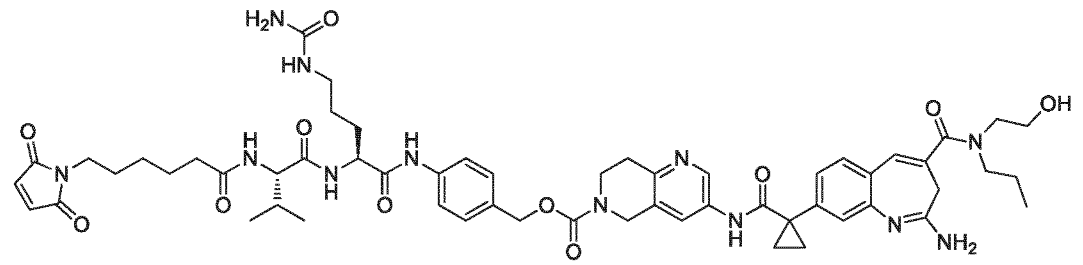

,

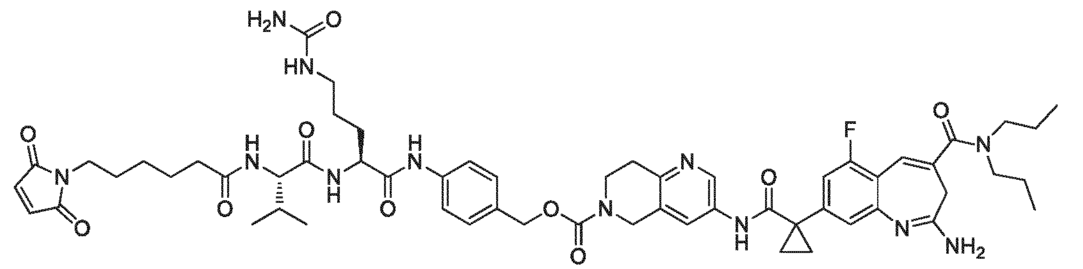

,

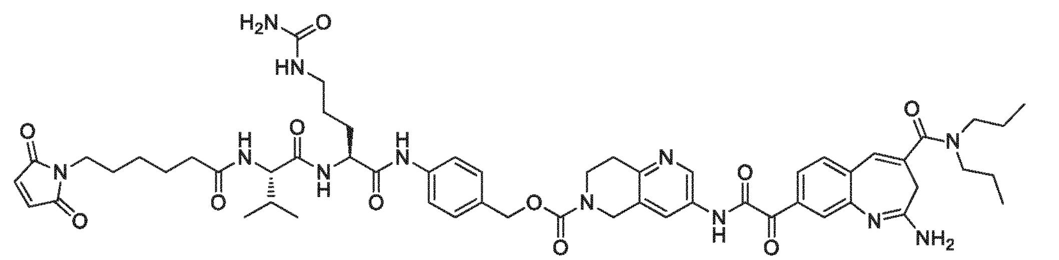

,

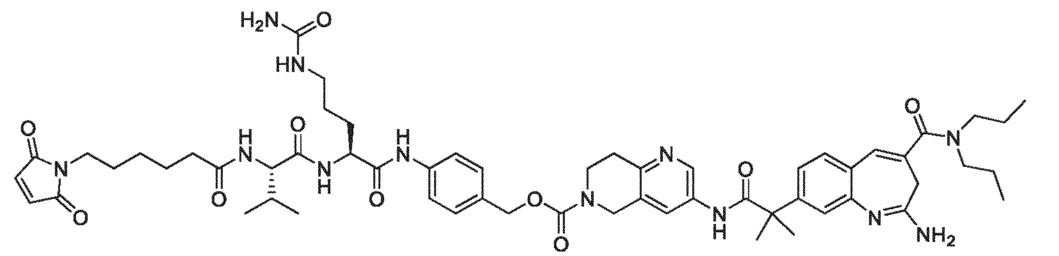

,

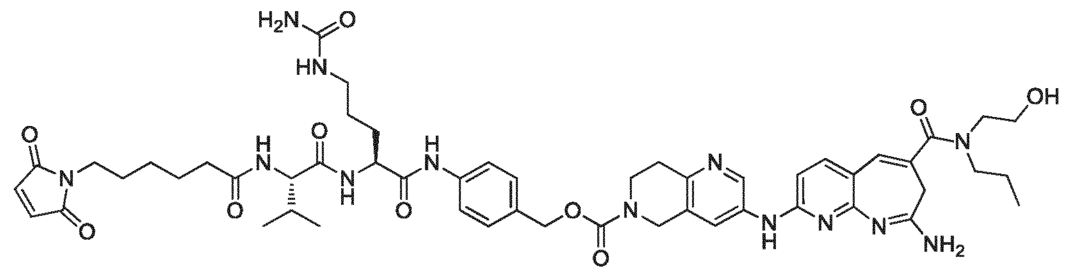

,

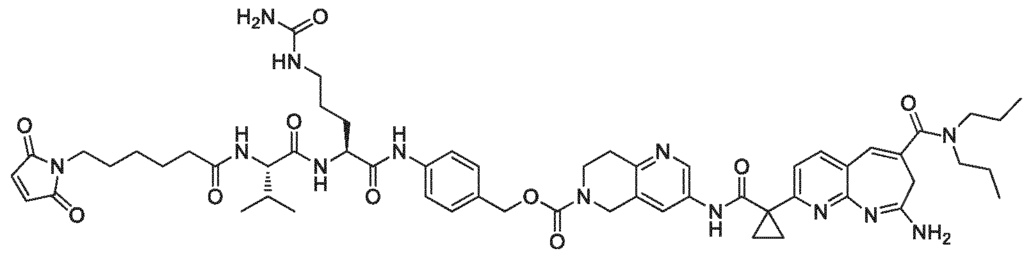

,

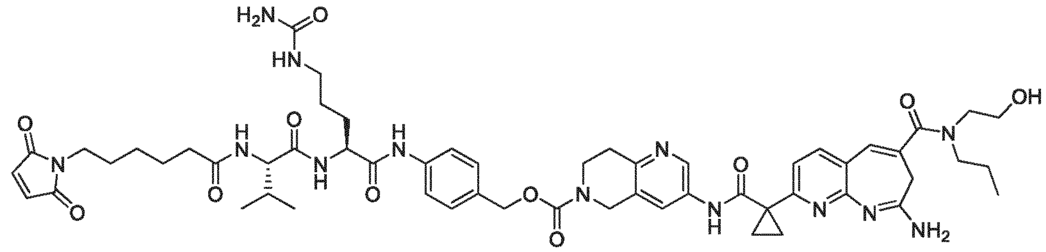

,

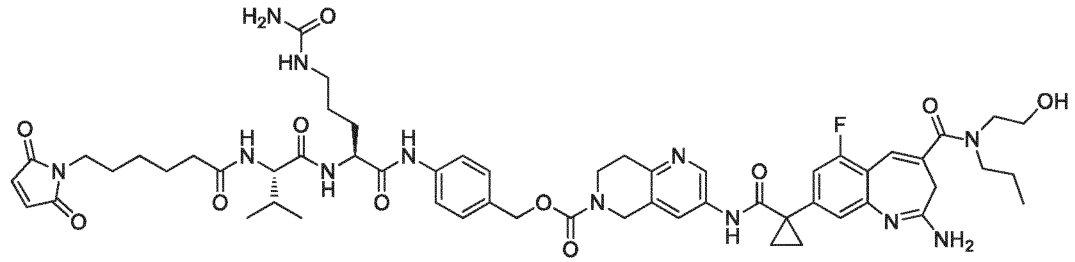

,

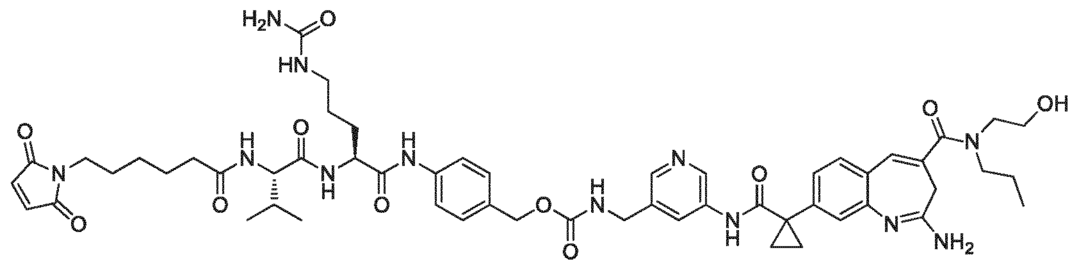

,

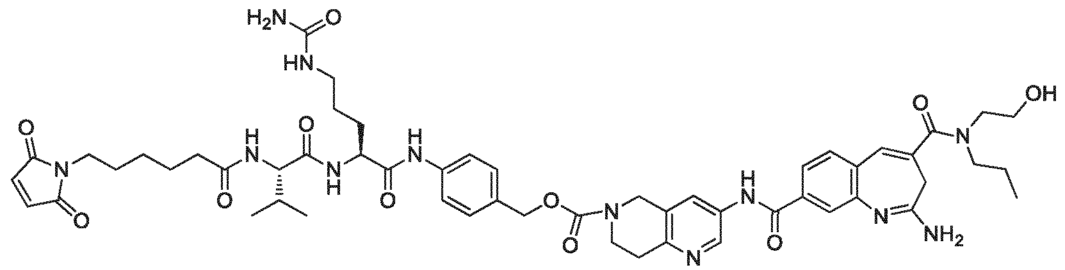

,

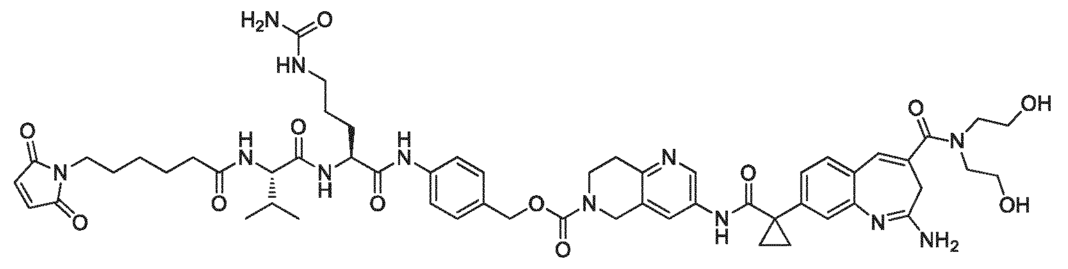

,

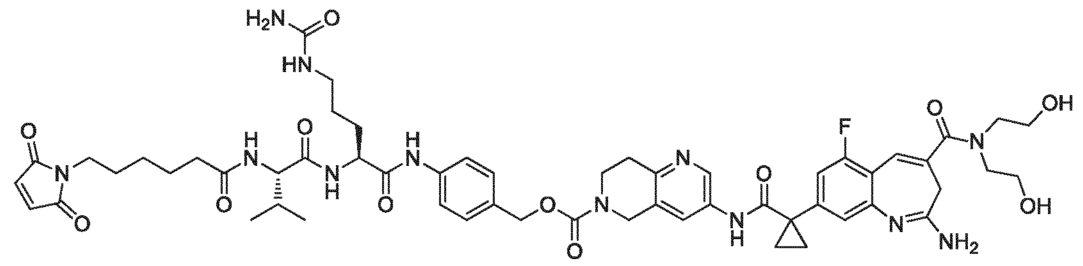

,

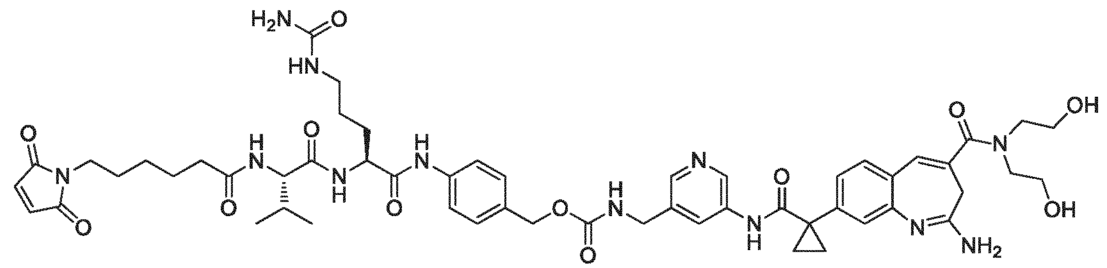

,

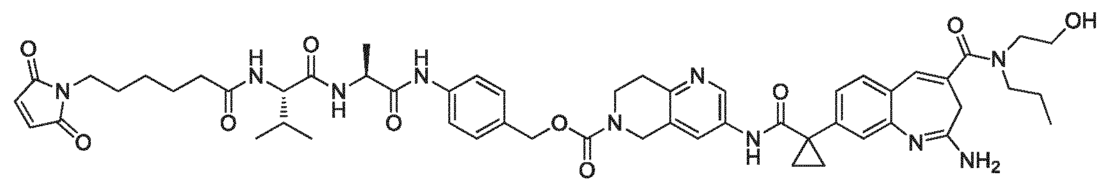

,

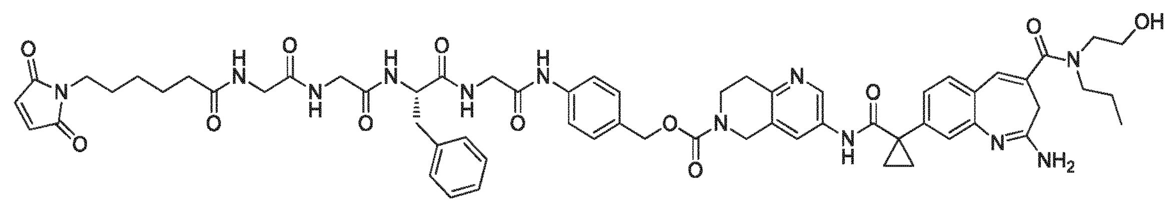

,

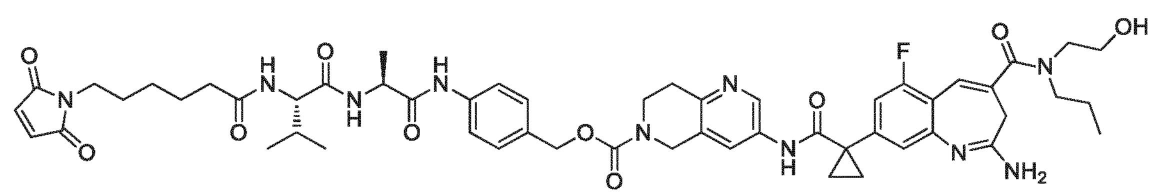

,

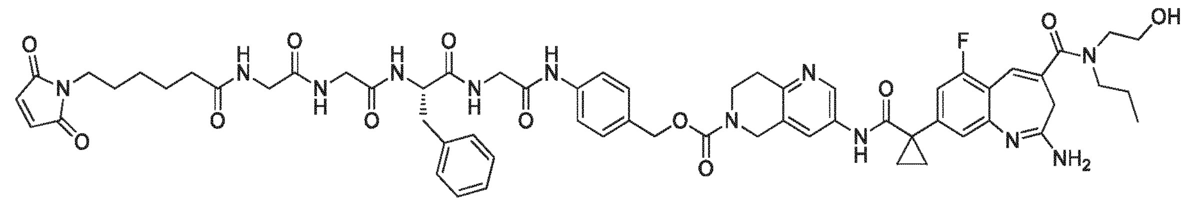

,

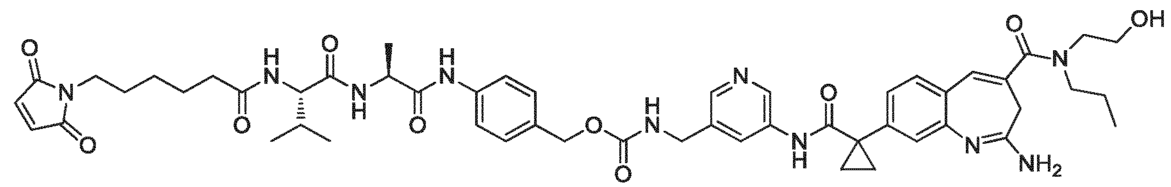

,

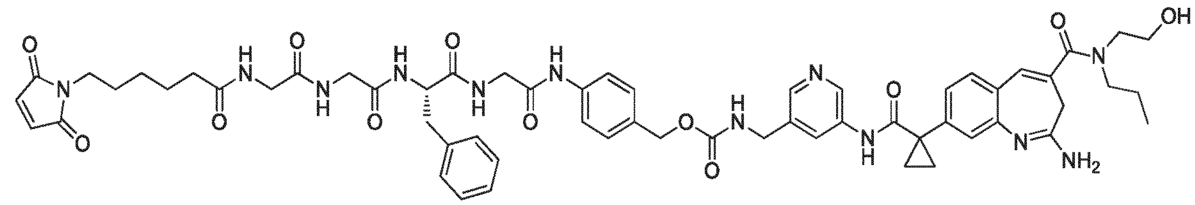

,

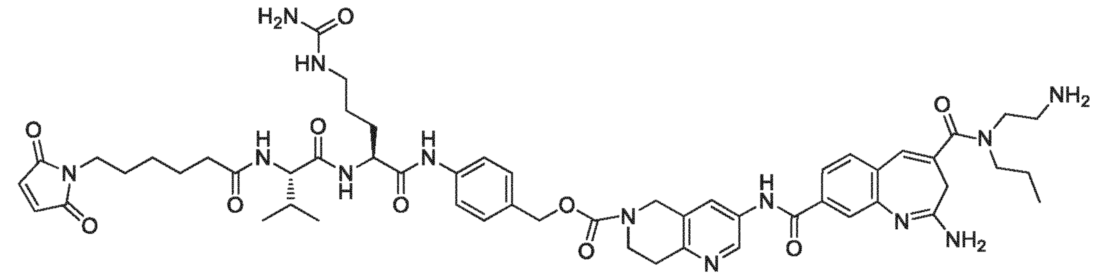

,

or

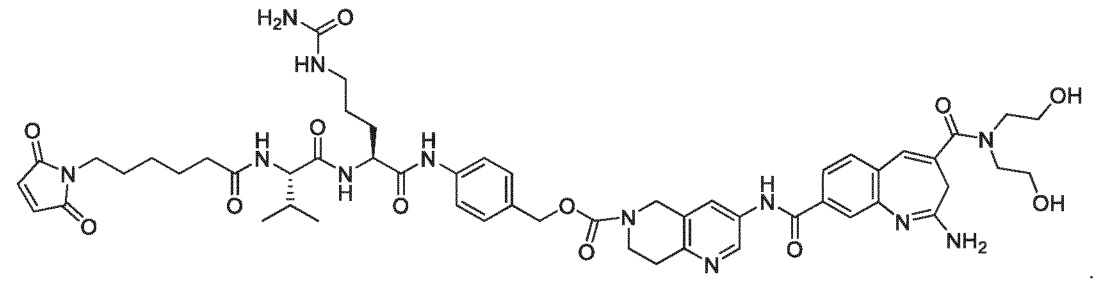

.

The present disclosure also provides an immune-stimulating antibody conjugate of formula III or a pharmaceutically acceptable salt thereof,

$$Ab\text{-}(L\text{—}D)_t$$

(III)

wherein Ab is an antibody;
L is a linker that attaches Ab to D;
D is a group formed by losing one hydrogen atom in the compound of formula I;

t is any value from 1 to 8.

In some embodiments, in the immune-stimulating antibody conjugate of formula III or the pharmaceutically acceptable salt thereof, some of the groups are defined as follows, and the rest of the groups are defined as described in any other embodiment (hereinafter referred to as "in some embodiments"): the antibody can contain one or more than one antigen-binding domain that can bind to an antigen.

In some embodiments, the antibody can contain one or two antigen-binding domains that can bind to the antigen.

In some embodiments, the antibody can contain one antigen-binding domain that can bind to the antigen.

In some embodiments, the antibody can contain only one antigen-binding domain that can bind to the antigen.

In some embodiments, the antibody can contain one Fc region.

In some embodiments, the antibody can contain only one Fc region.

In some embodiments, the antibody can contain only one antigen-binding domain that can bind to the antigen and one Fc region.

In some embodiments, the antibody can be a monoclonal antibody.

In some embodiments, the antibody can be an anti-HER2 antibody.

In some embodiments, the antibody can be Trastuzumab.

In some embodiments, the D can be a group formed by losing one hydrogen atom from $R_7$ of the compound of formula I.

In some embodiments, the D can be a group formed by losing one hydrogen atom from the secondary or primary amine of $R_7$ of the compound of formula I, i.e., attached to the linker via an N atom.

In some embodiments, the D can be attached to the linker via the a-end of

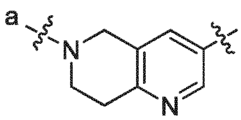 or 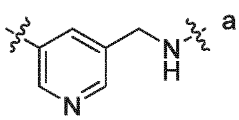 .

In the immune-stimulating antibody conjugate of formula III or the pharmaceutically acceptable salt thereof, the linker is a bivalent group attached to the D via one site and attached to the Ab via another site.

In the immune-stimulating antibody conjugate of formula III or the pharmaceutically acceptable salt thereof, the linker is a conventional linker in the field of ISAC.

In some embodiments, the L can be a degradable linker or a non-degradable linker.

In some embodiments, the L can be a linker that can be degraded by lysosomal enzymes.

In some embodiments, the L can be

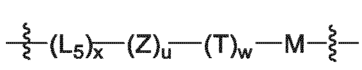 ;

x is 1;

u is 1, 2, 3, 4, 5, or 6;

w is 1;

$L_5$ is

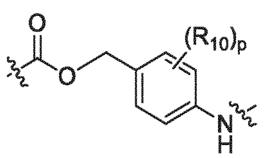 ;

p is 1; $R_{10}$ is hydrogen; the carbonyl end of the $L_5$ is attached to the D;

each Z is independently

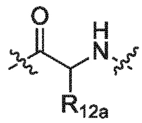;

each $R_{12a}$ is independently hydrogen, methyl, ethyl, n-propyl, isopropyl,

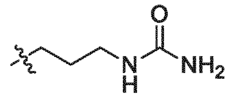, or 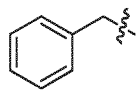;

the carbonyl end of the

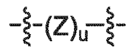

is attached to the amino end of the $L_5$;

T is

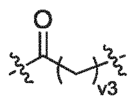;

v3 is 1, 2, 3, 4, 5, or 6; the carbonyl end of the T is attached to the amino end of

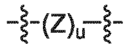;

M is

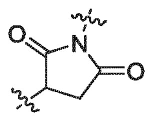,

the amino end of the M is attached to the non-carbonyl end of the T.

In some embodiments, Ab is an antibody;

L is a linker that attaches Ab to D;
D is a group formed by losing one hydrogen atom in the compound of formula I;
t is any value from 1 to 8.
In some embodiments,
Ab is an anti-HER2 monoclonal antibody;
L is

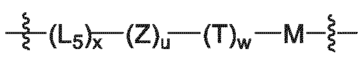,

x is 1;
u is 1, 2, 3, 4, 5, or 6;

w is 1;
$L_5$ is

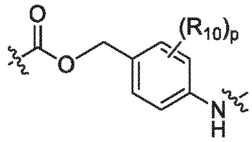
;

p is 1; $R_{10}$ is hydrogen; the carbonyl end of the $L_5$ is attached to the D;
each Z is independently

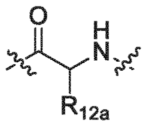
;

each $R_{12a}$ is independently hydrogen, methyl, ethyl, n-propyl, isopropyl,

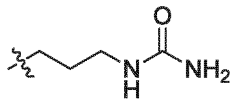
, or
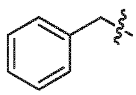
;

the carbonyl end of the

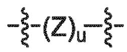

is attached to the amino end of the Ls;
T is

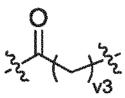
;

v3 is 1, 2, 3, 4, 5, or 6; the carbonyl end of the T is attached to the amino end of

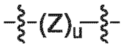
;

M is

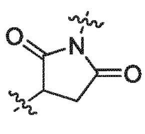
,

the amino end of the M is attached to the non-carbonyl end of the T;
the D is a group formed by losing one hydrogen atom from $R_7$ of the compound of formula I;
t is any value from 1 to 8.

In some embodiments, the $L_5$ can be

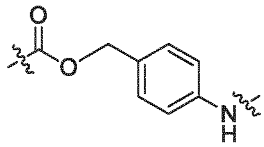.

In some embodiments, the carbonyl end of $L_5$ can be attached to the D.

In some embodiments, the Z can be an amino acid.

In some embodiments, the

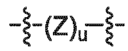

can be a peptide chain.

In some embodiments, the

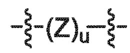

can be a dipeptide, tripeptide, or tetrapeptide linker.

In some embodiments, the carbonyl end of

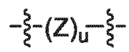

can be attached to the amino end of the $L_5$.

In some embodiments, the

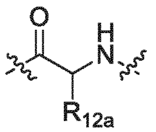

can be

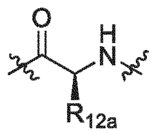 or 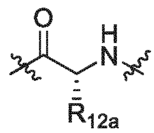,

and also can be

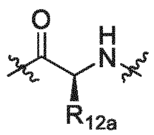.

In some embodiments, the carbonyl end of the T can be attached to the amino end of

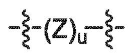.

In some embodiments, the amino end of the M can be attached to the non-carbonyl end of the T.

In the immune-stimulating antibody conjugate of formula III or the pharmaceutically acceptable salt thereof, the t is an integer or a non-integer. When it is a non-integer, it means that the immune-stimulating antibody conjugate of

formula III is a mixture of immune-stimulating antibody conjugates with different drug antibody ratios; when it is an integer, it can mean that the immune-stimulating antibody conjugate of formula III is a single immune-stimulating antibody conjugate with a fixed drug antibody ratio, or it can mean that the immune-stimulating antibody conjugate of formula III is a mixture of immune-stimulating antibody conjugates with different drug antibody ratios.

In some embodiments, the t can be any value from 2 to 5.

In some embodiments, the t can be any value from 3 to 5.

In some embodiments,

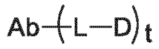

(III)

wherein Ab is an antibody;
L is a linker that attaches Ab to D;
t is any value from 1 to 8;
D is the compound of formula I;

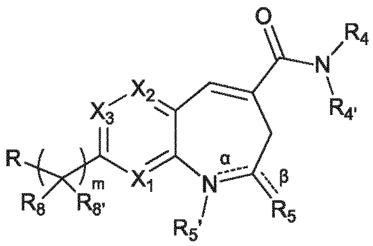

(I) ;

$\alpha$ and $\beta$ are independently a single bond or a double bond; at least one selected from the group of $\alpha$ and $\beta$ is a single bond;
m is 0 or 1;
$X_1$ is N or $CR_2$, $X_2$ is N or $CR_3$, and $X_3$ is N or $CR_1$; when $X_1$ is $CR_2$, $X_2$ is $CR_3$, and $X_3$ is $CR_1$, m is 1;
R is $-C(O)-L_1-R_7$, $-C(O)-NR_9-L_1-R_7$, $-C(S)-NR_9-L_1-R_7$, $-NR_9-L_1-R_7$, $-NR_9-C(O)-L_1-R_7$, $-NR_9-C(O)-NR_9-L_1-R_7$, $-O-L_1-R_7$, $-S(O)_2-NR_9-L_1-R_7$, $-CH=CH-L_1-R_7$, or $-S(O)_2-L_1-R_7$;
$R_1$, $R_2$, and $R_3$ are each independently hydrogen, deuterium, halogen, hydroxyl, amino, cyano, alkyl, haloalkyl, $-L_2-OR_a$, or $-L_2-NR_aR_b$;
$R_4$ and $R_{4'}$ are each independently hydrogen, alkyl, alkenyl, alkynyl, cycloalkyl, heterocycloalkyl, aryl, heteroaryl, cycloalkylalkyl, heterocycloalkylalkyl, arylalkyl, or heteroarylalkyl; the $R_4$ or $R_{4'}$ is unsubstituted or optionally substituted at any position by one or more than one substituent selected from halogen, cyano, $-L_2-OR_a$, $-L_2-OC(O)R_a$, $-L_2-NR_aR_b$, $-L_2-NR_aC(O)OR_b$, $-L_2-NR_aC(O)NR_aR_b$, $-L_2-NR_bC(NR_b)NR_aR_b$, and $-L_2-C(O)OR_b$; or, $R_4$ and $R_{4'}$ together with the N atom to which they are attached form a 3- to 8-membered heterocycloalkyl group; the 3- to 8-membered heterocycloalkyl group is unsubstituted or further substituted at any position by 1 to 3 substituents selected from halogen, cyano, $-L_2-OR_a$, $-L_2-OC(O)R_a$, $-L_2-NR_aR_b$, $-L_2-NR_aC(O)OR_b$, $-L_2-NR_aC(O)NR_aR_b$, $-L_2-NR_bC(NR_b)NR_aR_b$, and $-L_2-C(O)OR_b$;
$R_5$ is =O, $=NR_a$, $-OR_a$, or $-NR_aR_b$;
$R_{5'}$ is absent, or $R_{5'}$ is $-R_c$, $-L_2-OR_a$, $-L_2-NR_aR_b$, $-L_2-NR_aC(O)OR_b$, $-L_2-NR_aC(O)NR_aR_b$, $-L_2-NR_bC(NR_b)NR_aR_b$, or $-L_2-C(O)OR_b$;
$R_7$ is phenyl, 5- to 6-membered heteroaryl, 3- to 8-membered heterocycloalkyl, or an 8- to 12-membered fused ring group; the $R_7$ is unsubstituted or optionally substituted at any position by one or more than one substituent selected from $-L_3-W$, $-R_c$, halogen, cyano, nitro, alkyl, alkenyl, alkynyl, alkoxy, haloalkyl, haloalkoxy, and alkylamino;
$R_8$ and $R_{8'}$ are each independently hydrogen, halogen, or alkyl, and the $R_8$ or $R_{8'}$ is unsubstituted or optionally substituted at any position by one or more than one substituent selected from $-L_3-W$, halogen, cyano, nitro, alkyl, alkenyl, alkynyl, alkoxy, haloalkyl, haloalkoxy, and alkylamino; $R_8$ and Rs> are each independent substituents, or, $R_8$ and $R_{8'}$ together with the carbon atom to which they are attached form an oxo, thio, $C_{1-6}$ alkylene, $C_{3-10}$ cycloalkyl, or 3- to 10-membered heterocycloalkyl group; the $C_{1-6}$ alkylene, $C_{3-10}$ cycloalkyl, or 3- to 10-membered heterocycloalkyl group is unsubstituted or optionally substituted at any position by one or more than one substituent selected from deuterium, halogen, hydroxyl, amino, cyano, oxo, alkyl, haloalkyl, $-L_2-OR_a$, and $-L_2-NR_aR_b$;

$R_9$ is hydrogen, alkyl, haloalkyl, $-L_2-OR_a$, or $-L_2-NR_aR_b$;

W is Cyl, $-SR_d$, $-OR_d$, $-OC(O)R_e$, $-OC(O)NR_eR_{e'}$, $-C(O)OR_e$, $-C(O)R_e$, $-C(O)NR_eR_{e'}$, $-C(O)NR_eS(O)_2R_e$, $-NR_dR_e$, $-NR_dC(O)R_e$, $-N(R_d)C(O)OR_e$, $-N(R_d)C(O)NR_eR_{e'}$, $-NR_dS(O)_2R_e$, $-NR_dS(O)_2NR_eR_{e'}$, $-S(O)_{1-2}R_e$, $-S(O)_2NR_eR_{e'}$, $-S(O)(=NR_d)R_e$, $-S(O)_2N(R_e)C(O)R_{e'}$, $-P(O)(OR_e)_2$, $-P(O)(OR_e)R_{e'}$, $-OP(O)(OR_e)_2$, or $-B(OR_e)_2$;

$Cy^1$ is cycloalkyl, heterocycloalkyl, aryl, or heteroaryl; the $Cy^1$ is unsubstituted or optionally substituted at any position by one or more than one substituent selected from halogen, alkyl, haloalkyl, haloalkoxy, alkenyl, alkynyl, cyano, $-R_c$, $-L_4-SR_d$, $-L_4-OC(O)R_e$, $-L_4-C(O)OR_e$, $-L_4-C(O)R_e$, $-L_4-C(O)NR_eR_{e'}$, $-L_4-NR_dC(O)R_e$, $-L_4-NR_dS(O)_2R_e$, $-L_4-S(O)_{1-2}R_e$, $-L_4-S(O)_2NR_eR_{e'}$, $-L_4-OR_d$, and $-L_4-NR_eR_{e'}$;

$L_1$, $L_2$, $L_3$, and $L_4$ are each independently a linkage bond, $C_{1-6}$ alkylene, $C_{2-6}$ alkenylene, or $C_{2-6}$ alkynylene; the $L_1$, $L_2$, $L_3$, or $L_4$ is unsubstituted or optionally substituted at any position by one or more than one substituent selected from oxo, hydroxyl, amino, halogen, cyano, alkyl, haloalkyl, alkoxy, and haloalkoxy;

$R_a$, $R_b$, $R_d$, $R_e$, and $R_{e'}$ are each independently $-R_c$, amino, $C_{1-6}$ alkyl, $C_{2-6}$ alkenyl, $C_{2-6}$ alkynyl, $C_{3-10}$ cycloalkyl, 3- to 10-membered heterocycloalkyl, $C_{6-10}$ aryl, 5- to 10-membered heteroaryl, $C_{3-10}$ cycloalkyl-$C_{1-6}$ alkyl, 3- to 10-membered heterocycloalkyl-$C_{1-6}$ alkyl, phenyl-$C_{1-6}$ alkyl, or 5- to-10 membered heteroaryl-$C_{1-6}$ alkyl; the $R_a$, $R_b$, $R_d$, $R_e$, or $R_{e'}$ is unsubstituted or optionally substituted at any position by 1 to 3 substituents selected from $-OR_f$, $-OC(O)-L_4-R_f$, $-NR_fR_{f'}$, halogen, cyano, nitro, $C_{1-6}$ alkyl, halo-$C_{1-6}$ alkyl, and halo-$C_{1-6}$ alkoxy;

$R_f$ and $R_{f'}$ are each independently $-R_c$, $-NHR_c$, or $C_{1-6}$ alkyl;

each $R_c$ is independently hydrogen or a linkage bond attached to L; at least one $R_c$ in D is a linkage bond attached to L.

In some embodiments,

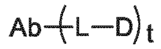

(III)

wherein Ab is an antibody;

L is a linker that attaches Ab to D;

t is any value from 1 to 8;

D is the compound of formula I, the stereoisomer, or the pharmaceutically acceptable salt thereof:

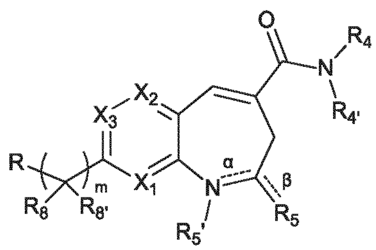

(I) ;

$\alpha$ and $\beta$ are independently a single bond or a double bond; at least one selected from the group of $\alpha$ and $\beta$ is a single bond;

m is 0 or 1;

$X_1$ is N or $CR_2$, $X_2$ is N or $CR_3$, and $X_3$ is N or $CR_1$; when $X_1$ is $CR_2$, $X_2$ is $CR_3$, and $X_3$ is $CR_1$, m is 1;

R is $-C(O)-L_1-R_7$, $-C(O)-NR_9-L_1-R_7$, $-C(S)-NR_9-L_1-R_7$, $-NR_9-L_1-R_7$, $-NR_9-C(O)-L_1-R_7$, $-NR_9-C(O)-NR_9-L_1-R_7$, $-O-L_1-R_7$, $-S(O)_2-NR_9-L_1-R_7$, $-CH=CH-L_1-R_7$, or $-S(O)_2-L_1-R_7$;

$R_1$, $R_2$, and $R_3$ are each independently hydrogen, deuterium, halogen, hydroxyl, amino, cyano, alkyl, haloalkyl, $-L_2-OR_a$, or $-L_2-NR_aR_b$;

$R_4$ and $R_{4'}$ are each independently hydrogen, alkyl, alkenyl, alkynyl, cycloalkyl, heterocycloalkyl, aryl, heteroaryl, cycloalkylalkyl, heterocycloalkylalkyl, arylalkyl, or heteroarylalkyl; the $R_4$ or $R_{4'}$ is unsubstituted or optionally substituted at any position by one or more than one substituent selected from halogen, cyano, $-L_2-OR_a$, $-L_2-NR_aR_b$, $-L_2-NR_aC(O)OR_b$, $-L_2-NR_aC(O)NR_aR_b$, $-L_2-NR_bC(NR_b)NR_aR_b$, and $-L_2-C(O)OR_b$; or, $R_4$ and $R_{4'}$ together with the N atom to which they are attached form a 3- to 8-membered heterocycloalkyl group; the 3- to 8-membered heterocycloalkyl group is unsubstituted or further substituted at any position by 1 to 3 substituents selected from halogen, cyano, $-L_2-OR_a$, $-L_2-NR_aR_b$, $-L_2-NR_aC(O)OR_b$, $-L_2-NR_aC(O)NR_aR_b$, $-L_2-NR_bC(NR_b)NR_aR_b$, and $-L_2-C(O)OR_b$;

$R_5$ is =O, $=NR_a$, $-OR_a$, or $-NR_aR_b$;

$R_{5'}$ is absent, or $R_{5'}$ is $-R_c$, $-L_2-OR_a$, $-L_2-NR_aR_b$, $-L_2-NR_aC(O)OR_b$, $-L_2-NR_aC(O)NR_aR_b$, $-L_2-NR_bC(NR_b)NR_aR_b$, or $-L_2-C(O)OR_b$;

$R_7$ is phenyl, 5- to 6-membered heteroaryl, 3- to 8-membered heterocycloalkyl, or an 8- to 12-membered fused ring group; the $R_7$ is unsubstituted or optionally substituted at any position by one or more than one substituent selected from $-L_3-W$, $-R_c$, halogen, cyano, nitro, alkyl, alkenyl, alkynyl, alkoxy, haloalkyl, haloalkoxy, and alkylamino;

$R_8$ and $R_{8'}$ are each independently hydrogen, halogen, or alkyl, and the $R_8$ or $R_{8'}$ is unsubstituted or optionally substituted at any position by one or more than one substituent selected from $-L_3-W$, halogen, cyano, nitro, alkyl, alkenyl, alkynyl, alkoxy, haloalkyl, haloalkoxy, and alkylamino; $R_8$ and $R_{8'}$ are each independent substituents, or, $R_8$ and $R_{8'}$ together with the carbon atom to which they are attached form an oxo, thio, $C_{1-6}$ alkylene, $C_{3-10}$ cycloalkyl, or 3- to 10-membered heterocycloalkyl group; the $C_{1-6}$ alkylene, $C_{3-10}$ cycloalkyl, or 3- to 10-membered heterocycloalkyl group is unsubstituted or optionally substituted at any position by one or more than one substituent selected from deuterium, halogen, hydroxyl, amino, cyano, oxo, alkyl, haloalkyl, $-L_2-OR_a$, and $-L_2-NR_aR_b$;

$R_9$ is hydrogen, alkyl, haloalkyl, $-L_2-OR_a$, or $-L_2-NR_aR_b$;

W is Cyl, $-SR_d$, $-OR_d$, $-OC(O)R_e$, $-OC(O)NR_eR_{e'}$, $-C(O)OR_e$, $-C(O)R_e$, $-C(O)NR_eR_{e'}$, $-C(O)NR_eS(O)_2R_e$, $-NR_dR_e$, $-NR_dC(O)R_e$, $-N(R_d)C(O)OR_e$, $-N(R_d)C(O)NR_eR_{e'}$, $-NR_aS(O)_2R_e$, $-NR_dS(O)_2NR_eR_{e'}$, $-S(O)_{1-2}R_e$, $-S(O)_2NR_eR_{e'}$, $-S(O)(=NR_d)R_e$, $-S(O)_2N(R_e)C(O)R_{e'}$, $-P(O)(OR_e)_2$, $-P(O)(OR_e)R_{e'}$, $-OP(O)(OR_e)_2$, or $-B(OR_e)_2$;

$Cy^1$ is cycloalkyl, heterocycloalkyl, aryl, or heteroaryl; the $Cy^1$ is unsubstituted or optionally substituted at any position by one or more than one substituent selected from halogen, alkyl, haloalkyl, haloalkoxy, alkenyl, alkynyl, cyano, $-R_c$, $-L_4-SR_d$, $-L_4-OC(O)R_e$, $-L_4-C(O)OR_e$, $-L_4-C(O)R_e$, $-L_4-C(O)NR_eR_{e'}$, $-L_4-NR_dC(O)R_e$, $-L_4-NR_dS(O)_2R_e$, $-L_4-S(O)_{1-2}R_e$, $-L_4-S(O)_2NR_eR_{e'}$, $-L_4-OR_d$, and $-L_4-NR_eR_{e'}$;

$L_1$, $L_2$, $L_3$, and $L_4$ are each independently a linkage bond, $C_{1-6}$ alkylene, $C_{2-6}$ alkenylene, or $C_{2-6}$ alkynylene; the $L_1$, $L_2$, $L_3$, or $L_4$ is unsubstituted or optionally substituted at any position by one or more than one substituent selected from oxo, hydroxyl, amino, halogen, cyano, alkyl, haloalkyl, alkoxy, and haloalkoxy;

$R_a$, $R_b$, $R_d$, $R_e$, and $R_{e'}$ are each independently $-R_c$, amino, $C_{1-6}$ alkyl, $C_{2-6}$ alkenyl, $C_{2-6}$ alkynyl, $C_{3-10}$ cycloalkyl, 3- to 10-membered heterocycloalkyl, $C_{6-10}$ aryl, 5- to 10-membered heteroaryl, $C_{3-10}$ cycloalkyl-$C_{1-6}$ alkyl, 3- to 10-membered heterocycloalkyl-$C_{1-6}$ alkyl, phenyl-$C_{1-6}$ alkyl, or 5- to-10 membered heteroaryl-$C_{1-6}$ alkyl; the $R_a$, $R_b$, $R_d$, $R_e$, or $R_{e'}$ is unsubstituted or optionally substituted at any position by 1 to 3 substituents selected from $-OR_f$, $-NR_fR_{f'}$, halogen, cyano, nitro, $C_{1-6}$ alkyl, halo-$C_{1-6}$ alkyl, and halo-$C_{1-6}$ alkoxy;

$R_f$ and $R_{f'}$ are each independently $-R_c$, $-NHR_c$, or $C_{1-6}$ alkyl;

each $R_c$ is independently hydrogen or a linkage bond attached to L; at least one $R_c$ in D is a linkage bond attached to L.

All embodiments of formula III described below and combinations of variables are included in the scope of the structural formula of formula III of the present disclosure.

In some embodiments, the linker is a degradable linker.

In some embodiments, the linker is a non-degradable linker.

In some embodiments, the linker can be degraded by lysosomal enzymes.

In some embodiments, the linker is $-[(L_5)_x-(Z)_u-(T)_w]_y-M-$; x is 0, 1, 2, or 3; u is 0, 1, 2, 3, 4, 5, or 6; w is 0, 1, 2, 3, 4, 5, or 6; y is 1, 2, or 3;

each $L_5$ is independently

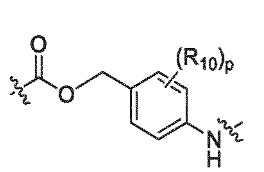,
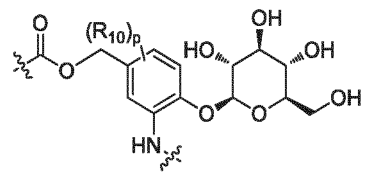,

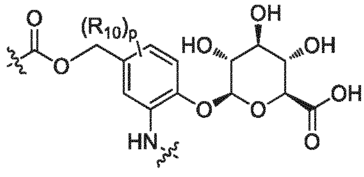,
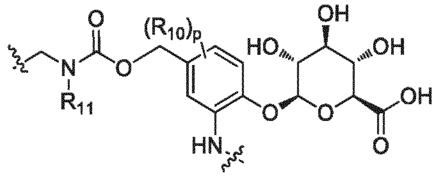, or
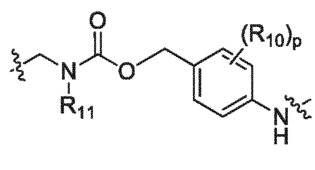;

p is 1, 2, or 3; each $R_{10}$ is independently hydrogen, halogen, $C_{1-6}$ alkyl, halo-$C_{1-6}$ alkyl, nitro, cyano, $C_{1-6}$ alkoxy, $C_{1-6}$ alkylamino, $-OC(O)R_{11}$, or $-C(O)N(R_{11})_2$; $R_{11}$ is hydrogen, $C_{1-6}$ alkyl, or $C_{1-6}$ alkylamino-$C_{1-6}$ alkyl;

each Z is independently

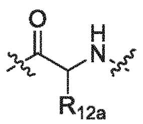, 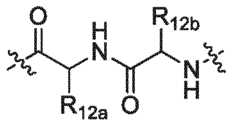, 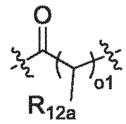,

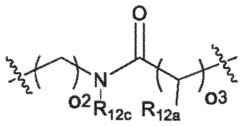, 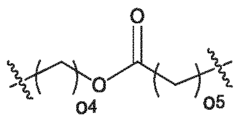, 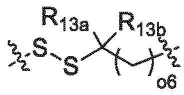,

and $-(CH_2CH_2O)_{o7}-$; o1, o2, o3, o4, o5, o6, o7 are each independently 0, 1, 2, 3, 4, 5, 6, 7, or 8; $R_{12a}$, $R_{12b}$, and $R_{12c}$ are each independently hydrogen, methyl, ethyl, n-propyl, isopropyl, $-SO_3H$,

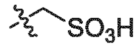, 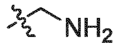,

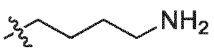, 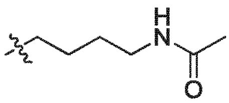, 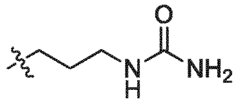, 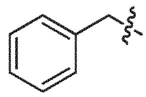, 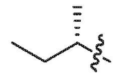,

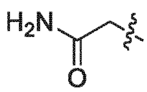, 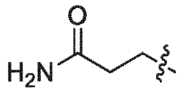, or 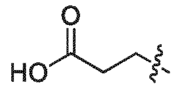;

$R_{13a}$ and $R_{13b}$ are each independently hydrogen or methyl;

each T is independently $-(CH_2)_{v1}-$, $-(CH_2CH_2O)_{v2}-$,

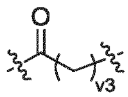, 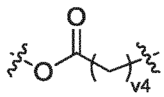,

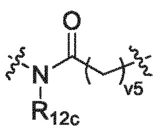, 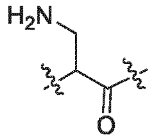, 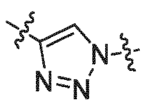, or 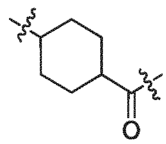;

v1, v2, v3, v4, and v5 are each independently 1, 2, 3, 4, 5, or 6;

M is a linkage bond or a linker head (such as

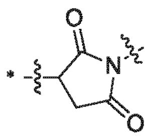, 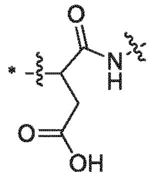, 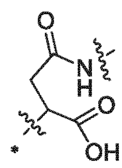,

or

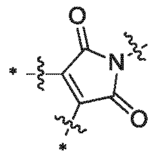,

the marked site * is the site attached to Ab).

In some embodiments, the linker is $-(L_5)_x-(Z)_u-(T)_w-M-$; x is 0, 1, or 2; u is 0, 1, 2, 3, 4, or 5; and w is 1, 2, or 3.

In some embodiments, $-L_5-$ is

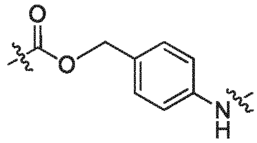;

$-(Z)_u-$ is

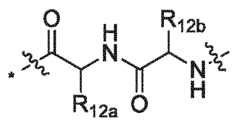, 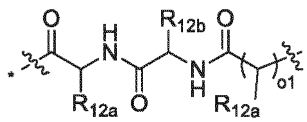,

or

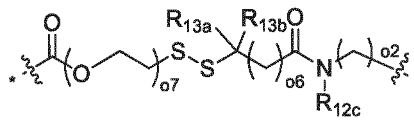;

o1, o2, o7, $R_{12a}$, $R_{12b}$, $R_{12c}$, $R_{13a}$, and $R_{13b}$ are defined as above; the marked site * is the site attached to Ls;

$-(T)_w-$ is $-(CH_2CH_2O)_{v2}-$*,

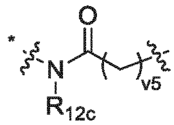, 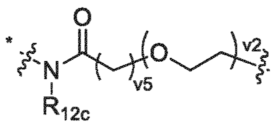, or 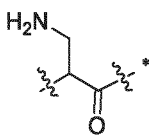;

v1, v2, v5, and $R_{12c}$ are defined as above; the marked site * is the site attached to Z.

In some embodiments, $-L_5-$ is

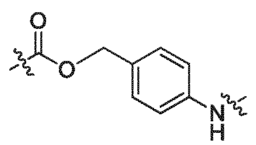;

$-(Z)_u-$ is

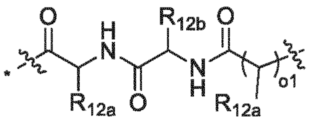
;

o1, $R_{12a}$, and $R_{12b}$ are defined as above; the marked site * is the site attached to $L_5$;

-$(T)_w$- is a linkage bond, -$(CH_2CH_2O)_{v2}$-*,

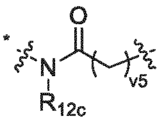
, or
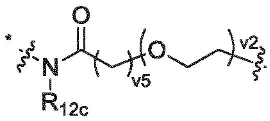
;

v1, v2, v5, and $R_{12c}$ are defined as above; the marked site * is the site attached to Z.

In some embodiments, -$(Z)_u$- is

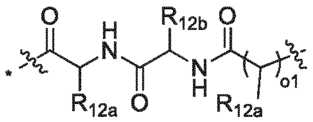
;

o1, $R_{12a}$, and $R_{12b}$ are defined as above; the marked site * is the site attached to $L_5$.

In some embodiments, -$(Z)_u$- is

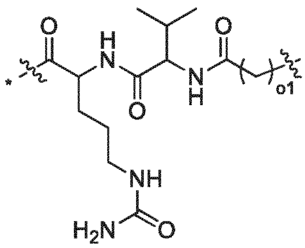
,
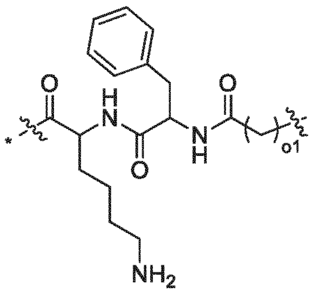
,

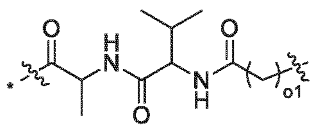
;

o1 is defined as above; the marked site * is the site attached to $L_5$.

In some embodiments, -$(Z)_u$- is

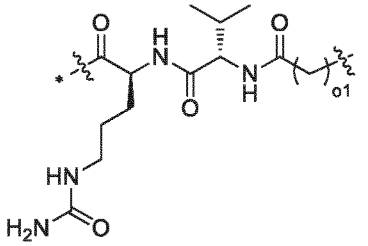

or

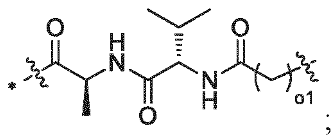

o1 is defined as above; the marked site * is the site attached to $L_5$.

In some embodiments, the linker has any one of the following structures:

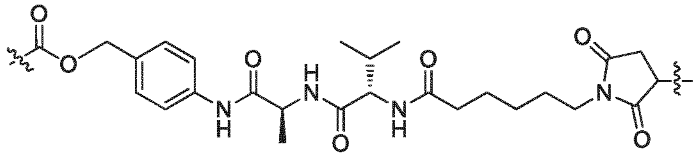

,

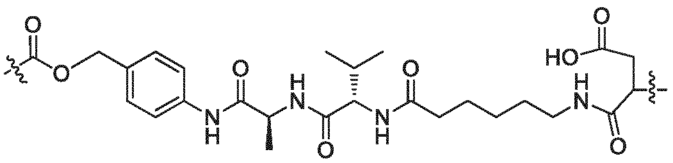

,

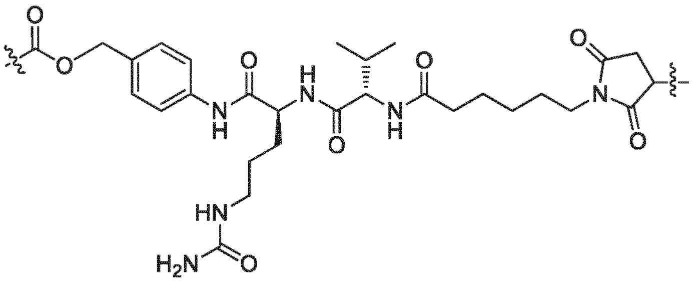

,

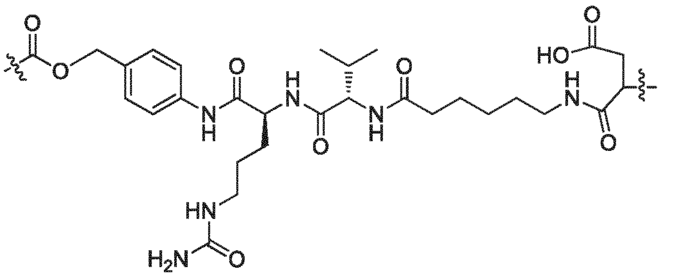

,

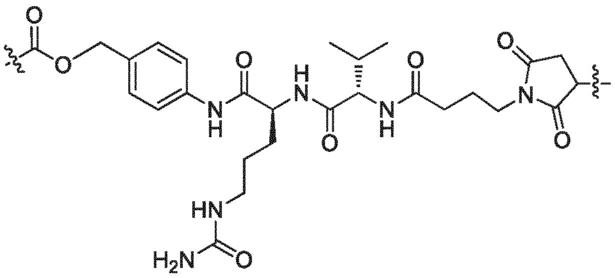

,

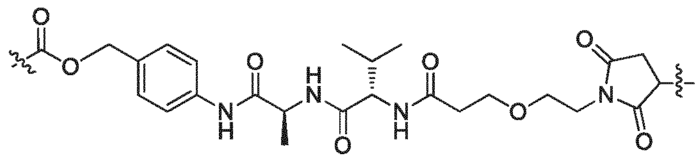

,

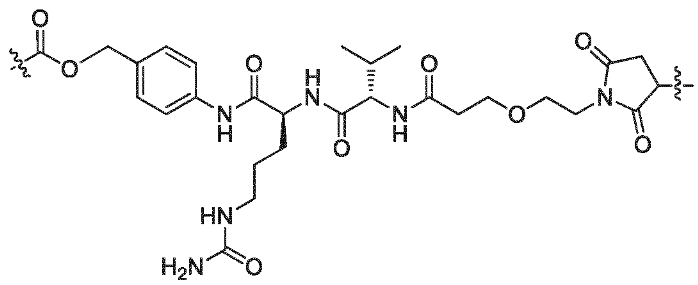

,

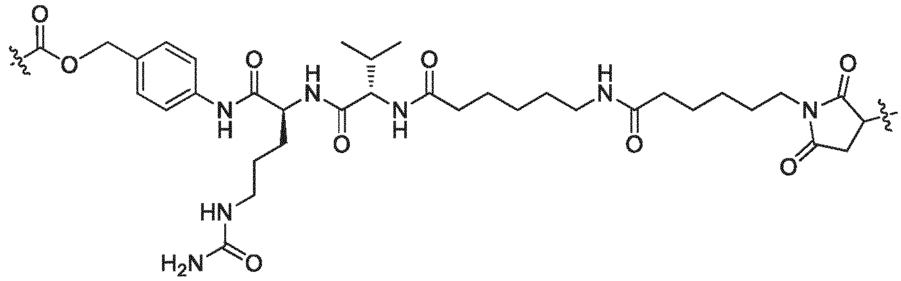

,

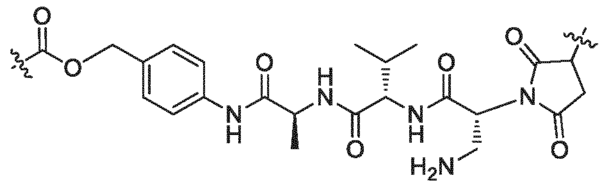

,

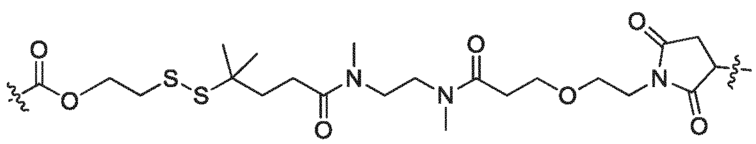

,

or

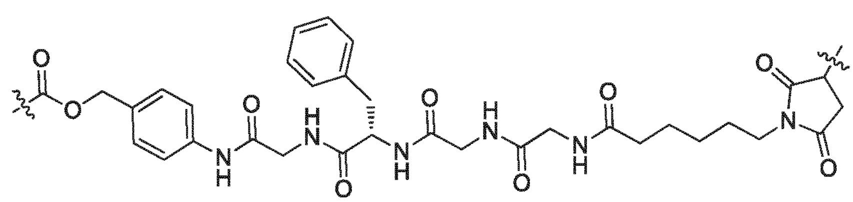

.

In some embodiments, the linker has any one of the following structures:

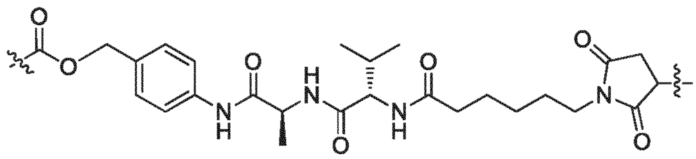

,

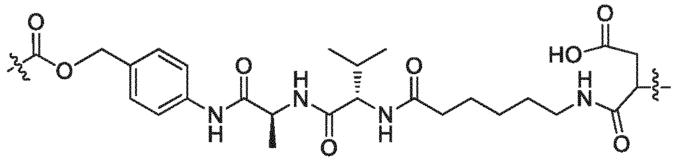

,

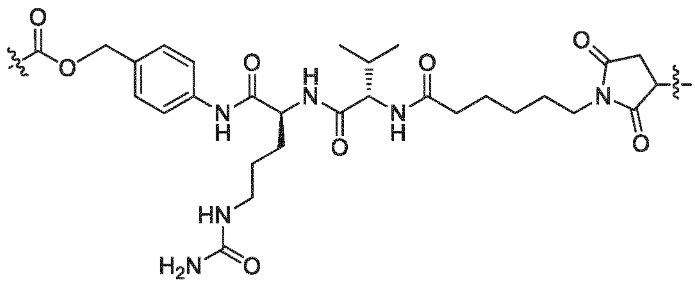

,

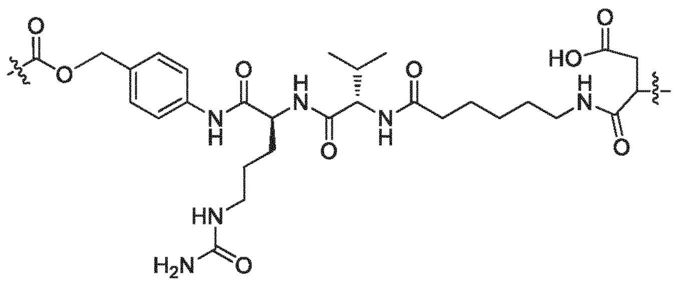

,

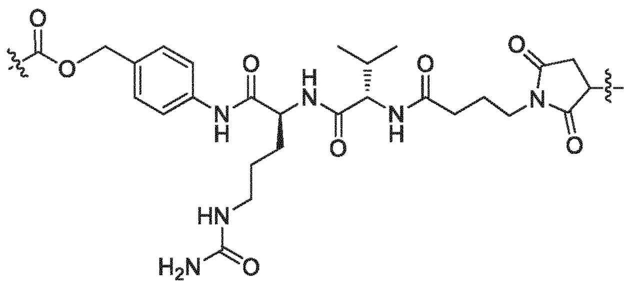

,

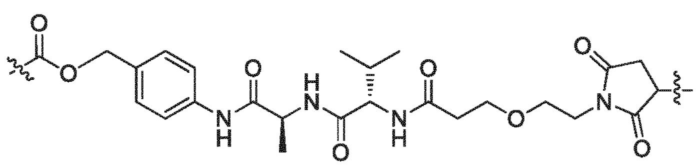

,

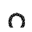

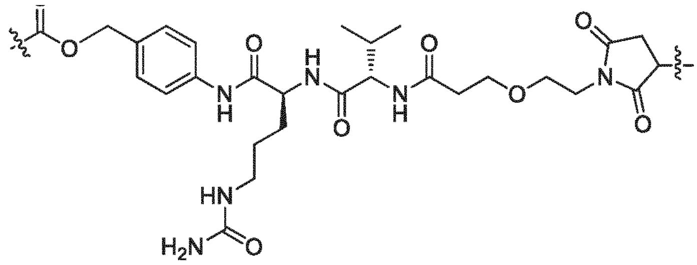

,

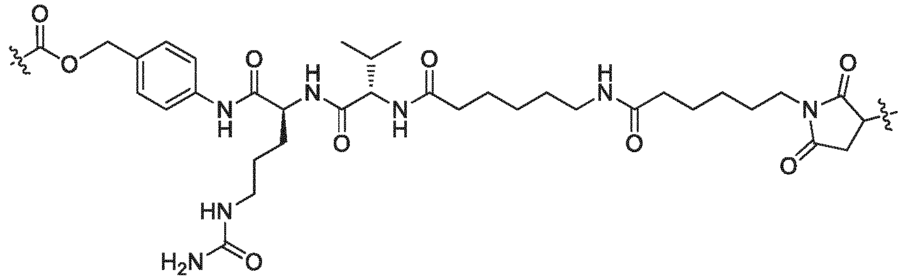

,

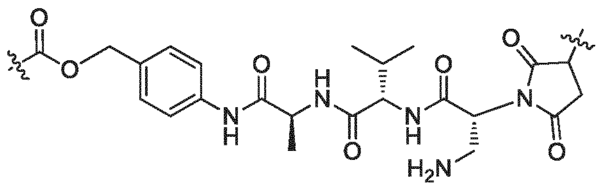

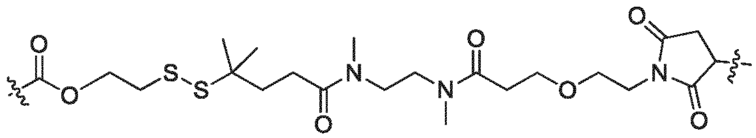

,

or

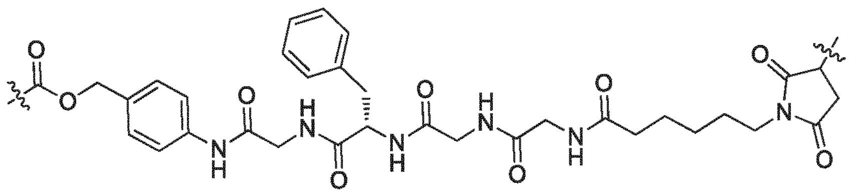

;

its carbonyl end is attached to the D.

In some embodiments, the Ab is an antibody, and the antibody contains one antigen-binding domain that can bind to the antigen.

In some embodiments, the Ab is an antibody, and the antibody contains one antigen-binding domain that can bind to the antigen and one Fc region.

In some embodiments, the Ab is an antibody, and the antibody contains one antigen-binding domain that can bind to HER2.

In some embodiments, the Ab is an anti-HER2 monoclonal antibody or an antibody fragment thereof.

In some embodiments, the anti-HER2 monoclonal antibody includes, but is not limited to: Trastuzumab, Trastuzumab biosimilar, Pertuzumab, Pertuzumab biosimilar, Margetuximab, HT-19, etc.

In some embodiments, the antibody is Trastuzumab, Pertuzumab, Margetuximab, or HT-19.

In some embodiments, t is any value from 1 to 6 or 1 to 5.

In some embodiments, t is any value from 2 to 8, 2 to 6, or 2 to 5.

In some embodiments, t is any value from 3 to 6 or 3 to 5.

In some embodiments, in D, $X_3$ is $CR_1$; $X_1$ is N; $X_2$ is $CR_3$.

In some embodiments, in D, $X_3$ is $CR_1$; $X_1$ is $CR_2$; $X_2$ is $CR_3$.

In some embodiments, in D, $R_a$, $R_b$, $R_d$, $R_e$, and $R_{e'}$ are each independently $-R_c$, amino, $C_{1-6}$ alkyl, $C_{2-6}$ alkenyl, $C_{2-6}$ alkynyl, $C_{3-10}$ cycloalkyl, 3- to 10-membered heterocycloalkyl, $C_{6-10}$ aryl, 5- to 10-membered heteroaryl, $C_{3-10}$ cycloalkyl-$C_{1-6}$ alkyl, 3- to 10-membered heterocycloalkyl-$C_{1-6}$ alkyl, phenyl-$C_{1-6}$ alkyl, or 5- to-10 membered heteroaryl-$C_{1-6}$ alkyl; the $R_a$, $R_b$, $R_d$, $R_e$, or $R_{e'}$ is unsubstituted or optionally substituted at any position by 1 to 3 substituents selected from $-OR_f$, $-OC(O)-(CH_2)_{1-5}-R_f$, $-NR_fR_{f'}$, halogen, cyano, $C_{1-6}$ alkyl, halo-$C_{1-6}$ alkyl, and halo-$C_{1-6}$ alkoxy.

In some embodiments, in D, R is $-C(O)-NR_9-L_1-R_7$.

In some embodiments, in D, $R_1$, $R_2$, and $R_3$ are each independently hydrogen, deuterium, halogen, hydroxyl, amino, cyano, $C_{1-6}$ alkyl, halo-$C_{1-6}$ alkyl, $-L_2-OR_a$, or $-L_2-NR_aR_b$.

In some embodiments, in D, $R_1$, $R_2$, and $R_3$ are each independently preferably H, F, Cl, Br, $-CH_3$, $-OCH_3$, $-CF_3$, $-CH_2F$, $-CHF_2$, $-OCF_3$, -CN, or $-(CH_2)_{0-5}-NH_2$.

In some embodiments, in D, $R_1$ is H; $R_2$ is H.

In some embodiments, in D, $R_4$ and $R_{4'}$ are each independently hydrogen, $C_{1-6}$ alkyl, $C_{2-6}$ alkenyl, $C_{2-6}$ alkynyl, $C_{3-8}$ cycloalkyl, 3- to 8-membered heterocycloalkyl, $C_{6-10}$ aryl, 5- to 10-membered heteroaryl, cycloalkyl-$C_{1-6}$ alkyl, 3- to 8-membered heterocycloalkyl-$C_{1-6}$ alkyl, $C_{6-10}$ aryl-$C_{1-6}$ alkyl, or 5- to 10-membered heteroaryl-$C_{1-6}$ alkyl; the $R_4$ or $R_{4'}$ is unsubstituted or optionally substituted at any position by 1 to 3 substituents selected from halogen, cyano, - $L_2-OR_a$, $-L_2-OC(O)R_a$, $-L_2-NR_aR_b$, $-L_2-NR_aC(O)OR_b$, $-L_2-NR_aC(O)NR_aR_b$, $-L_2-NR_bC(NR_b)NR_aR_b$, and $-L_2-C(O)OR_b$; or, $R_4$ and $R_{4'}$ together with the N atom to which they are attached form a 3- to 8-membered heterocycloalkyl group; the 3- to 8-membered heterocycloalkyl group is unsubstituted or further substituted at any position by 1 to 3 substituents selected from halogen, cyano, $-L_2-OR_a$, $-L_2-NR_aR_b$, $-L_2-NR_aC(O)OR_b$, $-L_2-NR_aC(O)NR_aRb$, $-L_2-NR_bC(NR_b)NR_aR_b$, and $-L_2-C(O)OR_b$.

In some embodiments, in D, $R_4$ and $R_{4'}$ are each independently $C_{1-6}$ alkyl; the $R_4$ or $R_{4'}$ is unsubstituted or optionally substituted at any position by 1 to 3 substituents selected from $-OR_a$, $-OC(O)R_a$, $-NR_aR_b$, $-NR_aC(O)OR_b$, $-NR_aC(O)NR_aR_b$, $-NR_bC(NR_b)NR_aR_b$, and - $C(O)OR_b$.

In some embodiments, in D, $R_4$ is $C_{1-6}$ alkyl; the $R_4$ is unsubstituted or optionally substituted at any position by one $-OR_a$, $-OC(O)R_a$, $-NR_aR_b$, $-NR_aC(O)OR_b$, $-NR_aC(O)NR_aR_b$, $-NR_bC(NR_b)NR_aR_b$, or $-C(O)OR_b$; $R_{4'}$ is $C_{1-6}$ alkyl; the $R_{4'}$ is unsubstituted or optionally substituted at any position by 1 to 3 substituents selected from halogen, $-OR_a$, and $-NR_aR_b$.

In some embodiments, in D, $R_4$ and $R_{4'}$ are each independently $C_{1-6}$ alkyl; the $R_4$ or $R_{4'}$ is unsubstituted or optionally substituted at any position by one substituent selected from halogen, $-OR_a$, and $-NR_aR_b$.

In some embodiments, in D, $R_4$ is $-CH_2CH_2OH$ or $-CH_2CH_2CH_3$; $R_{4'}$ is $-CH_2CH_2OH$, $-CH_2CH_2CH_3$, $-CH_2CH_2CF_3$, or $-CH_2CH_2CHF_2$.

In some embodiments, in D, $\alpha$ is a double bond, $\beta$ is a single bond, $R_{5'}$ is absent, and Rs is $-NR_aR_b$.

In some embodiments, in D, $\alpha$ is a double bond, $\beta$ is a single bond, $R_{5'}$ is absent, and $R_5$ is $-NH_2$.

In some embodiments, in D, $\alpha$ is a single bond, $\beta$ is a double bond, $R_{5'}$ is H, and $R_5$ is =O.

In some embodiments, in D, $R_8$ and $R_{8'}$ are each independently hydrogen, halogen, or $C_{1-6}$ alkyl; the $R_8$ or $R_{8'}$ is unsubstituted or optionally substituted at any position by 1 to 3 substituents selected from $-L_3-W$, halogen, cyano, nitro, $C_{1-6}$ alkyl, $C_{2-6}$ alkenyl, $C_{2-6}$ alkynyl, $C_{1-6}$ alkoxy, halo-$C_{1-6}$ alkyl, halo-$C_{1-6}$ alkoxy, and $C_{1-6}$ alkylamino.

In some embodiments, in D, $R_8$ and $R_{8'}$ are each independently $C_{1-6}$ alkyl, and the $C_{1-6}$ alkyl is preferably methyl.

In some embodiments, in D, $R_8$ and $R_{8'}$ together with the carbon atom to which they are attached form an oxo, thio, $C_{3-6}$ cycloalkyl, or 3- to 6-membered heterocycloalkyl group; the $C_{3-6}$ cycloalkyl or 3- to 6-membered heterocycloalkyl group is unsubstituted or optionally substituted at any position by 1 to 3 substituents selected from deuterium, halogen, hydroxyl, amino, cyano, oxo, $C_{1-6}$ alkyl, halo-$C_{1-6}$ alkyl, $-L_2-OR_a$, and $-L_2-NR_aR_b$.

In some embodiments, in D, $R_8$ and $R_{8'}$ together with the carbon atom to which they are attached form an oxo, thio, cyclopropyl, cyclobutyl, azetidinyl, or oxetanyl group; the cyclopropyl, cyclobutyl, azetidinyl, or oxetanyl group is unsubstituted or optionally substituted at any position by 1 to 3 substituents selected from deuterium, halogen, hydroxyl, amino, cyano, oxo, $C_{1-6}$ alkyl, halo-$C_{1-6}$ alkyl, $-L_2-OR_a$, and $-L_2-NR_aR_b$.

In some embodiments, in D, $R_8$ and $R_{8'}$ together with the carbon atom to which they are attached form an oxo, thio, cyclopropyl, or cyclobutyl group.

In some embodiments, in D, $R_9$ is hydrogen, $C_{1-6}$ alkyl, halo-$C_{1-6}$ alkyl, $-L_2-OR_a$, or - $L_2-NR_aR_b$.

In some embodiments, in D, $R_9$ is hydrogen.

In some embodiments, in D, $Cy^1$ is $C_{3-8}$ cycloalkyl, 3- to 8-membered heterocycloalkyl, $C_{6-10}$ aryl, or 5- to 10-membered heteroaryl; the $Cy^1$ is unsubstituted or optionally substituted at any position by 1 to 3 substituents selected from halogen, cyano, $C_{1-6}$ alkyl, halo-$C_{1-6}$ alkyl, halo-$C_{1-6}$ alkoxy, $C_{2-6}$ alkenyl, $C_{2-6}$ alkynyl, $-L_4-SR_d$, $-L_4-OC(O)R_e$, $-L_4-C(O)OR_e$, $-L_4-C(O)R_e$, $-L_4-C(O)NR_eR_{e'}$, $-L_4-NR_dC(O)R_e$, $-L_4-NR_dS(O)_2R_e$, $-L_4-S(O)_{1-2}R_e$, $-L_4-S(O)_2NR_eR_{e'}$, $-L_4-OR_d$, or $-L_4-NR_eR_{e'}$;

In some embodiments, in D, $L_1$ is a linkage bond or $C_{1-6}$ alkylene; the $L_1$ is unsubstituted or optionally substituted at any

position by 1 to 3 substituents selected from oxo, halogen, hydroxyl, amino, cyano, $C_{1-6}$ alkyl, halo-$C_{1-6}$ alkyl, $C_{1-6}$ alkoxy, or halo-$C_{1-6}$ alkoxy.

In some embodiments, in D, $L_1$ is a linkage bond or $-CH_2-$.

In some embodiments, in D, $L_2$ is a linkage bond or $C_{1-6}$ alkylene; the $L_2$ is unsubstituted or optionally substituted at any position by 1 to 3 substituents selected from oxo, halogen, hydroxyl, amino, cyano, $C_{1-6}$ alkyl, halo-$C_{1-6}$ alkyl, $C_{1-6}$ alkoxy, or halo-$C_{1-6}$ alkoxy.

In some embodiments, in D, $L_3$ is a linkage bond or $C_{1-6}$ alkylene; the $L_3$ is unsubstituted or optionally substituted at any position by 1 to 3 substituents selected from oxo, halogen, hydroxyl, amino, cyano, $C_{1-6}$ alkyl, halo-$C_{1-6}$ alkyl, $C_{1-6}$ alkoxy, or halo-$C_{1-6}$ alkoxy.

In some embodiments, in D, $L_3$ is a linkage bond, $-CH_2-$, $-CH_2CH_2-$, $-CH_2CH_2CH_2-$, $-CH_2CH_2C(CH_3)_2-$, $-CH_2CH_2CH_2CH_2-$, or $-CH_2CH(CH_3)CH_2-$.

In some embodiments, in D, $L_3$ is $-CH_2-$.

In some embodiments, in D, $L_4$ is a linkage bond or $C_{1-6}$ alkylene; the $L_4$ is unsubstituted or optionally substituted at any position by 1 to 3 substituents selected from oxo, halogen, hydroxyl, amino, cyano, $C_{1-6}$ alkyl, halo-$C_{1-6}$ alkyl, $C_{1-6}$ alkoxy, or halo-$C_{1-6}$ alkoxy.

In some embodiments, in D, $R_7$ is phenyl, 5- to 6-membered heteroaryl, 3- to 8-membered heterocycloalkyl, or an 8- to 12-membered fused ring group; the $R_7$ is unsubstituted or optionally substituted at any position by one or more than one substituent selected from $-L_3-W$, $-R_c$, halogen, cyano, nitro, $C_{1-6}$ alkyl, $C_{2-6}$ alkenyl, $C_{2-6}$ alkynyl, $C_{1-6}$ alkoxy, halo-$C_{1-6}$ alkyl, halo-$C_{1-6}$ alkoxy, and $C_{1-6}$ alkylamino.

In some embodiments, in D, $R_7$ is an 8- to 12-membered fused ring group; the $R_7$ is unsubstituted or optionally substituted at any position by one or more than one substituent selected from $-L_3-W$, $-R_c$, halogen, cyano, nitro, $C_{1-6}$ alkyl, $C_{2-6}$ alkenyl, $C_{2-6}$ alkynyl, $C_{1-6}$ alkoxy, halo-$C_{1-6}$ alkyl, halo-$C_{1-6}$ alkoxy, and $C_{1-6}$ alkylamino.

In some embodiments, in D, $R_7$ is

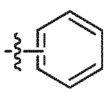, 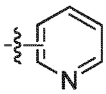, 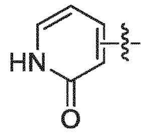, 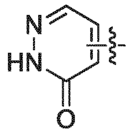,

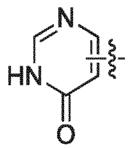, 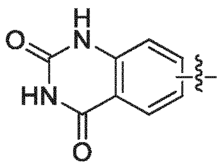, 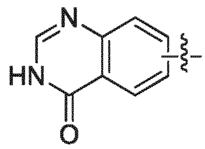, 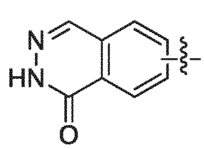, 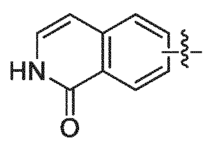,

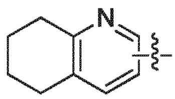, 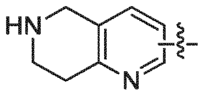, 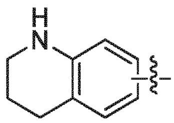, 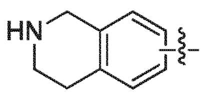, 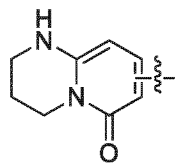,

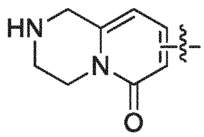, or 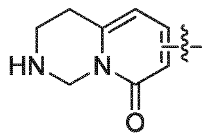;

the $R_7$ is unsubstituted or optionally substituted at any position by 1 to 3 substituents selected from $-L_3-W$, $-R_c$, halogen, cyano, nitro, $C_{1-6}$ alkyl, $C_{2-6}$ alkenyl, $C_{2-6}$ alkynyl, $C_{1-6}$ alkoxy, halo-$C_{1-6}$ alkyl, halo-$C_{1-6}$ alkoxy, and $C_{1-6}$ alkylamino.

In some embodiments, in D, $R_7$ is

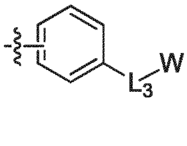, 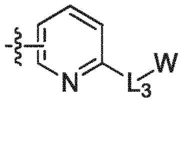, 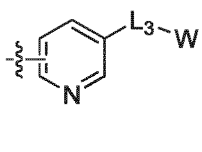,

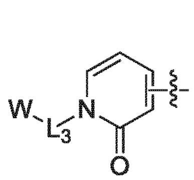, 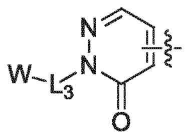, 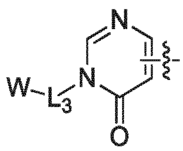, 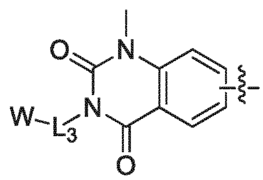, 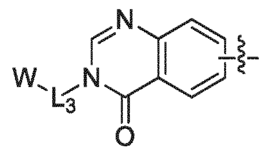,

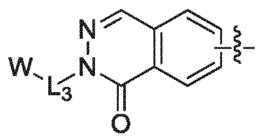, 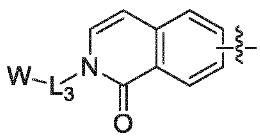, 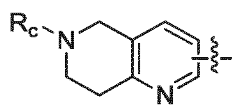, 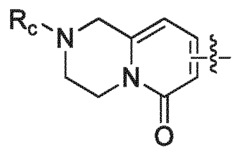,

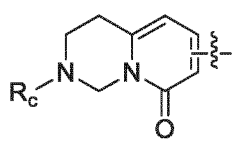, 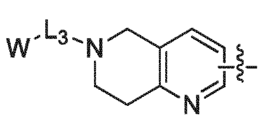, 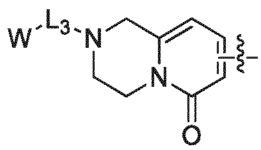, or 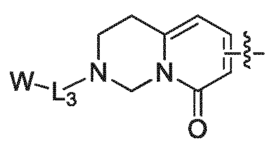.

In some embodiments, in D, $R_7$ is

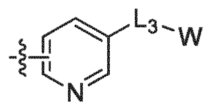 or 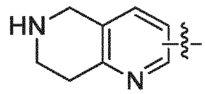.

In some embodiments, in D, $X_1$ is N or $CR_2$, $X_2$ is N or $CR_3$, and $X_3$ is N or $CR_1$; when $X_1$ is $CR_2$, $X_2$ is $CR_3$, and $X_3$ is $CR_1$, m is 1;

In some embodiments, in D, m is 0 or 1;
$X_1$ is N or $CR_2$, $X_2$ is N or $CR_3$, and $X_3$ is N or $CR_1$; when $X_1$ is $CR_2$, $X_2$ is $CR_3$, and $X_3$ is $CR_1$, m is 1;
R is $-C(O)-L_1-R_7$, $-C(O)-NR_9-L_1-R_7$, or $-C(S)-NR_9-L_1-R_7$.
In some embodiments, in D, m is 1;
$X_1$ is N or $CR_2$, $X_2$ is N or $CR_3$, and $X_3$ is N or $CR_1$;
R is $-C(O)-L_1-R_7$, $-C(O)-NR_9-L_1-R_7$, or $-C(S)-NR_9-L_1-R_7$.

In some embodiments, D is a compound of formula IA:

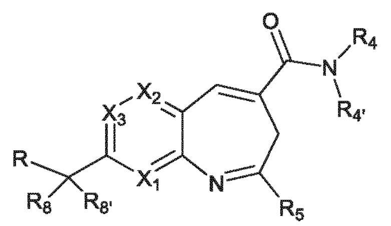

(IA) ;

R is $-C(O)-L_1-R_7$, $-C(O)-NR_9-L_1-R_7$, or $-C(S)-NR_9-L_1-R_7$;
$X_1$, $X_2$, $X_3$, R, $R_4$, $R_{4'}$, $R_5$, $R_8$, and $R_{8'}$ are defined as above.

In some embodiments, D is a compound of formula IB:

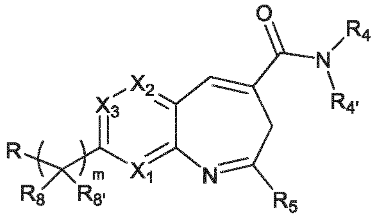

(IB)

R is $-C(O)-L_1-R_7$, $-C(O)-NR_9-L_1-R_7$, or $-C(S)-NR_9-L_1-R_7$;
$R_4$ is $C_{1-6}$ alkyl; the $R_4$ is optionally substituted at any position by one $-OR_a$,
$X_1$, $X_2$, $X_3$, R, $R_{4'}$, $R_5$, $R_8$, $R_{8'}$, $R_a$, and m are defined as above.

In some embodiments, in D, in IA, $L_1$ is a linkage bond.

In some embodiments, in D, in IA, $R_8$ and $R_{8'}$ together with the carbon atom to which they are attached form a $C_{3-6}$ cycloalkyl or 3- to 6-membered heterocycloalkyl group; the $C_{3-6}$ cycloalkyl or 3- to 6-membered heterocycloalkyl group is unsubstituted or optionally substituted at any position by 1 to 3 substituents selected from deuterium, halogen, hydroxyl, amino, cyano, oxo, $C_{1-6}$ alkyl, halo-$C_{1-6}$ alkyl, $-L_2-OR_a$, and $-L_2-NR_aR_b$.

In some embodiments, in D, in IA, $R_8$ and $R_{8'}$ together with the carbon atom to which they are attached form the following groups:

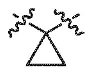, 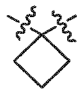, 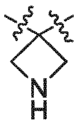, or 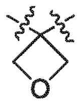.

In some embodiments, in D, in IA, $X_1$ is CH, $X_2$ is $CR_3$, and $X_3$ is CH; m is 1; $R_3$ is H or halogen.

In some embodiments, in D, in IA, $R_5$ is $-NH_2$.

In some embodiments, in D, in IB, $R_a$ is H.

In some embodiments, in D, in IB, $X_1$ is N or $CR_2$, $X_2$ is N or $CR_3$, and $X_3$ is N or $CR_1$; m is 1; $L_1$ is a linkage bond.

In some embodiments, in D, in IB, $R_8$ and $R_{8'}$ together with the carbon atom to which they are attached form a $C_{3-6}$ cycloalkyl or 3- to 6-membered heterocycloalkyl group; the $C_{3-6}$ cycloalkyl or 3- to 6-membered heterocycloalkyl group is unsubstituted or optionally substituted at any position by 1 to 3 substituents selected from deuterium, halogen, hydroxyl, amino, cyano, oxo, $C_{1-6}$ alkyl, halo-$C_{1-6}$ alkyl, $-L_2-OR_a$, and $-L_2-NR_aR_b$.

In some embodiments, in D, in IB, $R_8$ and $R_{8'}$ together with the carbon atom to which they are attached form the following groups:

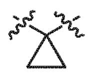, 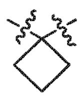, 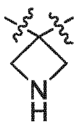, or 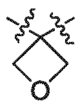.

In some embodiments, in D, in IB, $R_5$ is $-NH_2$.

In some embodiments, D is the compound shown in Table 1 and is covalently attached to the linker.

In some embodiments, D is the compound shown in Table 1 and is covalently attached to the linker via a nitrogen atom in the molecule.

In some embodiments, the immune-stimulating antibody conjugate of formula III has any one of the following structures:

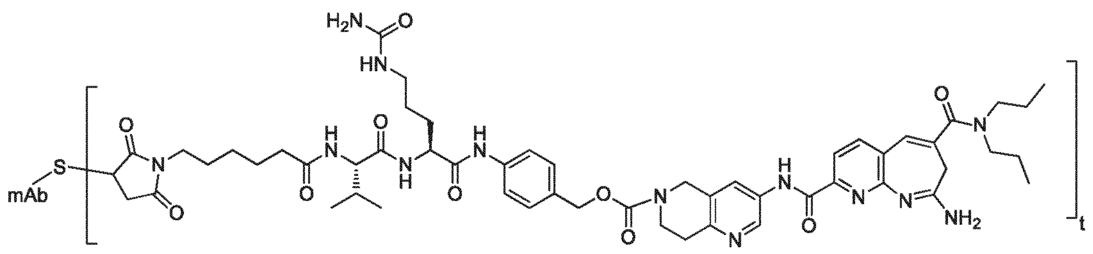

,

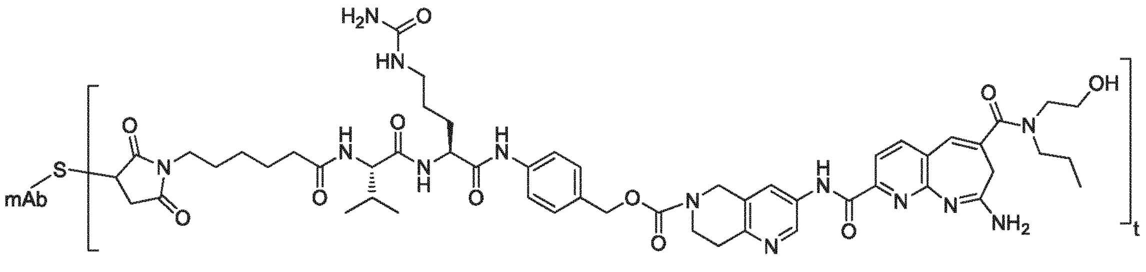

,

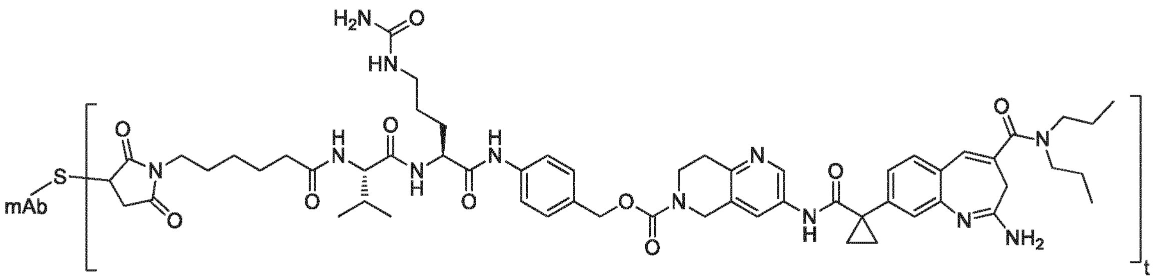

,

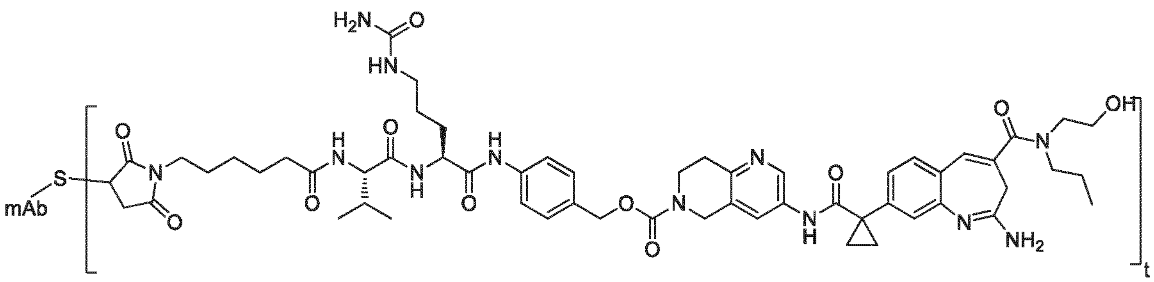

,

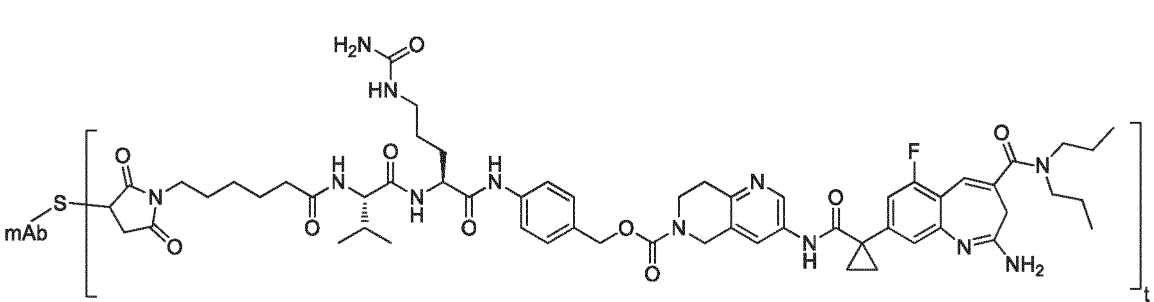

,

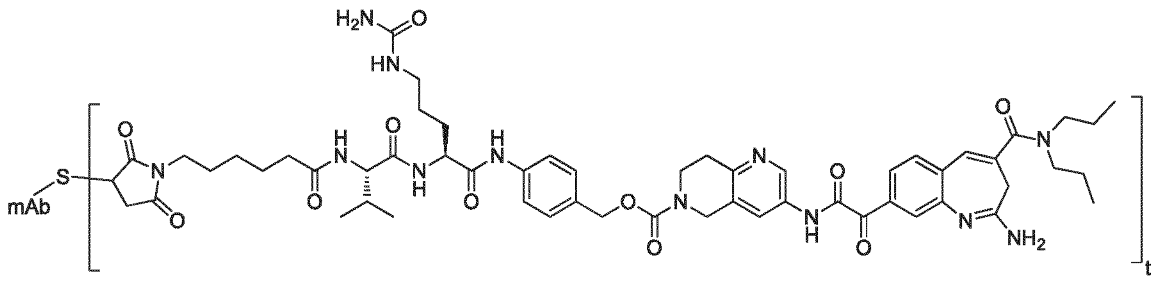

,

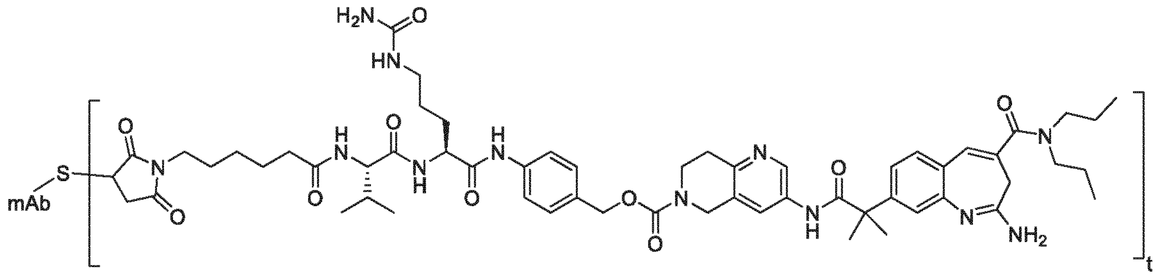

,

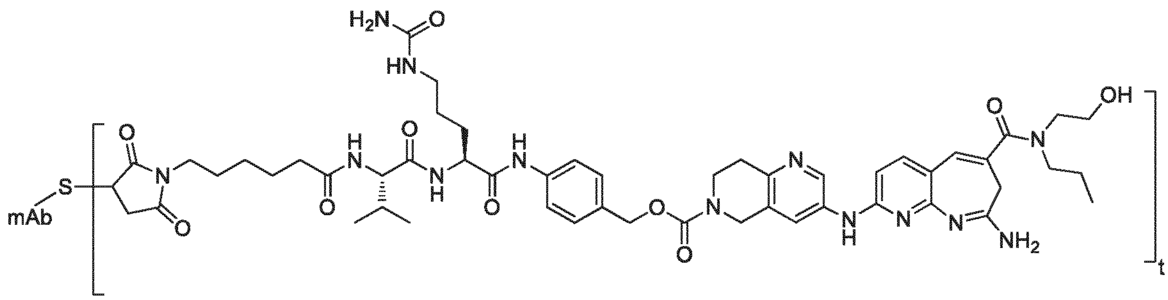

,

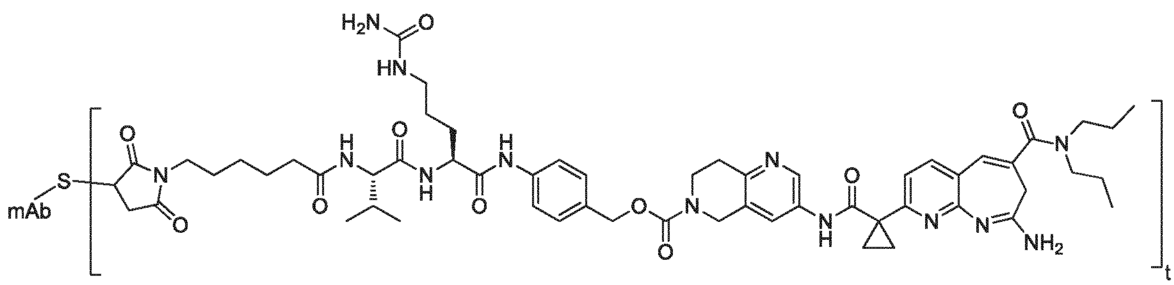

,

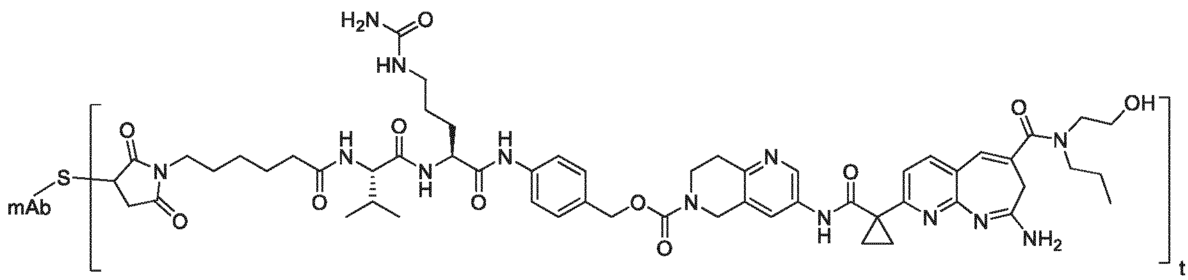

,

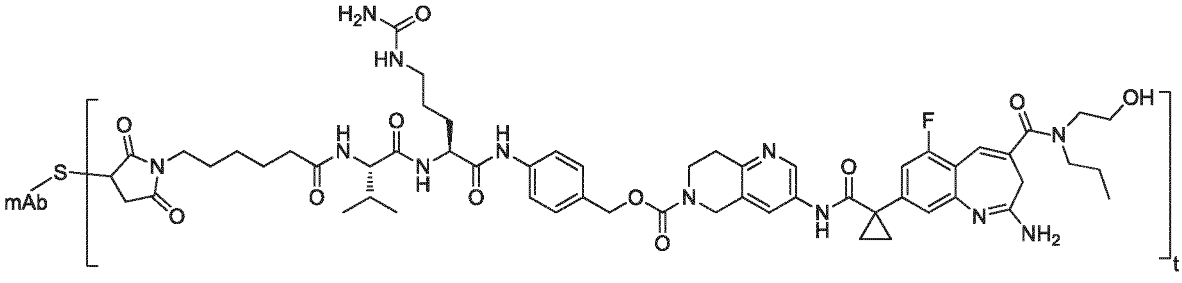,

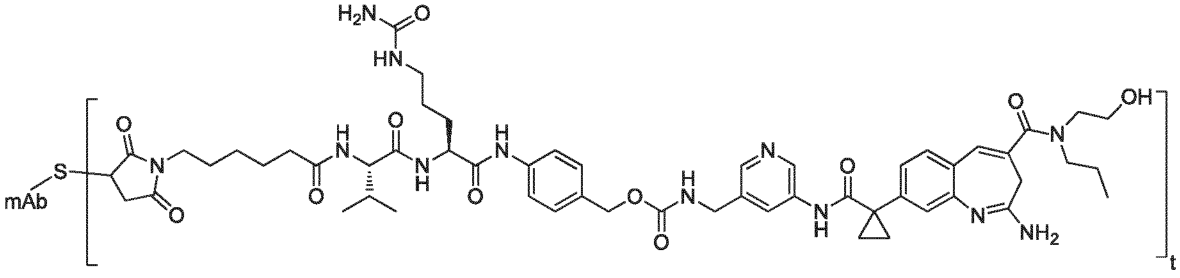,

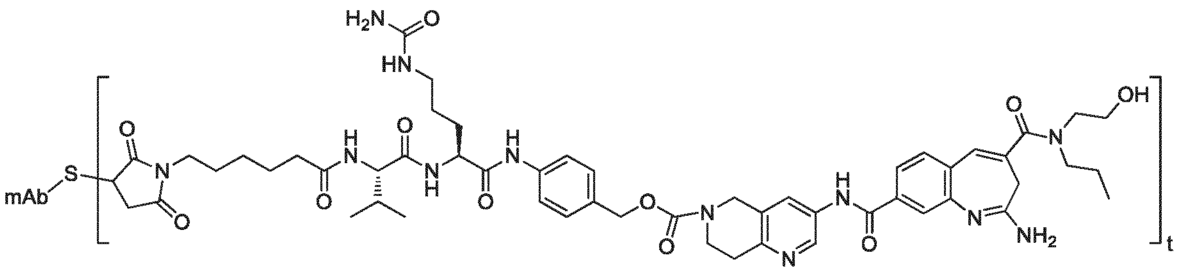,

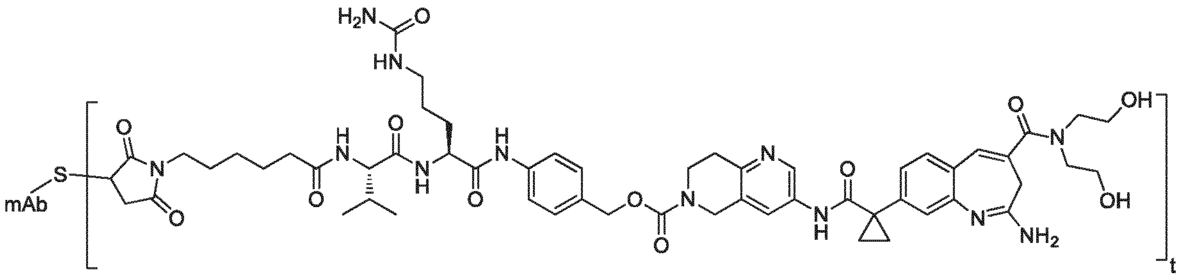,

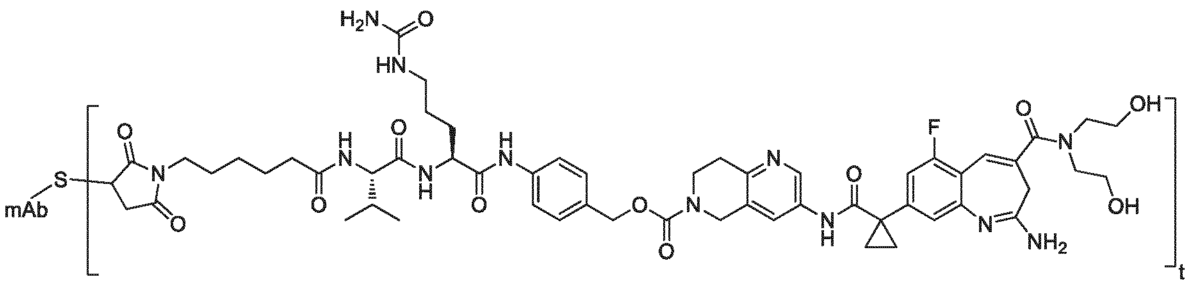,

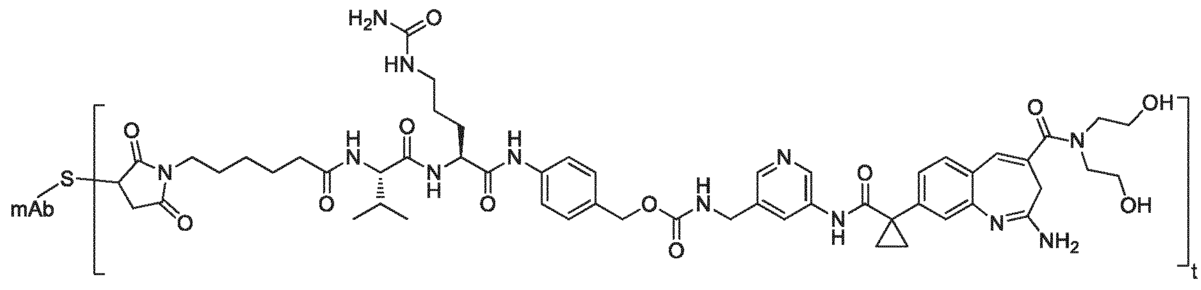

,

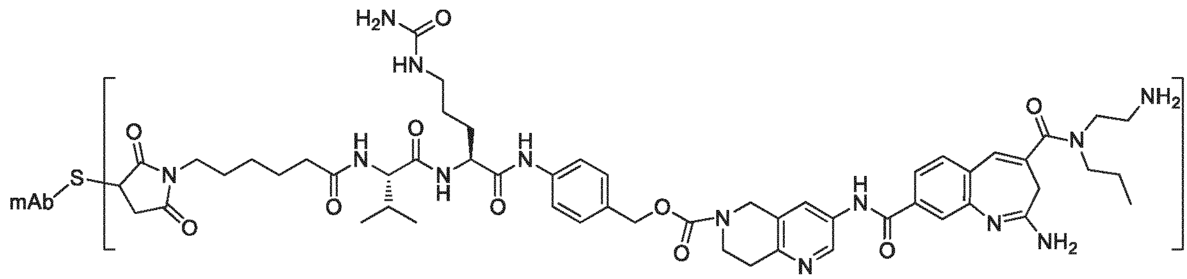

,

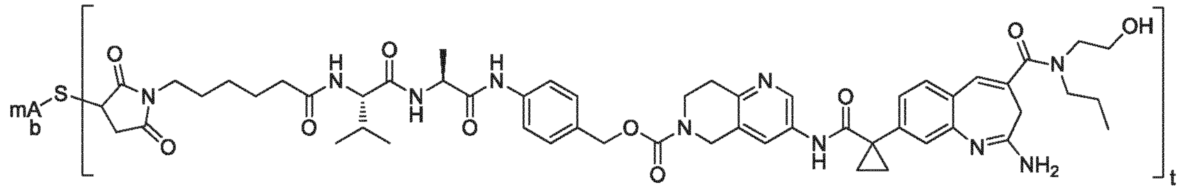

,

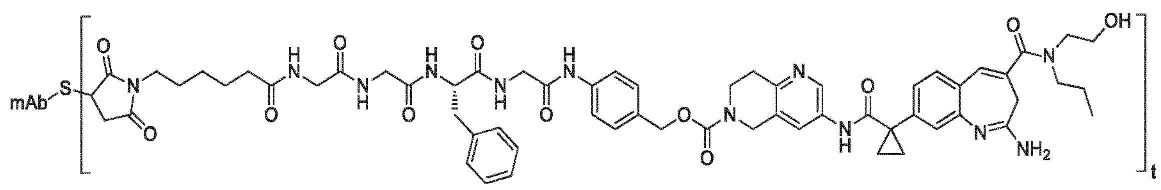

,

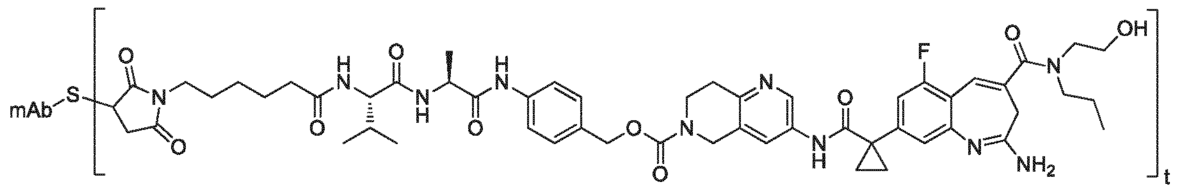

,

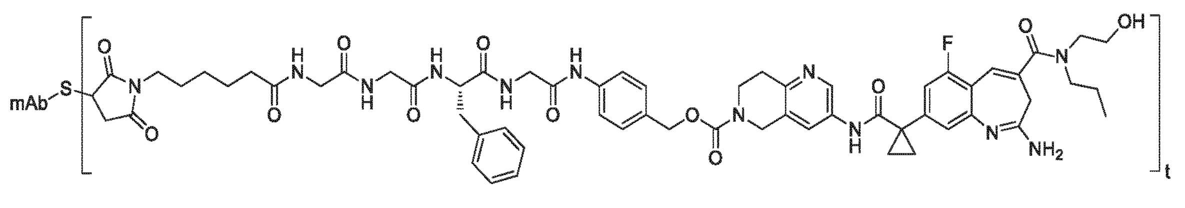

,

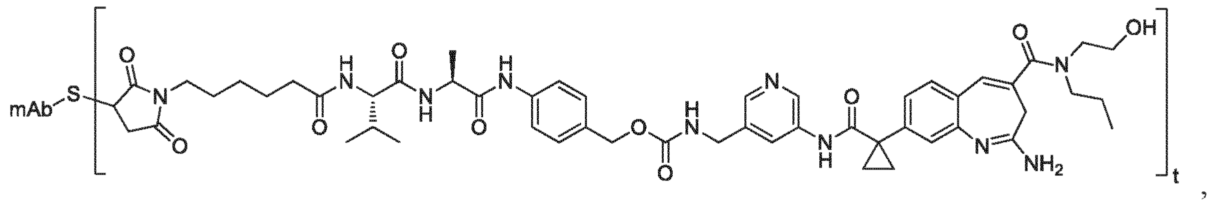

or

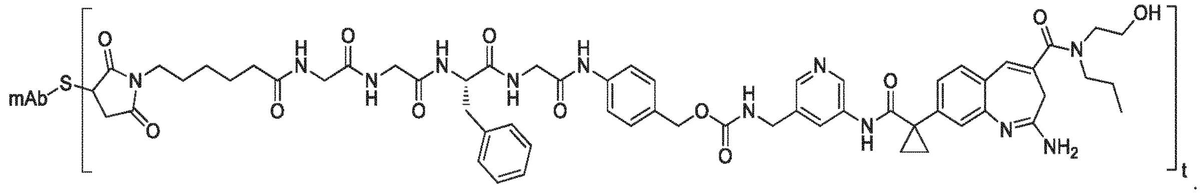

In some embodiments, the immune-stimulating antibody conjugate of formula III has any one of the following structures:

| Structure | t |
|---|---|
| 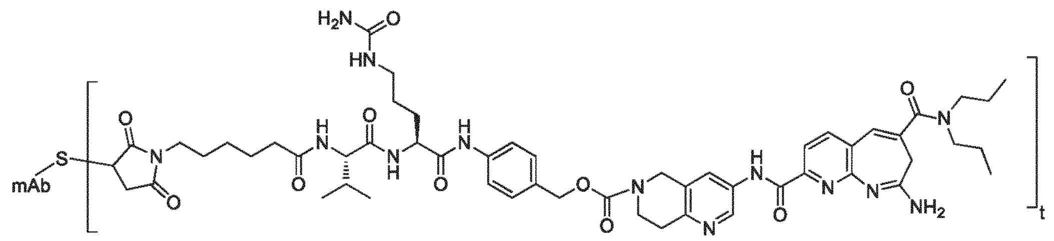 | 4.27 |
| 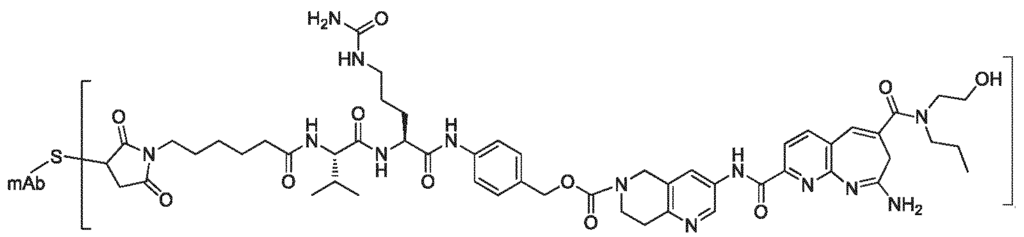 | 4.39 |
| 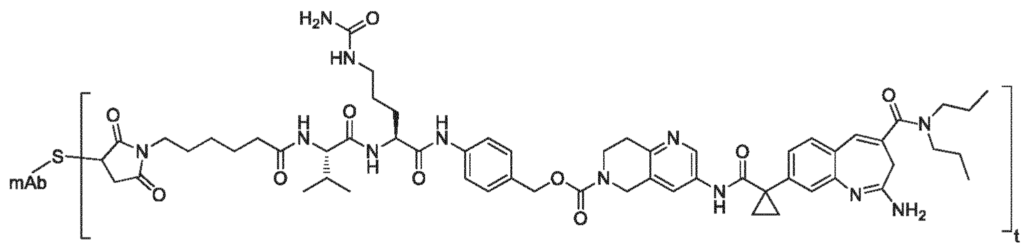 | 4.14 |
| 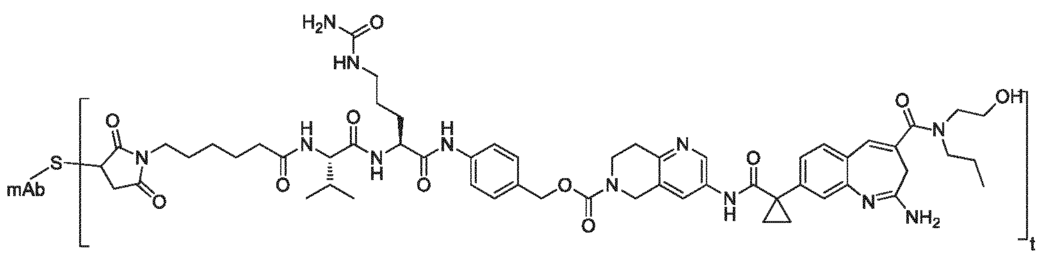 | 4.57 |

(continued)

| Structure | t |
|---|---|
| 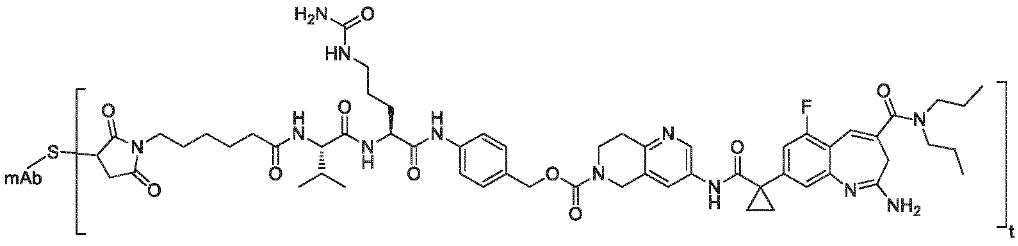 | 4.14 |
| 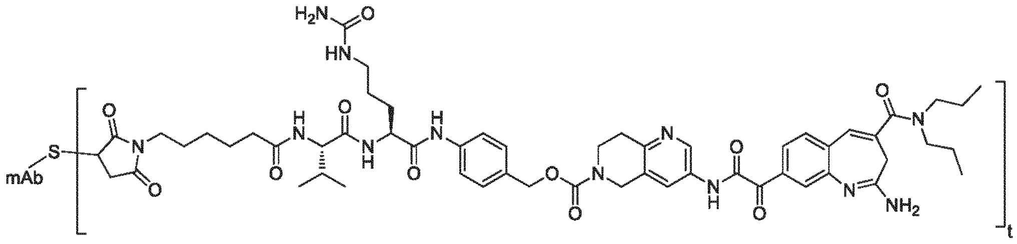 | 4.23 |
| 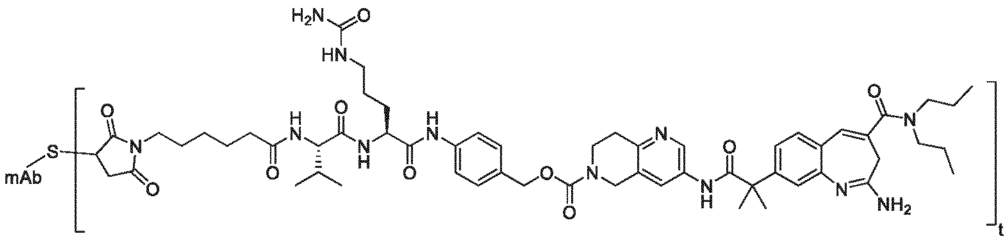 | 4.29 |
| 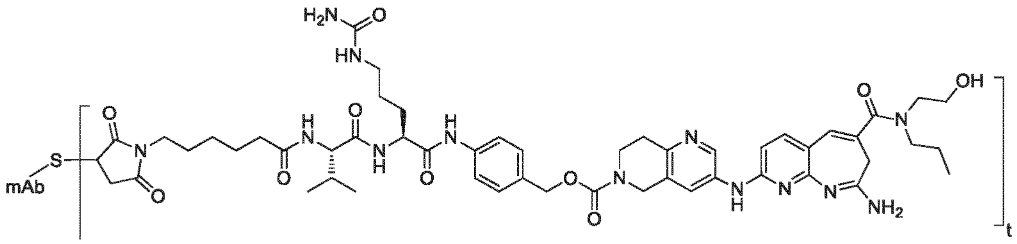 | 4.04 |
| 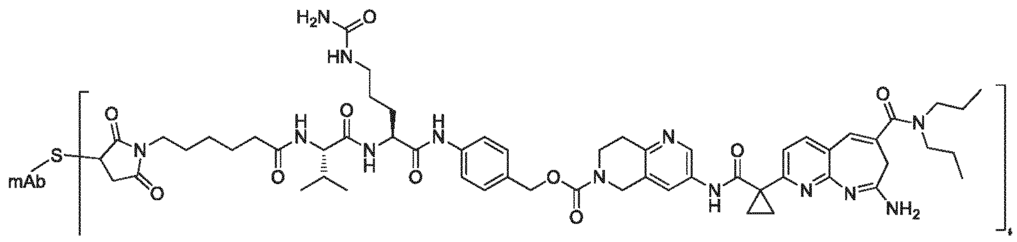 | 4.16 |
| 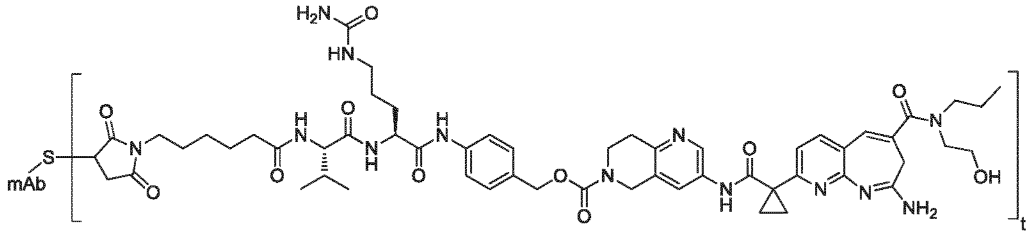 | 4.28 |

(continued)

| Structure | t |
|---|---|
| 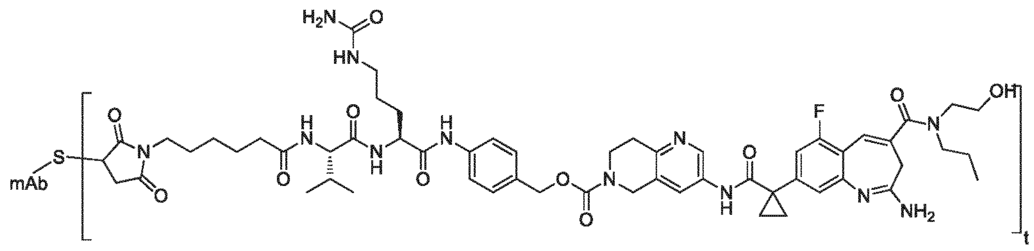 | 3.93 |
| 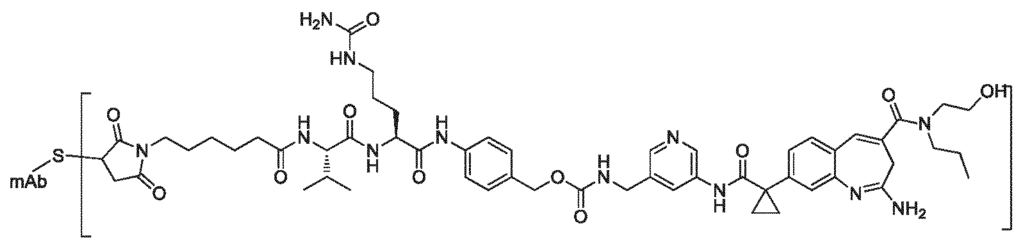 | 4.72 |
| 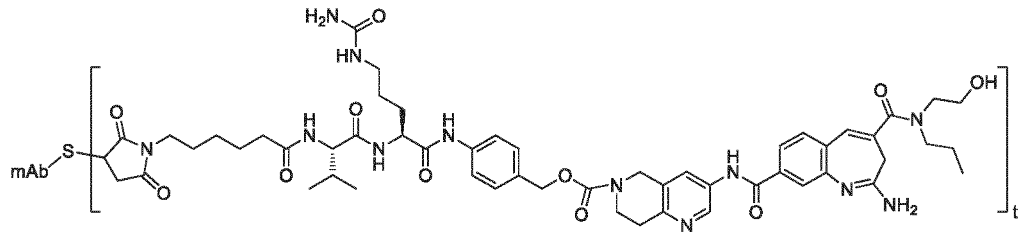 | 4.61 |
| 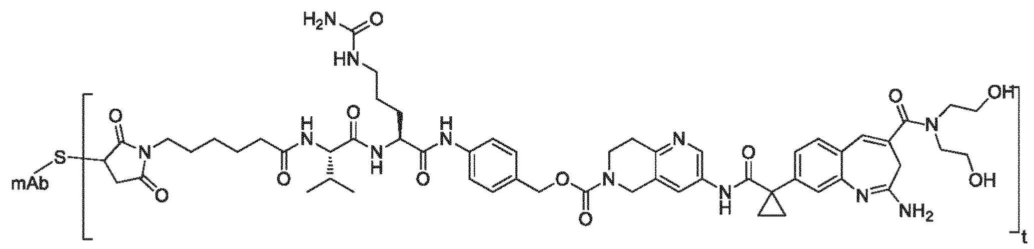 | 4.45 |
| 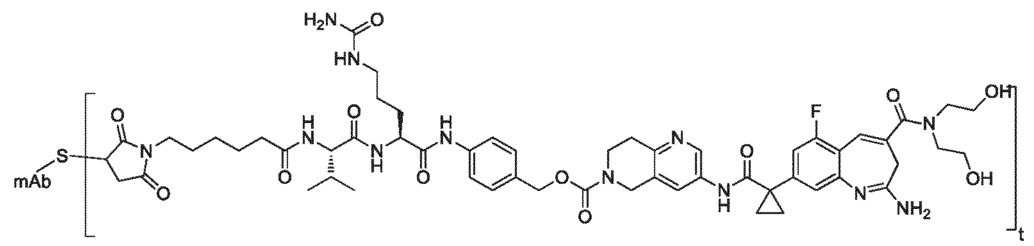 | 4.39 |
| 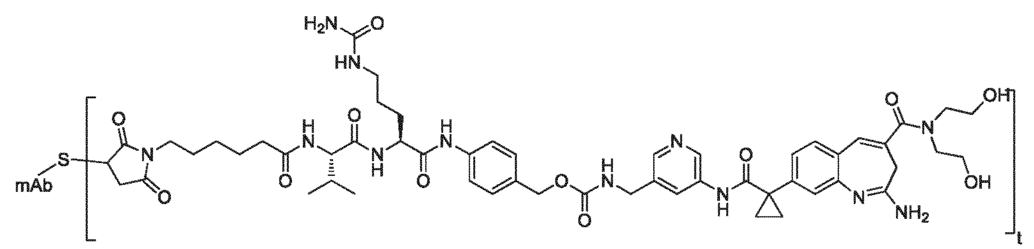 | 4.35 |

(continued)

| Structure | t |
|---|---|
| 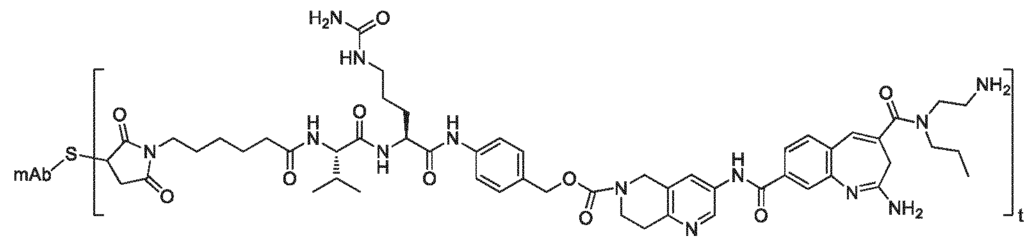 | 3.21 |
| 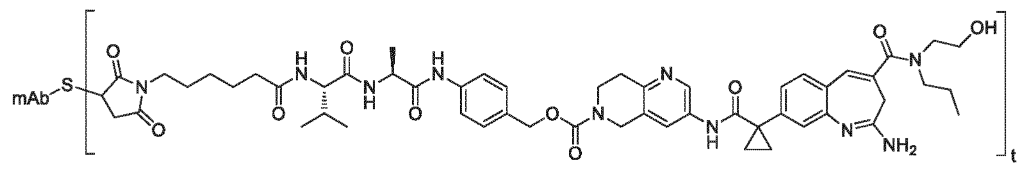 | 4.03 |
| 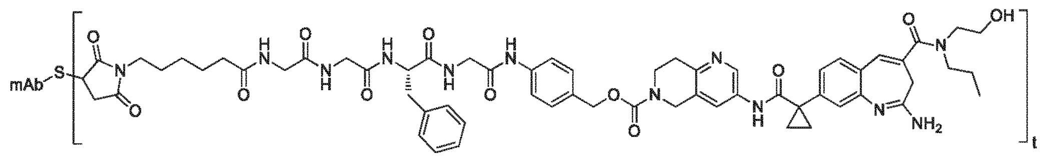 | 3.86 |
| 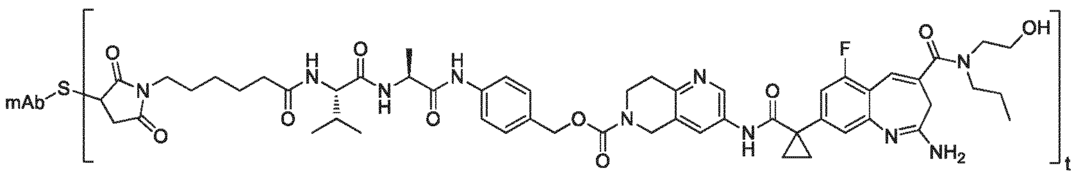 | 3.98 |
| 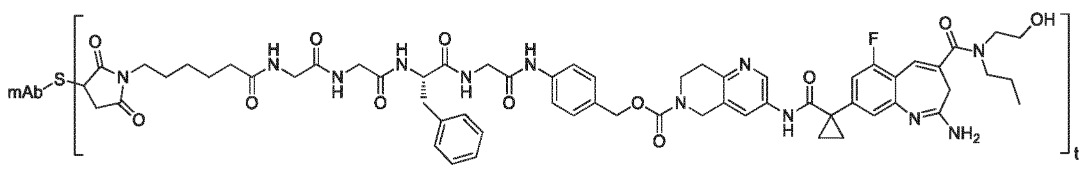 | 4.03 |
| 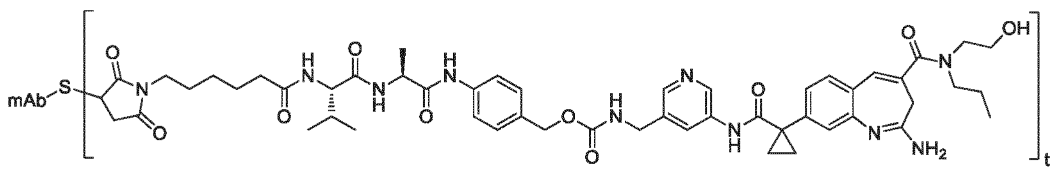 | 4.12 |
| 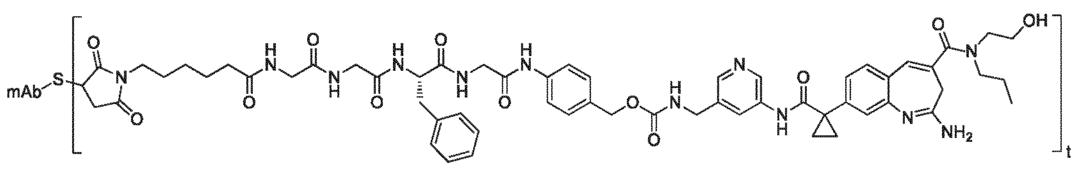 | 4.06 |

The present disclosure provides a pharmaceutical composition comprising substance K and a pharmaceutically acceptable excipient;

the substance K is substance K-1, substance K-2, or substance K-3;
the substance K-1 is the compound of formula I, the solvate thereof, the pharmaceutically acceptable salt thereof, or the solvate of the pharmaceutically acceptable salt thereof;
the substance K-2 is the compound of formula II', the solvate thereof, the pharmaceutically acceptable salt thereof, or the solvate of the pharmaceutically acceptable salt thereof;
the substance K-3 is the immune-stimulating antibody conjugate of formula III or the pharmaceutically acceptable

salt thereof.

In some embodiments, the substance K is the compound of formula I or the pharmaceutically acceptable salt thereof, the compound of formula II or the pharmaceutically acceptable salt thereof, or the immune-stimulating antibody conjugate of formula III or the pharmaceutically acceptable salt thereof.
In some embodiments, the amount of the substance K can be a therapeutically effective amount.
In some embodiments, the amount of the compound of formula I or the pharmaceutically acceptable salt thereof, the compound of formula II or the pharmaceutically acceptable salt thereof, or the immune-stimulating antibody conjugate of formula III or the pharmaceutically acceptable salt thereof can be a therapeutically effective amount.
The pharmaceutically acceptable excipient in the pharmaceutical composition can comprise a pharmaceutically acceptable carrier, diluent, and/or formulating agent.
The pharmaceutical composition can be administered by conventional routes, comprising (but not limited to): intramuscular, intraperitoneal, intravenous, subcutaneous, intradermal, local administration (such as intratumoral injection), etc.
The present disclosure also provides a use of substance K or the pharmaceutical composition in the manufacture of a medicament for regulating T cells and other immune cells, and the substance K is substance K-1, substance K-2, or substance K-3;

the substance K-1 is the compound of formula I, the solvate thereof, the pharmaceutically acceptable salt thereof, or the solvate of the pharmaceutically acceptable salt thereof;
the substance K-2 is the compound of formula II', the solvate thereof, the pharmaceutically acceptable salt thereof, or the solvate of the pharmaceutically acceptable salt thereof;
the substance K-3 is the immune-stimulating antibody conjugate of formula III or the pharmaceutically acceptable salt thereof.

In some embodiments, the substance K is the compound of formula I or the pharmaceutically acceptable salt thereof, the compound of formula II or the pharmaceutically acceptable salt thereof, or the immune-stimulating antibody conjugate of formula III or the pharmaceutically acceptable salt thereof.
In some embodiments, the amount of the substance K can be a therapeutically effective amount.
The present disclosure provides a use of substance K or the pharmaceutical composition in the manufacture of a medicament for treating and/or alleviating tumors or viral infectious diseases, and the substance K is substance K-1, substance K-2, or substance K-3;

the substance K-1 is the compound of formula I, the solvate thereof, the pharmaceutically acceptable salt thereof, or the solvate of the pharmaceutically acceptable salt thereof;
the substance K-2 is the compound of formula II', the solvate thereof, the pharmaceutically acceptable salt thereof, or the solvate of the pharmaceutically acceptable salt thereof;
the substance K-3 is the immune-stimulating antibody conjugate of formula III or the pharmaceutically acceptable salt thereof.

In some embodiments, the substance K is the compound of formula I or the pharmaceutically acceptable salt thereof, the compound of formula II or the pharmaceutically acceptable salt thereof, or the immune-stimulating antibody conjugate of formula III or the pharmaceutically acceptable salt thereof.
In some embodiments, the amount of the substance K can be a therapeutically effective amount.
The present disclosure provides a use of substance K or the pharmaceutical composition in the manufacture of a medicament for treating, alleviating, and/or preventing TLR8-mediated related diseases, and the substance K is substance K-1, substance K-2, or substance K-3;

the substance K-1 is the compound of formula I, the solvate thereof, the pharmaceutically acceptable salt thereof, or the solvate of the pharmaceutically acceptable salt thereof;
the substance K-2 is the compound of formula II', the solvate thereof, the pharmaceutically acceptable salt thereof, or the solvate of the pharmaceutically acceptable salt thereof;
the substance K-3 is the immune-stimulating antibody conjugate of formula III or the pharmaceutically acceptable salt thereof.

In some embodiments, the substance K is the compound of formula I or the pharmaceutically acceptable salt thereof, the compound of formula II or the pharmaceutically acceptable salt thereof, or the immune-stimulating antibody conjugate of formula III or the pharmaceutically acceptable salt thereof.

In some embodiments, the TLR8-mediated related diseases refer to tumors or viral infectious diseases.

In some embodiments, the amount of the substance K can be a therapeutically effective amount.

The tumor can be a malignant tumor, comprising metastatic and non-metastatic cancer, as well as familial hereditary and incidental cancer, and can also comprise solid tumors and non-solid tumors. In the present disclosure, the tumor is preferably a tumor with HER2 expression (either a tumor with high HER2 expression or a tumor with low HER2 expression). In the present disclosure, the tumor is preferably a tumor with high HER2 expression.

In the present disclosure, the terms "tumor" and "cancer" have the same meaning.

In the present disclosure, unless otherwise specified, the term "optionally substituted at any position by one or more than one substituent" means that any one or more than one hydrogen atom of one or more than one atom designated on the group is substituted with the designated substituent provided that the normal valence of the designated atom is not exceeded, and that the substitution in any position is a common and reasonable substitution in the art.

In the present disclosure, when the bonding to a substituent is shown to intersect the bonding of two atoms in a linking ring, then such a substituent can be bonded to any bondable ring atom on the ring.

In the present disclosure, any combination of variables is allowed only if such combination results in a stable compound.

In the present disclosure, when any variable occurs in the constitution or structure of the compound more than once, the definition of the variable at each occurrence is independent. For example, when R is substituted by one or more than one group, each substituent is an independent substituent and can be the same or different.

Unless otherwise specified, the following terms appearing in the specification and claims of the present disclosure have the meanings below:

The term "antibody" means any form of antibody that exhibits a desired biological activity (such as inhibiting the binding of a ligand to its receptor or inhibiting ligand-induced receptor signal transduction). Therefore, "antibody" is used in its broadest sense and expressly includes, but is not limited to, monoclonal antibodies (including full-length monoclonal antibodies), polyclonal antibodies, and multispecific antibodies (including bispecific antibodies). Naturally occurring "antibodies" are glycoproteins containing at least two heavy (H) chains and two light (L) chains interconnected by disulfide bonds. Each heavy chain consists of a heavy chain variable region and a heavy chain constant region. The heavy chain constant region consists of three domains (CH1, CH2, and CH3). Each light chain consists of a light chain variable region and a light chain constant region. The light chain constant region contains a CL structural domain. The variable regions of the heavy and light chains contain binding domains (antigen-binding domains) that interact with the antigen. The antigen-binding domain can be provided by one or more than one variable region on the antibody. Specifically, the antigen-binding domain contains the variable domain of light chain (VL) and variable domain of heavy chain (VH) on the antibody. The antibody can be a monoclonal antibody, a human antibody, a humanized antibody, or a chimeric antibody. These antibodies can be of any class (IgG, IgE, IgM, IgD, IgA, and IgY) or subtype (IgG1, IgG2, IgG3, IgG4, IgA1, and IgA2). The present disclosure includes not only complete antibodies, but also immunologically active antibody fragments (including Fab, F(ab')2, scFv, or Fv fragments) or fusion proteins formed by antibodies and other sequences. Therefore, "antibodies" as used in the present disclosure also include fragments, derivatives, and analogs of the antibodies.

The term "mAb", also known as "monoclonal antibody", refers to polypeptides (including antibodies, bispecific antibodies, etc.) that have essentially the same amino acid sequence or are derived from the same genetic source. Monoclonal antibodies are highly specific and can target a single antigenic site. Furthermore, in contrast to conventional (polyclonal) antibody preparations, which typically include multiple different antibodies directed against multiple different determinant clusters (epitopes), each monoclonal antibody is directed against only a single determinant cluster on the antigen. In some specific embodiments, the monoclonal antibody binds to optional antigens including, but not limited to, the following: HER2.

The term "HER2" (also known as ERBB2, NEU, NGL, TKR1, CD340, p185, MLN19, HER-2/neu) refers to the transmembrane tyrosine kinase receptor of the epidermal growth factor (EGF) receptor family. HER2 contains an extracellular binding domain, a transmembrane domain, and an intracellular tyrosine kinase domain. HER2 does not have its own ligand-binding domain and therefore cannot bind growth factors. However, HER2 binds tightly to other ligand-binding EGF receptor family members (such as HER1 or HER3) to form heterodimers that stabilize ligand binding and enhance kinase-mediated activation of downstream signaling pathways. In humans, there are isotypes of HER2 species: A, B, C, D, and E. "HER2" as used in the present disclosure includes all HER2 isotypes.

The term "anti-HER2 antibody" refers to an antibody that targets the HER2 protein, and the anti-HER2 antibody can be derived from any species, such as humans, rats, mice, and rabbits. The anti-HER2 antibody is preferably a monoclonal anti-HER2 antibody, and the anti-HER2 antibody is more preferably a humanized anti-HER2 antibody. The anti-HER2 antibodies include, but are not limited to: Pertuzumab, Trastuzumab, Trastuzumab biosimilar, Pertuzumab biosimilar, Margetuximab, HT-19, etc.;

wherein, Trastuzumab (also known as Herceptin or Herclon) is a humanized monoclonal antibody that can bind to the near-membrane region of the extracellular structure of the HER2 receptor (Hudis CA, N Engl J Med. 2007;

357(1):39-51). The amino acid sequences of heavy chain and light chain variable regions of Trastuzumab are disclosed in US Patent 5,821,337. Trastuzumab interacts with the tricyclic region formed by human HER2 residues 557 to 561, 570 to 573, and 593 to 603 (Cho et al., Nature 421: 756-760, 2003). Trastuzumab can interfere with HER2 signal transduction by preventing HER2 receptor dimerization, promoting HER2 receptor endocytosis, and inhibiting the shedding of the extracellular domain. In addition, another important mechanism of action of anti-HER2 antibodies is to mediate antibody-dependent cellular cytotoxicity (ADCC). In ADCC, anti-HER2 antibodies bind to tumor cells and then recruit immune cells, such as macrophages, through interaction with Fcy receptor (FcyR). Trastuzumab has a conserved human IgG Fc region and can recruit immune effector cells responsible for antibody-dependent cytotoxicity (Hudis CA, N Engl J Med. 2007; 357(1):39-51). Trastuzumab was approved by the US FDA in September 1998 for the treatment of tumors overexpressing HER2 and patients with metastatic breast cancer who have received one or more than one chemotherapy regimen;

Pertuzumab (also known as Perjeta, Omnitarg) is a humanized monoclonal antibody that binds to the extracellular domain of the HER2 receptor and inhibits the dimerization of HER2 and the HER receptor. The amino acid sequences of heavy chain and light chain variable regions of Pertuzumab are disclosed in US Patent 7,560,111. Pertuzumab mainly interacts with residues in the 245 to 333 region of human HER2, specifically residues His245, Val286, Ser288, Leu295, His296, or Lys311 (Franklin et al., Cancer Cell 5: 317-328, 2004). Studies have shown that Pertuzumab is more effective than Trastuzumab in disrupting HER1-HER2 and HER3-HER2 complex formation in breast and prostate cancer cell lines (Agus et al., J Clin Oncol. 2005; 23(11):2534-43. Epub Feb 7, 2005). Pertuzumab was approved by the US FDA in June 2012, and is used in combination with Trastuzumab and Docetaxel to treat patients with HER2-positive metastatic breast cancer who have not received anti-HER2 therapy or chemotherapy;

Margetuximab (also known as MGAH22, Margenza) is an Fc-engineered monoclonal antibody targeting the HER2 protein and binds to the extracellular region of HER2. Margetuximab only differs from the variable region sequence of Trastuzumab by a few amino acids. The Fc has been mutated at five sites: F243L/R292P/Y300L/L235V/P396L, which improves the affinity for CD16A and enhances the activity of ADCC. The modified Fc region of Margetuximab increases the binding to the activating Fc receptor FCGR3A (CD16A) and reduces the binding to the inhibitory Fc receptor FCGR2B (CD32B), resulting in stronger ADCC effects and NK cell activation (Nordstrom J. et al., Breast Cancer Research, 2011; 13: R123). Margetuximab was approved by the US FDA in December 2020 for the treatment of adult patients with HER2-positive metastatic breast cancer (MBC) who have received two or more than two anti-HER2-targeted therapies, wherein at least one anti-HER2-targeted therapy is for the treatment of metastatic breast cancer;

HT-19 is another anti-HER2 monoclonal antibody that binds the epitope of human HER2 that is different from the binding epitopes of Trastuzumab or Pertuzumab and has been shown to have comparable ability to inhibit HER2 signal transduction as Trastuzumab. HT-19 in combination with Trastuzumab and Pertuzumab can promote HER2 degradation (Bergstrom D. A. et al., Cancer Res. 2015; 75:LB-231).

In the present disclosure, the anti-HER2 antibody is not limited to the above-listed antibodies as long as it specifically binds to HER2 (for example, an anti-HER2 antibody that has the internalizing activity in HER2-expressing cells through binding to HER2).

In the present disclosure, the isotypes of the "anti-HER2 antibody" include IgG1, IgG2, IgG3, IgG4, etc., preferably IgG1, IgG2, or IgG4.

In the present disclosure, the "anti-HER2 antibody" or the antigen-binding fragment thereof can include Fc regions, and the Fc region can be further modified. In some cases, one or more than one mutation in the Fc regions results in improvements in drugs containing such modified Fc regions, such as reduced effector function, changes in the regulation of metabolic half-life of drugs, and drug stability. In some cases, the modified Fc regions can contain one or more than one mutation that will reduce or eliminate the interaction between the antibody and the immune system. Key interactions can include the interaction of the antibody Fc with the Fcy receptor, and the interaction with the C1q of the complement system. In the case of using IgG1 as the same isotype of the antibody of the present disclosure, the effector function can be adjusted by substituting part of the amino acid residues of the constant region. Variants of IgG1 that reduce or weaken the effector function include, but are not limited to: IgG1 LALA (IgG1-L234A, L235A), IgG1 LAGA (IgG1-L235A, G237A), etc., preferably IgG1 LALA. The L234A and L235A represent the leucine at positions 234 and 235 determined by the EU index (Proc. Natl. Acad. Sci. U.S.A., Vol. 63, No. 1 (May 15, 1969), p. 78-85) are replaced by alanine, and G237A represents that the glycine at position 237 determined by EUindex is replaced by alanine.

The term "low HER2 expression" generally refers to a clinical test result of HER2 expression level of IHC 1+, or IHC 2+/FISH negative (i.e., IHC 2+, and the FISH test is negative at the same time). The terms "high HER2 expression" and "HER2 positive" are used interchangeably and generally refer to a clinical test result of HER2 expression level of IHC 2+/FISH positive (i.e., IHC 2+, and the FISH test is positive at the same time), or IHC 3+. When IHC staining intensity is reported as a range, the term "low HER2 expression" herein includes not only IHC 1+, or IHC 2+/FISH negative, but also ranges from IHC 0 to 1+ and IHC 1+ to 2+. The terms "high HER2 expression" and "HER2 positive" each include not only IHC 2+/FISH positive, or IHC 3+, but also a range from IHC 2+ to 3+. In the present disclosure, FISH negative means a FISH test result showing no amplification of the HER2 gene, while FISH positive means a FISH test result

showing amplification of the HER2 gene.

The terms "fragment", "derivative", and "analog" refer to polypeptides that essentially maintain the same biological function or activity of the antibodies of the present disclosure. The polypeptide fragment, derivative, or analog of the present disclosure can be a) a polypeptide in which one or more than one conservative or non-conservative amino acid residues (preferably conservative amino acid residues) are replaced, and such replaced amino acid residues can or can not be encoded by genetic code, or b) a polypeptide having substituent groups in one or more than one amino acid residue, or c) a polypeptide formed by fusion of a mature polypeptide with another compound (such as a compound that prolongs the half-life of the polypeptide, such as polyethylene glycol), or d) a polypeptide formed by fusion of an additional amino acid sequence to this polypeptide sequence (such as a leader sequence, secretion sequence, a sequence used for purification of this peptide, or a proteinogenic sequence, or a fusion protein with a 6His tag).

In the present disclosure, the biosimilar of the monoclonal antibody refers to the biosimilar of the monoclonal antibody, despite minor differences in inactive components clinically, is highly similar to the monoclonal antibody and has no clinically meaningful differences in safety and/or efficacy.

The term "linker" refers to a degradable or non-degradable linking fragment used to attach the small molecule drug D to the antibody. One antibody molecule can be attached to a plurality of linkers carrying small molecule drug D. Normally, each linker can be attached to one or more than one small molecule drug. In the present disclosure, preferably, each linker is attached to one small molecule drug D. Normally, each antibody can be attached to a plurality of linkers. In the present disclosure, preferably, each antibody is preferably attached to 1 to 10 linkers; more preferably, each antibody is attached to 1 to 8 linkers.

In the present disclosure, the non-degradable linker means that the linker has enzyme stability and/or chemical stability *in vivo* and *in vitro,* and the release of small molecule drug D can not depend on the differential nature of plasma, tumor tissue, and intracellular enzyme levels. The release of small molecule drug D can be achieved through antigen-mediated phagocytosis by degradation of the antibody to the amino acid level after endocytosis of the immune-stimulating antibody conjugate, which in turn releases a derivative of small molecule drug D. The derivative of small molecule drug D is composed of a small molecule drug D, linker units, and amino acid residues or is a residue consisting of a small molecule drug D covalently attached to linker units.

In the present disclosure, the degradable linker can be degraded *in vivo or in vitro,* and it contains a linker unit that can be degraded by a specific enzyme *in vivo or in vitro* or a chemically unstable linker unit. The degradable linker can be degraded intracellularly to release the small molecule drug D. For example, the linker can be reduced in the cytoplasm, degraded under lysosomal acidic conditions, or degraded by specific proteases or other enzymes within the cell. The degradable linker includes one or more than one enzymatically degradable linker unit, chemically unstable linker unit, or other degradable linker unit, and the other components can be non-enzymatically degradable or chemically stable linker units. The chemically unstable linker unit includes oxime, hydrazone, and/or disulfide groups (such as

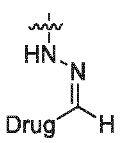, 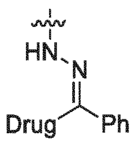, 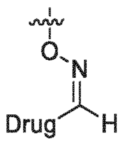, 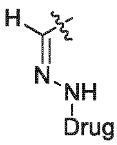, 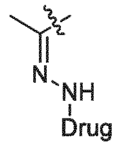, 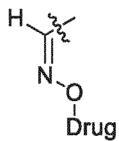, 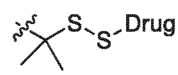

). The linker unit specifically degraded by the enzyme is 1) a linker unit formed based on the peptide. The peptide bond can have good serum stability due to the fact that lysosomal proteases are much less active in blood than in certain tumor tissues. Therefore, the linker unit can be selectively degraded in certain tumor tissues or cells to release the small molecule drug D. The lysosomal enzyme can be selected from cathepsin B, cathepsin S, plasmin, elastase, β-glucuronidase, β-galactosidase, etc. The linker unit based on peptide formation can be a tetrapeptide (for example: Gly-Phe-Leu-Gly, Ala-Leu-Ala-Leu, Gly-Gly-Phe-Gly), a tripeptide (Val-Leu-Lys, Ala-Pro-Val), a dipeptide (such as Val-Cit, Cit-Val, Val-Ala, Ala-Val, Ala-Cit, Cit-Ala, Asn-Cit, Cit-Asn, Cit-Cit, Val-Glu, Glu-Val, Ser-Cit, Cit-Ser, Lys-Cit, Cit-Lys, Asp-Cit, Cit-Asp, Ala-Val, Val-Ala, Phe-Lys, Lys-Phe, Val-Lys, Lys-Val, Ala-Lys, Lys-Ala, Phe-Cit, Cit-Phe, Leu-Cit, Cit-Leu, Ile-Cit, Cit-Ile, Phe-Arg, Arg-Phe, Cit-Trp, Trp-Cit), or an amino acid monomer, and preferably, the linker unit based on peptide formation is a dipeptide linker unit. The other degradable linker units can include ester linkage bonds formed by the reaction of PEG carboxylic acid or activated PEG carboxylic acid with the hydroxyl groups on the small molecule drug D, wherein such ester linkage bonds can be hydrolyzed under physiological conditions, thereby releasing the small molecule drug D. Hydrolytically degradable linkage bonds include, but are not limited to, carbonate linkage bonds, imine linkage bonds resulting from the reaction of amines and aldehydes, phosphate ester linkage bonds resulting from the reaction of hydroxyl groups and phosphoric acid groups, acetal linkage bonds resulting from the reaction of hydroxyl groups and aldehydes, and orthoester linkage bonds resulting from the reaction of formate groups and hydroxyl groups. The degradable linker units can also include non-degradable fragments, such as polyethylene glycol (PEG) and its

related polymers. 2) A linker unit formed via pyrophosphate or phosphate esters. Lysosomal acidic pyrophosphate and acidic phosphatase are enzymes that hydrolyze pyrophosphate and terminal monophosphate, respectively, into their parent alcohols in lysosomes. Targeting these enzymes can effectively release the small molecule drug D with an alkyl alcohol terminal.

In the present disclosure, the "linker" also includes a linker head that can be attached to the antibody. In the present disclosure, the linker can be attached via the linker head to the thiol group (such as cysteine group) in the antibody. The linker head is preferably a maleimide group and a ring-opened maleimide group (for example:

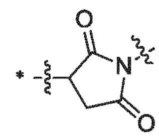,
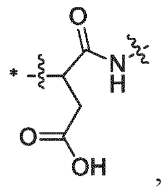,

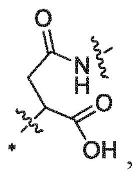, or
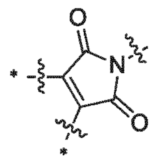,

the marked site * is the site attached to the antibody). The attachment between the maleimide group and the antibody's thiol group (for example: cysteine group) is specific (for example, as shown in formula 1 or 2).

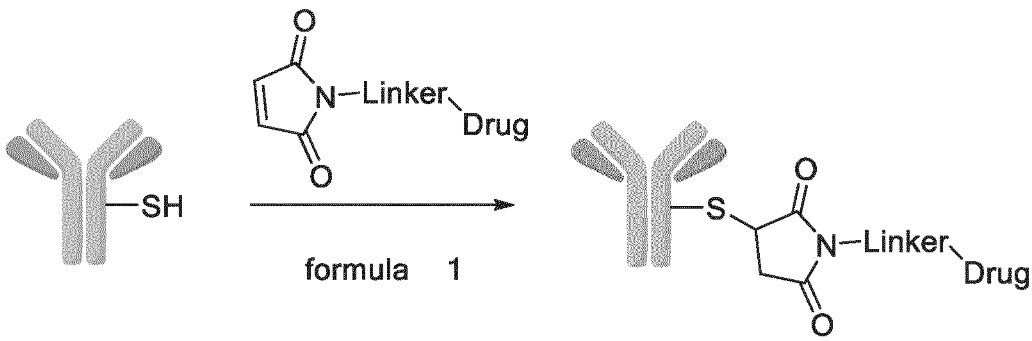
formula 1
;

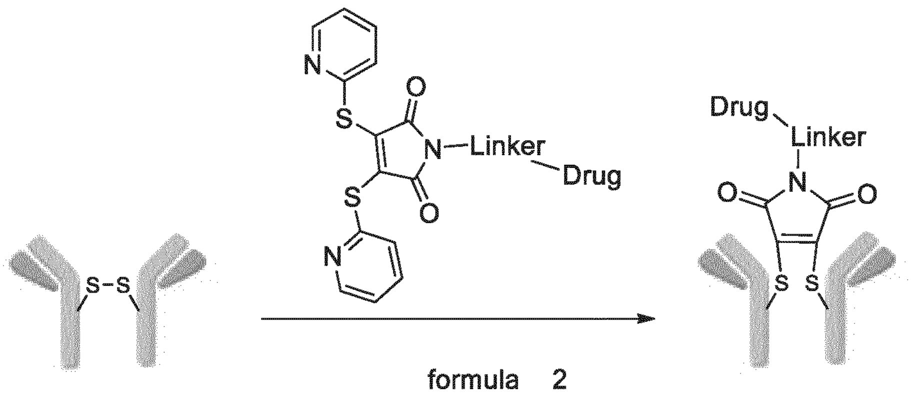
formula 2

In the present disclosure, the "linker" can further include a Spacer, and the linker head can be directly attached to a linker unit in the linker, or can be attached to a linker unit in the linker via a Spacer. The Spacer can be polyethylene glycol and its related polymer, an alkylene group containing 1 to 10 carbon atoms, a cyclohexyl group, an amide group, an ester group, an oxo group,

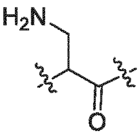, or 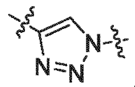,

and combinations of any one or more than one of the Spacers.

The "linker" can also form an amide bond (for example, shown in formula 3) through the reaction of the carboxyl or ester group in the linker unit or Spacer with the amino group (for example, lysine group) in the antibody.

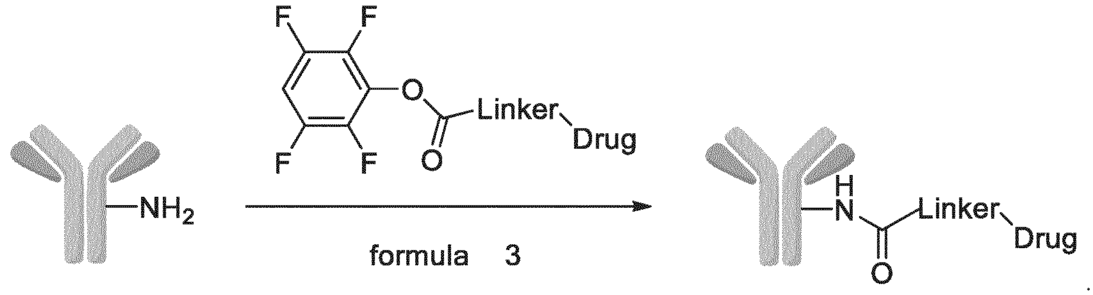


.

In the present disclosure, the "linker" may further include a self-immolative group, which spatially separates the small molecule drug D from the enzyme degradation site. Common self-immolative groups usually contain the following basic structures:

1)

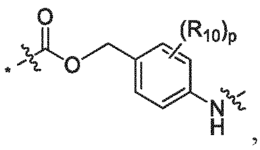, 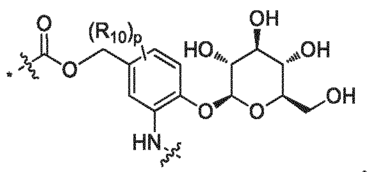, 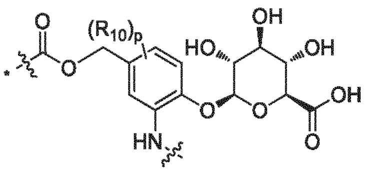;

the self-immolative groups can also be attached to the hydroxyl group on the small molecule D through an aminomethylene group, an amino-$C_{3-5}$ alkanoyl group, a phosphate group, or a pyrophosphate group;

2)

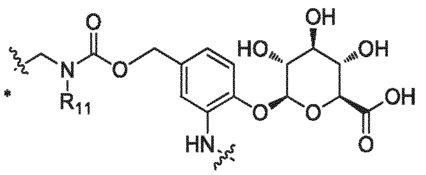, 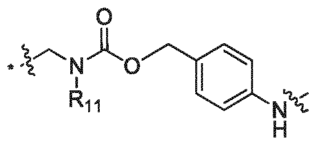, 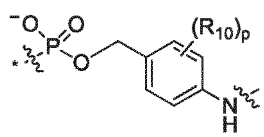,

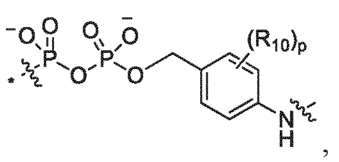, 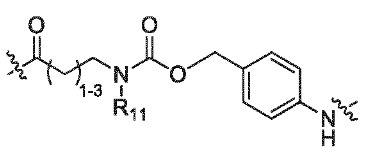;

the marked site * in 1) is the site attached to the small molecule drug D, preferably attached to the amino group on the small molecule drug D; the marked site * in 2) is the site attached to the small molecule drug D, preferably attached to the hydroxyl group on the small molecule drug D.

The term "linker 2" and "linker X" mean the degradable or non-degradable linking fragment to which small molecule drugs D is attached. They are not attached to the antibody molecule, but can be attached to the antibody

molecule via a reaction, thus forming the "linker". Normally, each linker X can be attached to one or more than one small molecule drug. In the present disclosure, preferably, each linker X is attached to one small molecule drug D. Except that the linker 2 is not attached to an antibody molecule, the rest of the definitions of the linker 2 are the same as the "linker".

In the present disclosure, the "linker 2" also includes a linker head that has not been attached to the antibody. In the present disclosure, the linker can be attached via the linker head to the thiol group (such as cysteine group) in the antibody. The linker head is preferably a maleimide group and a ring-opened maleimide group (for example, a linker head that has not been attached to the antibody, which includes, but is not limited to

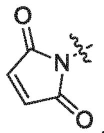,
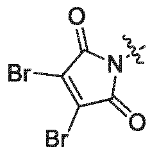,

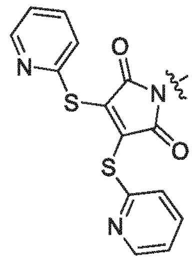, or
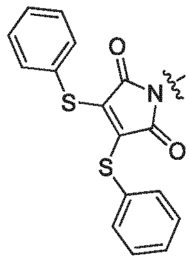

). The attachment between the maleimide group and the antibody's thiol group (for example: cysteine group) is specific (for example, as shown in formula 1 or 2).

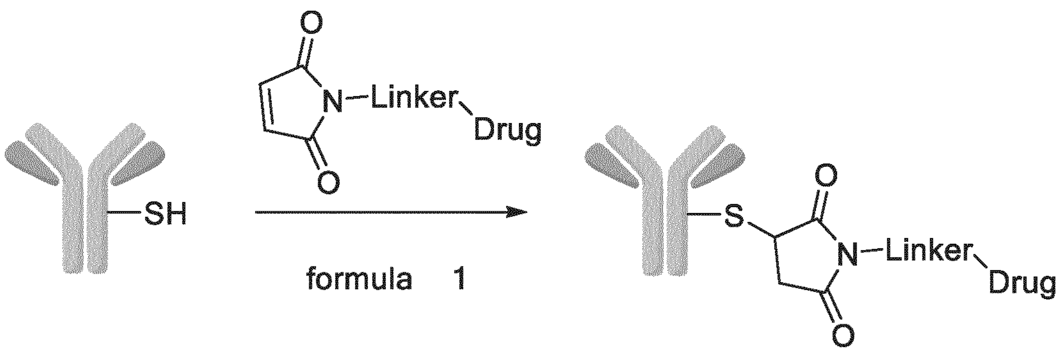

;

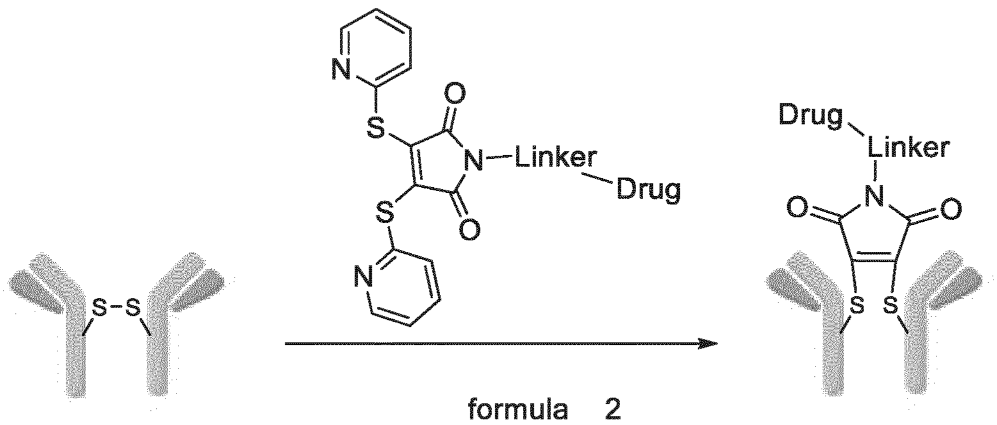

.

The term "and/or" includes any and all combinations of one or more than one related listed item.

The term "$C_{A-B}$" refers to the range from the starting point to the end point, wherein A, B, and each point in the range are integers, indicating the number of the carbon atom, for example, $C_{1-4}$ means that the number of the carbon atom is 1, 2, 3, or 4; $C_{1-6}$ means the number of the carbon atom is 1, 2, 3, 4, 5, or 6; $C_{3-8}$ means the number of the carbon atom is 3, 4, 5, 6, 7, or 8; $C_{A-B}$ can be used in combination with any group containing the carbon atom for the purpose of defining the number of the carbon atom, such as $C_{1-6}$ alkyl, $C_{3-8}$ cycloalkyl, $C_{6-10}$ aryl, $C_{1-4}$ alkoxy, $C_{3-8}$ cycloalkyl-$C_{1-4}$ alkyl, etc.

The term "alkyl" means a saturated linear or branched hydrocarbon group containing 1 to 20 carbon atoms, preferably 1 to 10 carbon atoms, more preferably 1 to 8, 1 to 6, 1 to 4, or 1 to 3 carbon atoms. Representative examples of alkyl include, but are not limited to: methyl, ethyl, *n*-propyl, isopropyl, *n*-butyl, *sec*-butyl, *tert*-butyl, isobutyl, *n*-pentyl, *n*-hexyl, *n*-heptyl, octyl, nonyl, decyl, 1,1-dimethylpropyl, 1,2-dimethylpropyl, 2,2-dimethylpropyl, 1-ethylpropyl, 2-methylbutyl, 3-methylbutyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2,3-dimethylbutyl, 2-ethylbutyl, 2-methylpentyl, 3-methylpentyl, 4-methylpentyl, 4,4-dimethylpentyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylhexyl, 3-ethylhexyl, 2,2,4-trimethylpentyl, undecyl, dodecyl, and various isomers thereof.

The term "alkenyl" means a linear, branched, or cyclic non-aromatic hydrocarbon group containing at least one carbon-carbon double bond. There can be 1 to 3 carbon-carbon double bonds, preferably one carbon-carbon double bond. The term "$C_{2-4}$ alkenyl" means an alkenyl group with 2 to 4 carbon atoms, and the term "$C_{2-6}$ alkenyl" means an alkenyl group with 2 to 6 carbon atoms, including vinyl, propenyl, butenyl, 2-methylbutenyl, and cyclohexenyl. The alkenyl can be substituted.

The term "alkynyl" means a linear, branched, or cyclic hydrocarbon group containing at least one carbon-carbon triple bond. There can be 1 to 3 carbon-carbon triple bonds, preferably one carbon-carbon triple bond. The term "$C_{2-6}$ alkynyl" means an alkynyl group with 2 to 6 carbon atoms, including ethynyl, propynyl, butynyl, and 3-methylbutynyl.

The term "alkylene" means a saturated linear or branched non-bridged divalent alkyl group containing 1 to 20 carbon atoms, preferably 1 to 8 carbon atoms, more preferably 1 to 6 or 1 to 4 carbon atoms, and the examples include but are not limited to $-CH_2-$, $-CH_2CH_2-$, $-CH_2CH_2CH_2-$, $-CH_2CH_2CH_2CH_2-$, $-CH_2CH(CH_3)-$, $-CH_2C(CH_3)_2CH_2-$, $-CH_2C(CH_3)_2CH_2CH_2-$, $=CH_2$, $=CHCH_3$, and $=C(CH_3)_2$.

The term "alkenylene" means a linear, branched, or cyclic non-aromatic divalent hydrocarbon group containing at least one carbon-carbon double bond, wherein there can be 1 to 3 carbon-carbon double bonds, and preferably one carbon-carbon double bond. The alkenylene preferably contains 2 to 8 carbon atoms, more preferably 2 to 6 or 2 to 4 carbon atoms.

The term "alkynylene" means a linear, branched, or cyclic non-aromatic divalent hydrocarbon group containing at least one carbon-carbon triple bond. There can be 1 to 3 carbon-carbon triple bonds, preferably one carbon-carbon triple bond. The alkynylene preferably contains 2 to 8 carbon atoms, more preferably 2 to 6 or 2 to 4 carbon atoms.

The term "cycloalkyl" means a saturated or partially unsaturated (containing 1 or 2 double bonds) monocyclic or polycyclic group containing 3 to 20 carbon atoms. The "monocyclic cycloalkyl" is preferably a 3- to 10-membered monocycloalkyl group, more preferably a 3- to 8-membered monocycloalkyl group, such as cyclopropyl, cyclobutyl, cyclopentyl, cyclohexyl, cycloheptyl, cyclooctyl, cyclodecyl, cyclododecyl, and cyclohexenyl. The "polycyclic cycloalkyl" includes "bridged cycloalkyl", "fused cycloalkyl", and "spirocycloalkyl". Representative examples of "bridged cycloalkyl" include, but are not limited to, bornyl, bicyclo[2.2.1]heptenyl, bicyclo[3.1.1]heptyl, bicyclo[2.2.1]heptyl, bicyclo[2.2.2]octyl, bicyclo[3.2.2]nonyl, bicyclo[3.3.1]nonyl, bicyclo[4.2.1]nonyl, adamantyl, etc. The "fused cycloalkyl" includes a cycloalkyl ring fused to phenyl, cycloalkyl, or heteroaryl. The fused cycloalkyl includes, but is not limited to: benzocyclobutenyl, 2,3-dihydroindenyl, decahydronaphthalenyl, etc. Monocyclic cycloalkyl or polycyclic cycloalkyl can be attached to the parent molecule via any carbon atom on the ring.

The term "heterocycloalkyl" means a saturated or partially unsaturated (containing 1 or 2 double bonds) non-aromatic cyclic group consisting of a carbon atom and a heteroatom selected from nitrogen, oxygen, sulfur, boron, etc., and/or a sulfur-containing heteroatom group, which can be monocyclic or polycyclic, wherein the sulfur-containing heteroatom group is selected from, but is not limited to, S(O), $S(O)_2$, and S(O)(NH). In the present disclosure, the number of the heteroatoms and/or the heteroatom groups in the heterocycloalkyl is preferably 1, 2, 3, or 4, and the boron, nitrogen, or carbon atom in the heterocycloalkyl can be optionally oxidized. The nitrogen atom can optionally be further substituted by other groups to form tertiary amines or quaternary ammonium salts. The "monocyclic heterocycloalkyl" is preferably a 3- to 10-membered monocyclic heterocycloalkyl group, and more preferably a 3-to 8-membered monocyclic heterocycloalkyl group. For example: pyrrolidinyl, dihydropyrrolidinyl, dihydroimidazolyl, dihydropyrazolyl, tetrahydrofuranyl, tetrahydropyrazinyl, dihydrofuranyl, tetrahydrothiophenyl, tetrahydropyranyl, dihydropyranyl, tetrahydrothiopyranyl, tetrahydropyridinyl, tetrahydropyrimidinyl, piperidinyl, aziridinyl, morpholinyl, thiomorpholinyl, thiomorpholin-S-oxide-4-yl, piperidinyl, piperazinyl, 1,4-dioxanyl, homopiperazinyl, 1-imino-1-oxo-tetrahydro-2*H*-thiopyranyl, 1,1-dioxo-tetrahydrothiophenyl, 1-imino-1-oxo-tetrahydrothiophenyl, 1,1-dioxo-3,4-dihydro-2*H*-thiopyranyl, 1-imino-1-oxo-3,4-dihydro-2*H*-thiopyranyl, 1,1-dioxo-2,3-dihydrothiophenyl, 1-imino-1-oxo-2,3-dihydrothiophenyl, etc. The "polycyclic heterocycloalkyl" includes "fused heterocycloalkyl", "spiroheterocyclyl", and "bridged heterocycloalkyl". The "fused heterocycloalkyl" includes a monocyclic heterocycloalkyl ring fused to phenyl, cycloalkyl, heterocycloalkyl, or heteroaryl. The fused heterocycloalkyl includes, but is not limited to: 2,3-dihydrobenzofuranyl, 1,3-dihydroisobenzofuranyl, dihydroindolyl, 2,3-dihydrobenzo[*b*]thiophenyl, dihydrobenzopyranyl, 1,2,3,4-tetrahydroquinolyl, 1,2,3,4-tetrahydroisoquinolyl, 1,2,3,4-tetrahydronaphthyridinyl, 5,6,7,8-tetrahydronaphthyridinyl, 1-hydroxy-1,3-dihydrobenzo[c][1,2]oxaborolanyl, etc. The monocyclic heterocycloalkyl and polycyclic heterocycloalkyl can be attached to the parent molecule via any ring atom on the ring. The ring atoms specifically means the carbon atom and/or nitrogen atom constituting the ring skeleton.

The term "cycloalkylalkyl" means the cycloalkyl is attached to the parent structure via an alkyl group. Thus, "cycloalkylalkyl" includes the above definitions of alkyl and cycloalkyl.

The term "heterocycloalkylalkyl" means the heterocycloalkyl is attached to the parent structure via an alkyl group. Thus, "heterocycloalkylalkyl" includes the above definitions of alkyl and heterocycloalkyl.

The term "alkoxy" means a cyclic or non-cyclic alkyl group having the stated number of the carbon atoms attached via an oxygen bridge, including alkyloxy, cycloalkyloxy, and heterocycloalkyloxy. Thus, "alkoxy" includes the above definitions of alkyl, heterocycloalkyl, and cycloalkyl.

The term "hydroxyalkyl" means any hydrogen atom on the alkyl group is substituted by a hydroxyl group, including but not limited to: $-CH_2OH$, $-CH_2CH_2OH$, $- CH_2CH_2C(CH_3)_2OH$.

The term "heterocycloalkenyl" means a cyclic, unsaturated monovalent hydrocarbon group having a specified number of ring atoms (e.g., 5- to 10-membered), a specified number of heteroatoms (e.g., 1, 2, or 3), and a specified type of heteroatoms (one or more than one of N, O, and S), having one or more than one (e.g., 1, 2, or 3) carbon-carbon $sp^2$ double bond, which is monocyclic, and which is not aromatic. The heterocycloalkenyl is attached to the rest of the molecule via a carbon atom or a heteroatom. The heterocycloalkenyl includes, but is not limited to:

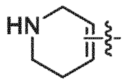, 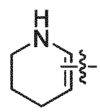, 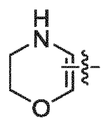, and 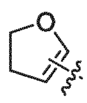.

The term "heterocycloalkene" meets any one of the following conditions and is otherwise defined as the term "heterocycloalkenyl": it shares two atoms and one bond with the rest of the molecule.

The term "aryl" means any stable 6- to 20-membered monocyclic or polycyclic aromatic group, such as phenyl, naphthyl, tetrahydronaphthyl, 2,3-dihydro-1*H*-indenyl, or biphenyl.

The term "heteroaryl" means an aromatic ring group formed by replacing at least one carbon atom on the ring by a heteroatom selected from nitrogen, oxygen, or sulfur. It can be a 5- to 7-membered monocyclic structure or a 7- to 20-membered fused ring structure, preferably a 5- to 6-membered heteroaryl group. In the present disclosure, the number of the heteroatoms is preferably 1, 2, or 3, including: pyridinyl, pyridonyl, pyrimidinyl, pyrimidine-2,4(1*H*,3*H*)-dione, pyrimidonyl, piperazinyl, pyridazinonyl, furanyl, thiophenyl, thiazolyl, pyrrolyl, imidazolyl, pyrazolyl, oxazolyl, isoxazolyl, 1,2,5-oxadiazolyl, 1,2,4-oxadiazolyl, 1,3,4-oxadiazolyl, 1,3,4-thiadiazolyl, 1,2,4-triazolyl, 1,2,3-triazolyl, tetrazolyl, indazolyl, isoindazolyl, indolyl, isoindolyl, benzofuranyl, benzothiophenyl, benzo[*d*][1,3]dioxolanyl, benzothiazolyl, benzoxazolyl, quinolinyl, isoquinolinyl, isoquinolinonyl, quinazolinyl, 4-hydroxythieno[3,2-c]pyridinyl, 4,5-dihydro-4-oxofuro[3,2]pyridinyl, 4-hydroxy-5-azaindolyl, furo[2,3-*c*]pyridin-7(6*H*)-one, thieno[2,3-c]pyridin-7(6*H*)-one, etc.

The term "fused ring group" refers to a fused ring structure formed by two, three, or four cyclic structures sharing two adjacent atoms with each other. The fused ring group can further include a spiro ring or a bridge ring. In the present disclosure, the fused ring group is a saturated, unsaturated, or partially saturated fused ring structure, and preferably at least one ring is an aromatic ring. More preferably, bicyclic or tricyclic fused ring groups, and at least one ring is an aromatic ring. In the present disclosure, the fused ring group is an 8- to 20-membered fused ring group, preferably an 8- to 12-membered fused ring group, and more preferably an 8- to 10-membered fused ring group. Specific examples of the fused ring group include, but are not limited to: benzocyclobutenyl, 2,3-dihydro-1*H*-indenyl, 1,2,3,4-tetrahydronaphthyl, 6,7,8,9-tetrahydro-5*H*-benzo[7]annulenyl, 6,9-dihydro-5*H*-benzo[7]annulenyl, 5,6,7,8,9,10-hexahydrobenzo[8]annulenyl, 2,3-cyclopentapyridinyl, 5,6-dihydro-4*H*-cyclopenta[*b*]thienyl, 5,6-dihydro-4*H*-cyclopenta[*b*]furanyl, 2,3-dihydrobenzofuranyl, 1,3-dihydroisobenzofuranyl, dihydroindolyl, 2,3-dihydrobenzo[*b*]thienyl, dihydrobenzopyranyl, 1,2,3,4-tetrahydroquinolyl, 2,3-dihydro-1,4-benzodioxanyl, 3,4-dihydro-2*H*-1,4-benzoxazinyl, naphthyridinyl, naphthyl, benzofuranyl, benzothiophenyl, benzopyrrolyl, benzothiazolyl, benzooxazolyl, indazolyl, benzopyridazinyl, benzimidazolyl, indolyl, quinolyl, isoquinolyl, purinyl, pteridinyl,

EP 4 342 884 A1

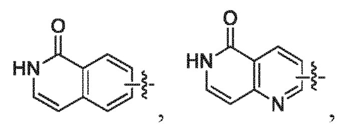

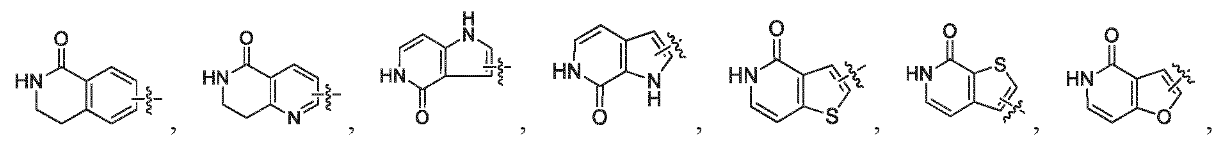

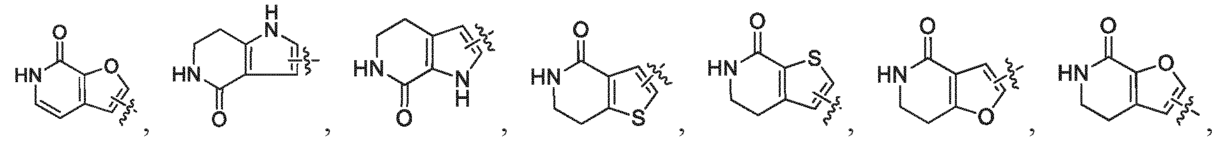

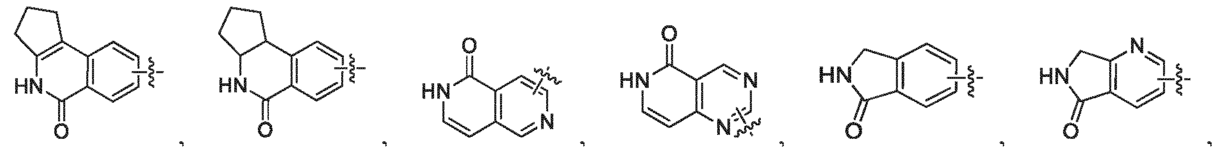

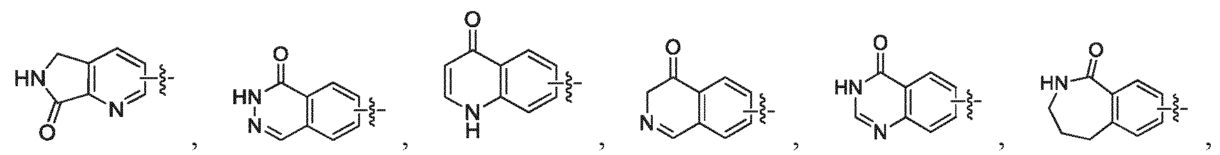

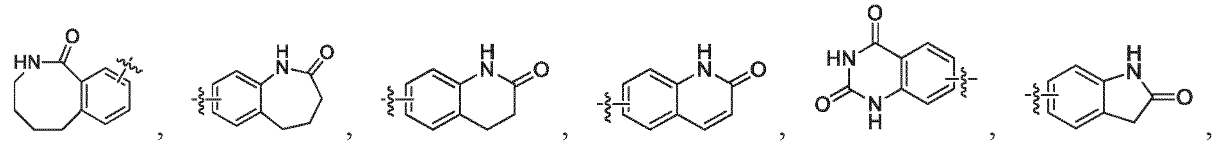

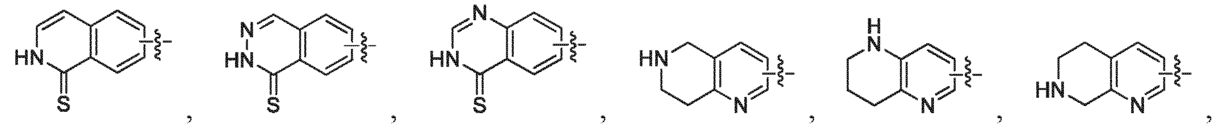

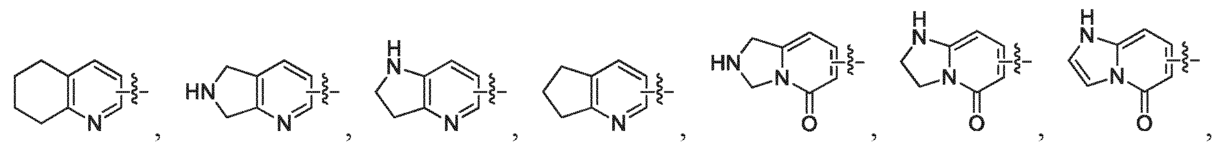

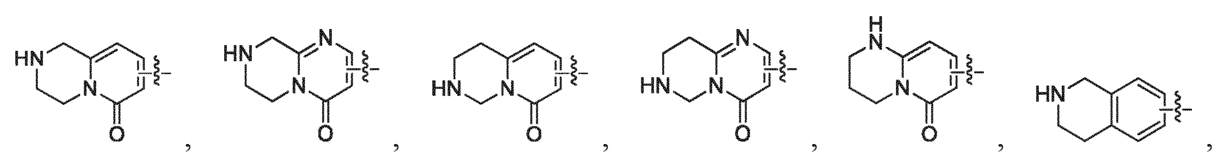

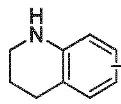, 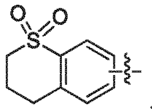, 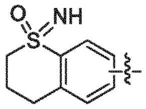, 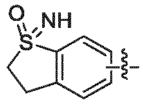, 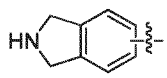, 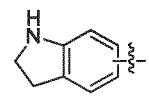, 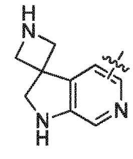,

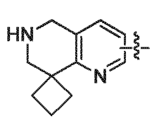, 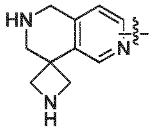, 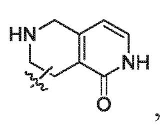, 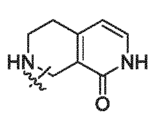, or 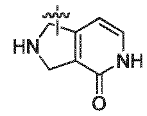;

the fused ring group can be attached to the parent molecule via a carbon atom on the ring, preferably via a carbon atom on an aromatic ring. The fused ring group can be unsubstituted or optionally substituted at any position by one or more than one substituent.

The term "arylalkyl" means the aryl group is attached to the parent structure by an alkyl group. Thus, "arylalkyl" includes the above definitions of alkyl and aryl.

The term "heteroarylalkyl" means the heterocycloalkyl group is attached to the parent structure via an alkyl group. Thus, "heteroarylalkyl" includes the above definitions of alkyl and heteroaryl.

The term "halogen" means fluorine, chlorine, bromine, or iodine.

The term "haloalkyl" means an alkyl group optionally substituted by halogen. Thus, "haloalkyl" includes the above definitions of halogen and alkyl.

The term "haloalkoxy" means an alkoxy group optionally substituted by halogen. Thus, "haloalkoxy" includes the above definitions of halogen and alkoxy.

The term "amino" means $-NH_2$, and the term "alkylamino" means that at least one hydrogen atom on the amino group is substituted by an alkyl group, including but not limited to: $-NHCH_3$, $-N(CH_3)_2$, $-NHCH_2CH_3$, and $-N(CH_2CH_3)_2$. Thus, "alkylamino" includes the above definitions of alkyl and amino.

The term "nitro" means $-NO_2$.

The term "cyano" means -CN.

The term "oxo" means =O.

In the present disclosure, the abbreviations of amino acids are conventional abbreviations, such as: alanine (Ala), arginine (Arg), aspartic acid (Asp), cysteine (Cys), asparagine (Asn), Glutamic acid (Glu), glycine (Gly), histidine (His), isoleucine (Ile), leucine (Leu), lysine (Lys), phenylalanine (Phe), serine (Ser), tryptophan (Trp), citrulline (Cit), valine (Val), and proline (Pro).

The "room temperature" in the present disclosure means 15 to 30°C.

In the substituents R, $R_1$, $R_2$, $R_3$, $R_4$, $R_{4'}$, $R_5$, $R_6$, $R_7$, $R_8$, $R_{8'}$, $R_9$, $R_{10}$, $R_{11}$, $R_{12a}$, $R_{12b}$, $R_{12c}$, $R_{13a}$, $R_{13b}$, $R_a$, $R_b$, $R_d$, $R_e$, $R_{e'}$, $R_f$, $R_{f'}$, and $L_{1-5}$ of the "immune-stimulating antibody conjugate", "compound", and "pharmaceutically acceptable salt" of the present disclosure, if there are tautomers, they can present in the form of a single tautomer or a mixture of them, preferably in the form dominated by the more stable tautomer.

The "pharmaceutically acceptable salt" of the present disclosure is discussed in Berge, et al., "Pharmaceutically acceptable salts", J. Pharm. Sci., 66, 1-19 (1977), and it is obvious to the medicinal chemist that the salts are essentially non-toxic and can provide the desired pharmacokinetic properties, palatability, absorption, distribution, metabolism, or excretion, etc. The compounds of the present disclosure can have an acidic group, a basic group, or an amphoteric group. Typical pharmaceutically acceptable salts include salts prepared by reacting the compounds of the present disclosure with acids.

The term "solvate" means a substance formed by combining a compound with a solvent, which includes, but is not limited to: water, methanol, ethanol, etc. Solvates are divided into stoichiometric solvates and non-stoichiometric solvates. Solvates include, but are not limited to: monohydrate.

The term "solvate of pharmaceutically acceptable salt" means a substance formed by combining a compound with a pharmaceutically acceptable acid or base, and a solvent, which includes, but is not limited to: water, methanol, ethanol, etc. The amount of solvent can be stoichiometric or non-stoichiometric. The solvate of the pharmaceutically acceptable salt include, but is not limited to: monohydrochloride monohydrate.

On the basis of not violating common knowledge in the art, the above-mentioned preferred conditions can be combined arbitrarily to obtain preferred examples of the present disclosure.

The reagents and raw materials used in the present disclosure are all commercially available.

The positive and progressive effect of the present disclosure is that the nitrogen-containing compound has a good regulatory effect on TLR8 and can effectively treat, alleviate, and/or prevent various related diseases caused by

immunosuppression, such as cancer or viral infection.

BRIEF DESCRIPTION OF THE DRAWINGS

Figure 1 shows a graph of the anti-tumor effects of the vehicle, the immune-stimulating antibody conjugate of the present disclosure, and the positive control in a subcutaneous xenograft tumor model in mice of MC38-HER2 mouse colon cancer cells, which shows that the anti-tumor effect of the immune-stimulating antibody conjugate of the present disclosure is significantly better than that of the positive control.

DETAILED DESCRIPTION OF THE PREFERRED EMBODIMENT

The present disclosure is further illustrated below by means of examples, but the present disclosure is not limited to the scope of the examples. The experimental methods that haven't illustrated specific conditions in the following examples are selected according to conventional methods and conditions, or according to the product instructions.
The structures of all compounds of the present disclosure can be characterized by nuclear magnetic resonance ($^1$H NMR) and/or mass spectrometric detection (MS).
$^1$H NMR chemical shifts ($\delta$) were recorded in ppm ($10^{-6}$). NMR was performed with a Bruker AVANCE-400 spectrometer. Suitable solvents were deuterated chloroform ($CDCl_3$), deuterated methanol ($CD_3OD$), deuterated dimethyl sulfoxide (DMSO-$d_6$), heavy water ($D_2O$), with tetramethylsilane as an internal standard (TMS).
Liquid chromatography-mass spectrometry (LCMS) was measured by Agilent 1200HPLC/6120 mass spectrometer, using a chromatographic column: Xtimate C18, 3.0 $\times$ 50 mm, 3 $\mu$m, with a column temperature of 40°C; or by Thermo UltiMate 3000HPLC/MSQ PLUS mass spectrometer using a chromatographic column of Xbridge C18, 3.0 $\times$ 50 mm, 3.5 $\mu$m, with a column temperature of 30°C. Agilent gradient elution condition I: 95% to 5% solvent $A_1$ and 5% to 95% solvent $B_1$ (0 to 2.0 minutes), then 95% solvent $B_1$ and 5% solvent $A_1$ (hold for 1.1 minutes), the percentage was the volume percentage of a specific solvent in the total solvent volume. Solvent $A_1$: 0.01% trifluoroacetic acid (TFA) aqueous solution; solvent $B_1$: 0.01% acetonitrile solution of trifluoroacetic acid; the percentage was the volume percentage of the solute in the solution. Thermo gradient elution condition II: 95% to 5% solvent $A_2$ and 5% to 95% solvent $B_2$ (0 to 2 minutes), then 95% solvent $B_2$ and 5% solvent $A_2$ (hold for 1.8 minutes), the percentage was the volume percentage of a specific solvent in the total solvent volume. Solvent $A_2$: 10 mM ammonium bicarbonate aqueous solution; solvent $B_2$: acetonitrile.
Preparative high performance liquid chromatography (*prep*-HPLC) was performed on Gilson GX-281 preparative liquid chromatography or Agela FLEXA-HP preparative liquid chromatography. The chromatographic column was Xtimate 21.2 * 250 mm, 10 $\mu$m. Separation condition 1: mobile phase A: 0.05% hydrochloric acid aqueous solution, mobile phase B: acetonitrile; separation condition 2: mobile phase A: 10 mmol/L ammonium bicarbonate aqueous solution, mobile phase B: acetonitrile; separation condition 3: mobile phase A: 0.1 % trifluoroacetic acid aqueous solution, mobile phase B: acetonitrile. Detection wavelength: 214 nm & 254 nm; flow rate: 15.0 mL/min.
Flash column chromatography (flash system/CheetahTM) was performed on Agela Technologies MP200, and the accompanying normal phase separation column was Flash columm Silica-CS (25 g, 40 g, 80 g, 120 g, or 330 g) from Tianjin Bonna-Agela. The elution system was ethyl acetate/petroleum ether, or dichloromethane/methanol; the reverse phase separation column was C18 reverse phase column (Spherical C18, 40 to 75 $\mu$m, 100 Å, SepaFlash® model: SW-040), and the elution system was 10 mM ammonium bicarbonate aqueous solution/acetonitrile or 0.05% trifluoroacetic acid aqueous solution/acetonitrile.
The abbreviations used in the examples of the present disclosure have the following meanings:
$(Boc)_2O$: di-*tert*-butyl dicarbonate; BINAP: 2,2'-bis(diphenylphosphino)-1,1'-binaphthyl; DMF: *N,N*-dimethylformamide; DMSO: dimethyl sulfoxide; DIPEA: *N,N*-diisopropylethylamine; EDCI: 1-ethyl-3-(3-dimethylaminopropyl)carbodiimide hydrochloride; HATU: 2-(7-azabenzotriazol-1-yl)-*N,N,N',N'*-tetramethyluronium hexafluorophosphate; LiHMDS: lithium bis(trimethylsilyl)amide; $PdCl_2dppf$ $CH_2Cl_2$: [1,1'-bis(diphenylphosphino)ferrocene]dichloropalladium(II) dichloromethane complex; $Pd_2(dba)_3$: tris(dibenzylideneacetone)dipalladium; $Pd(PPh_3)_4$: tetrakis(triphenylphosphine)palladium; TBSCl: *tert*-butyl(chloro)dimethylsilane; Xantphos: 4,5-bis(diphenylphosphino)-9,9-dimethylxanthene; Xphos: 2-dicyclohexylphosphino-2',4',6'-triisopropylbiphenyl.

**Synthesis of intermediates:**

**Synthesis of linker-1**

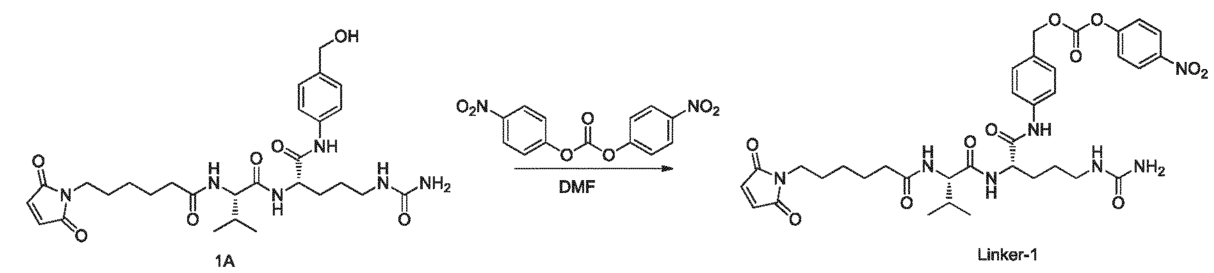


To a solution of compound 1A(150 mg, 0.26 mmol) in DMF (4 mL) were sequentially added bis(4-nitrophenyl) carbonate (158 mg, 0.52 mmol) and DIPEA (101 mg, 0.78 mmol), and the reaction mixture was stirred at room temperature for overnight. The reaction mixture was filtered and concentrated under reduced pressure, and the residue was purified by flash column chromatography (10 mM ammonium bicarbonate aqueous solution/acetonitrile = 0% to 65%) to obtain linker-1 (125 mg) as a pale yellow solid. m/z: $[M+H]^+$738.1.

**Synthesis of linker-2**

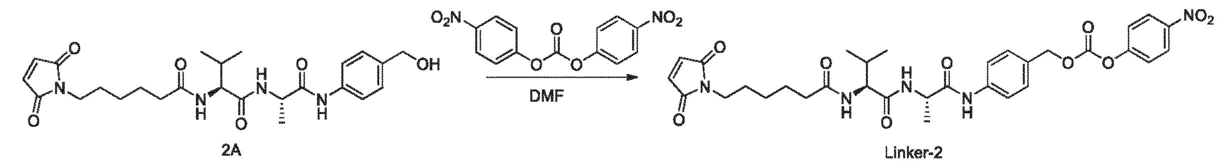


To a solution of compound 2A(120 mg, 0.25 mmol) in DMF (3 mL) were sequentially added bis(4-nitrophenyl) carbonate (152 mg, 0.50 mmol) and DIPEA (97 mg, 0.75 mmol), and the reaction mixture was stirred at room temperature for overnight. The reaction mixture was filtered and concentrated under reduced pressure, and the residue was purified by flash column chromatography (10 mM ammonium bicarbonate aqueous solution/acetonitrile = 0% to 85%) to obtain linker-2 (115 mg) as a pale yellow solid. m/z: $[M+H]^+$652.1.

**Synthesis of linker-3**

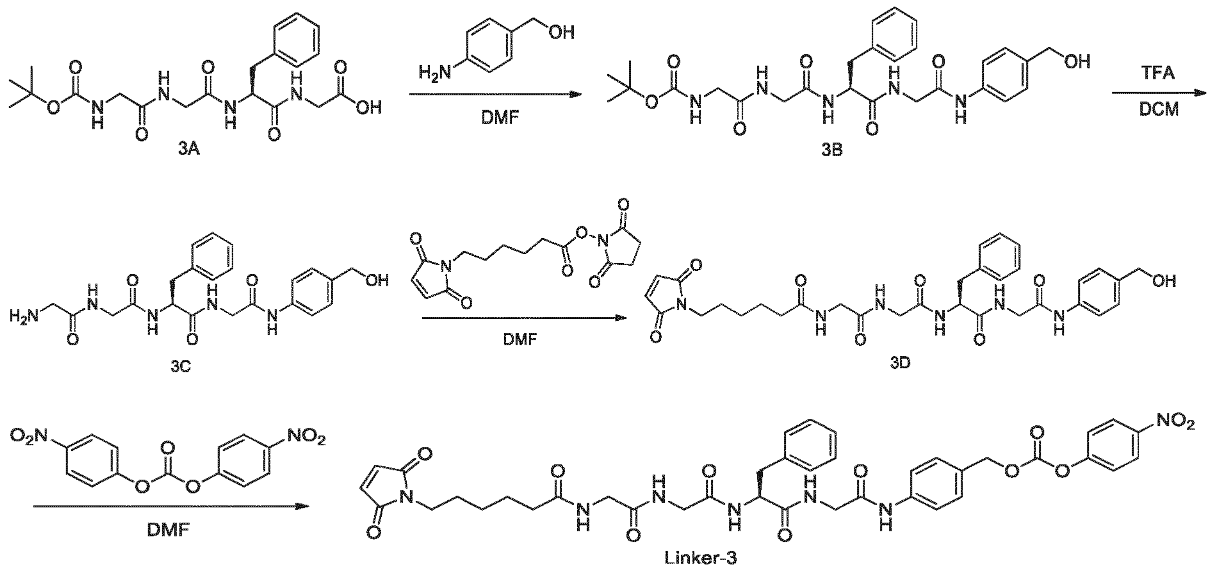


Step 1: To a solution of compound 3A (436 mg, 1.0 mmol) in DMF (5 mL) were sequenti ally added 2-(7-azabenzotriazol-1-yl)-*N,N,N,N'*-tetramethyluronium hexafluorophosphate (456 mg, 1.2 mmol) and DIPEA (258 mg, 2.0 mmol), and the reaction system was stirred at room temperature for 5 minutes, and then (4-aminophenyl)methanol (135 mg, 1.1 mmol) was added thereto. The reaction system was stirred at room temperature for 16 hours and then directly purified by flash column chromatography (10 mM ammonium bicarbonate aqueous solution/acetonitrile = 5% to 75%) to obtain compound 3B (350 mg) as a white solid. m/z: $[M+H]^+$542.1.

Step 2: To a solution of compound 3B (350 mg, 0.65 mmol) in dichloromethane (3 mL) was added dropwise trifluoroacetic acid (2 mL) under an ice bath condition. The reaction mixture was slowly warmed to room temperature, stirred for 2 hours, concentrated under reduced pressure to remove most of the solvent, and directly purified by flash column chromatography (10 mM ammonium bicarbonate aqueous solution/acetonitrile = 5% to 85%) to obtain compound 3C (270 mg) as a white solid. m/z:$[M+H]^+$442.0.

Step 3: To a solution of compound 3C (260 mg, 0.59 mmol) in DMF (2 mL) were added dropwise DIPEA(152 mg, 1.18 mmol) and *N*-succinimidyl 6-maleimidohexanoate (200 mg, 0.65 mmol). The reaction mixture was stirred at room temperature for 2 hours and directly purified by flash column chromatography (10 mM ammonium bicarbonate aqueous solution/acetonitrile = 5% to 50%) to obtain compound 3D (300 mg) as a white solid. m/z:$[M+H]^+$635.3.

Step 4: To a solution of compound 3D (280 mg, 0.44 mmol) in DMF (3 mL) were added dropwise DIPEA (170 mg, 1.32 mmol) and bis(4-nitrophenyl) carbonate (240 mg, 0.79 mmol). The reaction mixture was stirred at room temperature for 2 hours and then directly purified by flash column chromatography (10 mM ammonium bicarbonate aqueous solution/acetonitrile = 0% to 60%) to obtain linker-3 (290 mg) as a white solid. m/z:$[M+H]^+$800.1.

**Synthesis of 8-((*tert*-butoxycarbonyl)amino)-2-chloro-7*H*-pyrido[2,3-*b*]azepine-6-carboxylic acid (intermediate 1.7)**

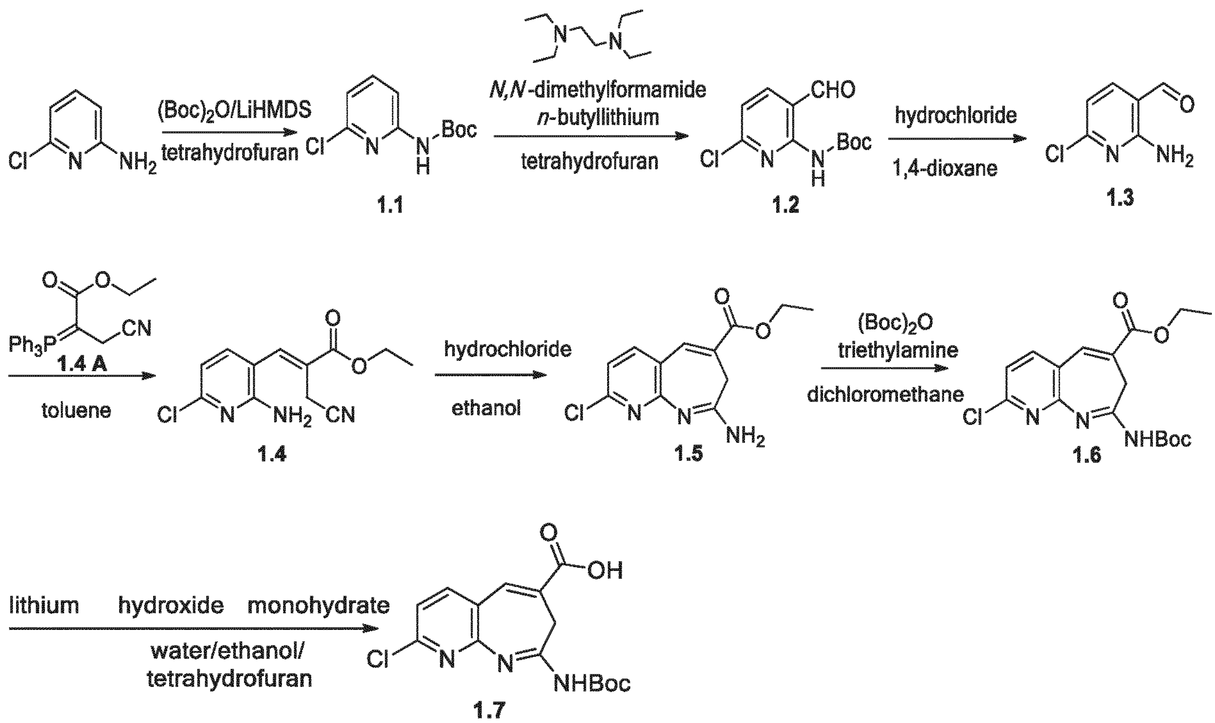


Step 1: To a solution of 2-amino-6-chloropyridine (40 g, 311 mmol) and LiHMDS (685 mL, 685 mmol, 1 M tetrahydrofuran solution) in tetrahydrofuran (400 mL) was added dropwise $(Boc)_2O$ (74.7 g, 342 mmol) under an ice bath condition. The reaction system was stirred at room temperature for overnight, then concentrated, and diluted with ethyl acetate (400 mL). The organic phase was washed with hydrochloric acid (1 M), saturated sodium bicarbonate aqueous solution, and saturated brine respectively, separated, dried over sodium sulfate, and filtered. The filtrate was concentrated under reduced pressure, then the residue was recrystallized with ethanol and filtered, and the filter cake was dried under vacuum to obtain intermediate 1.1 (39.5 g, yield: 56%) as a yellow solid.

Step 2: To a solution of intermediate 1.1 (39.5 g, 173 mmol) and *N,N,N',N'*-tetramethylethylenediamine (74.4 g, 432 mmol) in tetrahydrofuran (400 mL) was added dropwise *n*-butyllithium (173 mL, 432 mmol, 2.5 M hexane solution) under nitrogen atmosphere at -78°C. After the addition was completed, the reaction system was slowly warmed to -10°C

and stirred at this temperature for 2 hours. The reaction system was cooled to -78°C again, and DMF (25.3 g, 347 mmol) was added thereto. After the addition was completed, the reaction system was slowly warmed to room temperature and stirred for overnight. The reaction was quenched with hydrochloric acid (1 M) at -10°C, and the pH was adjusted to 2 to 3. The aqueous phase was extracted with ethyl acetate, and the organic phase was washed with water and saturated brine respectively, separated, dried over anhydrous sodium sulfate, and filtered. The filtrate was concentrated under reduced pressure to obtain intermediate 1.2 (32.9 g, yield: 74%) as a yellow solid.

Step 3: To a solution of hydrogen chloride in 1,4-dioxane (300 mL, 4 M) was added intermediate 1.2 (32.9 g, 128 mmol), and the reaction mixture was stirred at room temperature for overnight. The reaction mixture was concentrated under reduced pressure, and the pH of the residue was adjusted to 7 to 8 with saturated sodium bicarbonate aqueous solution. The aqueous phase was extracted with ethyl acetate, and the organic phases were combined, washed with water and saturated brine respectively, separated, dried over anhydrous sodium sulfate, and filtered. The filtrate was concentrated under reduced pressure to obtain intermediate 1.3 (18.9 g, yield: 94%) as a yellow solid. m/z: $[M+H]^+$157.0.

Step 4: A solution of intermediate 1.3 (18.9 g, 121 mmol) and compound 1.4A (46.8 g, 121 mmol) in toluene (400 mL) was stirred under reflux for overnight. The solvent was removed by concentration under reduced pressure, and the residue was recrystallized with ethanol (150 mL) and filtered. The filter cake was dried under vacuum to obtain intermediate 1.4 (13.1 g, yield: 41%) as a yellow solid. m/z: $[M+H]^+$266.0.

Step 5: Intermediate 1.4 (6.6 g, 24.9 mmol) was stirred in a solution of hydrogen chloride in ethanol (80 mL, 2 M) at 30°C for two days. The solvent was removed by concentration under reduced pressure, and the pH of the residue was adjusted to 7 to 8 with saturated sodium bicarbonate aqueous solution. The aqueous phase was extracted with a mixed solvent of dichloromethane and methanol (10/1), and the organic phase was washed with water and saturated brine respectively, separated, dried over anhydrous sodium sulfate, and filtered. The filtrate was concentrated under reduced pressure to obtain intermediate 1.5 (6.5 g, yield: 98%) as a yellow solid. m/z: $[M+H]^+$266.0; $^1H$ NMR (400MHz, DMSO-$d_6$): δ 7.90 (d, $J$ = 8.0 Hz, 1H), 7.75 (s, 1 H), 7.39 (br. s, 2H), 7.02 (d, $J$ = 8.0 Hz, 1H), 4.24 (q, $J$ = 7.6 Hz, 2H), 2.98 (s, 2H), 1.29 (t, $J$ = 7.0 Hz, 3H).

Step 6: To a solution of intermediate 1.5 (6.5 g, 24.5 mmol) in dichloromethane (130 mL) was added $(Boc)_2O$ (13.4 g, 61.2 mmol). The reaction system was stirred at room temperature for 5 days and then diluted with dichloromethane (100 mL). The organic phase was washed with citric acid aqueous solution (2 M), saturated sodium bicarbonate aqueous solution, and saturated brine respectively. The organic phase was separated, dried over anhydrous sodium sulfate, and filtered. The filtrate was concentrated under reduced pressure, and the residue was purified by flash column chromatography (petroleum ether/ethyl acetate = 100 to 1/1) to obtain intermediate 1.6 (8.6 g, yield: 78%) as a yellow solid.

Step 7: To a mixed solution of intermediate 1.6 (4 g, 10.9 mmol) in tetrahydrofuran (21 mL), ethanol (14 mL), and water (7 mL) was added lithium hydroxide monohydrate (1.38 g, 32.8 mmol). The reaction system was stirred at room temperature for overnight and then poured into water. Then the mixture was added with citric acid aqueous solution (2 M) to adjust the pH to 4 and filtered. The filter cake was washed with water and methyl *tert*-butyl ether respectively, and the organic phase was concentrated under reduced pressure to obtain intermediate 1.7 (2.97 g, yield: 80%) as an earthy yellow solid.

**Synthesis of 8-amino-2-chloro-7*H*-pyrido[2,3-*b*]azepine-6-carboxilic acid (intermediate 1.8)**

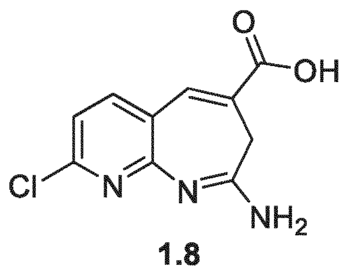

**1.8**

Intermediate 1.8 was obtained by reacting intermediate 1.5 using the synthesis method of intermediate 1.7 in step 7.

**Synthesis of** 8-bromo-2-((*tert*-butoxycarbonyl)amino)-3*H*-benzo[*b*]azepine-4-**carboxylic acid (intermediate 2.6)**

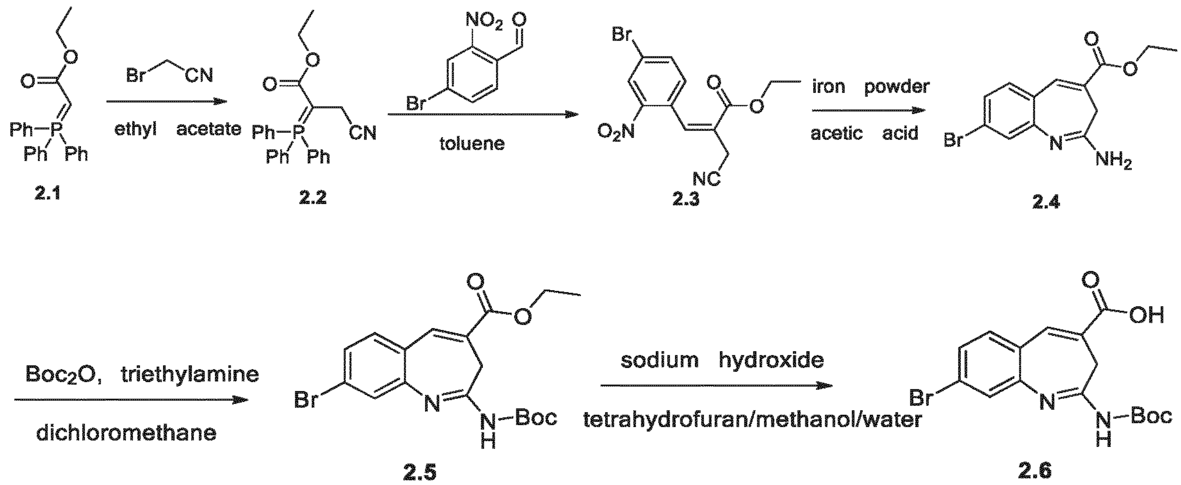


2.1 2.2 2.3 2.4 2.5 2.6

Step 1: A solution of intermediate 2.1 (20 g, 57.4 mmol) and bromoacetonitrile (6.9 g, 57.4 mmol) in ethyl acetate (200 mL) was stirred under reflux for 3 hours and filtered to remove the solid, and the filter cake was washed with ethyl acetate. The filtrate was concentrated under reduced pressure to obtain intermediate 2.2 (17 g, yield: 76%) as a pale yellow oil.

Step 2: A solution of 4-bromo-2-nitrobenzaldehyde (10 g, 43.9 mmol) and intermediate 2.2 (17 g, 43.9 mmol) in toluene (170 mL) was stirred under reflux for 2 hours. The mixture was cooled to room temperature, then filtered using a short silica gel column, eluted with a 25% ethyl acetate-petroleum ether solution, and concentrated under reduced pressure to remove most of the eluent. The rest of the solution was placed at -18°C for 16 hours. The solid was precipitated, then the mixture was filtered, and the filter cake was dried under vacuum to obtain intermediate 2.3 (8.2 g, yield: 55%) as an off-white solid.

Step 3: A solution of intermediate 2.3 (4.2 g, 12.4 mmol) in acetic acid (80 mL) was heated to 80°C and added with iron powder (4.1 g, 74.3 mmol) in batches within 15 minutes. The internal temperature was maintained at no more than 90°C. After the addition was completed, the reaction mixture was stirred for 3 hours. The reaction system was cooled to room temperature, filtered through diatomite, and rinsed with ethyl acetate. The filtrate was concentrated under reduced pressure, and the residue was diluted with cold water and adjusted to pH > 8 with saturated sodium bicarbonate aqueous solution. The aqueous phase was extracted with ethyl acetate, and the organic phases were combined, washed with saturated brine, separated, dried over anhydrous sodium sulfate, and filtered. The filtrate was concentrated under reduced pressure, and the residue was triturated with a 10% ethyl acetate-petroleum ether solution and filtered, and the filter cake was dried under vacuum to obtain intermediate 2.4 (3 g, yield: 78%) as a yellow solid.

Step 4: To a solution of intermediate 2.4 (3 g, 9.7 mmol) and triethylamine (1.47 g, 14.6 mmol) in dichloromethane (50 mL) was added $(BOC)_2O$ (3.2 g, 14.6 mmol). The reaction system was stirred at room temperature for 2 days and then diluted with dichloromethane. The organic phase was washed with hydrochloric acid (3 M), saturated sodium bicarbonate aqueous solution, and saturated brine respectively, separated, dried over sodium sulfate, and filtered. The filtrate was concentrated under reduced pressure, then the residue was triturated with a 10% ethyl acetate-petroleum ether solution and filtered, and the filter cake was dried under vacuum to obtain intermediate 2.5 (1.6 g, yield: 40%) as an off-white solid.

Step 5: To a solution of intermediate 2.5 (1.6 g, 3.91 mmol) in tetrahydrofuran (50 mL) was added sodium hydroxide aqueous solution (1.0 M, 5.9 mL) under an ice bath condition. The reaction system was stirred at room temperature for 16 hours, and then the pH of the reaction system was adjusted to 6 with hydrochloric acid (0.5 M). The aqueous phase was extracted with ethyl acetate, and the organic phases were combined, washed with saturated brine, separated, dried over anhydrous sodium sulfate, and filtered. The filtrate was concentrated under reduced pressure to obtain intermediate 2.6 (1.1 g, yield: 74%) as a pale yellow foamy solid.

**Synthesis of 8-bromo-2-((*tert*-butoxycarbonyl)amino)-6-fluoro-3*H*-benzo[b]azepine-4-carboxylic acid (intermediate 3.1)**

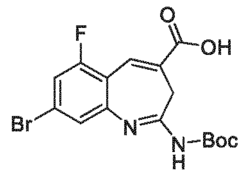

**3.1**

Intermediate 3.1 was obtained by replacing 4-bromo-2-nitrobenzaldehyde in step 2 with 4-bromo-2-fluoro-6-nitrobenzaldehyde using the synthesis method of intermediate 2.6.

**Synthesis of 2-amino-8-bromo-6-fluoro-3H-benzo[b]azepine-4-carboxylic acid (intermediate 3.2)**

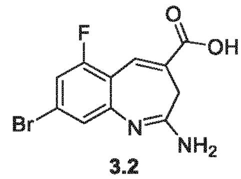

**3.2**

Intermediate 3.2 was obtained by reacting ethyl 2-amino-8-bromo-6-fluoro-3*H*-benzo[*b*]azepine-4-carboxylate using the synthesis method of intermediate 2.6 in step 5.

**Synthesis of 2-amino-8-bromo-3H-benzo[b]azepine-4-carboxylic acid (intermediate 4.1)**

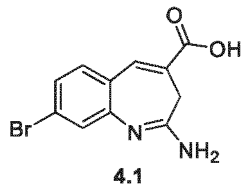

**4.1**

Intermediate 4.1 was obtained by reacting intermediate 2.4 using the synthesis method of intermediate 2.6 in step 5.

**Synthesis of *tert*-butyl (2-(propylamino)ethyl)carbamate (intermediate 7.1)**

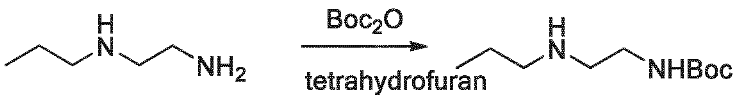


**7.1**

To a solution of *N*-propylethylenediamine (3.40 g, 33.3 mmol) in tetrahydrofuran (40 mL) was added dropwise a solution of di-tert-butyl dicarbonate (2.2 g, 10.1 mmol) in tetrahydrofuran (30 mL) under an ice bath condition. After the dropwise addition was completed in 30 minutes, the reaction system was stirred at room temperature for overnight. Then the reaction mixture was concentrated under reduced pressure, added with saturated brine (50 mL), and extracted with ethyl acetate (3 × 30 mL). The organic phases were combined, washed with saturated brine, dried over anhydrous sodium sulfate, filtered, and concentrated under reduced pressure. The residue was dissolved with *n*-pentane (30 mL) and placed in a refrigerator for overnight, and the precipitated solid was collected by filtration to obtain intermediate 7.1 (1.26 g, yield: 61%), as a colorless oil at room temperature. m/z:$[M+H]^+$ 203.2.

## Synthesis of compounds

**Example 1: Synthesis of 8-amino-*N*[6],*N*[6]-di-*n*-propyl-*N*[2]-(5,6,7,8-tetrahydro-1,6-naphthyridin-3-yl)-7*H*-pyrido[2,3-*b*]azepine-2,6-dicarboxamide (compound 1)**

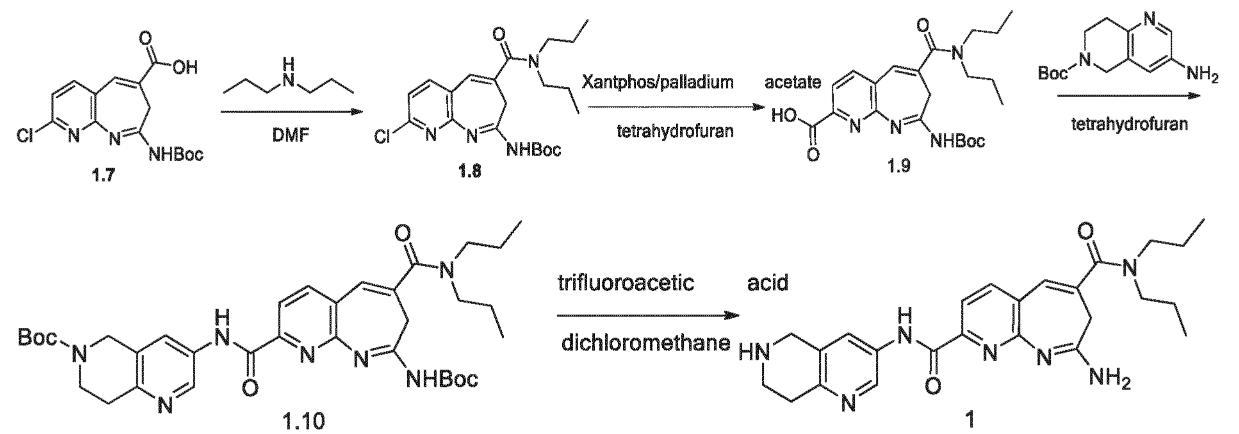


Step 1: To DMF (30 mL) were sequentially added compound 1.7 (2.97 g, 8.8 mmol), DIPEA (2.84 g, 22 mmol), di-*n*-propylamine (2.22 g, 22 mmol), and HATU (7.13 g, 22 mmol) at -70°C. After the addition was completed, the reaction system was stirred at 0°C for 20 minutes, and the reaction mixture was poured into water. The aqueous phase was extracted with ethyl acetate, and the organic phases were combined, washed with saturated brine, separated, dried over anhydrous sodium sulfate, and filtered. The filtrate was concentrated under reduced pressure. The residue was purified by flash column chromatography (petroleum ether/ethyl acetate = 1/1) to obtain compound 1.8 (1.2 g, yield: 32%) as a yellow oil. m/z: $[M+H]^+$421.0.

Step 2: A mixed solution of compound 1.8 (1 g, 2.38 mmol), Xantphos (69 mg, 0.12 mmol), palladium acetate (53 mg, 0.24 mmol), and tripotassium phosphate (1.51 g) in tetrahydrofuran (10 mL) and water (10 mL) was replaced with nitrogen and carbon monoxide three times, respectively, and then stirred at 70°C for 6 hours under carbon monoxide atmosphere. The reaction system was cooled to room temperature and filtered, and the filter cake was washed with methanol. The filtrate was concentrated under reduced pressure and directly purified by*prep*-HPLC (separation condition 2) to obtain compound 1.9 (220 mg, yield: 22%) as a brown solid. m/z: $[M+H]^+$ 431.2.

Step 3: To a solution of compound 1.9 (100 mg, 0.23 mmol) and *N*-methylmorpholine (28 mg, 0.28 mmol) in tetrahydrofuran (10 mL) was added isobutyl chloroformate (38 mg, 0.28 mmol) at -78°C, and the reaction mixture was stirred at -78°C for 1 hour. Then *tert*-butyl 3-amino-7,8-dihydro-1,6-naphthyridine-6(5*H*)-carboxylate (69 mg, 0.28 mmol) was added thereto, and the reaction system was stirred at this temperature for another 1 hour, then warmed to room temperature, and stirred again for another 1 hour. The reaction was quenched with water, and the aqueous phase was extracted with ethyl acetate. The organic phases were combined and concentrated under reduced pressure. The residue was purified by *prep*-TLC (dichloromethane/methanol = 10/1) to obtain compound 1.10 (100 mg, yield: 65%) as a brown oil. m/z: $[M+H]^+$ 662.4.

Step 4: A solution of compound 1.10 (100 mg, 0.15 mmol) and trifluoroacetic acid (1 mL) in dichloromethane (3 mL) was stirred at room temperature for 2 hours and directly concentrated under reduced pressure, and the residue was purified by *prep*-HPLC (separation condition 2) to obtain compound 1 (24.7 mg, yield: 35%) as a white solid. m/z: $[M+H]^+$ 462.3; $^1H$ NMR (400 MHz, DMSO-$d_6$): δ 10.49 (s, 1H), 8.73 (d, *J* = 2.4Hz, 1H), 8.07-7.89 (m, 2H), 7.69 (d, *J* = 7.6Hz, 1H), 7.25 (d, *J* = 21.2Hz, 2H), 6.86 (s, 1H), 3.87 (s, 2H), 3.34-3.25 (m, 5H), 3.03 (t, *J* = 6.0Hz, 2H), 2.86 (s, 2H), 2.75 (t, *J* = 6.0 Hz, 2H), 1.68-1.46 (m, 4H), 1.02-0.59 (m, 6H).

**Example 2: Synthesis of 8-amino-*N*[6]-(2-hydroxyethyl)-*N*[6]-*n*-propyl-*N*[2]-(5,6,7,8-tetrahydro-1,6-naphthyridin-3-yl)-7*H*-pyrido[2,3-b]azepine-2,6-dicarboxamide (compound 2)**

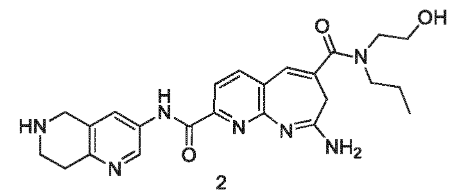

2

Compound 2 was obtained as a white solid by replacing di-*n*-propylamine in step 1 with 2-(propylamino)ethanol using the synthesis method of compound 1. m/z: $[M+H]^+$ 464.2; $^1H$ NMR (400 MHz, DMSO-$d_6$): δ 10.48 (s, 1H), 8.73 (d, *J* = 2.4 Hz, 1H), 8.02-7.89 (m, 2H), 7.69 (d, *J* = 8.0Hz, 1H), 7.24 (s, 2H), 6.90 (s, 1H), 4.82 (s, 1H), 3.86 (s, 2H), 3.63-3.50 (m, 3H), 3.44 (t, *J* = 6.0Hz, 3H), 3.02 (t, *J* = 6.0Hz, 3H), 2.85 (s, 2H), 2.74 (t, *J* = 6.0Hz, 2H), 1.64-1.51 (m, 2H), 0.93-0.77 (m, 3H).

**Example 3: Synthesis of 2-amino-*N,N*-di-*n*-propyl-8-(1-((5,6,7,8-tetrahydro-1,6-naphthyridin-3-yl)carbamoyl)cyclopropyl)-3*H*-benzo[*b*]azepine-4-carboxamide (compound 3)**

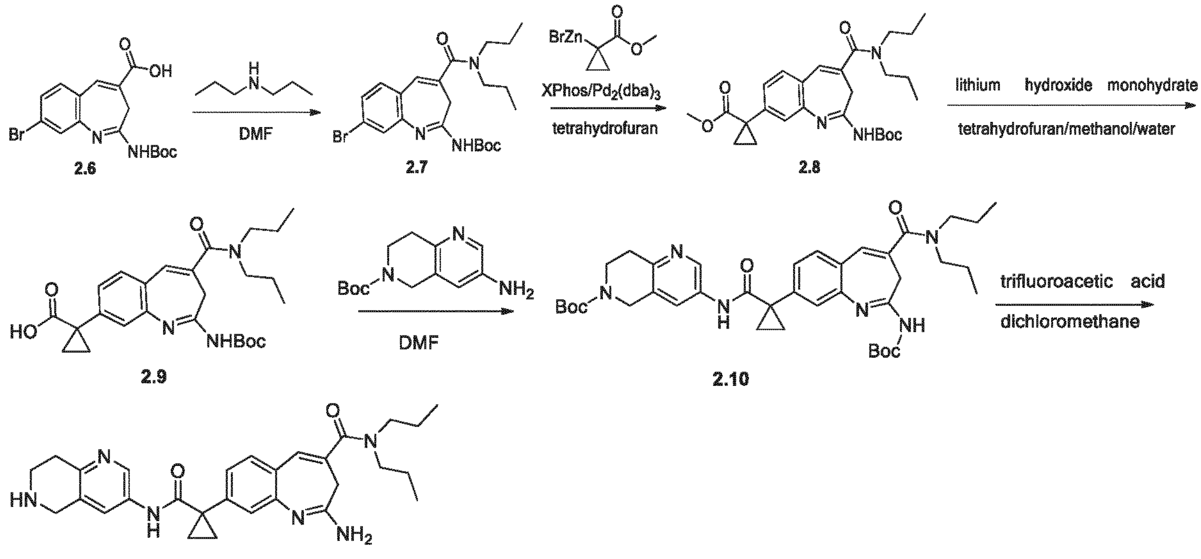


3

Step 1: A solution of compound 2.6 (1.1 g, 2.8 mmol), HATU (1.6 g, 4.33 mmol), di*n*-propylamine (580 mg, 22 mmol), and DIPEA (560 mg, 4.37 mmol) in DMF (10 mL) was stirred for 3 hours at room temperature. The reaction system was diluted with ethyl acetate (100 mL) and then washed with water and saturated brine respectively. The organic phase was separated, dried over anhydrous sodium sulfate, and filtered, and the filtrate was concentrated under reduced pressure. The residue was purified by flash column chromatography (methanol/dichloromethane = 1/20) to obtain compound 2.7 (500 mg, yield: 39%) as a pale yellow solid.

Step 2: Compound 2.7 (2 g, 4.31 mmol), Xphos (0.2 g), and $Pd_2(dba)_3$ (0.2 g) were dissolved in a freshly prepared tetrahydrofuran solution of (1-(methoxycarbonyl)cyclopropyl)zinc bromide (1 eq, 30 mL) (referring to WO2018/138356A1), and the reaction system was replaced with nitrogen for three times, then stirred for 2 hours at 75°C under nitrogen atmosphere, then cooled to room temperature, and the reaction was quenched with ice water. The aqueous phase was extracted with ethyl acetate, and the organic phases were combined and washed with saturated brine, dried over anhydrous sodium sulfate, and filtered. The filtrate was concentrated under reduced pressure, and the residue was purified by flash column chromatography (petroleum ether/ethyl acetate = 3/1) to obtain compound 2.8 (500 mg, yield: 24%) as a light yellow solid. m/z: $[M+H]^+$484.2.

Step 3: To a mixed solution of compound 2.8 (500 mg, 1.03 mmol) in tetrahydrofuran (5 mL), methanol (0.5 mL), and water (0.5 mL) was added lithium hydroxide monohydrate (130 mg, 3.1 mmol). The reaction system was stirred for 16 hours at room temperature, and then the reaction mixture was neutralized with hydrochloric acid (1 M). The aqueous phase was extracted with ethyl acetate, and the organic phase was combined, washed with saturated brine, dried over anhydrous sodium sulfate, and filtered. The filtrate was concentrated under reduced pressure to obtain

compound 2.9 (400 mg, yield: 82%) as an off-white solid. m/z: $[M+H]^+$ 470.2.

Step 4: To a solution of compound 2.9 (100 mg, 0.21 mmol) in DMF (1 mL) was sequentially added *tert*-butyl 3-amino-7,8-dihydro-1,6-naphthyridine-6(5*H*)-carboxylate (64 mg, 0.25 mmol), HATU (97 mg, 0.25 mmol), and DIPEA (55 mg, 0.42 mmol). The reaction mixture was stirred at room temperature for 4 hours. Then the reaction mixture was directly purified by flash column chromatography (10 mM ammonium bicarbonate aqueous solution/acetonitrile = 15/85) to obtain compound 2.10 (28 mg, yield: 19%) as a white solid. m/z: $[M+H]^+$701.3.

Step 5: To a solution of compound 2.10 (28 mg, 0.04 mmol) in dichloromethane (2 mL) was added trifluoroacetic acid (1 mL). The reaction mixture was stirred at room temperature for 2 hours and then concentrated directly under reduced pressure, and the residue was neutralized with ammonia methanol solution (7 M) and then purified by flash column chromatography (10 mM ammonium bicarbonate aqueous solution/acetonitrile = 40/60) to obtain compound 3 (17 mg, yield: 85%) as a white solid. m/z: $[M+H]^+$ 501.3; $^1H$ NMR (400 MHz, DMSO-$d_6$): δ 9.19 (s, 1H), 8.42 (s, 1H), 7.63 (s, 1H), 7.25 (d, *J* = 7.6Hz, 1H), 7.00 (s, 1H), 6.91 (d, *J* = 8.4Hz, 1H), 6.74 (s, 2H), 6.69 (s, 1H) , 3.78 (s, 2H), 2.97 (br. s, 2H), 2.63 (s, 6H), 1.55-0.74 (m, 17H).

**Example 4: Synthesis of 2-amino-*N*-(2-hydroxyethyl)-*N*-*n*-propyl-8-(1-((5,6,7,8-tetrahydro-1,6-naphthyridin-3-yl)carbamoyl)cyclopropyl)-3*H*-benzo[*b*]azepine-4-carboxamide (compound 4)**

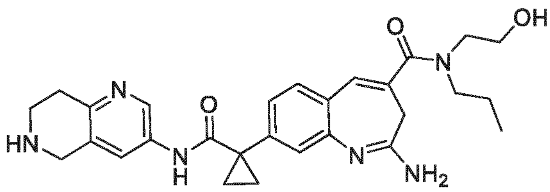

**4**

Compound 4 was obtained as a white solid by replacing di-*n*-propylamine in step 1 with 2-(propylamino)ethanol using the synthesis method of compound 3. m/z: $[M+H]^+$ 503.3.

**Example 5: Synthesis of 2-amino-G-fluoro-*N*,*N*-di-*n*-propyl-8-(1-((5,6,7,8-tetrahydro-1,6-naphthyridin-3-yl)carbamoyl)cyclopropyl)-3H-benzo[*b*]azepine-4-carboxamide (compound 5)**

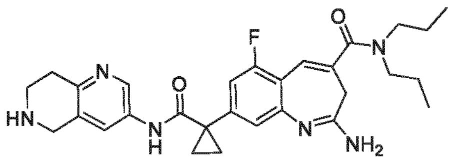

**5**

Compound 5 was obtained as a white solid by replacing intermediate 2.6 in step 1 with intermediate 3.1 using the synthesis method of compound 3. m/z: $[M+H]^+$ 519.1.

**Example 6: Synthesis of 2-amino-8-(2-oxo-2-((5,6,7,8-tetrahydro-1,6-naphthyridin-3-yl)amino)acetyl)-*N*,*N*-di-*n*-propyl-3*H*-benzo[*b*]azepine-4-carboxamide (compound 6)**

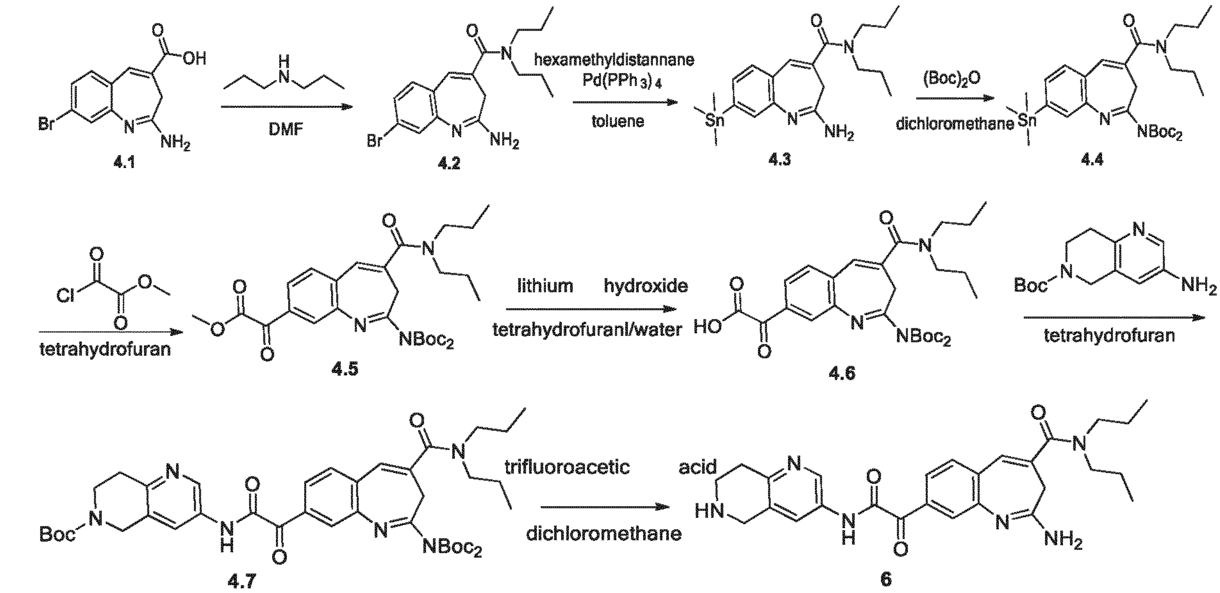


Step 1: Compound 4.2 was obtained by reacting compound 4.1 using the synthesis method of compound 2.7.

Step 2: A mixture of compound 4.2 (2 g, 5.49 mmol), hexamethyldistannane (1.89 g, 5.76 mmol), and $Pd(PPh_3)_4$ (127 mg, 0.11 mmol) in toluene (25 mL) was replaced with nitrogen three times, and the reaction system was stirred at 100°C for 6 hours under nitrogen atmosphere. Then the reaction system was cooled to room temperature and filtered, and the filtrate was concentrated under reduced pressure. The residue was purified by flash column chromatography (petroleum ether/ethyl acetate = 1/1) to obtain compound 4.3 (1.45 g, yield: 59%)as a pale brown solid. m/z: $[M+H]^+$ 450.2.

Step 3: To a solution of compound 4.3 (1.45 g, 3.24 mmol) in dichloromethane (30 mL) were added triethylamine (0.98 g, 9.71 mmol), 4-dimethylaminopyridine (79 mg, 0.65 mmol), and $(Boc)_2O$ (2.12 g, 9.71 mmol), respectively. The reaction mixture was stirred at room temperature for overnight and then directly concentrated under reduced pressure, and the residue was purified by flash column chromatography (petroleum ether/ethyl acetate = 10/1) to obtain compound 4.4 (1.2 g, yield: 57%) as a pale yellow solid. m/z: $[M+H]^+$ 650.0.

Step 4: To a solution of compound 4.4 (400 mg, 0.62 mmol), DIPEA (120 mg, 0.92 mmol), and potassium carbonate (17 mg, 0.12 mmol) in anhydrous tetrahydrofuran (6 mL) was added $Pd_2(dba)_3$ (33 mg, 0.06 mmol) under an ice bath condition, and the reaction system was replaced with nitrogen three times and then added dropwise with a solution of monomethyl oxalyl chloride (113 mg, 0.92 mmol) in tetrahydrofuran (1 mL). After the addition was completed, the reaction system was warmed to room temperature and stirred for overnight, and the reaction was quenched with water. The aqueous phase was extracted with ethyl acetate, and the organic phases were combined and concentrated under reduced pressure. The residue was purified by flash column chromatography (petroleum ether/ethyl acetate=2/1) to obtain compound 4.5 (145 mg, yield: 41%) as a pale yellow solid. m/z: $[M+Na]^+$594.3.

Step 5: To a solution of compound 4.5 (145 mg, 0.25 mmol) in tetrahydrofuran (5 mL) was added an aqueous solution (2 mL) of lithium hydroxide monohydrate (289 mg, 0.88 mmol) under an ice bath condition. The reaction system was stirred at 0°C for 1 hour. Then the pH of the reaction mixture was adjusted to 7 with hydrochloric acid (1 M), and the mixture was directly purified by flash column chromatography ($MeCN/NH_4HCO_3$ (10 mM) = 85/15) to obtain compound 4.6 (90 mg, yield: 65%) as a pale brown solid. m/z: $[M+H]^+$558.3.

Step 6: To a solution of compound 4.6 (80 mg, 0.14 mmol) in DMF (4 mL) were sequentially added *tert*-butyl 3-amino-7,8-dihydro-1,6-naphthyridine-6(5*H*)-carboxylate (43 mg, 0.17 mmol), HATU (65 mg, 0.17 mmol), and DIPEA (22 mg, 0.17 mmol), and the reaction mixture was reacted under microwave irradiation at 50°C for 1 hour. Then the reaction was quenched with water, and the aqueous phase was extracted with ethyl acetate. The organic phases were combined and concentrated under reduced pressure, and the residue was purified by *prep*-TLC (petroleum ether/ethyl acetate = 1/2) to obtain compound 4.7 (39 mg, yield: 34%) as a pale yellow solid. m/z: $[M+H]^+$ 789.5.

Step 7: To a solution of compound 4.7 (39 mg, 0.05 mmol) in dichloromethane (4 mL) was added trifluoroacetic acid (2 mL). The reaction mixture was stirred at room temperature for 2 hours and then concentrated directly under reduced pressure, and the residue was neutralized with ammonia methanol solution (7M) and then purified by flash column chromatography (10 mM ammonium bicarbonate aqueous solution/acetonitrile = 40/60) to obtain compound 6 (15 mg, yield: 62%) as a yellow solid. m/z: $[M+H]^+$489.3.

**Example 7: Synthesis of 2-amino-8-(2-methyl-1-oxo-1-((5,6,7,8-tetrahydro-1,6-naphthyridin-3-yl)amino)propan-2-yl)-N,N-di-n-propyl-3H-benzo[b]azepine-4-carboxamide (compound 7)**

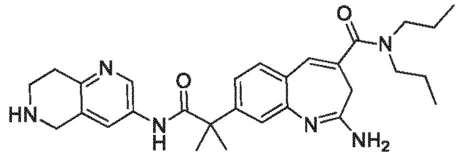

7

Compound 7 was obtained as a white solid by replacing (1-(methoxycarbonyl)cyclopropyl)zinc bromide in step 2 with (1-methoxy-2-methyl-1-oxopropan-2-yl)zinc bromide using the synthesis method of compound 3. m/z:$[M+H]^+$ 503.3; $^1$H NMR (400 MHz, $CD_3OD$): δ 8.45 (d, *J* = 2.0Hz, 1H), 7.77 (d, *J* = 2.0Hz, 1 H), 7.31 (d, *J* = 8.4Hz, 1H), 7.20 (d, *J* = 1.6Hz, 1H), 7.09-7.05 (m, 1H), 6.82 (s, 1H), 3.96 (s, 2H), 3.44-3.37 (m, 4H), 3.19-3.14 (m, 2H), 2.94-2.73 (m, 4H), 1.75-1.56 (m, 10H), 1.05-0.78 (m, 6H).

**Example 8: Synthesis of 8-amino-*N*-(2-hydroxyethyl)-*N*-*n*-propyl-2-((5,6,7,8-tetrahydro-1,6-naphthyridin-3-yl)amino)-7*H*-pyrido[2,3-*b*]azepine-6-carboxamide (compound 8)**

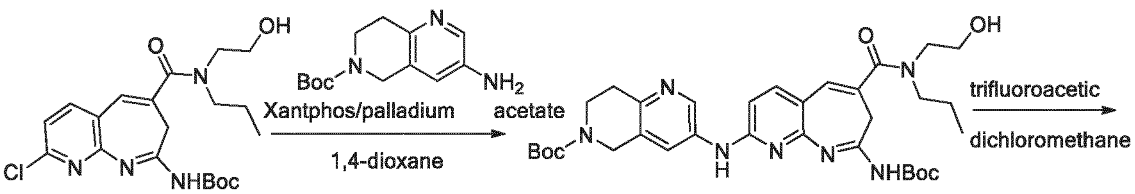


5.1

5.2

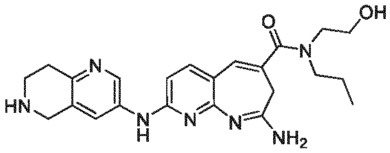

8

Step 1: A mixture of compound 5.1 (obtained by reacting intermediate 1.7 with 2-(propylamino)ethanol using the synthesis method of compound 2.7) (200 mg, 0.47 mmol), *tert*-butyl 3-amino-7,8-dihydro-l,6-naphthyridine-6(5*H*)-carboxylate (142 mg, 0.57 mmol), cesium carbonate (170 mg, 0.52 mmol), palladium acetate (32 mg, 0.14 mmol), and Xantphos (82 mg, 0.14 mmol) in 1,4-dioxane (6 mL) was replaced with nitrogen for three times, and the reaction system was stirred at 90°C for 4 hours under nitrogen atmosphere. The reaction was quenched with water, and the aqueous phase was extracted with ethyl acetate. The organic phases were combined and concentrated under reduced pressure, and the residue was purified by *prep*-TLC (dichloromethane/methanol = 10/1) to obtain compound 5.2 (200 mg, yield: 67%) as a pale brown solid.

Step 2: To a solution of compound 5.2 (200 mg, 0.31 mmol) in dichloromethane (10 mL) was added trifluoroacetic acid (2 mL). The reaction system was stirred at room temperature for 2 hours. The reaction mixture was concentrated under reduced pressure, and the residue was purified by *prep*-HPLC (separation condition 3) to obtain compound 8 (31 mg, yield: 23%) as a pale yellow solid. m/z: $[M+H]^+$436.2; $^1$H NMR (400 MHz, DMSO-$d_6$): δ 9.08 (s, 1H), 8.63 (d, *J* = 2.4Hz, 1H), 8.00 (d, *J* = 2.0Hz, 1H), 7.48 (d, *J* = 8.8Hz, 1H), 7.09 (br. s, 1H), 6.87 (br. s, 1H), 6.66 (s, 1H), 6.44 (d, *J* = 8.4Hz, 1H), 4.79 (s, 1H), 3.82 (s, 2H), 3.30-3.61 (m, 2H), 3.10-3.20 (m, 5H), 3.00 (t, *J* = 6.0Hz, 2H), 2.82-2.62 (m, 4H), 1.65-1.47 (m, 2H), 0.81 (br. s, 3H).

**Example 9: Synthesis of 8-amino-*N,N*-di-*n*-propyl-2-(1-((5,6,7,8-tetrahydro-1,6-naphthyridin-3-yl)carbamoyl)cyclopropyl)-7*H*-pyrido[2,3-*b*]azepine-6-carboxamide (compound 9)**

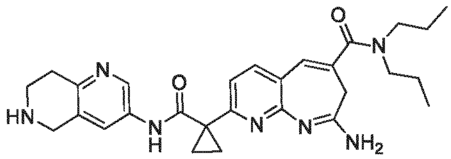

9

Compound 9 was obtained as a white solid by replacing intermediate 2.6 in step 1 with intermediate 1.8 using the synthesis method of compound 3. m/z: $[M+H]^+$ 502.4; $^1H$ NMR (400 MHz, DMSO-$d_6$): δ 12.43 (s, 1H), 8.51 (d, *J* = 2.4Hz, 1H), 7.81 (d, *J* = 2.4Hz, 1H), 7.70 (d, *J* = 8.0Hz, 1H), 7.26 (s, 1H), 7.15 (s, 1H), 6.80 (d, *J* = 8.4Hz, 1H), 6.73 (s, 1H), 3.84 (s, 2H), 3.29-3.19 (m, 5H), 3.01 (t, *J* = 6.0Hz, 2H), 2.85-2.66 (m, 4H), 1.67-1.48 (m, 6H), 1.40 (q, *J* = 3.6Hz, 2H), 0.82 (s, 6H).

**Example 10: Synthesis of 8-amino-*N*-(2-hydroxyethyl)-*N-n*-propyl-2-(1-((5,6,7,8-tetrahydro-1,6-naphthyridin-3-yl)carbamoyl)cyclopropyl)-7*H*-pyrido[2.3-*b*]azepine-6-carboxamide (compound 10)**

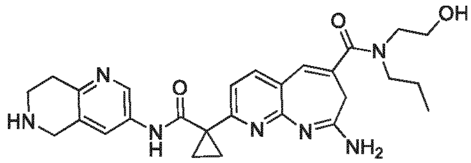

10

Compound 10 was synthesized as a white solid using 1.7 and 2-(propylamino)ethanol as the starting materials using the synthesis method of compound 3. m/z: $[M+H]^+$ 504.2.

**Example 11: Synthesis of 2-amino-6-fluoro-*N*-(2-hydroxyethyl)-*N-n*-propyl-8-(1-((5,6,7,8-tetrahydro-1,6-naphthyridin-3-yl)carbamoyl)cyclopropyl)-3*H*-benzo[b]azepine-4-carboxamide (compound 11)**

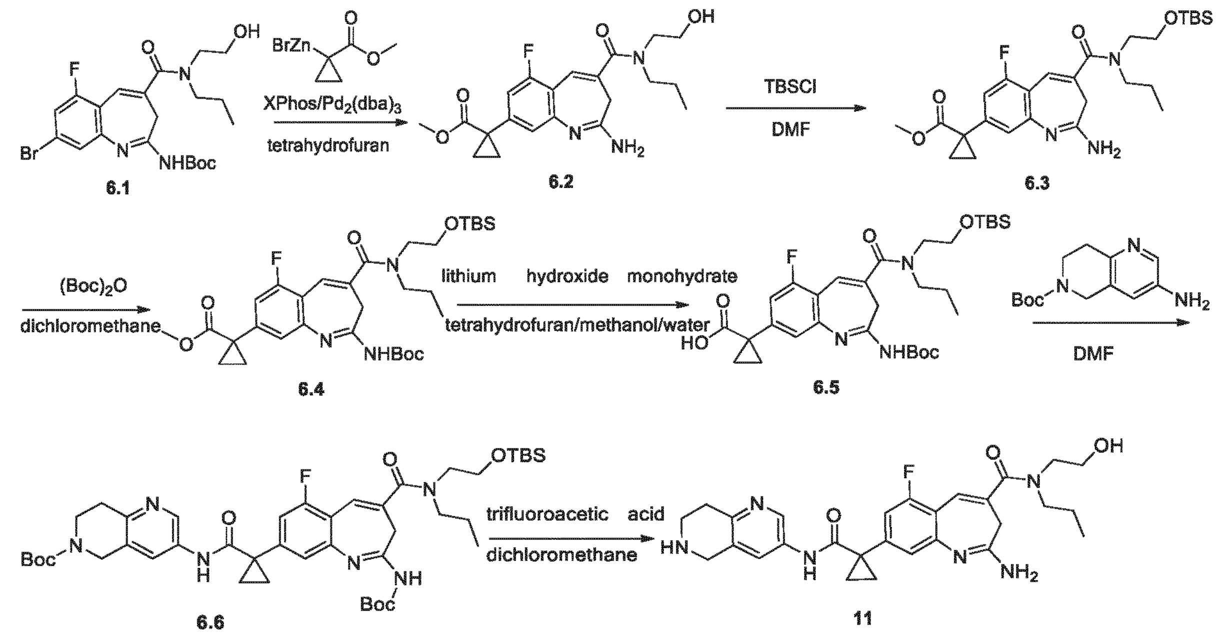


6.1 6.2 6.3 6.4 6.5 6.6 11

Step 1: Compound 6.1 (obtained by reacting intermediate 3.1 with 2-(propylamino)ethanol using the synthesis method of compound 2.7) (1 g, 2.06 mmol), Xphos (0.1 g), and $Pd_2(dba)_3$ (0.2 g) were dissolved in a freshly prepared solution (35 mL) of (1-(methoxycarbonyl)cyclopropyl)zinc bromide in tetrahydrofuran, and the reaction system was replaced with nitrogen for three times, then stirred at 75°C for 2 hours under nitrogen atmosphere, and cooled to room temperature. The reaction was quenched with hydrogen chloride methanol solution (1 mL, 4 M), and the reaction mixture was directly purified by flash column chromatography (10 mM ammonium bicarbonate aqueous solution/acetonitrile = 0 to 7/3) to obtain compound 6.2 (600 mg, yield: 72%) as an off-white solid. m/z: $[M+H]^+$ 404.2.

Step 2: To a solution of compound 6.2 (500 mg, 1.24 mmol) in DMF (5 mL) were sequentially added imidazole (422 mg, 6.2 mmol) and TBSCl (561 mg, 3.72 mmol), and the reaction system was stirred at room temperature for 3 hours, and then directly purified by flash column chromatography (10 mM ammonium bicarbonate aqueous solution/acetonitrile = 0 to 4/1) to obtain compound 6.3 (510 mg, yield: 79%) as a white solid. m/z: $[M+H]^+$ 518.2.

Step 3: To a solution of compound 6.3 (500 mg, 0.97 mmol) in dichloromethane (15 mL) were sequentially added triethylamine (220 mg, 2.18 mmol) and $(Boc)_2O$ (285 mg, 1.31 mmol), and the reaction system was stirred at room temperature for 12 hours. The reaction was quenched with water, then the aqueous phase was extracted with dichloromethane, and the organic phases were combined, washed with saturated brine, dried over anhydrous sodium sulfate, and filtered. The filtrate was concentrated under reduced pressure, and the residue was purified by flash column chromatography (petroleum ether/ethyl acetate = 10/1) to obtain compound 6.4 (510 mg, yield: 85%) as an off-white solid. m/z: $[M+H]^+$ 618.3.

Step 4: To a mixed solution of compound 6.4 (500 mg, 0.81 mmol) in tetrahydrofuran (2 mL), methanol (1 mL), and water (1 mL) was added lithium hydroxide monohydrate (102 mg, 2.43 mmol). The reaction system was stirred at room temperature for 3 hours, and then the pH of the reaction mixture was adjusted to 6 with hydrochloric acid (1M). The reaction mixture was directly purified by flash column chromatography (10 mM ammonium bicarbonate aqueous solution/acetonitrile = 0 to 1/1) to obtain compound 6.5 (330 mg, yield: 68%) as a white solid. m/z: $[M+H]^+$ 604.3.

Step 5: To a solution of compound 6.5 (50 mg, 0.08 mmol) in DMF (5 mL) were sequentially added HATU (46 mg, 0.12 mmol) and DIPEA (31 mg, 0.24 mmol), and the reaction mixture was stirred at room temperature for 30 minutes. Then tert-butyl 3-amino-7,8-dihydro-1,6-naphthyridine-6(5*H*)-carboxylate (25 mg, 0.1 mmol) was added thereto. The resulting reaction mixture was stirred at room temperature for another 16 hours and then directly purified by flash column chromatography (10 mM ammonium bicarbonate aqueous solution/acetonitrile = 1/20 to 3/1) to obtain compound 6.6 (35 mg, yield: 52%) as a white solid. m/z: $[M+H]^+$ 835.4.

Step 6: To a solution of compound 6.6 (30 mg, 0.04 mmol) in dichloromethane (2 mL) was added trifluoroacetic acid (1 mL). The reaction system was stirred at room temperature for 1 hour, and the reaction mixture was concentrated under reduced pressure, then added with ammonia methanol (1 mL, 7M) solution, and then directly purified by flash column chromatography (10 mM ammonium bicarbonate aqueous solution/acetonitrile = 0 to 2/1) to obtain compound 11 (12 mg, yield: 58%) as a white solid. m/z: $[M+H]^+$ 521.3.

**Example 12: Synthesis of 2-amino-8-(1-((5-(aminomethyl)pyridin-3-yl)carbamoyl)cyclopropyl)-*N*-(2-hydroxyethyl)-*N*-*n*-propyl-3*H*-benzo[*b*]azepine-4-carboxamide (compound 12)**

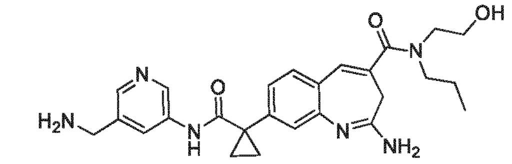

**12**

Compound 12 was obtained as a white solid by replacing di-n-propylamine in step 1 with 2-(propylamino)ethanol and replacing *tert*-butyl 3-amino-7,8-dihydro-1,6-naphthyridine-6(5*H*)-carboxylate in step 3 with *tert*-butyl ((5-aminopyridin-3-yl)methyl)carbamate using the synthesis method of compound 3. m/z: $[M+H]^+$ 477.3.

**Example 13: Synthesis of 2-amino-$N^4$-(2-hydroxyethyl)-$N^4$-*n*-propyl-$N^8$-(5,6,7,8-tetrahydro-1,6-naphthyridin-3-yl)-3*H*-benzo[*b*]azepine-4,8-dicarboxamide (compound 13)**

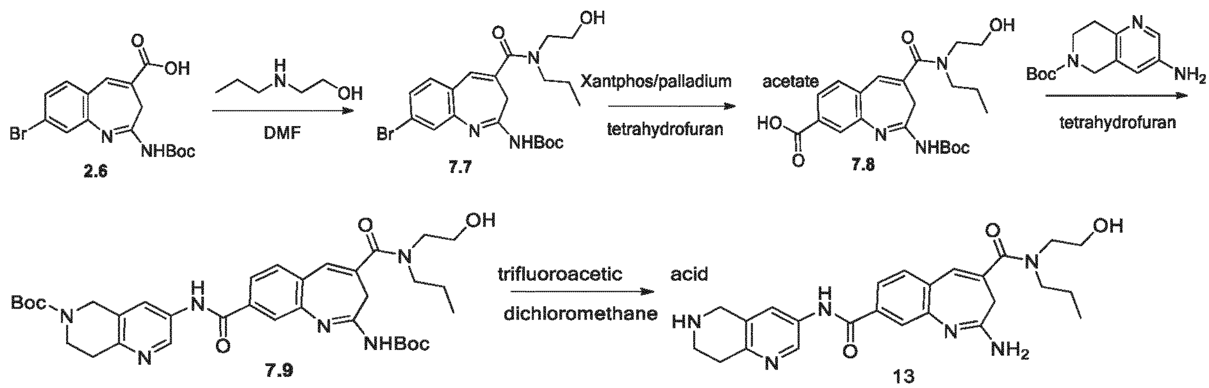


2.6 7.7 7.8 7.9 13

Step 1: To a solution of compound 2.6 (8.8 g, 23.1 mmol) in DMF (100 mL) were sequentially added DIPEA (8.9 g, 69.2 mmol), 2-(propylamino)ethanol (2.9 g, 27.7 mmol), and HATU (13.2 g, 34.63 mmol). After the addition was completed, the reaction system was stirred at room temperature for 2 hours, and then the reaction was quenched with water. The aqueous phase was extracted with ethyl acetate (100 mL × 2), and the organic phases were combined, washed with saturated brine, separated, dried over anhydrous sodium sulfate, filtered, and concentrated. The residue was purified by silica gel column chromatography (petroleum ether/ethyl acetate = 1/1) to obtain compound 7.7 (10 g, yield: 93%) as an off-white solid. m/z: $[M+H]^+$466.2.

Step 2: A mixed solution of compound 7.7 (600 mg, 1.29 mmol), potassium phosphate (820 mg, 3.86 mmol), palladium acetate (29 mg, 0.13 mmol), and Xantphos (37 mg, 0.06 mmol) in tetrahydrofuran (10 mL) and water (5 mL) was replaced with nitrogen and carbon monoxide for three times, respectively, and then the mixture was stirred at 70°C for 4 hours under carbon monoxide atmosphere. The reaction system was cooled to room temperature and concentrated under reduced pressure, and the crude product was dispersed into methanol and filtered. The filter cake was washed with methanol, and the filtrate was concentrated under reduced pressure and directly purified by flash column chromatography (10 mM ammonium bicarbonate aqueous solution/acetonitrile = 30/70) to obtain compound 7.8 (100 mg, yield: 18%) as a pale brown solid. m/z: $[M+H]^+$ 432.2.

Step 3: To a solution of compound 7.8 (50 mg, 0.11 mmol), HATU (49 mg, 0.13 mmol), and DIPEA (17 mg, 0.13 mmol) in DMF (3 mL) was added *tert*-butyl 3-amino-7,8-dihydro-1,6-naphthyridine-6(5*H*)-carboxylate (32 mg, 0.13 mmol), and the reaction mixture was stirred at room temperature for overnight. The reaction mixture was poured into water to quench the reaction, then the aqueous phase was extracted with ethyl acetate, and the organic phases were combined and concentrated under reduced pressure. The residue was purified by flash column chromatography (10 mM ammonium bicarbonate aqueous solution/acetonitrile = 30/70) to obtain compound 7.9 (25 mg, yield: 32%) as a pale brown solid. m/z: $[M+H]^+$663.2.

Step 4: A solution of compound 7.9 (25 mg, 0.04 mmol) and trifluoroacetic acid (2 mL) in dichloromethane (5 mL) was stirred at room temperature for 2 hours and directly concentrated under reduced pressure, and the residue was prepared by *prep*-HPLC (separation condition 2) to obtain compound 13 (16 mg, yield: 94%) as a white solid. m/z: $[M+H]^+$ 463.2; $^1H$ NMR (400 MHz, DMSO-$d_6$): δ 10.36 (s, 1H), 8.69 (d, *J* = 2.0Hz, 1H), 8.00 (s, 1H), 7.69 (s, 1H), 7.53-7.45 (m, 1H), 7.42 (d, *J* = 8.4 Hz, 1H), 6.99-6.76 (m, 2H), 4.81 (s, 1H), 4.01 (s, 2H), 3.55 (br.s, 4H), 3.38 (m, 5H), 3.17 (t, *J* = 6.0 Hz, 3H), 2.83 (t, *J* = 6.0 Hz, 2H), 2.73 (s, 2H), 1.67-1.43 (m, 2H), 0.86 (s, 3H).

**Example 14: Synthesis of 2-amino-*N*,*N*-bis(2-hydroxyethyl)-8-(1-((5,6,7,8-tetrahydro-1,6-naphthyridin-3-yl)carbamoyl)cyclopropyl)-3*H*-benzo[*b*]azepine-4-carboxamide (compound 14)**

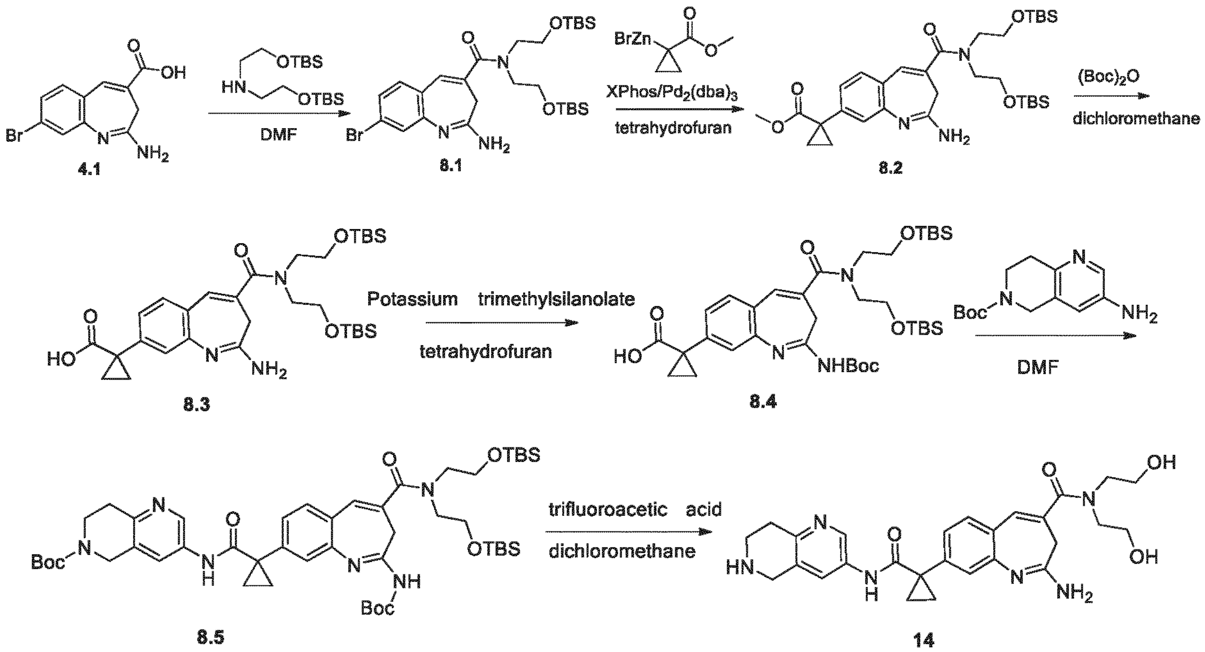


Step 1: A solution of compound 4.1 (3 g, 10.7 mmol), HATU (5.36 g, 14.1 mmol), bis(2-((*tert*-butyldimethylsilyl)oxy)ethyl)amine (3.76 g, 11.3 mmol), and DIPEA (3.64 g, 28.2 mmol) in DMF (30 mL) was stirred at room temperature for overnight. The reaction system was diluted with ethyl acetate (100 mL) and washed with saturated sodium bicarbonate aqueous solution and saturated brine, respectively. The organic phase was separated, dried over anhydrous sodium sulfate, and filtered, and the filtrate was concentrated under reduced pressure. The residue was purified by flash column chromatography (methanol/dichloromethane = 1/20) to obtain compound 8.1 (6.6 g, yield: 100%) as a yellow solid. m/z: $[M+H]^+$ 596.2.

Step 2: Compound 8.1 (6.5 g, 10.9 mmol), Xphos (0.5 g, 1 mmol), $Pd_2(dba)_3$ (0.5 g, 0.54 mmol) were dissolved in a freshly prepared solution of (1-(methoxycarbonyl)cyclopropyl)zinc bromide (1 eq, 160 mL) in tetrahydrofuran, and the reaction system was replaced with nitrogen for three times, then stirred for 5 hours at 75°C under nitrogen atmosphere, and then cooled to room temperature. The reaction was quenched with ice water, then the aqueous phase was extracted with ethyl acetate, and the organic phases were combined, washed with saturated brine, dried over anhydrous sodium sulfate, and filtered. The filtrate was concentrated under reduced pressure, and the residue was purified by flash column chromatography (petroleum ether/ethyl acetate = 3/1) to obtain compound 8.2 (4.23 g, yield: 63%) as a yellow solid. m/z: $[M+H]^+$616.4.

Step 3: To a solution of compound 8.2 (4.23 g, 6.87 mmol) and triethylamine (1.74 g, 17.2 mmol) in dichloromethane (50 mL) was added $(BOC)_2O$ (2.25 g, 10.3 mmol). The reaction system was stirred at room temperature for overnight, and the organic phase was washed with saturated sodium bicarbonate aqueous solution and saturated brine, respectively, separated, dried over anhydrous sodium sulfate, and filtered. The filtrate was concentrated under reduced pressure, and the residue was purified by flash column chromatography (petroleum ether/ethyl acetate = 2/1) to obtain compound 8.3 (1.24 g, yield: 25%) as a yellow solid. m/z: $[M+H]^+$ 716.4.

Step 4: To a mixed solution of compound 8.3 (1.23 g, 1.72 mmol) in tetrahydrofuran (2.55 mL), methanol (0.5 mL), and water (0.5 mL) was added potassium trimethylsilanolate (0.88 g, 6.88 mmol). The reaction system was stirred at room temperature for 1 hour and then added with saturated ammonium chloride aqueous solution under an ice bath

condition. The aqueous phase was extracted with ethyl acetate, and the organic phases were combined, washed with saturated brine, dried over anhydrous sodium sulfate, and filtered. The filtrate was concentrated under reduced pressure, and the residue was purified by flash column chromatography (dichloromethane/methanol = 10/1) to obtain compound 8.4 (400 mg, yield: 83%) as a yellow solid. m/z: $[M+H]^+$ 702.4.

Step 5: To a solution of compound 8.4 (200 mg, 0.28 mmol) in DMF (5 mL) were sequentially added *tert*-butyl 3-amino-7,8-dihydro-1,6-naphthyridine-6(5*H*)-carboxylate (84 mg, 0.34 mmol), HATU (160 mg, 0.42 mmol), and DIPEA (109 mg, 0.84 mmol), and the reaction mixture was stirred at room temperature for overnight. The reaction mixture was added with saturated sodium bicarbonate aqueous solution, then the aqueous phase was extracted with ethyl acetate, and the organic phase was concentrated under reduced pressure. The residue was purified by*prep-TLC* (dichloromethane/methanol = 10/1) to obtain compound 8.5 (229 mg, yield: 88%) as a yellow solid. m/z: $[M+H]^+$ 993.4.

Step 6: To a solution of compound 8.5 (200 mg, 0.21 mmol) in dichloromethane (4 mL) was added trifluoroacetic acid (1 mL). The reaction mixture was stirred at room temperature for 1 hour, directly concentrated under reduced pressure, and directly purified by *prep*-HPLC to obtain compound 14 (23 mg, yield: 21%) as a white solid. m/z:$[M+H]^+$505.2; $^1$H NMR (400 MHz, $CD_3OD$): δ 8.38 (d, *J* = 2.4Hz, 1H), 7.68 (d, *J* = 2.0Hz, 1H), 7.37 (d, *J* = 8.0Hz, 1H), 7.24 (d, *J* = 1.6Hz, 1H), 7.17- 7.11 (m, 1H), 7.00 (s, 1H), 3.94 (s, 2H), 3.86-3.69 (m, 4H), 3.69-3.57 (m, 4H), 3.18-3.11 (m, 2H), 3.07-2.95 (m, 1H), 2.90-2.79 (m, 3H), 1.63-1.56 (m, 2H), 1.26-1.19 (m, 2H).

**Example 15: Synthesis of 2-amino-6-fluoro-*N,N*-bis(2-hydroxyethyl)-8-(1-((5,6,7,8-tetrahydro-1,6-naphthyridin-3-yl)carbamoyl)cyclopropyl)-3*H*-benzo[*b*]azepine-4-carboxamide (compound 15)**

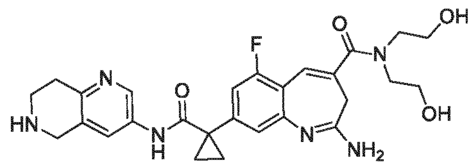

**15**

Compound 15 was obtained as a white solid using intermediate 3.2 as the starting material using the synthesis method of compound 14. m/z: $[M+H]^+$ 523.1; $^1$H NMR (400 MHz, $CD_3OD$): δ 8.42 (d, *J* = 2.4Hz, 1H), 7.72 (d, J = 2.4Hz, 1H), 7.12 (d, *J* = 1.6Hz, 1H), 7.08 (s, 1H), 6.92 (dd, *J* = 1.2, 10.8Hz, 1H), 3.97 (s, 2H), 3.68-3.81 (m, 8H), 3.32-3.34 (m, 2H), 3.17-3.20 (m, 2H), 2.88-2.91 (m, 2H), 1.60- 1.62 (m, 2H), 1.23-1.26 (m, 2H).

**Example 16: Synthesis of 2-amino-8-(1-((5-(aminomethyl)pyridin-3-yl)carbamoyl)cyclopropyl)-*N,N*-bis(2-hydroxyethyl)-3*H*-benzo[*b*]azepine-4-carboxamide (compound 16)**

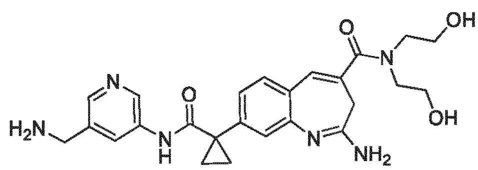

**16**

Compound 16 was obtained as a white solid by replacing *tert*-butyl 3-amino-7,8-dihydro-1,6-naphthyridine-6(5*H*)-carboxylate in step 5 with *tert*-butyl ((5-aminopyridin-3-yl)methyl)carbamate using the synthesis method of compound 14. m/z:$[M+H]^+$479.2; $^1$H NMR (400 MHz, $CD_3OD$): δ 8.53 (d, *J* = 2.4Hz, 1H), 8.24 (d, *J* = 2.0Hz, 1H), 7.93 (t, *J* = 2.4Hz, 1H), 7.37 (d, *J* = 8.0 Hz, 1H), 7.24 (d, *J* = 2.0Hz, 1H), 7.14 (dd, *J* = 1.8, 8.0Hz, 1H), 7.00 (s, 1H), 4.62 (s, 2H), 3.81 (s, 2H), 3.79-3.61 (m, 8H), 1.65-1.58 (m, 2H), 1.27-1.19 (m, 2H).

**Example 17: Synthesis of 2-amino-$N^4$-(2-aminoethyl)-$N^4$-*n*-propyl-$N^8$-(5,6,7,8-tetrahydro-1,6-naphthyridin-3-yl)-3*H*-benzo[*b*]azepine-4,8-dicarboxamide (compound** 17)

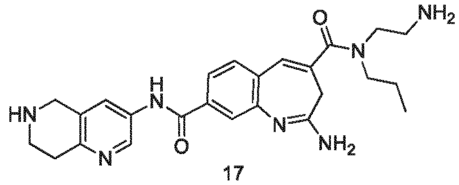

17

Compound 17 was obtained as a white solid using intermediates 2.6 and 7.1 as the starting materials using the synthesis method of compound 13. m/z:[M+H]+ 462.2; $^{1}H$ NMR (400 MHz, $CD_3OD$): δ 8.81 (s, 1 H), 8.26 (s, 1H), 7.92 (d, *J* = 7.6Hz, 1H), 7.82 (s, 1H), 7.52 (d, *J* = 8.0Hz, 1H), 4.48 (s, 2H), 3.69-3.60 (m, 2H), 3.59-3.44 (m, 4H), 3.41 -3.34 (m, 1H), 3.26-3.19 (m, 3H), 3.17-3.05 (m, 2H), 2.65-2.55 (m, 1H), 1.73-1.57 (m, 2H), 0.96 (t, *J* = 7.2Hz, 3H).

**Example 18: Synthesis of 2-amino-*N*4,*N*4-bis(2-hydroxyethyl)-*N*8-(5,6,7,8-tetrahydro-1,6-naphthyridin-3-yl)-3*H*-benzo[*b*]azepine-4,8-dicarboxamide (compound 18)**

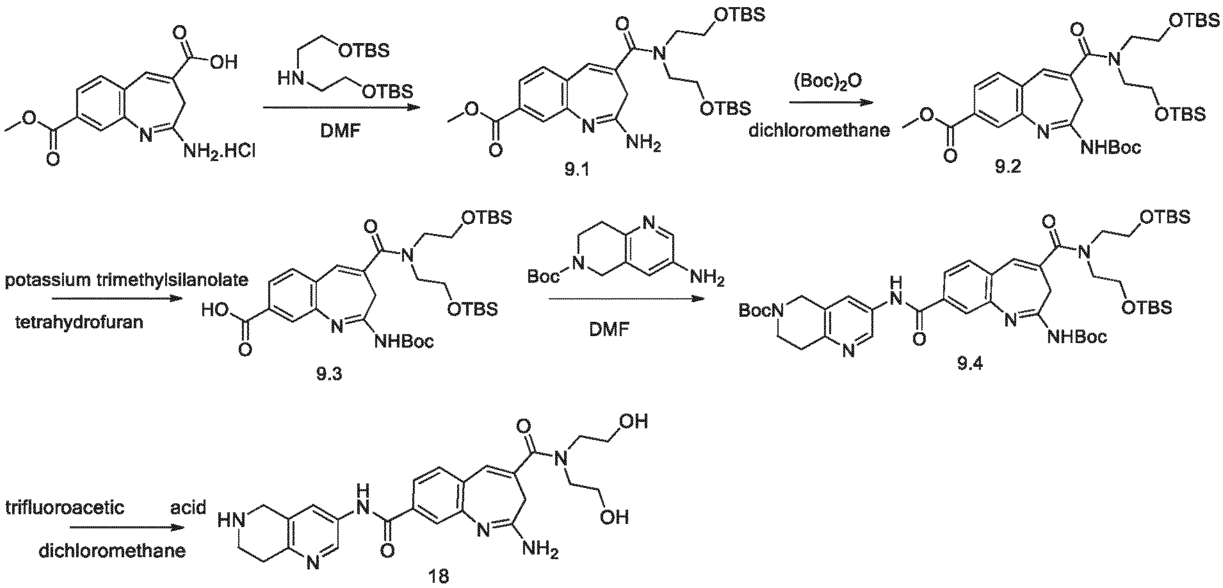


9.1 9.2 9.3 9.4 18

Step 1: Compound 9.1 was obtained as a pale yellow solid by reacting 2-amino-8-(methoxycarbonyl)-3*H*-benzo[*b*]azepine-4-carboxylic acid hydrochloride using the synthesis method of compound 14 in step 1. m/z: [M+H]+ 640.1.

Steps 2 to 5: Compound 18 was obtained as a white solid by reacting compound 9.1 using the synthesis method of compound 14 in steps 3 to 6. m/z:[M+H]+ 465.2.

The structure and identification data of the intermediates of examples 1 to 18 are shown below:

| Intermediate No. | Structure | Data |
|---|---|---|
| 17.1 | 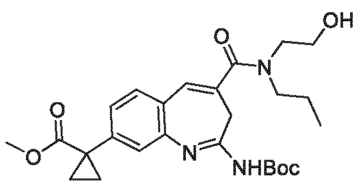 | m/z: [M+H]+ 486.2 |
| 17.2 | 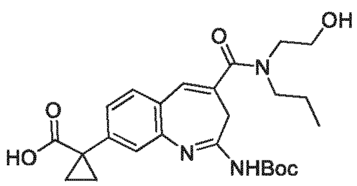 | m/z: [M+H]+ 472.2 |

(continued)

| Intermediate No. | Structure | Data |
|---|---|---|
| 17.3 | 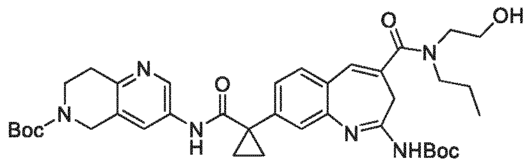 | m/z: [M+H]+ 703.4 |
| 10.1 | 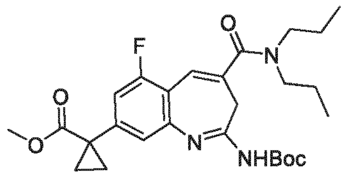 | m/z: [M+H]+ 502.1 |
| 10.2 | 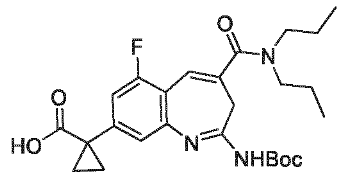 | m/z: [M+H]+ 488.1 |
| 10.3 | 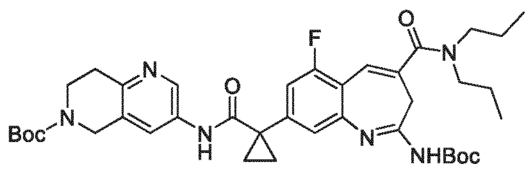 | m/z: [M+H]+ 719.0 |
| 11.1 | 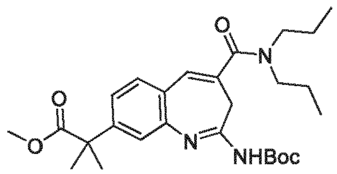 | m/z: [M+H]+ 486.2 |
| 11.2 | 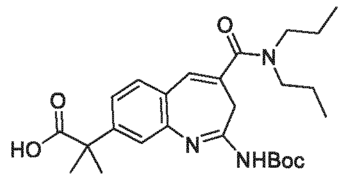 | m/z: [M+H]+ 472.2 |
| 11.3 | 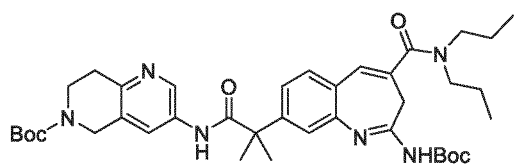 | m/z: [M+H]+ 703.4 |
| 12.1 | 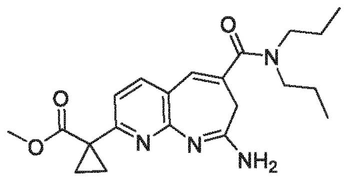 | m/z: [M+H]+ 385.3 |

(continued)

| Intermediate No. | Structure | Data |
|---|---|---|
| 12.2 | 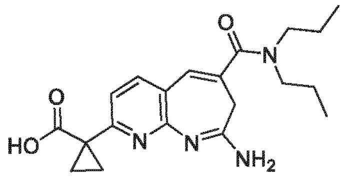 | m/z: $[M+H]^+$ 371.2 |
| 12.3 | 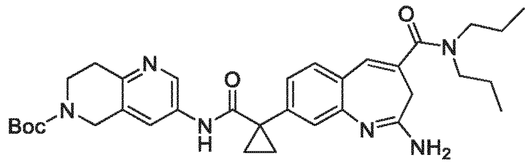 | m/z: $[M+H]^+$ 602.3 |
| 13.1 | 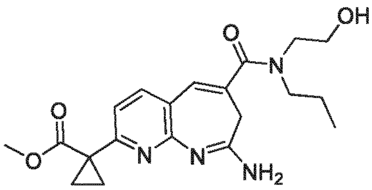 | m/z: $[M+H]^+$ 387.2 |
| 13.2 | 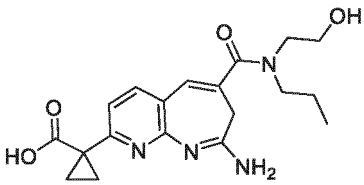 | m/z: $[M+H]^+$ 383.0 |
| 13.3 | 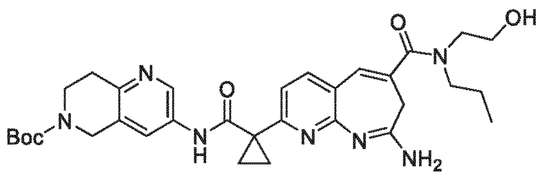 | m/z: $[M+H]^+$ 604.2 |
| 14.1 | 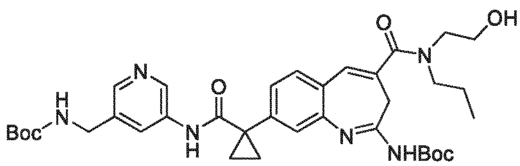 | m/z: $[M+H]^+$ 677.3 |
| 15.1 | 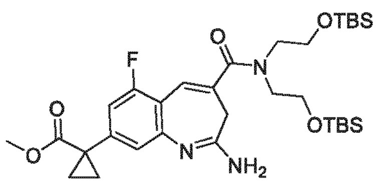 | m/z: $[M+H]^+$ 634.2 |
| 15.2 | 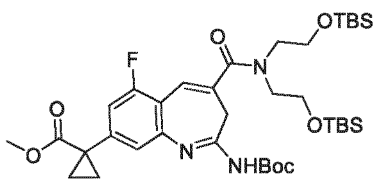 | m/z: $[M+H]^+$ 734.4 |

(continued)

| Intermediate No. | Structure | Data |
|---|---|---|
| 15.3 | 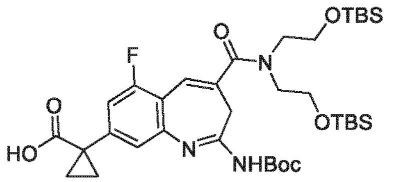 | m/z: $[M+H]^+$ 720.4 |
| 15.4 | 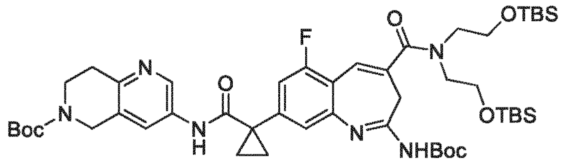 | m/z: $[M+H]^+$ 951.3 |
| 16.1 | 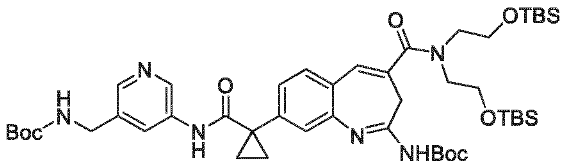 | m/z: $[M+H]^+$ 907.3 |
| 9.2 | 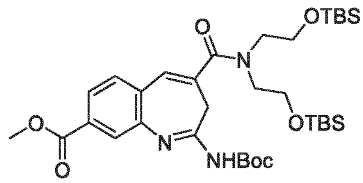 | m/z: $[M+H]^+$ 676.3 |
| 9.3 | 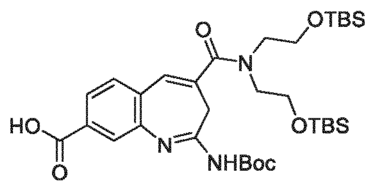 | m/z: $[M+H]^+$ 640.1 |
| 9.4 | 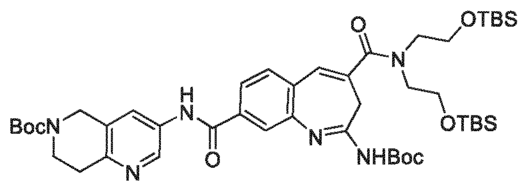 | m/z: $[M+H]^+$ 893.4 |

**Example 19: Synthesis of Compound II-1**

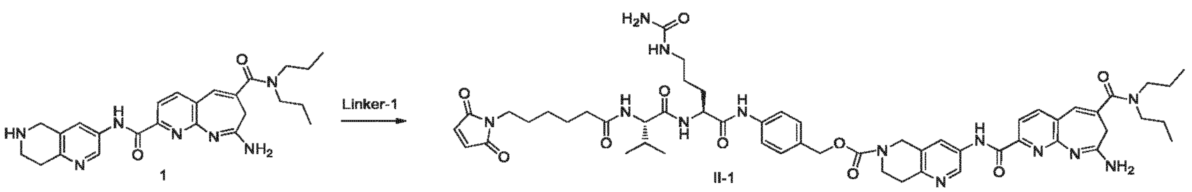


1 II-1

A solution of compound 1 (20 mg, 0.04 mmol), linker-1 (32 mg, 0.04 mmol), and DIPEA (11 mg, 0.09 mmol) in DMF (3 mL) was stirred at room temperature for 2 hours. The reaction mixture was then directly purified by *prep-HPLC*

(separation condition 2) to obtain compound II-1 (24.4 mg, yield: 53%) as a white solid. m/z: $[M+H]^+$ 1060.6.

**Examples 20 to 42: Synthesis of compounds II-2 to II-24**

Compounds II-2 to II-24 were obtained by reacting compounds 2 to 18 respectively using the synthesis method of compound II-1:

Table 2:

| No. | Structure | m/z: $[M+H]^+$ |
|---|---|---|
| II-2 | 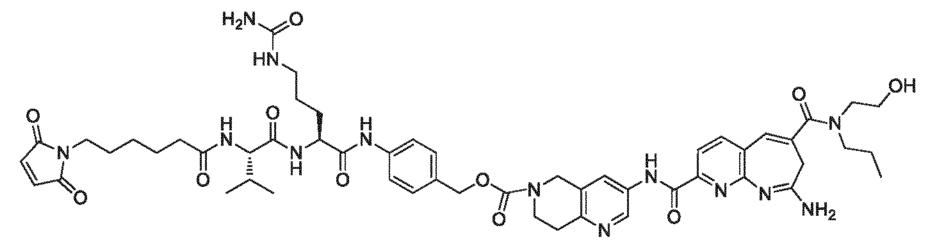 | 1062.5 |
| II-3 | 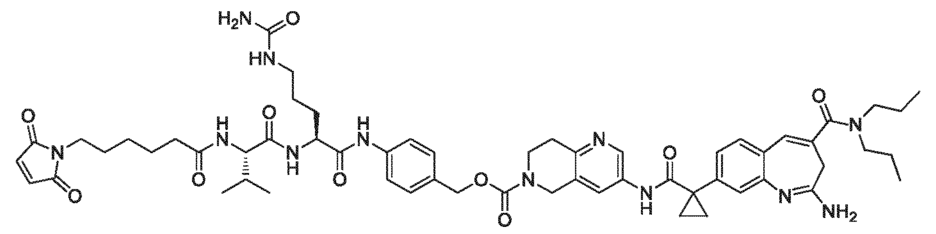 | 1099.6 |
| II-4 | 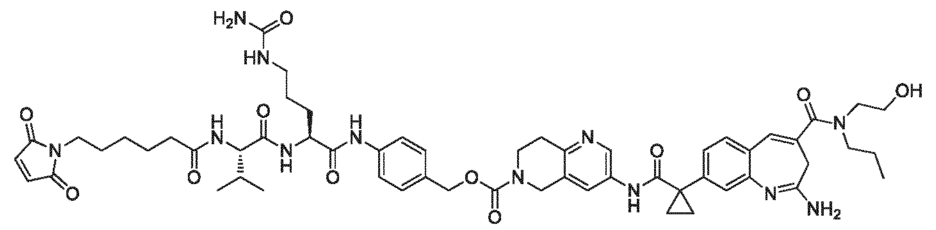 | 1101.6 |
| II-5 | 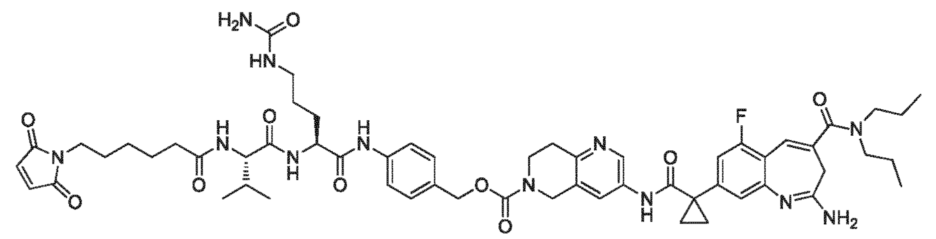 | 1117.2 |
| II-6 | 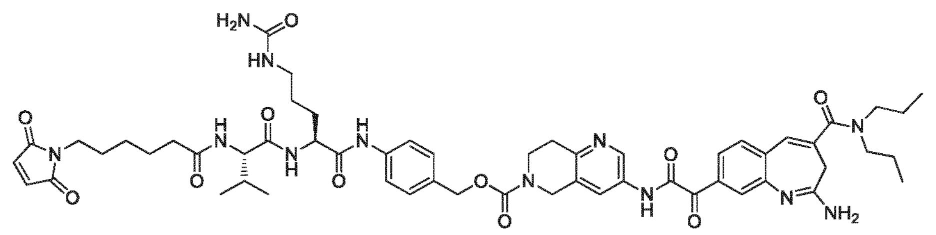 | 1087.7 |
| II-7 | 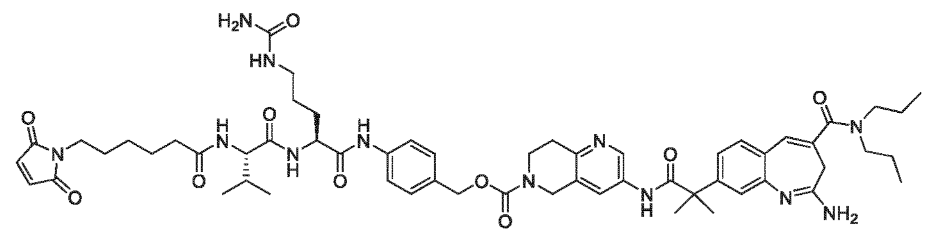 | 1101.7 |

(continued)

| No. | Structure | m/z: [M+H]+ |
|---|---|---|
| II-8 | 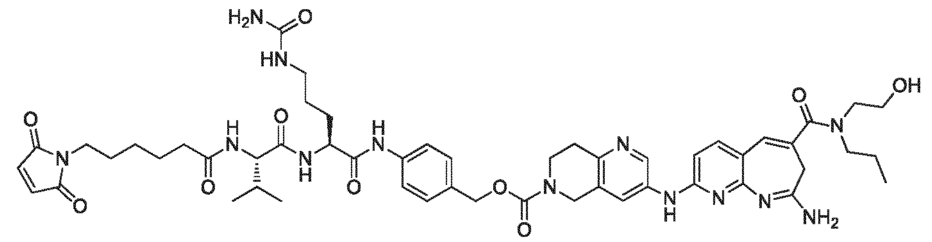 | 1034.3 |
| II-9 | 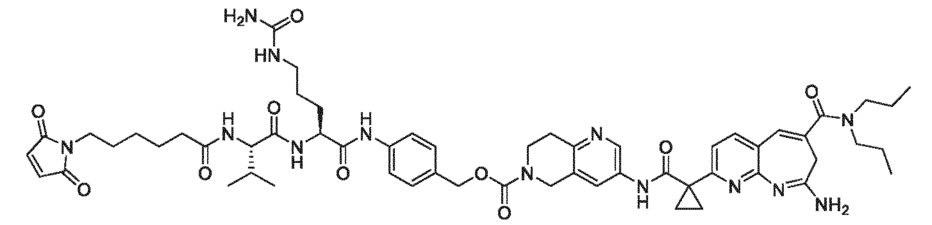 | 1100.5 |
| II-10 | 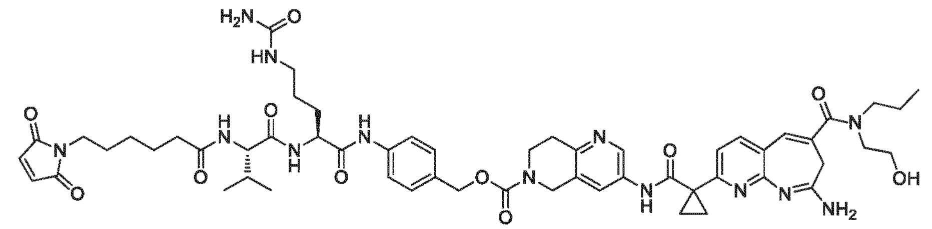 | 1102.5 |
| II-11 | 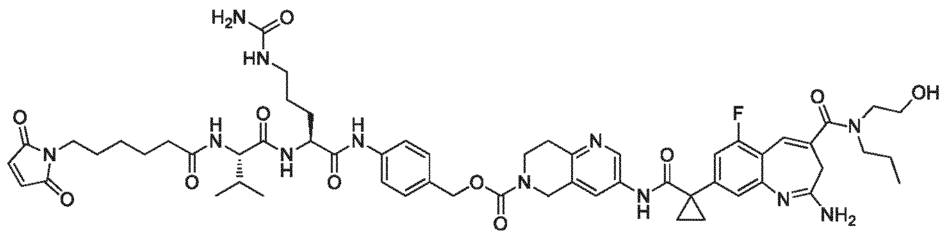 | 1119.6 |
| II-12 | 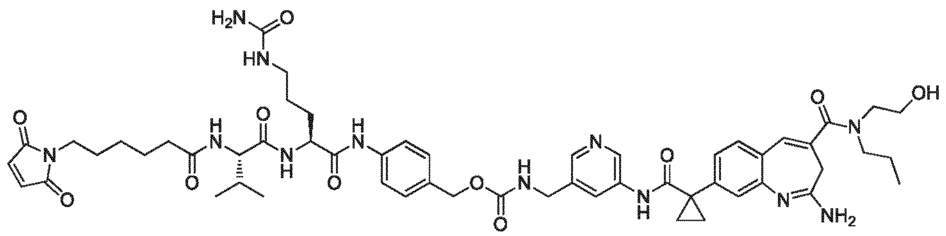 | 1075.7 |
| II-13 | 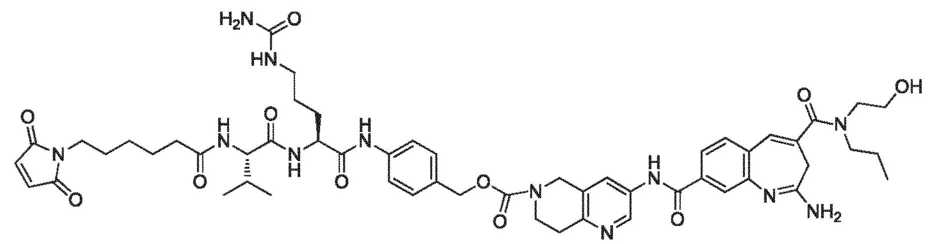 | 1061.7 |

(continued)

| No. | Structure | m/z: [M+H]+ |
|---|---|---|
| II-14 | 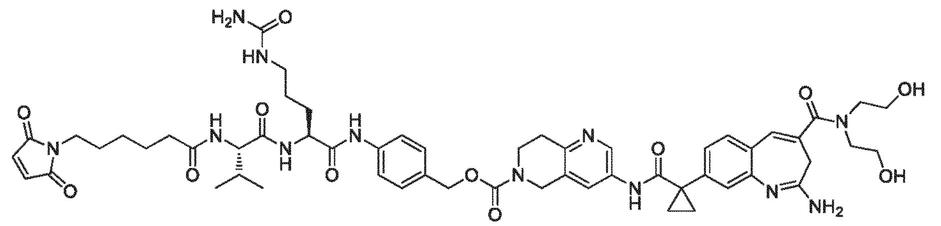 | 1103.4 |
| II-15 | 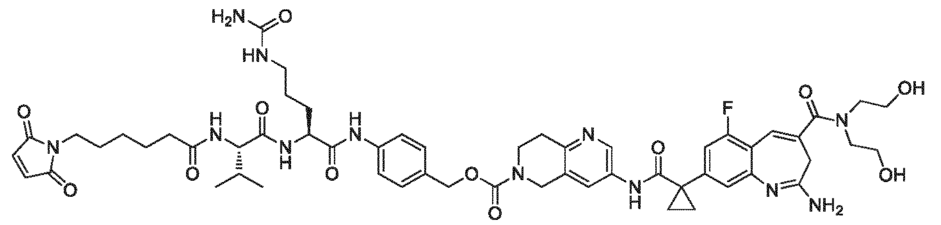 | 1121.5 |
| II-16 | 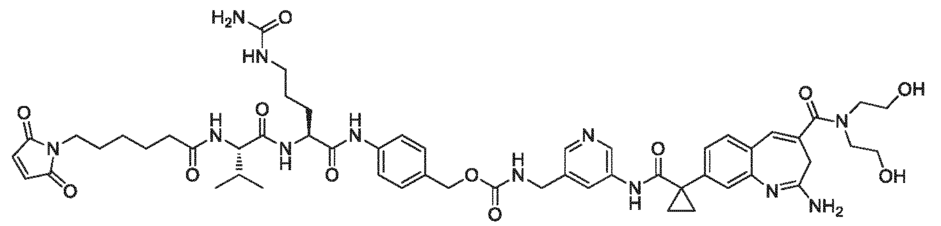 | 1077.2 |
| II-17 | 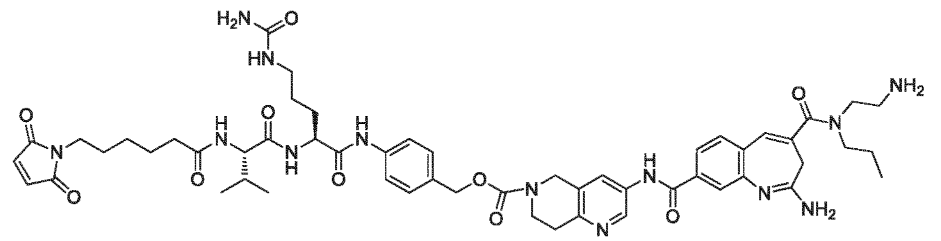 | 1060.5 |
| II-18 | 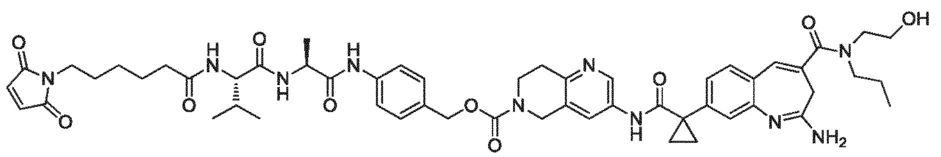 | 1015.2 |
| II-19 | 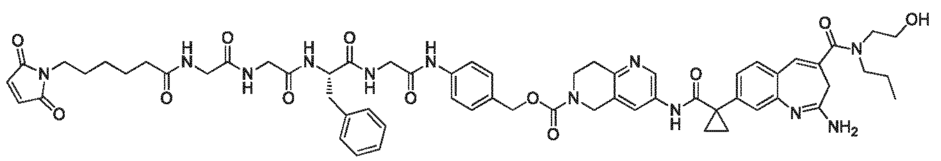 | 1163.2 |
| II-20 | 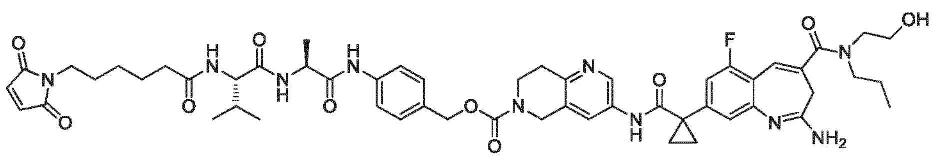 | 1033.2 |
| II-21 | 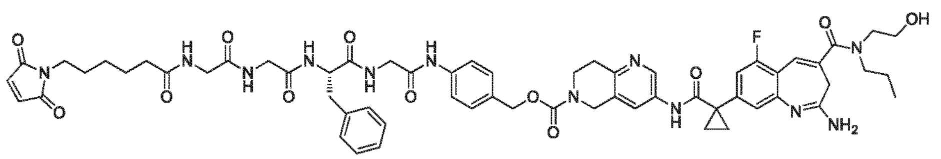 | 1181.1 |

(continued)

| No. | Structure | m/z: [M+H]+ |
|---|---|---|
| II-22 | 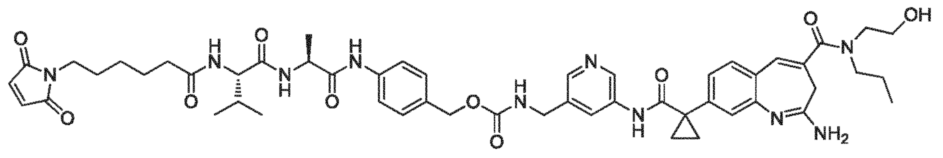 | 989.2 |
| II-23 | 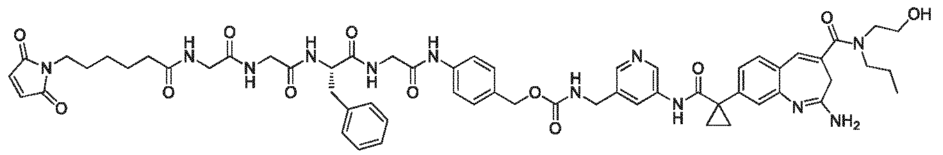 | 1137.3 |
| II-24 | 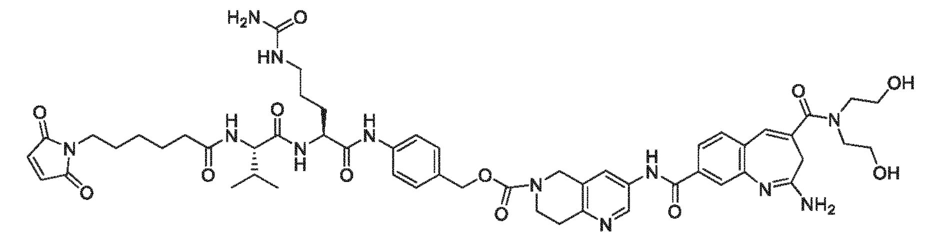 | 1063.4 |

**Example 43: Antibody-drug conjugation reaction**

**I. General method for antibody-drug conjugation reaction:**

**1) Determination of antibody concentration:** A UV/visible spectrophotometer (Nanodrop ONE) and Lambert-Beer's law ($A = \varepsilon cl$) are used to determine the concentration of the antibody.

**2) Antibody reduction:** 2 mL of the antibody solution (10.4 mg/mL) is put into the container of Amicon Ultra-15 (30,000 MWCO, Millipore Corporation), and a centrifuge is used to perform centrifugation (centrifuge at 3800 G for 5 minutes), and the antibody solution is concentrated. The concentration of the antibody solution is determined to be 25 mg/mL using the determination method for the antibody concentration. A NAP-25 chromatography column (5 mL/12 mL) loaded with Sephadex G-25 carrier is equilibrated with phosphate buffer solution (50 mM, pH 6.5; referred to as PBS6.5/EDTA) containing sodium chloride (50 mM) and EDTA (2 mM). One of the NAP-25 columns is loaded with 1.27 mL of the antibody aqueous solution, and then the fraction eluted with PBS6.5/EDTA is obtained via separation. The concentration of the antibody solution is determined to be 13 mg/mL using the determination method for the antibody concentration, and then the antibody concentration is adjusted to 10 mg/mL using PBS 6.5/EDTA. The solution is taken into a 15 mL tube, and after the addition of TCEP (74 μL, 2 mg/mL, sigma-aldrich) aqueous solution (5 eq. relative to one molecule of antibody), the mixture is incubated at 25°C for 2 hours.

**3) Antibody conjugation:** After the reduction reaction is completed, the concentration of the reduced antibody is adjusted to 5 mg/mL using 3 mL of PBS6.5/EDTA solution. 179 μL of *N,N*-dimethylacetamide (DMA) is added thereto, followed by 121 μL (10 mg/mL; 10 eq. relative to one molecule of antibody) of a dimethyl sulfoxide solution of the compound of formula II (linker-payload), with the final proportion of DMA controlled at about 10%. The mixture is stirred for 60 minutes at room temperature to conjugate the drug linker head to the antibody.

**4) Purification of antibody conjugation sample:** The NAP-25 chromatographic column is equilibrated with phosphate buffer (PB 7.4). 6.6 mL of the antibody-drug conjugate aqueous solution is loaded into this NAP-25 column, and then the fraction eluted with PB 7.4 is obtained via separation. After repeating this operation 2 to 3 times, the eluted fractions are collected, and Amicon Ultra-15 (30000MWCO, Millipore Corporation) is used to concentrate the coupling solution by dialysis, and then the residue is sterile-filtered. Sample concentrations are determined using the antibody-drug conjugate concentration determination method.

**II: Determination of antibody concentration in antibody-drug conjugate**

The concentration of the linked drug in the antibody-drug conjugate could be calculated by determining the UV

absorbance at 280 nm of an aqueous solution of the antibody-drug conjugate, and then performing the following calculations, from which it was calculated.

Since the total absorbance at a given wavelength is equal to the sum of the absorbances of all absorbing chemical species present in the system (additivity of absorbance), the antibody concentration and the drug concentration in the antibody-drug conjugate are shown in the following equation, assuming that the molar absorbance coefficients of the antibody and the drug do not change before and after the antibody-drug conjugation.

$$A_{280} = A_{Drug,280} + A_{ab,280} = \varepsilon_{Drug}^{280} \times c_{Drug} + \varepsilon_{ab}^{280} \times c_{ab} = \varepsilon_{Drug}^{280} \times c_{ab} \times DAR + \varepsilon_{ab}^{280} \times c_{ab}$$

Therefore, the concentration of the immune-stimulating antibody conjugate is

$$c_{ab} = \frac{A_{280}}{\left(\varepsilon_{ab}^{280} + \varepsilon_{Drug}^{280} \times DAR\right)}$$

wherein $\varepsilon_{Drug}^{280} = 4741$, DAR (the average number of drugs linked to each antibody molecule in the immune-stimulating antibody conjugate) is determined as described in Common Operation III.

**III: Determination of the average number of drugs linked to each antibody molecule (DAR) in the antibody-drug conjugate**

For the average number of drugs linked to each antibody molecule in the antibody-drug conjugate, it could be determined by high performance liquid chromatography (HPLC) analysis using the following method.

1): Pretreatment of samples for analysis

The sample was diluted to 5 mg/mL and added to a sample vial.

2): HPLC analysis

HPLC instrument: Waters/Waters e2695
Mobile phase A: 1.5 M $(NH_4)_2SO_4$ + 50 mM potassium phosphate (pH 7.0)
Mobile phase B: 50 mM sodium phosphate (pH 7.0)/isopropanol (75:25 V/V)
Analytical column: Thermo MabPac™ HIC-Butyl 5 μm 4.6 × 100 mm, PN. 088558
Injection volume: 5 μL
Flow rate: 1 mL/min
Column temperature: 30°C
Detector: PDA detector
Detection wavelength: 280 nm

Elution gradient:

| Time | Mobile phase A/% | Mobile phase B/% |
|---|---|---|
| 0.00 | 100 | 0 |
| 2.00 | 80 | 20 |
| 22.00 | 20 | 80 |
| 24.00 | 0 | 100 |
| 26.00 | 100 | 0 |
| 30.00 | 100 | 0 |

3): Data analysis

Hydrophobic interaction chromatography could be used to determine the drug-antibody conjugation ratio (DAR)

in antibody-drug conjugates. Unconjugated antibody drugs have the weakest hydrophobicity and are eluted first; antibodies linked with 8 drugs have the strongest hydrophobicity and are eluted last. The peak area percentage represented the relative distribution of ADCs with a specific number of linked drugs, and the weighted average DAR was calculated by the peak area percentage and the number of linked drugs, DAR = Σ**(relative peak area * number of linked drugs)/total peak area.**

**IV: Analysis of molecular size heterogeneity (SEC) of immune-stimulating antibody conjugate**

1): Pretreatment of samples for analysis
The sample was diluted to about 2.0 mg/mL with mobile phase A. The appropriate amount of dilution was centrifuged at 12000 rpm for 5 minutes, and the supernatant was introduced as a sample for analysis.
2): HPLC analysis

HPLC instrument: Waters Acquity Arc
Mobile phase: 100 mM PB + 200 mM Arg·HCl + 5% IPA, pH 6.8
Analytical column: TOSOH TSKgel G3000 SWxl, 7.8 * 300 mm, 5 μM, 450 Å, PN0008541
Injection volume: 10 μL
Flow rate: 0.6 mL/min
Column temperature: 30°C
Detector: PDA detector
Detection wavelength: 280 nm
Gradient: isocratic elution

3): Data analysis
The chromatogram was integrated by comparing the chromatogram with that of the blank buffer, and the peak area percentages of monomer, polymer, and impurities with low molecular weight were calculated by peak area normalization method, respectively.

**Examples 44 to 66: Preparation of III-1 to III-23**

III-1 to III-23 were prepared using an antibody (Trastuzumab, Shanghai Roche Pharmaceutical Co., Ltd.) and compounds II-1 to II-23, and according to the general preparation method of example 43.

Table 3:

| No. | Structure | DAR | SEC |
|---|---|---|---|
| III-1 | 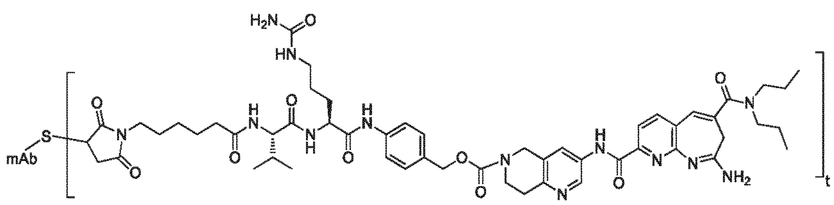 | 4.27 | 100% |
| III-2 | 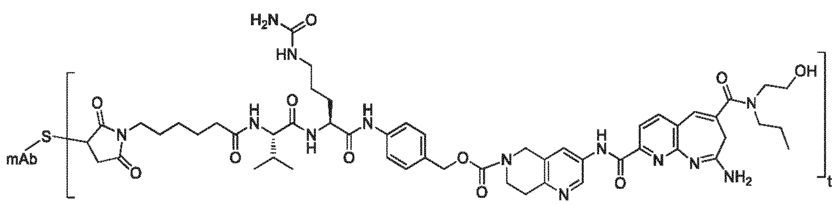 | 4.39 | 100% |

(continued)

| No. | Structure | DAR | SEC |
|---|---|---|---|
| III-3 | 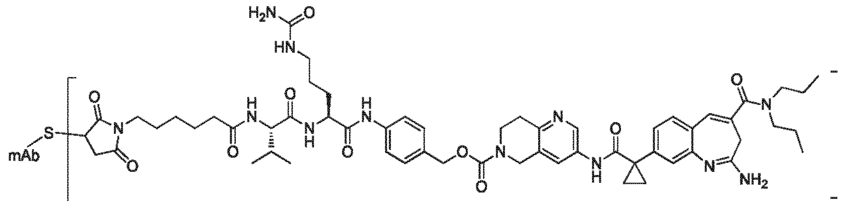 | 4.14 | 100% |
| III-4 | 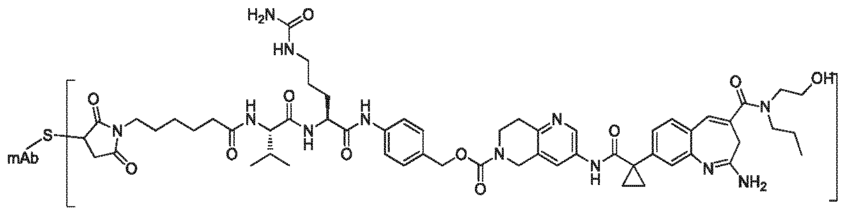 | 4.57 | 100% |
| III-5 | 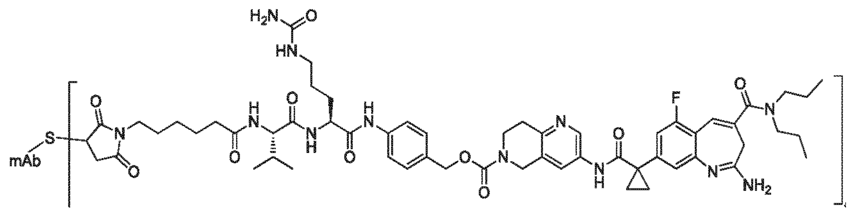 | 4.14 | 100% |
| III-6 | 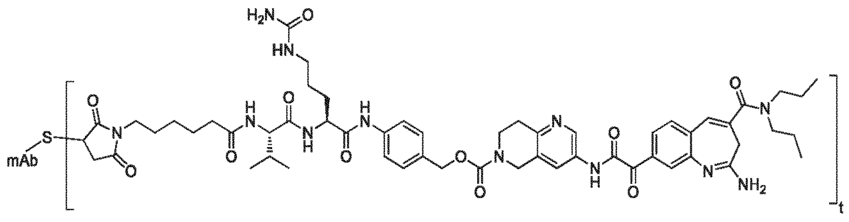 | 4.23 | 98.2% |
| III-7 | 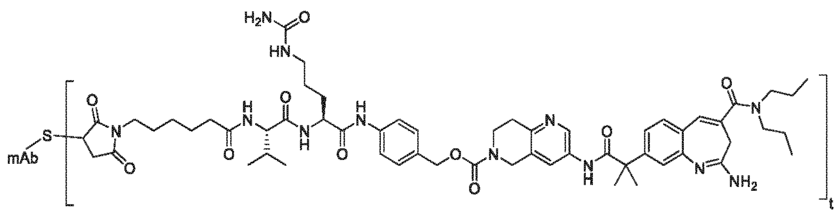 | 4.29 | 96.1% |
| III-8 | 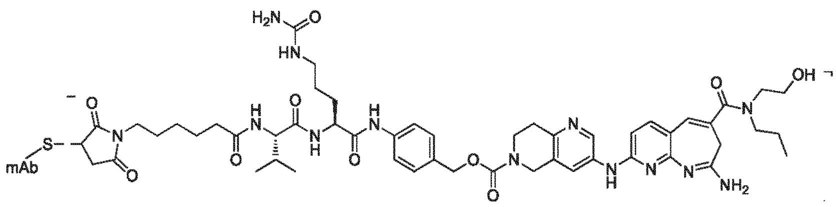 | 4.04 | 100% |
| III-9 | 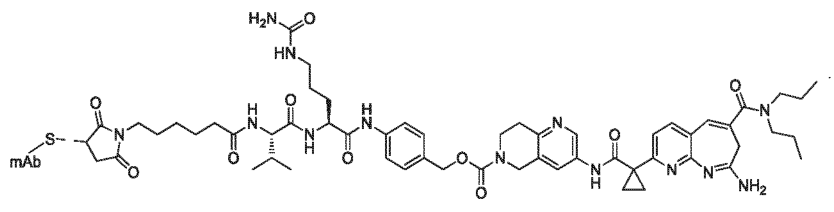 | 4.16 | 99.2% |

(continued)

| No. | Structure | DAR | SEC |
|---|---|---|---|
| III-10 | 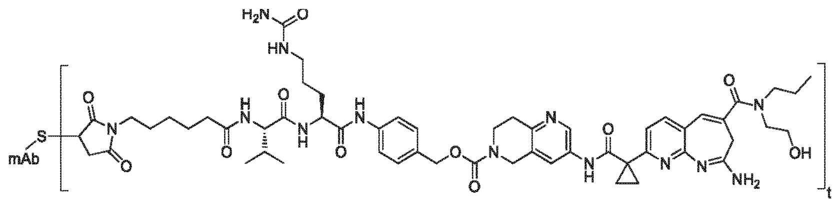 | 4.28 | 98.9% |
| III-11 | 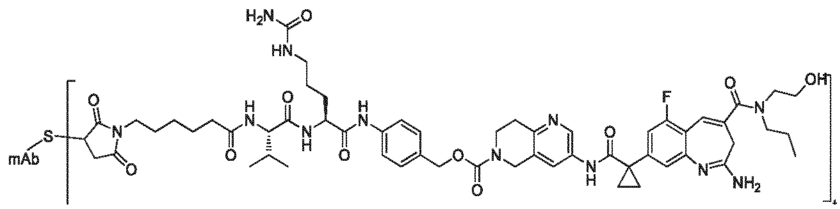 | 3.93 | 99.0% |
| III-12 | 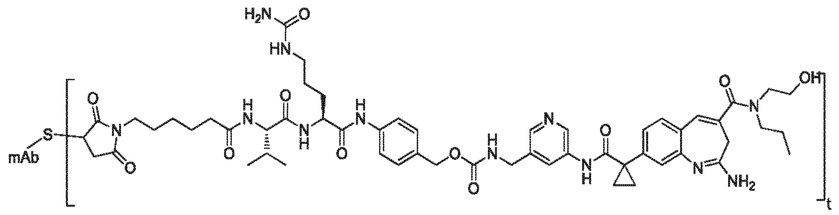 | 4.72 | 99.3% |
| III-13 | 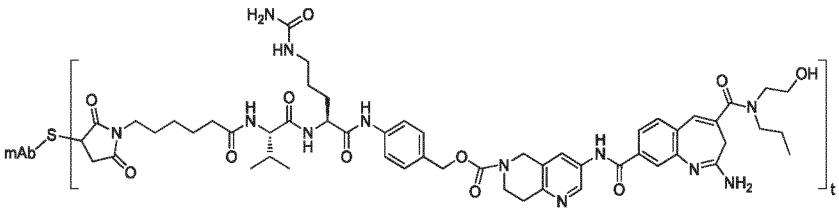 | 4.61 | 100% |
| III-14 | 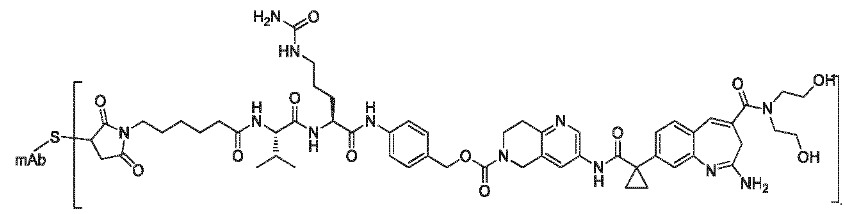 | 4.45 | 99.4% |
| III-15 | 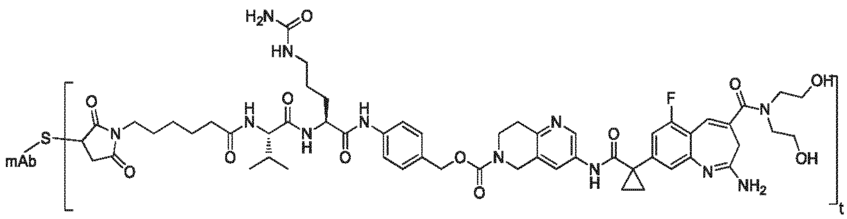 | 4.39 | 99.5% |
| III-16 | 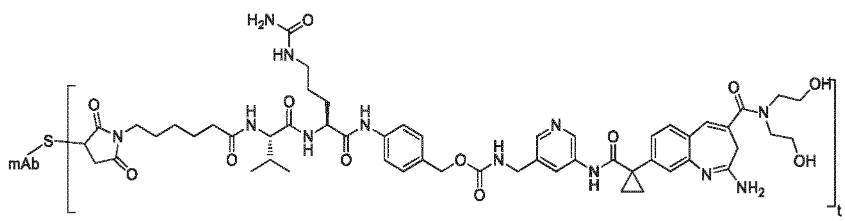 | 4.35 | 99.5% |

(continued)

| No. | Structure | DAR | SEC |
|---|---|---|---|
| III-17 | 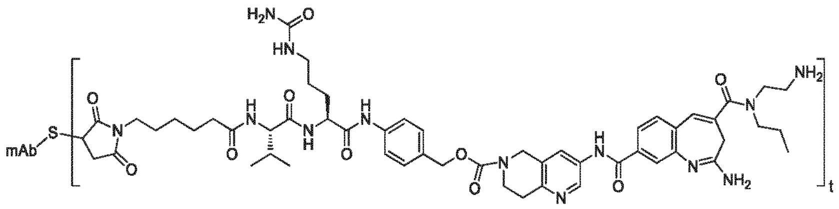 | 3.21 | 100% |
| III-18 | 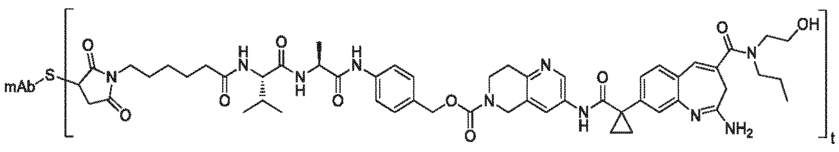 | 4.03 | 99.4% |
| III-19 | 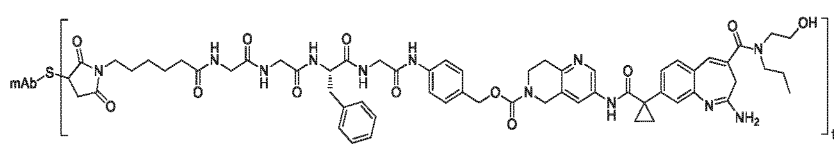 | 3.86 | 99.5% |
| III-20 | 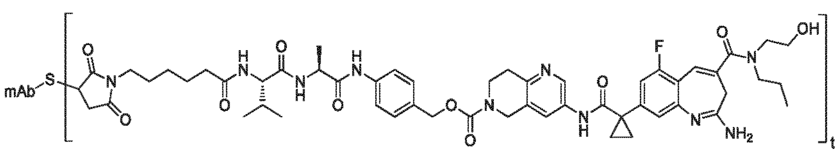 | 3.98 | 99.5% |
| III-21 | 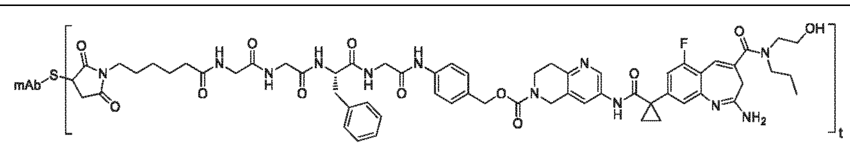 | 4.03 | 99.5% |
| III-22 | 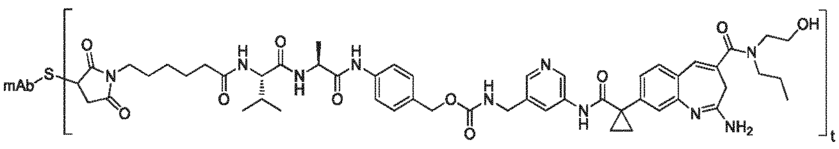 | 4.12 | 99.6% |
| III-23 | 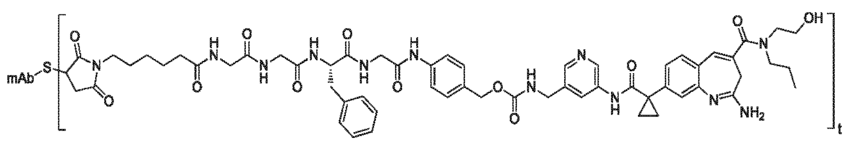 | 4.06 | 99.5% |

**Example 67: Synthesis of III-24**

III-24 was prepared using an antibody (Trastuzumab-LALA, B801901, Shanghai Biointron Biotechnology Co., Ltd.) and compound II-4, and according to the general preparation method of example 43, with a DAR value of 4.53.

**Example 68: Synthesis of Ref. A**

Ref. A was prepared using an antibody (Trastuzumab, Shanghai Roche Pharmaceutical Co., Ltd.) and compound A (compound 2.14 in CN110612104A), and according to the general preparation method of example 43, with a DAR value of 3.21.

**Effect Example 1: Cell activity assays of TLR7 and TLR8**

In this experiment, TLR7 and TLR8 bioactivity assays of the compound of formula I were determined using a

cellular test. This method was performed in human embryonic kidney cells (HEK293) expressing TLR family members such as TLR4, TLR7, TLR8, or TLR9. TLR agonist activates TLR, causing downstream NF-kB activation, thus activating the secreted embryonic alkaline phosphatase (SEAP) reporter gene. Quanti-Blue (InvivoGen) reagent was used to assay the SEAP activity, thus reflecting the activity of TLR7 and TLR8 agonists.

The detailed experimental method is as follows:
HEK-BLUE-hTLR7 and HEK-BLUE-hTLR8 cell lines were purchased from Invivogen company and cultured in DMEM medium containing 4.5 g/L glucose (Sigma-Aldrich) and 10% fetal bovine serum under a condition of a temperature of 37°C, a humidity of 95%, and 5% $CO_2$.

The test concentration of the compound ranged from 0.5 nM to 15 μM, with a total of 10 concentration gradients. TLR7 or TLR8 agonists with known activity were added thereto as positive controls, and 1 μL of DMSO as a negative control.

Cell treatment was as follows: The cells were removed from the incubator and centrifuged to remove the culture medium, resuspended in a T-150 flask with 10 mL of pre-warmed PBS. 12 mL of pre-warmed culture medium was added thereto, and the cells were pipetted gently up and down, and counted under a microscope. A single cell suspension of 200,000 cells/mL was prepared immediately with culture medium, and 200 μL/well (40,000 cells/well) of the cell suspension was added to a 96-well plate. The final concentration of DMSO was 0.5%.

The compound was added thereto, and the mixture was incubated in a 5% $CO_2$ incubator at 37°C for 24 hours.

20 μL/well of supernatant was pipetted into 180 μL of Quanti-Blue preheated to 37°C, and the mixture was incubated at 37°C for 1.5 hours, and the absorbance (OD value) at 650 nm was measured with a spectrophotometer. The calculation formula for the agonistic effect is as follows:

Effect % = (average OD value of the administration group - average OD value of the DMSO group) / (average OD value of the positive drug group - average OD value of the DMSO group) × 100

The concentration-effect curves were fitted with Graphpad software, and $EC_{50}$ was calculated. The test results are shown in Table 4 (A represents <100 nM, B represents 100 nM to 250 nM, C represents 250 nM to 1 μM, D represents >2 μM, / represents not tested). Except for the compounds in Table 4, the rest as shown in formula I also possessed better activity:

Table 4:

| Compound No. | TLR8 $EC_{50}$ | TLR7 $EC_{50}$ |
|---|---|---|
| 1 | C | / |
| 3 | B | D |
| 4 | C | D |
| 5 | A | D |
| 11 | C | D |
| 12 | B | / |
| 13 | C | D |

**Effect Example 2: Activity screening in hPBMC system**

Frozen human peripheral blood mononuclear cells (Allcells) were rapidly thawed in a 37°C water bath and added with 9 mL of RPMI 1640 medium containing 10% fetal bovine serum, 100 U/mL penicillin, 100 μg/mL streptomycin, and 1 mM sodium pyruvate (all from Gibco). The mixture was centrifuged at 400 × g for 5 minutes at room temperature, followed by discarding the supernatant. The cells were resuspended in the culture medium, and the cell density was adjusted to 4 × $10^6$/mL. To a 96-well flat-bottom plate (Corning) was added the mixture at 50 μL per well. To the culture system was added 50 μL of the culture medium.

The test sample dilution was prepared at three times the working concentration using the culture medium and added to the cell suspension at 50 μL per well. The total system was 150 μL, and the final concentration of the compound of formula I was 400 nM. 50 μL of the culture medium was supplemented to the blank control, and the mixture was incubated in a 5% $CO_2$ incubator at 37°C for 20 hours. The mixture was centrifuged at 500 × g for 5 minutes at room temperature, and the supernatant was collected. The concentrations of TNF-α were detected and analyzed by HTRF (Cisbio) and Infinite M1000 PRO (TECAN). The results showed that in the human peripheral blood mononuclear cell system, the TNF-α secretion values of compounds 6, 7, 9, and 10 were between 500 pg/mL to 2500 pg/mL at a concentration of 400 nM; the other compounds of formula I also possessed better safety, such as TNF-α secretion value between 0 pg/mL to 10000 pg/mL.

**Effect Example 3: Activity Screening in hPBMC and BT474 co-culture system**

Frozen human peripheral blood mononuclear cells (Allcells) were rapidly thawed in a 37°C water bath and added with 9 mL of RPMI 1640 medium containing 10% fetal bovine serum, 100 U/mL penicillin, 100 μg/mL streptomycin, and 1 mM sodium pyruvate (all from Gibco). The mixture was centrifuged at 400 × g for 5 minutes at room temperature, followed by discarding the supernatant. The cells were resuspended in the culture medium, and the cell density was adjusted to 4 × $10^6$/mL. To a 96-well flat-bottom plate (Corning) was added the mixture at 50 μL per well. Human breast cancer epithelial cells BT474 (Nanjing Cobioer Biosciences Co., Ltd.) in normal growth state were collected, resuspended in RPMI 1640 complete medium, and adjusted to a density of 4 × $10^5$/mL. 50 μL of the mixture was added to each well of a 96-well plate and mixed with hPBMC.

The test sample dilution was prepared at three times the working concentration using the culture medium and added to the cell suspension at 50 μL per well. The total system was 150 μL, and the final concentration of the compound of formula III was 500 nM. 50 μL of the culture medium was supplemented to the blank control, and the mixture was incubated in a 5% $CO_2$ incubator at 37°C for 20 hours. The mixture was centrifuged at 500 × g for 5 minutes at room temperature, and the supernatant was collected. The concentrations of TNF-α and IFN-γ were detected and analyzed by HTRF (Cisbio) and Infinite M1000 PRO (TECAN). The results showed that in human peripheral blood mononuclear cells and BT474 co-culture system, the TNF-α secretion values of compounds III-6, III-7, III-9, and III-10 were between 5000 pg/mL to 6000 pg/mL at a concentration of 500 nM; the other compounds of formula III also possessed better activity, such as TNF-α secretion value between 0 pg/mL to 10000 pg/mL (but not including 0 pg/mL).

**Effect Example 4: Activity assay in hPBMC system**

Frozen human peripheral blood mononuclear cells (Allcells) were rapidly thawed in a 37°C water bath and added with 9 mL of RPMI 1640 medium containing 10% fetal bovine serum, 100 U/mL penicillin, 100 μg/mL streptomycin, and 1 mM sodium pyruvate (all from Gibco). The mixture was centrifuged at 400 × g for 5 minutes at room temperature, followed by discarding the supernatant. The cells were resuspended in the culture medium, and the cell density was adjusted to 4 × $10^6$/mL. To a 96-well flat-bottom plate (Corning) was added the mixture at 50 μL per well. To the culture system was added 50 μL of the culture medium.

The test sample dilution was prepared at three times the working concentration using the culture medium and added to the cell suspension at 50 μL per well. The total system was 150 μL, and the final concentration of the compound of formula III was 100 nM to 0.032 nM (6 gradients) and the final concentration of the compound of formula I was 400 nM to 0.128 nM (6 gradients). 50 μL of the culture medium was supplemented to the blank control, and the mixture was incubated in a 5% $CO_2$ incubator at 37°C for 20 hours. The mixture was centrifuged at 500 × g for 5 minutes at room temperature, and the supernatant was collected. The concentrations of TNF-α and IFN-γ were detected and analyzed by HTRF (Cisbio) and Infinite M1000 PRO (TECAN). The test results of TNF-α are shown in Table 5 (D represents >400 nM).

**Effect Example 5: Activity assay in hPBMC and BT474 co-culture system**

Frozen human peripheral blood mononuclear cells (Allcells) were rapidly thawed in a 37°C water bath and added with 9 mL of RPMI 1640 medium containing 10% fetal bovine serum, 100 U/mL penicillin, 100 μg/mL streptomycin, and 1 mM sodium pyruvate (all from Gibco). The mixture was centrifuged at 400 × g for 5 minutes at room temperature, followed by discarding the supernatant. The cells were resuspended in the culture medium, and the cell density was adjusted to 4 × $10^6$/mL. To a 96-well flat-bottom plate (Corning)was added the mixture at 50 μL per well. Human breast cancer epithelial cells BT474 (Nanjing Cobioer Biosciences Co., Ltd.) in normal growth state were collected, resuspended in RPMI 1640 complete medium, and adjusted to a density of 4 × $10^5$/mL. 50 μL of the mixture was added to each well of a 96-well plate and mixed with hPBMC.

The test sample dilution was prepared at three times the working concentration using the culture medium and

added to the cell suspension at 50 μL per well. The total system was 150 μL, and the final concentration of the compound of formula III was 100 nM to 0.032 nM (6 gradients). 50 μL of the culture medium was supplemented to the blank control, and the mixture was incubated in a 5% $CO_2$ incubator at 37°C for 20 hours. The mixture was centrifuged at 500 × g for 5 minutes at room temperature, and the supernatant was collected. The concentrations of TNF-α and IFN-γ were detected and analyzed by HTRF (Cisbio) and Infinite M1000 PRO (TECAN). The test results of TNF-α are shown in Table 5 (A represents <1 nM, B represents 1 nM to 10 nM, and C represents >10 nM):

Table 5:

| No. | TNF-α (BT474/hPBMC) $EC_{50}$ | TNF-α (hPBMC) $EC_{50}$ |
|---|---|---|
| III-2 | B | D |
| III-3 | B | D |
| III-4 | A | D |
| III-11 | A | D |
| III-12 | A | D |
| III-13 | A | D |

**Effect Example 6:** PK assay for total antibody concentration

Purpose: PK assay for total antibody concentration of two ISACs and Trastuzumab (mice were injected intraperitoneally at 20 mg/kg).

Steps: A 96-well plate was coated with goat anti-human IgG antibody, placed in a refrigerator for overnight at 4°C, washed three times with PBS/0.05% Tween20 on the next day, and then the plate was sealed with PBS/0.05% Tween20/1% BSA. The mixture was incubated at 37°C for 1 to 2 hours and washed three times. The wells were added with the diluted standards and test samples, and the mixture was incubated at room temperature for 2 hours, washed three times, subsequently added with the buffer-diluted HRP-labeled anti-human IgG antibody, and incubated for 1 hour at room temperature. Finally, the color development solution was added thereto, the reaction was terminated after incubation for 10 minutes at room temperature, the OD value was read at an absorbance of 450 nm, and the experimental results are shown in Table 6:

Table 6:

| Sample | Total antibody | | |
|---|---|---|---|
| | $C_{max}$ (mcg/mL) | t½ (h) | AUC (h*mcg/mL) |
| Trastuzumab (20 mg/kg) | 505 | 86 | 52201 |
| III-4 (20 mg/kg) | 406 | 62 | 29371 |
| Ref.A (20 mg/kg) | 335 | 51 | 19757 |

Conclusion: After intraperitoneal administration of 20 mg/kg to mice, the AUC (total antibody) and $C_{max}$ of III-4 are higher than those of Ref. A. The $t_{1/2}$ of III-4 is longer than that of Ref. A. The AUC, $C_{max}$, and AUC of Trastuzumab are all higher than those of III-4 and Ref.A.

**Effect Example 7:** *In vivo* pharmacodynamic experiment of subcutaneous xenograft tumor model in mice of MC38-HER2 mouse colon cancer cells

Cell culture: MC38-HER2 mouse colon cancer cells were maintained in monolayer culture in DMEM medium containing 10% fetal bovine serum and 4 μg/mL puromycin in a constant temperature incubator containing 5% $CO_2$ at 37°C. Tumor cells were passaged twice a week. Cells in the exponential growth phase were harvested and counted for inoculation.

Experimental animals: B6-hTLR8-HO mice, 6 to 8 weeks, 18 to 22 g.

For Vehicle, III-11, III-12, III-24, and Ref.A, a total of 5 experimental groups are set up as shown in Table 7 below:

Table 7:

| Group | Number of mice | Test Compound | Dosage | Route of administration | Administration plan |
|---|---|---|---|---|---|
| 1 | 6 | Vehicle | -- | i.p. | Single dose |
| 2 | 6 | III-11 | 20 mg/kg | i.p. | Single dose |
| 3 | 6 | III-12 | 20 mg/kg | i.p. | Single dose |
| 4 | 6 | III-24 | 20 mg/kg | i.p. | Single dose |
| 5 | 6 | Ref A | 20 mg/kg | i.p. | Single dose |
| Note: i.p.: intraperitoneal administration | | | | | |

Experimental method: MC38-HER2 cell line ($5.0\times10^6$/mouse) was inoculated subcutaneously on the right back of experimental mice. The inoculation volume of each mouse was 0.1 mL. The growth of the tumor was observed regularly. When the tumor grew to about 100 $mm^3$, the mice were randomly grouped according to the size of the tumor and their body weights, and the drug was administrated in accordance with the administration plan shown in Table 7, and the body weights and tumor sizes of the mice were measured two times per week throughout the experimental process.
Tumor size calculation formula: tumor volume ($mm^3$) = 0.5 $\times$ (tumor long diameter $\times$ tumor short diameter$^2$).
The experimental results are shown in Table 8 and Figure 1:

Table 8:

| Group | Tumor volume (6 days, $mm^3$) | Tumor volume (19 days, $mm^3$) | TGI (%) | T/C (%) | P value |
|---|---|---|---|---|---|
| 1 | 125.74 $\pm$ 10.14 | 1430.28 $\pm$ 271.54 | N/A | N/A | N/A |
| 2 | 125.60 $\pm$ 10.10 | 128.93 $\pm$ 71.83 | 90.98 | 9.02 | 0.0004 |
| 3 | 126.12 $\pm$ 10.79 | 133.86 $\pm$ 133.86 | 90.64 | 9.36 | 0.0008 |
| 4 | 125.92 $\pm$ 10.41 | 142.16 $\pm$ 53.43 | 90.06 | 9.94 | 0.0004 |
| 5 | 125.77 $\pm$ 10.96 | 293.43 $\pm$ 185.79 | 79.48 | 20.52 | 0.0039 |

## Claims

1. A compound of formula I, a solvate thereof, a pharmaceutically acceptable salt thereof, or a solvate of the pharmaceutically acceptable salt thereof;

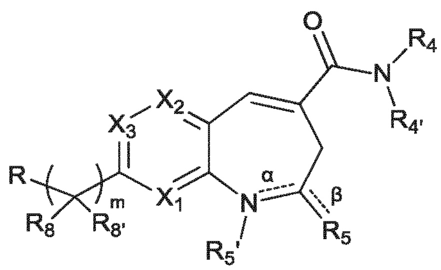

(I)

wherein

$\alpha$ and $\beta$ are independently a single bond or a double bond; at least one selected from the group of $\alpha$ and $\beta$ is a single bond;
m is 0 or 1;
$X_1$ is N or $CR_2$, $X_2$ is N or $CR_3$, and $X_3$ is N or $CR_1$;
R is $-C(O)-L_1-R_7$, $-C(O)-NR_9-L_1-R_7$, $-C(S)-NR_9-L_1-R_7$, $-NR_9-L_1-R_7$, $-NR_9-C(O)-L_1-R_7$, $-NR_9-C(O)-NR_9-L_1-R_7$,

$-O-L_1-R_7$, $-S(O)_2-NR_9-L_1-R_7$, $-CH=CH-L_1-R_7$, or $-S(O)_2-L_1-R_7$;
$R_1$, $R_2$, and $R_3$ are each independently hydrogen, deuterium, halogen, hydroxyl, amino, cyano, alkyl, haloalkyl, $-L_2-OR_a$, or $-L_2-NR_aR_b$;
$R_4$ and $R_{4'}$ are each independently hydrogen, alkyl, alkenyl, alkynyl, cycloalkyl, heterocycloalkyl, aryl, heteroaryl, cycloalkylalkyl, heterocycloalkylalkyl, arylalkyl, or heteroarylalkyl; the $R_4$ or $R_{4'}$ is unsubstituted or optionally substituted at any position by one or more than one substituent selected from halogen, cyano, $-L_2-OR_a$, $-L_2-OC(O)R_a$, $-L_2-NR_aR_b$, $-L_2-NR_aC(O)OR_b$, $-L_2-NR_aC(O)NR_aR_b$, $-L_2-NR_bC(NR_b)NR_aR_b$, and $-L_2-C(O)OR_b$; or, $R_4$ and $R_{4'}$ together with the N atom to which they are attached form a 3- to 8-membered heterocycloalkyl group; the 3- to 8-membered heterocycloalkyl group is unsubstituted or further substituted at any position by 1 to 3 substituents selected from halogen, cyano, $-L_2-OR_a$, $-L_2-OC(O)R_a$, $-L_2-NR_aR_b$, $-L_2-NR_aC(O)OR_b$, $-L_2-NR_aC(O)NR_aR_b$, $-L_2-NR_bC(NR_b)NR_aR_b$, and $-L_2-C(O)OR_b$;
$R_5$ is $=O$, $=NR_a$, $-OR_a$, or $-NR_aR_b$;
$R_{5'}$ is absent, or $R_{5'}$ is $-R_c$, $-L_2-OR_a$, $-L_2-NR_aR_b$, $-L_2-NR_aC(O)OR_b$, $-L_2-NR_aC(O)NR_aR_b$, $- L_2-NR_bC(NR_b)NR_aR_b$, or $-L_2-C(O)OR_b$;
$R_7$ is phenyl, 5- to 6-membered heteroaryl, 3- to 8-membered heterocycloalkyl, or an 8-to 12-membered fused ring group; the $R_7$ is unsubstituted or optionally substituted at any position by one or more than one substituent selected from $-L_3-W$, $-R_c$, halogen, cyano, nitro, alkyl, alkenyl, alkynyl, alkoxy, haloalkyl, haloalkoxy, and alkylamino;
$R_8$ and $R_{8'}$ are each independently hydrogen, halogen, or alkyl, and the $R_8$ or $R_{8'}$ is unsubstituted or optionally substituted at any position by one or more than one substituent selected from $-L_3-W$, halogen, cyano, nitro, alkyl, alkenyl, alkynyl, alkoxy, haloalkyl, haloalkoxy, and alkylamino; $R_8$ and $R_{8'}$ are each independent substituents, or, $R_8$ and $R_{8'}$ together with the carbon atom to which they are attached form an oxo, thio, $C_{1-6}$ alkylene, $C_{3-10}$ cycloalkyl, or 3- to 10-membered heterocycloalkyl group; the $C_{1-6}$ alkylene, $C_{3-10}$ cycloalkyl, or 3- to 10-membered heterocycloalkyl group is unsubstituted or optionally substituted at any position by one or more than one substituent selected from deuterium, halogen, hydroxyl, amino, cyano, oxo, alkyl, haloalkyl, $-L_2-OR_a$, and $-L_2-NR_aR_b$;
$R_9$ is hydrogen, alkyl, haloalkyl, $-L_2-OR_a$, or $-L_2-NR_aR_b$;
W is $Cy^1$, -SRa, $-OR_d$, $-OC(O)R_e$, $-OC(O)NR_eR_{e'}$, $-C(O)OR_e$, $-C(O)R_e$, $-C(O)NR_eR_{e'}$, $- C(O)NR_eS(O)_2R_e$, $-NR_dR_e$, $-NR_dC(O)R_e$, $-N(R_d)C(O)OR_e$, $-N(R_d)C(O)NR_eR_{e'}$, $-NR_dS(O)_2R_e$, $- NR_dS(O)_2NR_eR_{e'}$, $-S(O)_{1-2}R_e$, $-S(O)_2NR_eR_{e'}$, $-S(O)(=NR_d)R_e$, $-S(O)_2N(R_e)C(O)R_{e'}$, $- P(O)(OR_e)_2$, $-P(O)(OR_e)R_{e'}$, $-OP(O)(OR_e)_2$, or $-B(OR_e)_2$;
$Cy^1$ is cycloalkyl, heterocycloalkyl, aryl, or heteroaryl; the $Cy^1$ is unsubstituted or optionally substituted at any position by one or more than one substituent selected from halogen, alkyl, haloalkyl, haloalkoxy, alkenyl, alkynyl, cyano, $-R_c$, $-L_4-SR_d$, $-L_4-OC(O)R_e$, $-L_4-C(O)OR_e$, $-L_4-C(O)R_e$, $-L_4-C(O)NR_eR_{e'}$, $-L_4-NR_dC(O)R_e$, $-L_4-NR_dS(O)_2R_e$, $-L_4-S(O)_{1-2}R_e$, $-L_4-S(0)_2NR_eR_{e'}$, $-L_4-OR_d$, and $-L_4-NR_eR_{e'}$;
$L_1$, $L_2$, $L_3$, and $L_4$ are each independently a linkage bond, $C_{1-6}$ alkylene, $C_{2-6}$ alkenylene, or $C_{2-6}$ alkynylene; the $L_1$, $L_2$, $L_3$, or $L_4$ is unsubstituted or optionally substituted at any position by one or more than one substituent selected from oxo, hydroxyl, amino, halogen, cyano, alkyl, haloalkyl, alkoxy, and haloalkoxy;
$R_a$, $R_b$, $R_d$, $R_e$, and $R_{e'}$ are each independently $-R_c$, amino, $C_{1-6}$ alkyl, $C_{2-6}$ alkenyl, $C_{2-6}$ alkynyl, $C_{3-10}$ cycloalkyl, 3- to 10-membered heterocycloalkyl, $C_{6-10}$ aryl, 5- to 10-membered heteroaryl, $C_{3-10}$ cycloalkyl-$C_{1-6}$ alkyl, 3- to 10-membered heterocycloalkyl-$C_{1-6}$ alkyl, phenyl-$C_{1-6}$ alkyl, or 5- to-10 membered heteroaryl-$C_{1-6}$ alkyl; the $R_a$, $R_b$, $R_d$, $R_e$, or $R_{e'}$ is unsubstituted or optionally substituted at any position by 1 to 3 substituents selected from $-OR_f$, $-OC(O)-L_4-R_f$, $-NR_fR_{f'}$, halogen, cyano, nitro, $C_{1-6}$ alkyl, halo-$C_{1-6}$ alkyl, and halo-$C_{1-6}$ alkoxy;
Rf and $R_{f'}$ are each independently $-R_c$, $-NHR_c$, or $C_{1-6}$ alkyl;
each $R_c$ is independently hydrogen;
the compound of formula I satisfies 1, 2, 3, or 4 of the following conditions:

(1) m is 1;
(2) R is $-C(S)-NR_9-L_1-R_7$, $-NR_9-L_1-R_7$, $-NR_9-C(O)-L_1-R_7$, $-NR_9-C(O)-NR_9-L_1-R_7$, $-O-L_1-R_7$, $-S(O)_2-NR_9-L_1-R_7$, $-CH=CH-L_1-R_7$, or $-S(O)_2-L_1-R_7$;
(3) at least one selected from the group of $X_1$, $X_2$ and $X_3$ is N;
(4) at least one selected from the group of $R_4$ and $R_{4'}$ is substituted at any position by one or more than one substituent selected from halogen, cyano, $-L_2-OR_a$, $-L_2-OC(O)R_a$, $-L_2-NR_aR_b$, $-L_2-NR_aC(O)OR_b$, $-L_2-NR_aC(O)NR_aR_b$, $-L_2-NR_bC(NR_b)NR_aR_b$, and $-L_2-C(O)OR_b$.

**2.** The compound of formula I, the solvate thereof, the pharmaceutically acceptable salt thereof, or the solvate of the pharmaceutically acceptable salt thereof according to claim 1, wherein the compound of formula I satisfies one or more than one of the following conditions:

a) in $R_7$, the 8- to 12-membered fused ring group is ring A-fused ring B, the ring A is 5-to 6-membered heteroaryl, and the ring B is 5- to 6-membered heterocycloalkene; in the 5- to 6-membered heteroaryl, the heteroatom is selected from one or more than one of N, O, and S, and the number of heteroatoms is 1, 2, or 3; in the 5- to 6-membered heterocycloalkene, the heteroatom is selected from one or more than one of N, O, and S, and the number of heteroatoms is 1, 2, or 3;
b) in $R_7$, in the 5- to 6-membered heteroaryl, the heteroatom is selected from one or more than one of N, O, and S, and the number of heteroatoms is 1, 2, or 3;
c) in $L_3$, $C_{1-6}$ alkylene is $C_{1-3}$ alkylene;
d) in $R_8$ and $R_{8'}$, the alkyl is $C_{1-6}$ alkyl;
e) the $C_{3-10}$ cycloalkyl group formed by $R_8$ and $R_{8'}$ together with the carbon atom to which they are attached is a saturated monocyclic group;
f) the $C_{3-10}$ cycloalkyl group formed by $R_8$ and $R_{8'}$ together with the carbon atom to which they are attached is a $C_{3-6}$ cycloalkyl group;
g) in $R_1$, $R_2$, and $R_3$, the halogen is fluorine, chlorine, bromine, or iodine;
h) in $R_4$ and $R_{4'}$, the alkyl is $C_{1-6}$ alkyl.

**3.** The compound of formula I, the solvate thereof, the pharmaceutically acceptable salt thereof, or the solvate of the pharmaceutically acceptable salt thereof according to claim 2, wherein the compound of formula I satisfies one or more than one of the following conditions:

i) in $R_7$, the 8- to 12-membered fused ring group is ring A-fused ring B, the ring A is 5-to 6-membered heteroaryl, and the ring B is 5- to 6-membered heterocycloalkene, which is attached to the $L_1$ through the ring A; in the 5- to 6-membered heteroaryl, the heteroatom is N, and the number of heteroatoms is 1 or 2; in the 5- to 6-membered heterocycloalkene, the heteroatom is N, and the number of heteroatoms is 1 or 2;
j) in $R_7$, in the 5- to 6-membered heteroaryl, the heteroatom is N, and the number of heteroatoms is 1 or 2;
k) in $L_3$, the $C_{1-6}$ alkylene is $-CH_2-$, $-CH_2CH_2-$, or $-CH_2CH_2CH_2-$;
l) in $R_8$ and $R_{8'}$, the alkyl is methyl, ethyl, n-propyl, isopropyl, n-butyl, isobutyl, *sec*-butyl, or *tert*-butyl;
m)the $C_{3-10}$ cycloalkyl group formed by $R_8$ and $R_{8'}$ together with the carbon atom to which they are attached is cyclopropyl, cyclobutyl, cyclopentyl, or cyclohexyl;
n) in $R_1$, $R_2$, and $R_3$, the halogen is fluorine;
o) in $R_4$ and $R_{4'}$, the alkyl is methyl, ethyl, n-propyl, isopropyl, n-butyl, isobutyl, *sec*-butyl, or *tert*-butyl.

**4.** The compound of formula I, the solvate thereof, the pharmaceutically acceptable salt thereof, or the solvate of the pharmaceutically acceptable salt thereof according to claim 3, wherein the compound of formula I satisfies one or more than one of the following conditions:

p) in $R_7$, the 8- to 12-membered fused ring group is

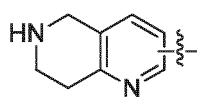
;

q) in $R_7$, the 5- to 6-membered heteroaryl is pyridyl;
r) $R_4$ is $-CH_2CH_2OH$ or $-CH_2CH_2CH_3$;
s) $R_{4'}$ is $-CH_2CH_2OH$ or $-CH_2CH_2CH_3$.

**5.** The compound of formula I, the solvate thereof, the pharmaceutically acceptable salt thereof, or the solvate of the pharmaceutically acceptable salt thereof according to claim 4, wherein the compound of formula I satisfies one or more than one of the following conditions:

t) in $R_7$, the 8- to 12-membered fused ring group is

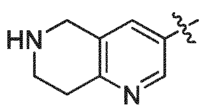
;

u) in $R_7$, the 5- to 6-membered heteroaryl substituted by one $-L_3$-W is

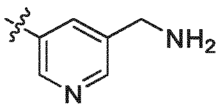

;

v) $R_4$ is $-CH_2CH_2OH$ or $-CH_2CH_2CH_3$; $R_{4'}$ is $-CH_2CH_2OH$ or $-CH_2CH_2CH_3$.

**6.** The compound of formula I, the solvate thereof, the pharmaceutically acceptable salt thereof, or the solvate of the pharmaceutically acceptable salt thereof according to at least one selected from the group of claims 1 to 5, wherein the compound of formula I satisfies one or more than one of the following conditions:

w) m is 1; $R_8$ and $R_{8'}$ are each independently unsubstituted alkyl; or, $R_8$ and $R_{8'}$ together with the carbon atom to which they are attached form an oxo or unsubstituted $C_{3-10}$ cycloalkyl group;
x) R is $-C(S)-NR_9-L_1-R_7$, $-NR_9-L_1-R_7$, $-NR_9-C(O)-L_1-R_7$, $-NR_9-C(O)-NR_9-L_1-R_7$, $-O-L_1-R_7$, $-S(O)_2-NR_9-L_1-R_7$, $-CH=CH-L_1-R_7$, or $-S(O)_2-L_1-R_7$;
y) at least one selected from the group of $X_1$, $X_2$, and $X_3$ is N;
z) at least one selected from the group of $R_4$ and $R_{4'}$ is substituted at any position by one or more than one substituent selected from halogen, cyano, $-L_2-OR_a$, $-L_2-OC(O)R_a$, $-L_2-NR_aR_b$, $-L_2-NR_aC(O)OR_b$, $-L_2-NR_aC(O)NR_aR_b$, $-L_2-NR_bC(NR_b)NR_aR_b$, and $-L_2-C(O)OR_b$.

**7.** The compound of formula I, the solvate thereof, the pharmaceutically acceptable salt thereof, or the solvate of the pharmaceutically acceptable salt thereof according to claim 6, wherein the compound of formula I satisfies one or more than one of the following conditions:

aa) R is $-C(S)-NR_9-L_1-R_7$ or $-NR_9-L_1-R_7$;
bb) $X_1$ is N;
cc) at least one selected from the group of $R_4$ and $R_{4'}$ is substituted at any position by one or more than one substituent selected from $-L_2-OR_a$ and $-L_2-NR_aR_b$.

**8.** The compound of formula I, the solvate thereof, the pharmaceutically acceptable salt thereof, or the solvate of the pharmaceutically acceptable salt thereof according to claim 7, wherein the compound of formula I satisfies one or more than one of the following conditions:

dd) R is $-NR_9-L_1-R_7$;
ee) $X_1$ is N, $X_2$ and $X_3$ are CH;
ff) $R_4$ and $R_{4'}$ are each independently alkyl; at least one selected from the group of $R_4$ and $R_{4'}$ is substituted at any position by one or more than one substituent selected from $-L_2-OR_a$ and $-L_2-NR_aRb$.

**9.** The compound of formula I, the solvate thereof, the pharmaceutically acceptable salt thereof, or the solvate of the pharmaceutically acceptable salt thereof according to at least one selected from the group of claims 1 to 5, wherein the compound of formula I satisfies one or more than one of the following conditions:

gg) R is $-C(O)-NR_9-L_1-R_7$, $-C(S)-NR_9-L_1-R_7$, or $-NR_9-L_1-R_7$; $R_9$ is hydrogen, $L_1$ is a linkage bond; $R_7$ is an unsubstituted 8- to 12-membered fused ring group, or 5- to 6-membered heteroaryl substituted by one $-L_3$-W; $L_3$ is $C_{1-6}$ alkylene, W is $-NR_dR_e$; $R_d$ and $R_e$ are $-R_c$, and $R_c$ is hydrogen;
hh) m is 0 or 1; $R_8$ and $R_{8'}$ are each independently unsubstituted alkyl; or, $R_8$ and $R_{8'}$ together with the carbon atom to which they are attached form an oxo or unsubstituted $C_{3-10}$ cycloalkyl group;
ii) $X_1$ is N or $CR_2$, $X_2$ is N or $CR_3$, and $X_3$ is N or $CR_1$; $R_1$, $R_2$, and $R_3$ are each independently hydrogen, deuterium, halogen, or cyano;
jj) $\alpha$ is a double bond and $R_{5'}$ is absent; $\beta$ is a single bond and $R_5$ is $-NR_aR_b$; $R_a$ and $R_b$ are $-R_c$, and $R_c$ is hydrogen;
kk) $R_4$ and $R_{4'}$ are each independently alkyl; the $R_4$ and $R_{4'}$ are each independently unsubstituted or optionally substituted at any position by one or more than one substituent selected from $-L_2-OR_a$ and $-L_2-NR_aR_b$; $L_2$ is a linkage bond; $R_a$ and $R_b$ are $-R_c$, and $R_c$ is hydrogen.

**10.** The compound of formula I, the solvate thereof, the pharmaceutically acceptable salt thereof, or the solvate of the pharmaceutically acceptable salt thereof according to at least one selected from the group of claims 1 to 5, wherein

the definition of the compound of formula I is as described in any one of the following schemes:

scheme 10-1:

R is -C(O)-NR$_9$-L$_1$-R$_7$, -C(S)-NR$_9$-L$_1$-R$_7$, or -NR$_9$-L$_1$-R$_7$; R$_9$ is hydrogen, L$_1$ is a linkage bond; R$_7$ is an unsubstituted 8- to 12-membered fused ring group, or 5- to 6-membered heteroaryl substituted by one -L$_3$-W; L$_3$ is C$_{1-6}$ alkylene, W is -NR$_d$R$_e$;
m is 0 or 1; R$_8$ and R$_{8'}$ are each independently unsubstituted alkyl; or, R$_8$ and R$_{8'}$ together with the carbon atom to which they are attached form an oxo or unsubstituted C$_{3-10}$ cycloalkyl group;
X$_1$ is N or CR$_2$, X$_2$ is N or CR$_3$, and X$_3$ is N or CR$_1$; R$_1$, R$_2$, and R$_3$ are each independently hydrogen, deuterium, halogen, or cyano;
$\alpha$ is a double bond, and R$_{5'}$ is absent; $\beta$ is a single bond, and R$_5$ is -NR$_a$R$_b$;
R$_4$ and R$_{4'}$ are each independently alkyl; the R$_4$ and R$_{4'}$ are each independently unsubstituted or optionally substituted at any position by one or more than one substituent selected from -L$_2$-OR$_a$ and -L$_2$-NR$_a$R$_b$; L$_2$ is a linkage bond;
R$_a$, R$_b$, R$_d$, and R$_e$ are -R$_c$, and R$_c$ is hydrogen;
the compound of formula I satisfies 1, 2, 3, or 4 of the following conditions:

(1) m is 1;
(2) R is -C(S)-NR$_9$-L$_1$-R$_7$ or -NR$_9$-L$_1$-R$_7$;
(3) at least one selected from the group of X$_1$, X$_2$ and X$_3$ is N;
(4) at least one selected from the group of R$_4$ and R$_{4'}$ is substituted at any position by one or more than one substituent selected from -L$_2$-OR$_a$ and -L$_2$-NR$_a$R$_b$;

scheme 10-2:

$\alpha$ and $\beta$ are independently a single bond or a double bond; at least one selected from the group of $\alpha$ and $\beta$ is a single bond;
m is 0 or 1;
X$_1$ is N or CR$_2$, X$_2$ is N or CR$_3$, and X$_3$ is N or CR$_1$; when X$_1$ is CR$_2$, X$_2$ is CR$_3$, and X$_3$ is CR$_1$, m is 1;
R is -C(O)-L$_1$-R$_7$, -C(O)-NR$_9$-L$_1$-R$_7$, -C(S)-NR$_9$-L$_1$-R$_7$, -NR$_9$-L$_1$-R$_7$, -NR$_9$-C(O)-L$_1$-R$_7$, -NR$_9$-C(O)-NR$_9$-L$_1$-R$_7$, -O-L$_1$-R$_7$, -S(O)$_2$-NR$_9$-L$_1$-R$_7$, -CH=CH-L$_1$-R$_7$, or -S(O)$_2$-L$_1$-R$_7$;
R$_1$, R$_2$, and R$_3$ are each independently hydrogen, deuterium, halogen, hydroxyl, amino, cyano, alkyl, haloalkyl, -L$_2$-OR$_a$, or -L$_2$-NR$_a$R$_b$;
R$_4$ and R$_{4'}$ are each independently hydrogen, alkyl, alkenyl, alkynyl, cycloalkyl, heterocycloalkyl, aryl, heteroaryl, cycloalkylalkyl, heterocycloalkylalkyl, arylalkyl, or heteroarylalkyl; the R$_4$ or R$_{4'}$ is unsubstituted or optionally substituted at any position by one or more than one substituent selected from halogen, cyano, -L$_2$-OR$_a$, -L$_2$-OC(O)R$_a$, -L$_2$-NR$_a$R$_b$, -L$_2$-NR$_a$C(O)OR$_b$, -L$_2$-NR$_a$C(O)NR$_a$R$_b$, -L$_2$-NR$_b$C(NR$_b$)NR$_a$R$_b$, and -L$_2$-C(O)OR$_b$; or, R$_4$ and R$_{4'}$ together with the N atom to which they are attached form a 3- to 8-membered heterocycloalkyl group; the 3- to 8-membered heterocycloalkyl group is unsubstituted or further substituted at any position by 1 to 3 substituents selected from halogen, cyano, -L$_2$-OR$_a$, -L$_2$-OC(O)R$_a$, -L$_2$-NR$_a$R$_b$, -L$_2$-NR$_a$C(O)OR$_b$, -L$_2$-NR$_a$C(O)NR$_a$R$_b$, -L$_2$-NR$_b$C(NR$_b$)NR$_a$R$_b$, and -L$_2$-C(O)OR$_b$;
R$_5$ is =O, =NR$_a$, -OR$_a$, or -NR$_a$R$_b$;
R$_{5'}$ is absent, or R$_{5'}$ is -R$_c$, -L$_2$-OR$_a$, -L$_2$-NR$_a$R$_b$, -L$_2$-NR$_a$C(O)OR$_b$, -L$_2$-NR$_a$C(O)NR$_a$R$_b$, -L$_2$-NR$_b$C(NR$_b$)NR$_a$R$_b$, or -L$_2$-C(O)OR$_b$;
R$_7$ is phenyl, 5- to 6-membered heteroaryl, 3- to 8-membered heterocycloalkyl, or an 8-to 12-membered fused ring group; the R$_7$ is unsubstituted or optionally substituted at any position by one or more than one substituent selected from -L$_3$-W, -R$_c$, halogen, cyano, nitro, alkyl, alkenyl, alkynyl, alkoxy, haloalkyl, haloalkoxy, and alkylamino;
R$_8$ and R$_{8'}$ are each independently hydrogen, halogen, or alkyl, and the R$_8$ or R$_{8'}$ is unsubstituted or optionally substituted at any position by one or more than one substituent selected from -L$_3$-W, halogen, cyano, nitro, alkyl, alkenyl, alkynyl, alkoxy, haloalkyl, haloalkoxy, and alkylamino; R$_8$ and R$_{8'}$ are each independent substituents, or, R$_8$ and R$_{8'}$ together with the carbon atom to which they are attached form an oxo, thio, C$_{1-6}$ alkylene, C$_{3-10}$ cycloalkyl, or 3- to 10-membered heterocycloalkyl group; the C$_{1-6}$ alkylene, C$_{3-10}$ cycloalkyl, or 3- to 10-membered heterocycloalkyl group is unsubstituted or optionally substituted at any position by one or more than one substituent selected from deuterium, halogen, hydroxyl, amino, cyano, oxo, alkyl, haloalkyl, -L$_2$-OR$_a$, and -L$_2$-NR$_a$R$_b$;
R$_9$ is hydrogen, alkyl, haloalkyl, -L$_2$-OR$_a$, or -L$_2$-NR$_a$R$_b$;

W is $Cy^1$, $-SR_d$, $-OR_d$, $-OC(O)R_e$, $-OC(O)NR_eR_{e'}$, $-C(O)OR_e$, $-C(O)R_e$, $-C(O)NR_eR_{e'}$, - $C(O)NR_eS(O)_2R_e$, $-NR_dR_e$, $-NR_dC(O)R_e$, $-N(R_d)C(O)OR_e$, $-N(R_d)C(O)NR_eR_{e'}$, $-NR_dS(O)_2R_e$, - $NR_dS(O)_2NR_eR_{e'}$, $-S(O)_{1-2}R_e$, $-S(O)_2NR_eR_{e'}$, $-S(O)(=NR_d)R_e$, $-S(O)_2N(R_e)C(O)R_{e'}$, - $P(O)(OR_e)_2$, $-P(O)(OR_e)R_{e'}$, $-OP(O)(OR_e)_2$, or $-B(OR_e)_2$;

$Cy^1$ is cycloalkyl, heterocycloalkyl, aryl, or heteroaryl; the $Cy^1$ is unsubstituted or optionally substituted at any position by one or more than one substituent selected from halogen, alkyl, haloalkyl, haloalkoxy, alkenyl, alkynyl, cyano, $-R_c$, $-L_4-SR_d$, $-L_4-OC(O)R_e$, $-L_4-C(O)OR_e$, $-L_4-C(O)R_e$, $-L_4-C(O)NR_eR_{e'}$, $-L_4-NR_dC(O)R_e$, $-L_4-NR_dS(O)_2R_e$, $-L_4-S(O)_{1-2}R_e$, $-L_4-S(O)_2NR_eR_{e'}$, $-L_4-OR_d$, and $-L_4-NR_eR_{e'}$;

$L_1$, $L_2$, $L_3$, and $L_4$ are each independently a linkage bond, $C_{1-6}$ alkylene, $C_{2-6}$ alkenylene, or $C_{2-6}$ alkynylene; the $L_1$, $L_2$, $L_3$, or $L_4$ is unsubstituted or optionally substituted at any position by one or more than one substituent selected from oxo, hydroxyl, amino, halogen, cyano, alkyl, haloalkyl, alkoxy, and haloalkoxy;

$R_a$, $R_b$, $R_d$, $R_e$, and $R_{e'}$ are each independently $-R_c$, amino, $C_{1-6}$ alkyl, $C_{2-6}$ alkenyl, $C_{2-6}$ alkynyl, $C_{3-10}$ cycloalkyl, 3- to 10-membered heterocycloalkyl, $C_{6-10}$ aryl, 5- to 10-membered heteroaryl, $C_{3-10}$ cycloalkyl-$C_{1-6}$ alkyl, 3- to 10-membered heterocycloalkyl-$C_{1-6}$ alkyl, phenyl-$C_{1-6}$ alkyl, or 5- to-10 membered heteroaryl-$C_{1-6}$ alkyl; the $R_a$, $R_b$, $R_d$, $R_e$, or $R_{e'}$ is unsubstituted or optionally substituted at any position by 1 to 3 substituents selected from $-OR_f$, $-OC(O)-L_4-R_f$, $-NR_fR_{f'}$, halogen, cyano, nitro, $C_{1-6}$ alkyl, halo-$C_{1-6}$ alkyl, and halo-$C_{1-6}$ alkoxy;

$R_f$ and $R_{f'}$ are each independently $-R_c$, $-NHR_c$, or $C_{1-6}$ alkyl;

each $R_c$ is independently hydrogen;

scheme 10-3:

$\alpha$ and $\beta$ are independently a single bond or a double bond; at least one selected from the group of $\alpha$ and $\beta$ is a single bond;

m is 0 or 1;

$X_1$ is N or $CR_2$, $X_2$ is N or $CR_3$, and $X_3$ is N or $CR_1$; when $X_1$ is $CR_2$, $X_2$ is $CR_3$, and $X_3$ is $CR_1$, m is 1;

R is $-C(O)-L_1-R_7$, $-C(O)-NR_9-L_1-R_7$, $-C(S)-NR_9-L_1-R_7$, $-NR_9-L_1-R_7$, $-NR_9-C(O)-L_1-R_7$, - $NR_9-C(O)-NR_9-L_1-R_7$, $-O-L_1-R_7$, $-S(O)_2-NR_9-L_1-R_7$, $-CH=CH-L_1-R_7$, or $-S(O)_2-L_1-R_7$;

$R_1$, $R_2$, and $R_3$ are each independently hydrogen, deuterium, halogen, hydroxyl, amino, cyano, alkyl, haloalkyl, $-L_2-OR_a$, or $-L_2-NR_aR_b$;

$R_4$ and $R_{4'}$ are each independently hydrogen, alkyl, alkenyl, alkynyl, cycloalkyl, heterocycloalkyl, aryl, heteroaryl, cycloalkylalkyl, heterocycloalkylalkyl, arylalkyl, or heteroarylalkyl; the $R_4$ or $R_{4'}$ is unsubstituted or optionally substituted at any position by one or more than one substituent selected from halogen, cyano, $-L_2-OR_a$, $-L_2-NR_aR_b$, $-L_2-NR_aC(O)OR_b$, $-L_2-NR_aC(O)NR_aR_b$, $-L_2-NR_bC(NR_b)NR_aR_b$, and $-L_2-C(O)OR_b$; or, $R_4$ and $R_{4'}$ together with the N atom to which they are attached form a 3- to 8-membered heterocycloalkyl group; the 3- to 8-membered heterocycloalkyl group is unsubstituted or further substituted at any position by 1 to 3 substituents selected from halogen, cyano, $-L_2-OR_a$, $-L_2-NR_aR_b$, $-L_2-NR_aC(O)OR_b$, $-L_2-NR_aC(O)NR_aR_b$, $-L_2-NR_bC(NR_b)NR_aR_b$, and $-L_2-C(O)OR_b$;

$R_5$ is =O, $=NR_a$, $-OR_a$, or $-NR_aR_b$;

$R_{5'}$ is absent, or $R_{5'}$ is $-R_c$, $-L_2-OR_a$, $-L_2-NR_aR_b$, $-L_2-NR_aC(O)OR_b$, $-L_2-NR_aC(O)NR_2R_b$, - $L_2-NR_bC(NR_b)NR_aR_b$, or $-L_2-C(O)OR_b$;

$R_7$ is phenyl, 5- to 6-membered heteroaryl, 3- to 8-membered heterocycloalkyl, or an 8-to 12-membered fused ring group; the $R_7$ is unsubstituted or optionally substituted at any position by one or more than one substituent selected from $-L_3-W$, $-R_c$, halogen, cyano, nitro, alkyl, alkenyl, alkynyl, alkoxy, haloalkyl, haloalkoxy, and alkylamino;

$R_8$ and $R_{8'}$ are each independently hydrogen, halogen, or alkyl, and the $R_8$ or $R_{8'}$ is unsubstituted or optionally substituted at any position by one or more than one substituent selected from $-L_3-W$, halogen, cyano, nitro, alkyl, alkenyl, alkynyl, alkoxy, haloalkyl, haloalkoxy, and alkylamino; $R_8$ and $R_{8'}$ are each independent substituents, or, $R_8$ and $R_{8'}$ together with the carbon atom to which they are attached form an oxo, thio, $C_{1-6}$ alkylene, $C_{3-10}$ cycloalkyl, or 3- to 10-membered heterocycloalkyl group; the $C_{1-6}$ alkylene, $C_{3-10}$ cycloalkyl, or 3- to 10-membered heterocycloalkyl group is unsubstituted or optionally substituted at any position by one or more than one substituent selected from deuterium, halogen, hydroxyl, amino, cyano, oxo, alkyl, haloalkyl, $-L_2-OR_a$, and $-L_2-NR_aR_b$;

$R_9$ is hydrogen, alkyl, haloalkyl, $-L_2-OR_a$, or $-L_2-NR_aR_b$;

W is $Cy^1$, $-SR_d$, $-OR_d$, $-OC(O)R_e$, $-OC(O)NR_eR_{e'}$, $-C(O)OR_e$, $-C(O)R_e$, $-C(O)NR_eR_{e'}$, - $C(O)NR_eS(O)_2R_e$, $-NR_dR_e$, $-NR_dC(O)R_e$, $-N(R_d)C(O)OR_e$, $-N(R_d)C(O)NR_eR_{e'}$, $-NR_dS(O)_2R_e$, - $NR_dS(O)_2NR_eR_{e'}$, $-S(O)_{1-2}R_e$, $-S(O)_2NR_eR_{e'}$, $-S(O)(=NR_d)R_e$, $-S(O)_2N(R_e)C(O)R_{e'}$, - $P(O)(OR_e)_2$, $-P(O)(OR_e)R_{e'}$, $-OP(O)(OR_e)_2$, or $-B(OR_e)_2$;

$Cy^1$ is cycloalkyl, heterocycloalkyl, aryl, or heteroaryl; the $Cy^1$ is unsubstituted or optionally substituted at any position by one or more than one substituent selected from halogen, alkyl, haloalkyl, haloalkoxy, alkenyl, alkynyl, cyano, $-R_c$, $-L_4-SR_d$, $-L_4-OC(O)R_e$, $-L_4-C(O)OR_e$, $-L_4-C(O)R_e$, $-L_4-C(O)NR_eR_{e'}$, $-L_4-NR_dC(O)R_e$, $-L_4-NR_dS(O)_2R_e$, $-L_4-S(O)_{1-2}R_e$, $-L_4-S(O)_2NR_eR_{e'}$, $-L_4-OR_d$, and $-L_4-NR_eR_{e'}$;

$L_1$, $L_2$, $L_3$, and $L_4$ are each independently a linkage bond, $C_{1-6}$ alkylene, $C_{2-6}$ alkenylene, or $C_{2-6}$ alkynylene; the $L_1$, $L_2$, $L_3$, or $L_4$ is unsubstituted or optionally substituted at any position by one or more than one substituent selected from oxo, hydroxyl, amino, halogen, cyano, alkyl, haloalkyl, alkoxy, and haloalkoxy;

$R_a$, $R_b$, $R_d$, $R_e$, and $R_{e'}$ are each independently $-R_c$, amino, $C_{1-6}$ alkyl, $C_{2-6}$ alkenyl, $C_{2-6}$ alkynyl, $C_{3-10}$ cycloalkyl, 3- to 10-membered heterocycloalkyl, $C_{6-10}$ aryl, 5- to 10-membered heteroaryl, $C_{3-10}$ cycloalkyl-$C_{1-6}$ alkyl, 3- to 10-membered heterocycloalkyl-$C_{1-6}$ alkyl, phenyl-$C_{1-6}$ alkyl, or 5- to-10 membered heteroaryl-$C_{1-6}$ alkyl; the $R_a$, $R_b$, $R_d$, $R_e$, or $R_{e'}$ is unsubstituted or optionally substituted at any position by 1 to 3 substituents selected from $-OR_f$, $-NR_fR_{f'}$, halogen, cyano, nitro, $C_{1-6}$ alkyl, halo-$C_{1-6}$ alkyl, and halo-$C_{1-6}$ alkoxy;

$R_f$ and $R_{f'}$ are each independently $-R_c$, $-NHR_c$, or $C_{1-6}$ alkyl;

each $R_c$ is independently hydrogen.

**11.** The compound of formula I, the solvate thereof, the pharmaceutically acceptable salt thereof, or the solvate of the pharmaceutically acceptable salt thereof according to at least one selected from the group of claims 1 to 5, wherein the compound of formula I is any one of the following compounds:

| | |
|---|---|
| 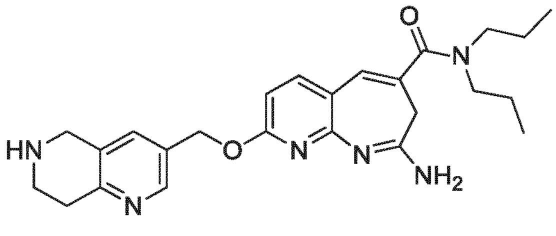 | 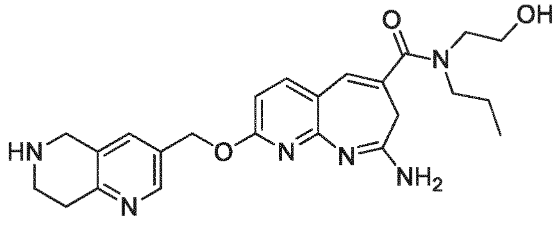 |
| 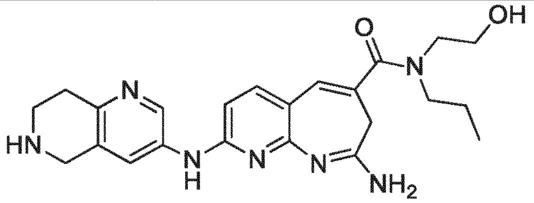 | 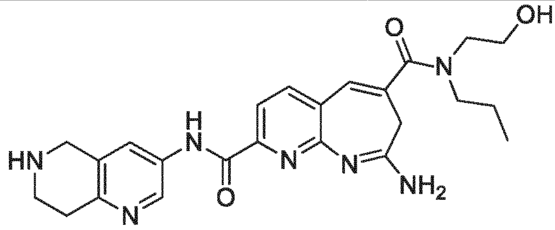 |
| 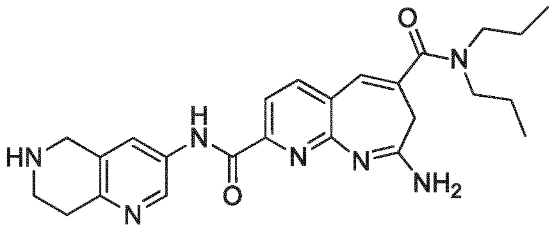 | 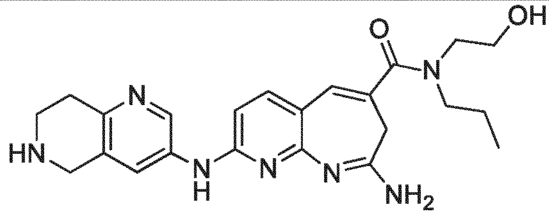 |
| 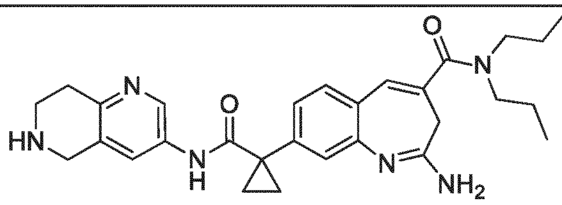 | 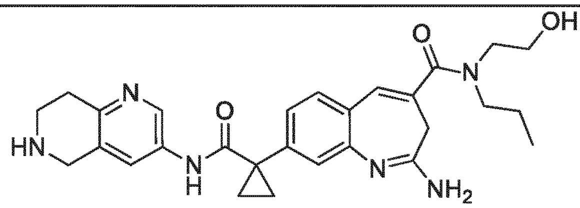 |
| 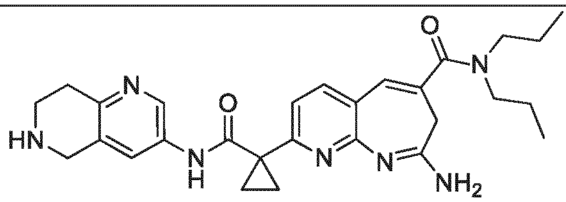 | 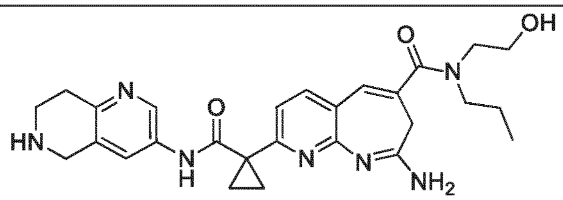 |
| 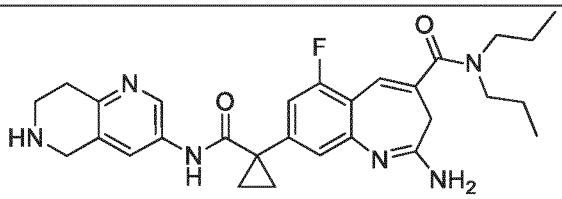 | 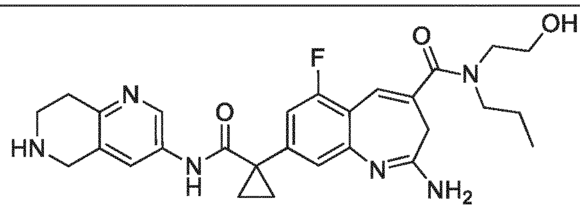 |

| | |
|---|---|
| 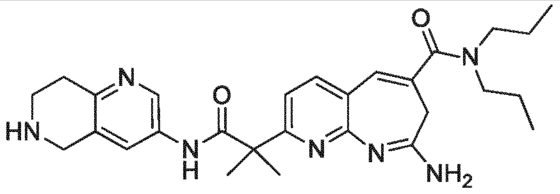 | 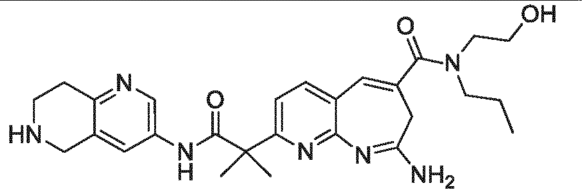 |
| 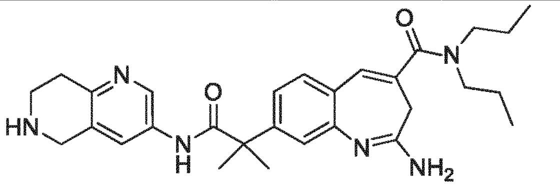 | 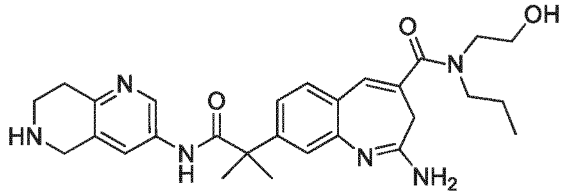 |
| 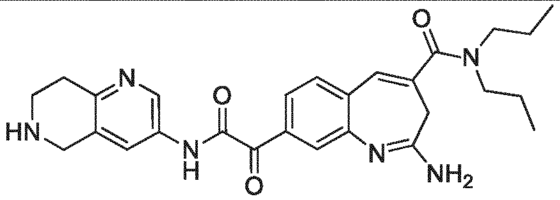 | 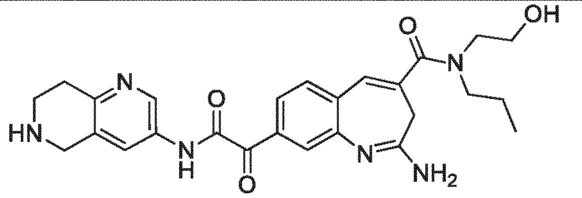 |
| 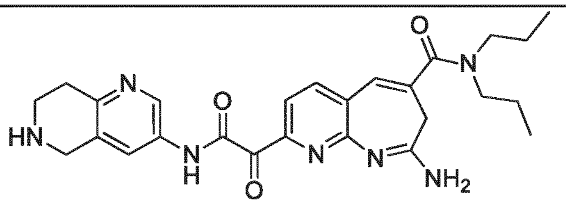 | 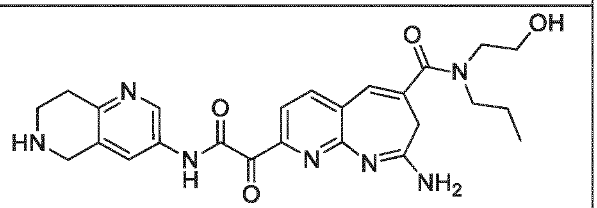 |
| 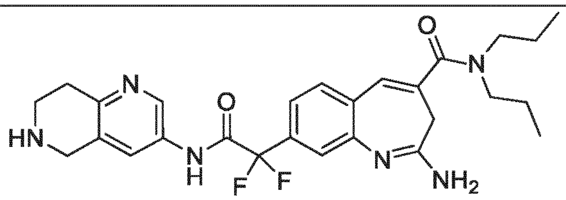 | 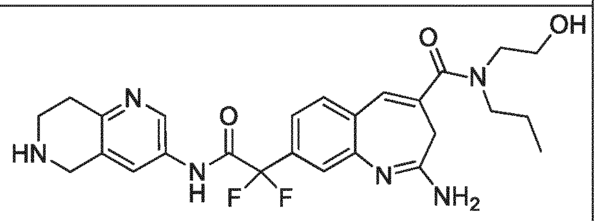 |
| 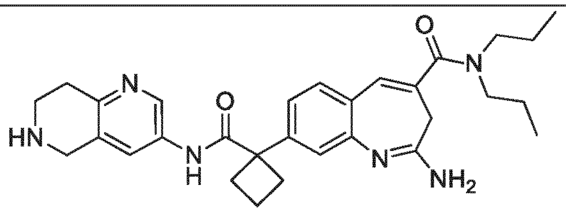 | 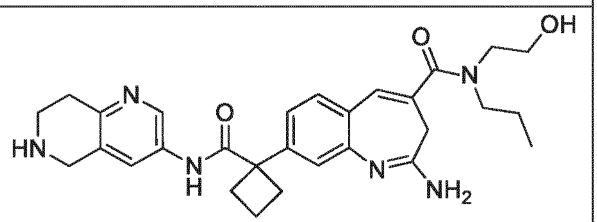 |
| 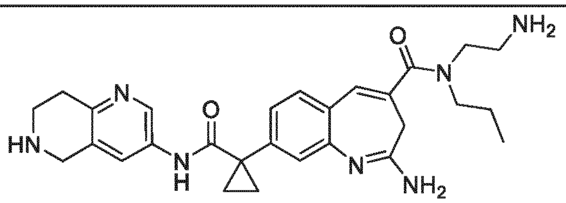 | 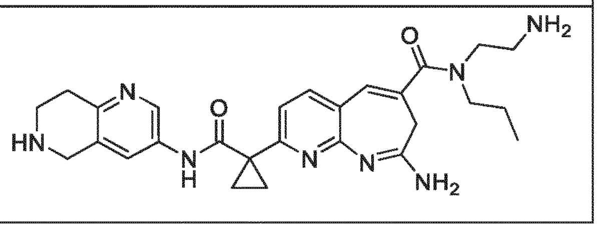 |

| | |
|---|---|
| 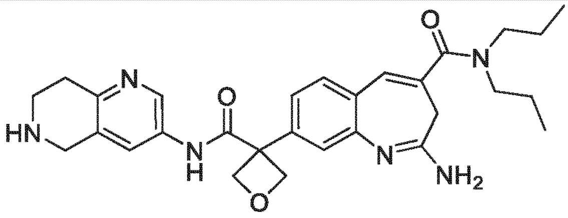 | 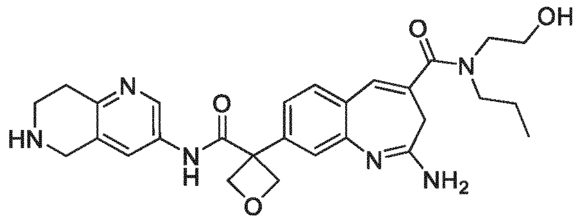 |
| 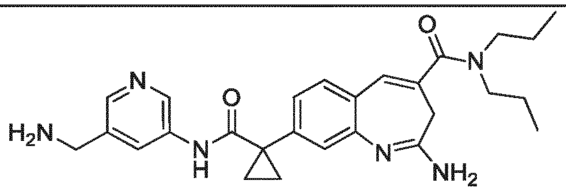 | 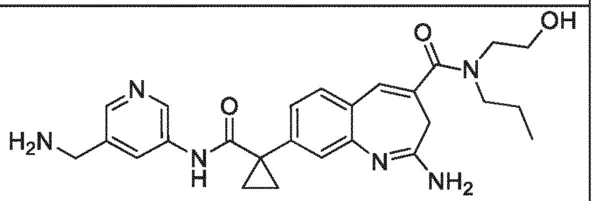 |
| 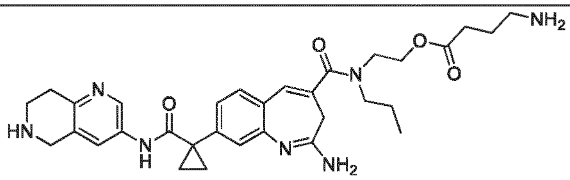 | 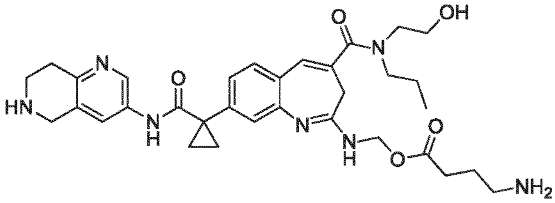 |
| 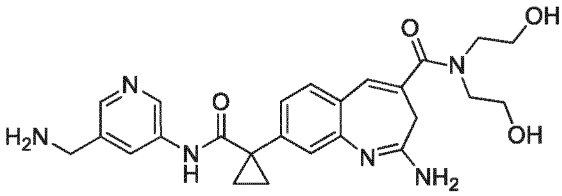 | 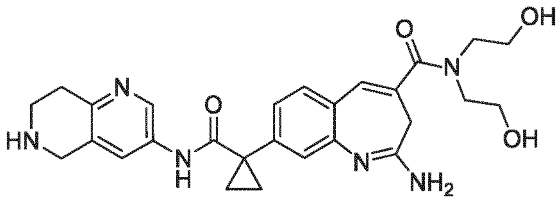 |
| 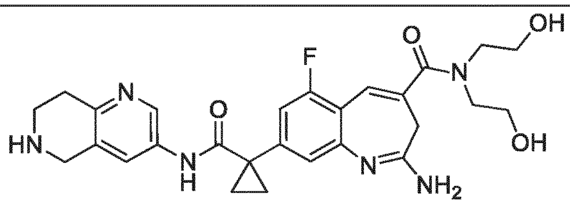 | 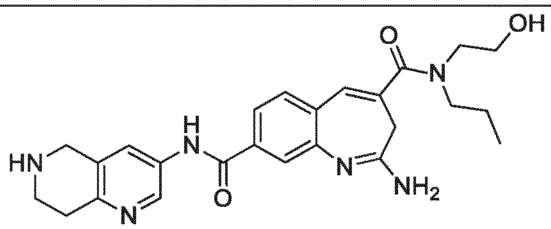 |
| 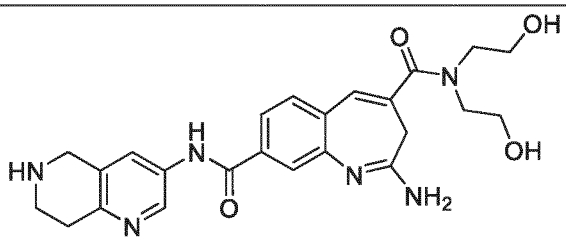 | |

**12.** A nitrogen-containing compound, wherein the nitrogen-containing compound is any one of the following compounds:

| | |
|---|---|
| 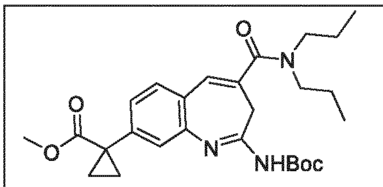 | 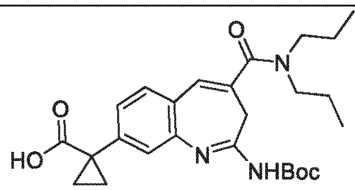 |
| 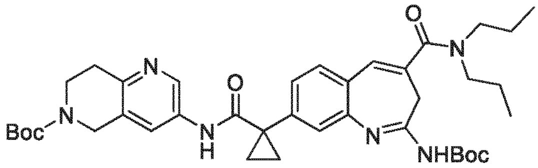 | 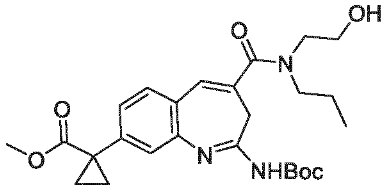 |
| 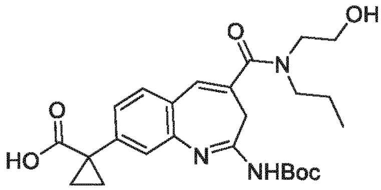 | 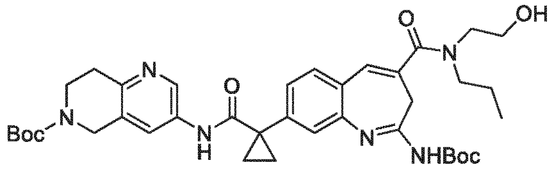 |
| 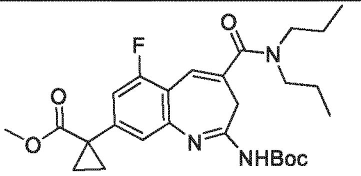 | 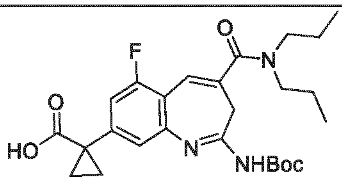 |
| 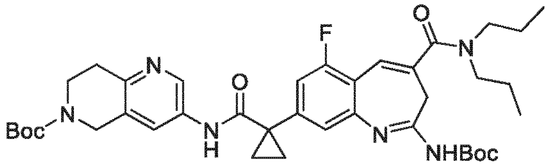 | 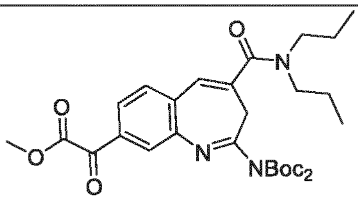 |
| 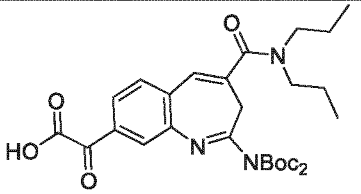 | 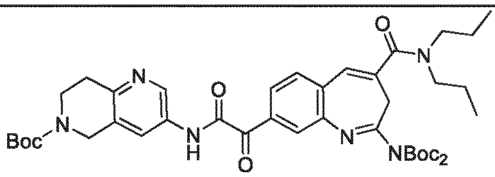 |
| 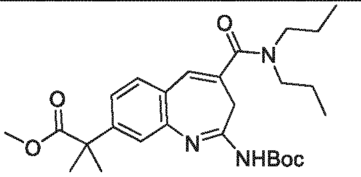 | 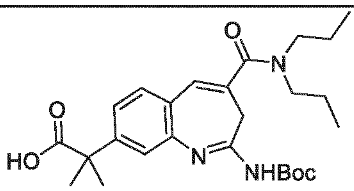 |
| 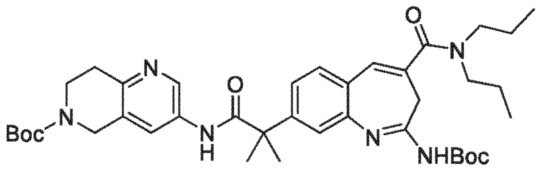 | 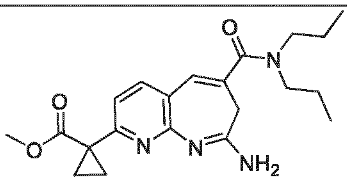 |

| | |
|---|---|
| 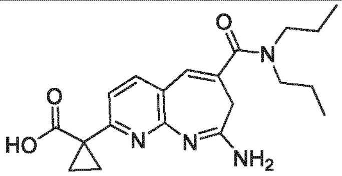 | 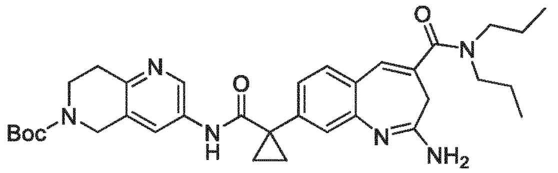 |
| 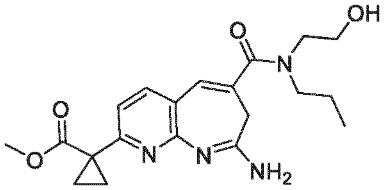 | 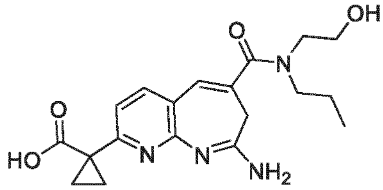 |
| 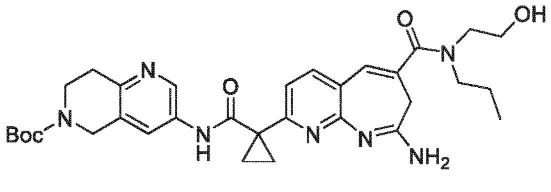 | 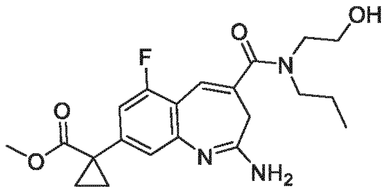 |
| 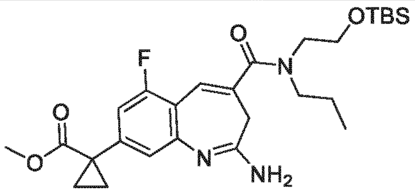 | 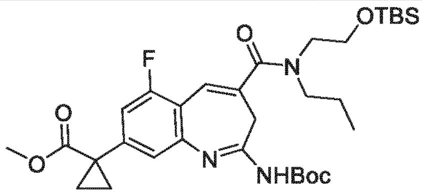 |
| 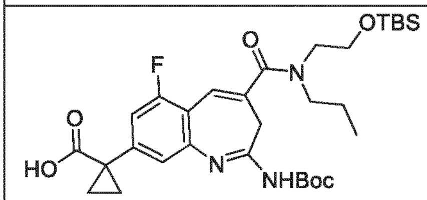 | 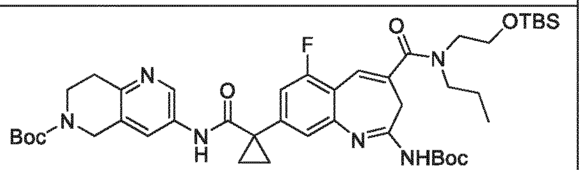 |
| 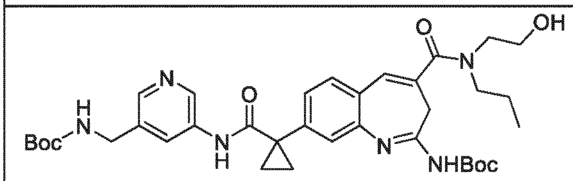 | 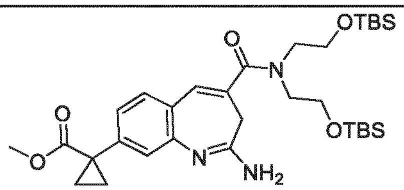 |
| 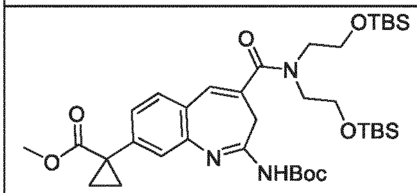 | 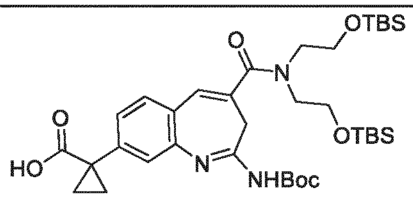 |
| 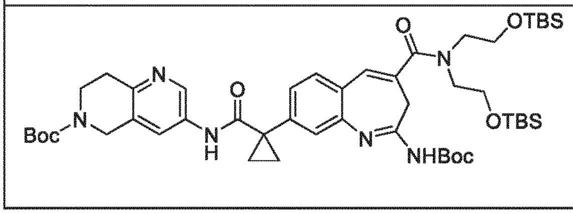 | 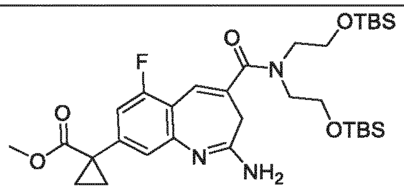 |

| | |
|---|---|
| 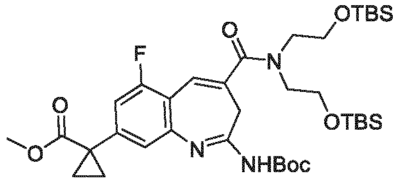 | 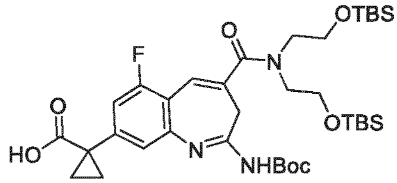 |
| 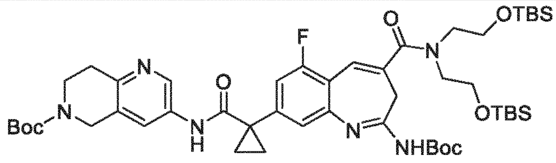 | 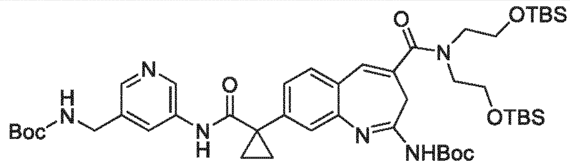 |
| 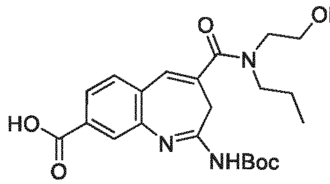 | 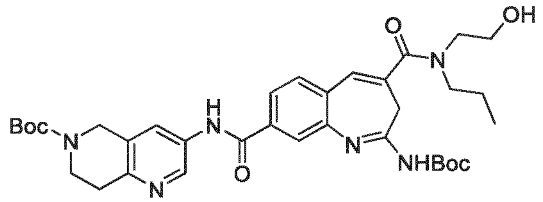 |
| 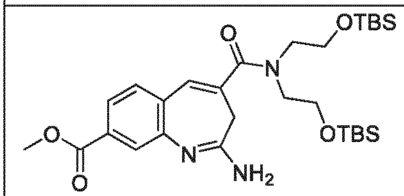 | 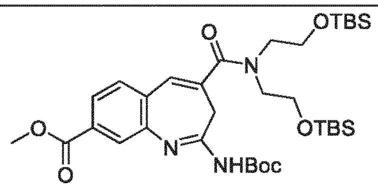 |
| 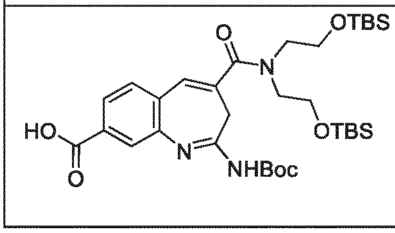 | 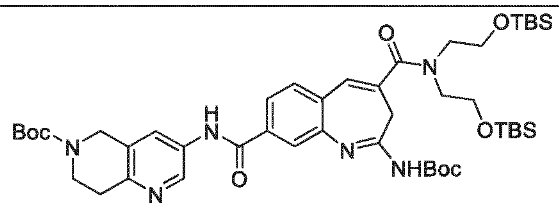 |

.

13. A compound of formula II', a solvate thereof, a pharmaceutically acceptable salt thereof, or a solvate of the pharmaceutically acceptable salt thereof,

**D-LinkerX** **(II')** ;

D is a group formed by losing one hydrogen atom in the compound of formula I according to at least one selected from the group of claims 1 to 11;
linker X is linker 2.

14. The compound of formula II', the solvate thereof, the pharmaceutically acceptable salt thereof, or the solvate of the pharmaceutically acceptable salt thereof according to claim 13, wherein the compound of formula II' satisfies one or more than one of the following conditions:

ll) the D is a group formed by losing one hydrogen atom from $R_7$ of the compound of formula I;
mm) the linker X is a degradable linker or a non-degradable linker.

15. The compound of formula II', the solvate thereof, the pharmaceutically acceptable salt thereof, or the solvate of the pharmaceutically acceptable salt thereof according to claim 14, wherein the compound of formula II' satisfies one or more than one of the following conditions:

nn) the D is a group formed by losing one hydrogen atom from the secondary or primary amine of $R_7$ of the compound of formula I;

oo) the linker X is a linker that can be degraded by lysosomal enzymes.

**16.** The compound of formula II', the solvate thereof, the pharmaceutically acceptable salt thereof, or the solvate of the pharmaceutically acceptable salt thereof according to claim 15, wherein the compound of formula II' satisfies one or more than one of the following conditions:

pp) the D is attached to the linker X via the a-end of

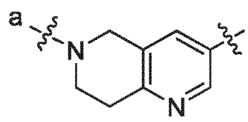

or

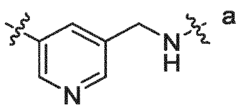

;

qq) the linker X is

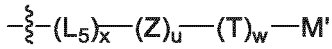

;

x is 0, 1, 2, or 3;

u is 0, 1, 2, 3, 4, 5, or 6;

w is 0, 1, 2, 3, 4, 5, or 6;

each $L_5$ is independently

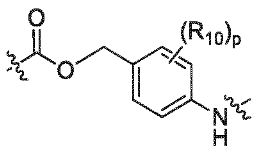

,

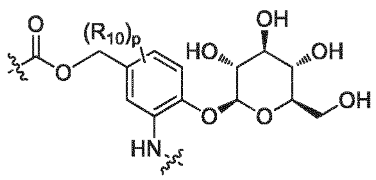

,

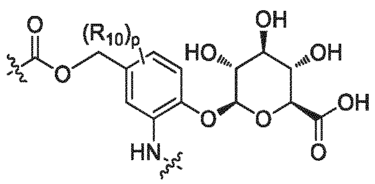

,

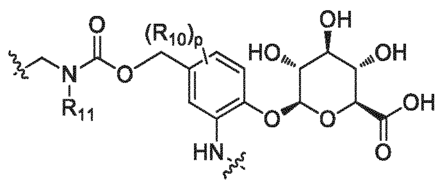

, or

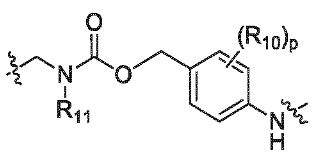

;

p is 1, 2, or 3; each $R_{10}$ is independently hydrogen, halogen, $C_{1-6}$ alkyl, halo-$C_{1-6}$ alkyl, nitro, cyano, $C_{1-6}$ alkoxy, $C_{1-6}$ alkylamino, $-OC(O)R_{11}$, or $-C(O)N(R_{11})_2$; $R_{11}$ is hydrogen, $C_{1-6}$ alkyl, or $C_{1-6}$ alkylamino-$C_{1-6}$ alkyl;

each Z is independently

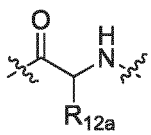

,

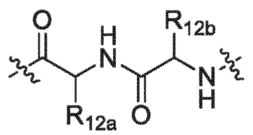

,

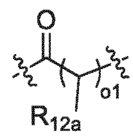

,

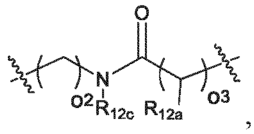, 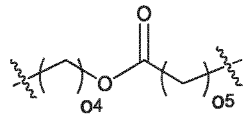, 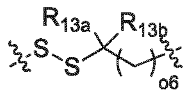,

and $-(CH_2CH_2O)_{o7}-$; o1, o2, o3, o4, o5, o6, o7 are each independently 0, 1, 2, 3, 4, 5, 6, 7, or 8; $R_{12a}$, $R_{12b}$, and $R_{12c}$ are each independently hydrogen, methyl, ethyl, n-propyl, isopropyl, $-SO_3H$,

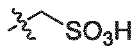, 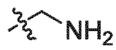,

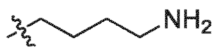, 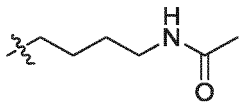, 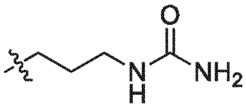, 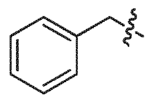, 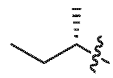,

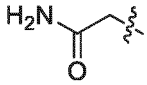, 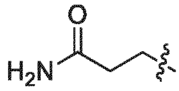, or 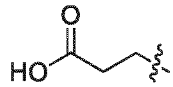;

$R_{13a}$ and $R_{13b}$ are each independently hydrogen or methyl;
each T is independently $-(CH_2)_{v1}-$, $-(CH_2CH_2O)_{v2}-$,

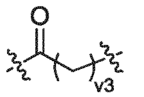, 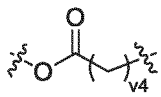,

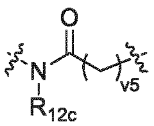, 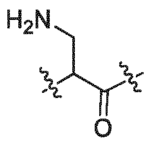, 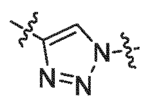, or 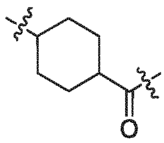;

v1, v2, v3, v4, and v5 are each independently 1, 2, 3, 4, 5, or 6;
M' is

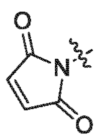, 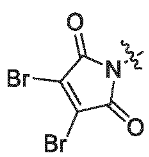, 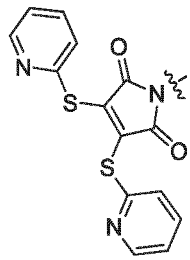, or 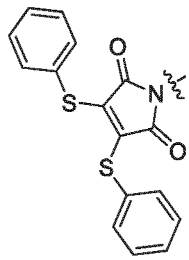.

**17.** The compound of formula II', the solvate thereof, the pharmaceutically acceptable salt thereof, or the solvate of the pharmaceutically acceptable salt thereof according to claim 16, wherein the compound of formula II' satisfies one or more than one of the following conditions:

rr) the $L_5$ is

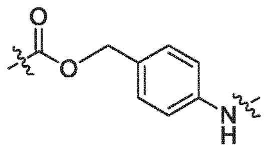;

ss)the carbonyl end of the $L_5$ is attached to the D;
tt) the Z is an amino acid;
uu) the

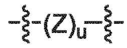

is a peptide chain;
vv) the carbonyl end of the

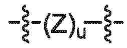

is attached to the amino end of the $L_5$;
ww) the

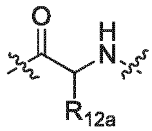

is

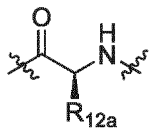 or 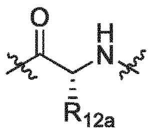;

xx) the carbonyl end of the T is attached to the amino end of the

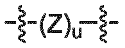,

yy) the amino end of the M' is attached to the non-carbonyl end of the T.

**18.** The compound of formula II', the solvate thereof, the pharmaceutically acceptable salt thereof, or the solvate of the pharmaceutically acceptable salt thereof according to claim 17, wherein the linker X is

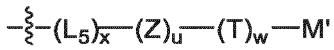,

x is 1;
u is 1, 2, 3, 4, 5, or 6;
w is 1;
$L_5$ is

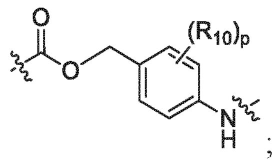;

p is 1; $R_{10}$ is hydrogen; the carbonyl end of the $L_5$ is attached to the D;
each Z is independently

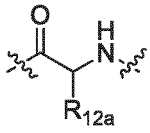;

each $R_{12a}$ is independently hydrogen, methyl, ethyl, n-propyl, isopropyl,

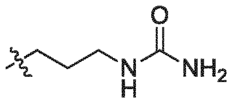, or 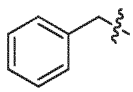;

the carbonyl end of the

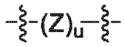

is attached to the amino end of the $L_5$;
Tis

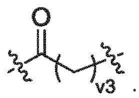;

v3 is 1, 2, 3, 4, 5, or 6; the carbonyl end of the T is attached to the amino end of

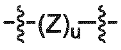;

M' is

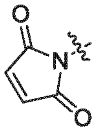,

the amino end of the M' is attached to the non-carbonyl end of the T.

**19.** The compound of formula II', the solvate thereof, the pharmaceutically acceptable salt thereof, or the solvate of the pharmaceutically acceptable salt thereof according to at least one selected from the group of claims 13 to 18, wherein the definition of the compound of formula II' is as described in any one of the following schemes:
scheme 19-1:
the compound of formula II' is

$$D-(L_5)_x-(Z)_u-(T)_w-M'$$

(II) ;

D is a group formed by losing one hydrogen atom in the compound of formula I;
x is 0, 1, 2, or 3;
u is 0, 1, 2, 3, 4, 5, or 6;
w is 0, 1, 2, 3, 4, 5, or 6;
each $L_5$ is independently

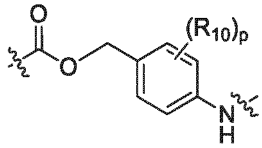 , 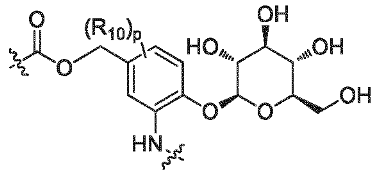 ,

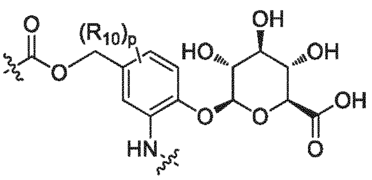 , 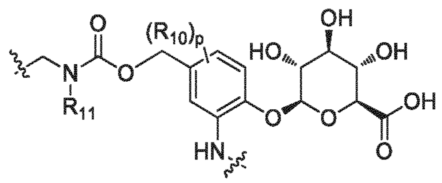 , or 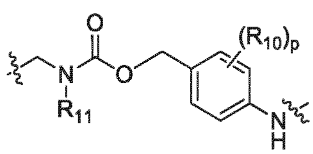 ;

p is 1, 2, or 3; each $R_{10}$ is independently hydrogen, halogen, $C_{1-6}$ alkyl, halo-$C_{1-6}$ alkyl, nitro, cyano, $C_{1-6}$ alkoxy, $C_{1-6}$ alkylamino, -OC(O)$R_{11}$, or -C(O)N$(R_{11})_2$; $R_{11}$ is hydrogen, $C_{1-6}$ alkyl, or $C_{1-6}$ alkylamino-$C_{1-6}$ alkyl;
each Z is independently

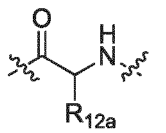 , 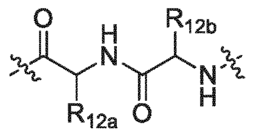 , 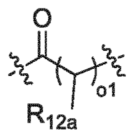 ,

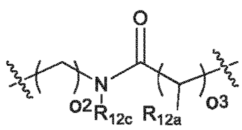 , 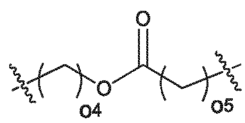 , 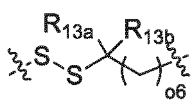 ,

and $-(CH_2CH_2O)_{o7}-$; o1, o2, o3, o4, o5, o6, o7 are each independently 0, 1, 2, 3, 4, 5, 6, 7, or 8; $R_{12a}$, $R_{12b}$, and $R_{12c}$ are each independently hydrogen, methyl, ethyl, n-propyl, isopropyl, $-SO_3H$,

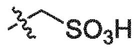 , 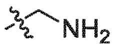 ,

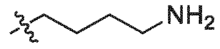 , 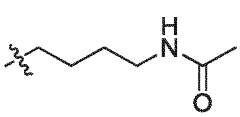 , 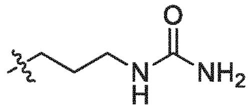 , 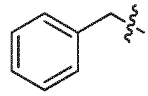 , 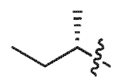 ,

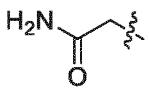, 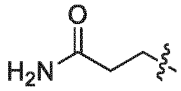, or 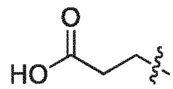;

$R_{13a}$ and $R_{13b}$ are each independently hydrogen or methyl;
each T is independently $-(CH_2)_{v1}-$, $-(CH_2CH_2O)v_2-$,

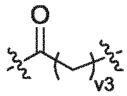, 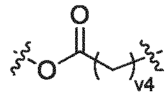,

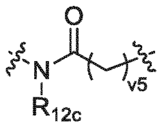, 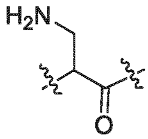, 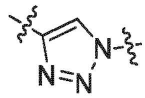, or 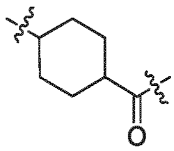;

v1, v2, v3, v4, and v5 are each independently 1, 2, 3, 4, 5, or 6;
M' is

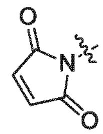, 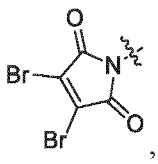, 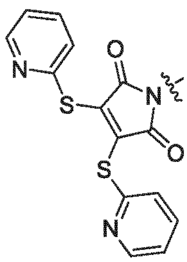, or 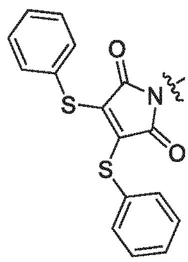;

scheme 19-2:

the D is a group formed by losing one hydrogen atom from $R_7$ of the compound of formula I;
linker X is

$$-(L_5)_x-(Z)_u-(T)_w-M'$$

x is 1;
u is 1, 2, 3, 4, 5, or 6;
w is 1;
$L_5$ is

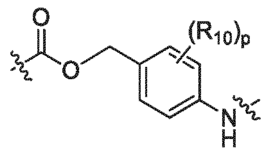;

p is 1; $R_{10}$ is hydrogen; the carbonyl end of the $L_5$ is attached to the D;
each Z is independently

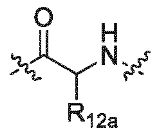;

each $R_{12a}$ is independently hydrogen, methyl, ethyl, n-propyl, isopropyl,

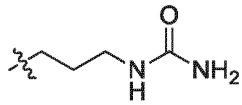, or 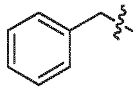;

the carbonyl end of the

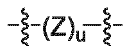

is attached to the amino end of the $L_5$;
Tis

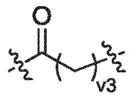;

v3 is 1, 2, 3, 4, 5, or 6; the carbonyl end of the T is attached to the amino end of

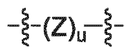;

M' is

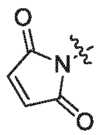,

the amino end of the M' is attached to the non-carbonyl end of the T;

scheme 19-3:

the compound of formula II' is

$$D-(L_5)_x-(Z)_u-(T)_w-M'$$

(II) ;

wherein D is the compound of formula I,

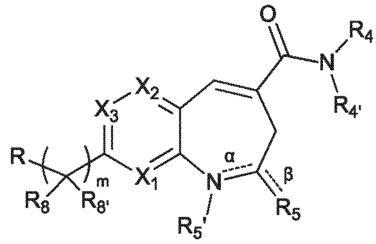

(I) ;

$\alpha$ **and** $\beta$ **are independently a single bond or a double bond;** at least one selected from the group of $\alpha$ and $\beta$ is a single bond;

m is 0 or 1;

$X_1$ is N or $CR_2$, $X_2$ is N or $CR_3$, and $X_3$ is N or $CR_1$; when $X_1$ is $CR_2$, $X_2$ is $CR_3$, and $X_3$ is $CR_1$, m is 1;

R is $-C(O)-L_1-R_7$, $-C(O)-NR_9-L_1-R_7$, $-C(S)-NR_9-L_1-R_7$, $-NR_9-L_1-R_7$, $-NR_9-C(O)-L_1-R_7$, $-NR_9-C(O)-NR_9-L_1-R_7$, $-O-L_1-R_7$, $-S(O)_2-NR_9-L_1-R_7$, $-CH=CH-L_1-R_7$, or $-S(O)_2-L_1-R_7$;

$R_1$, $R_2$, and $R_3$ are each independently hydrogen, deuterium, halogen, hydroxyl, amino, cyano, alkyl, haloalkyl, $-L_2-OR_a$, or $-L_2-NR_aR_b$;

$R_4$ and $R_{4'}$ are each independently hydrogen, alkyl, alkenyl, alkynyl, cycloalkyl, heterocycloalkyl, aryl, heteroaryl, cycloalkylalkyl, heterocycloalkylalkyl, arylalkyl, or heteroarylalkyl; the $R_4$ or $R_{4'}$ is unsubstituted or optionally substituted at any position by one or more than one substituent selected from halogen, cyano, $-L_2-OR_a$, $-L_2-OC(O)R_a$, $-L_2-NR_aR_b$, $-L_2-NR_aC(O)OR_b$, $-L_2-NR_aC(O)NR_aR_b$, $-L_2-NR_bC(NR_b)NR_aR_b$, and $-L_2-C(O)OR_b$; or, $R_4$ and $R_{4'}$ together with the N atom to which they are attached form a 3- to 8-membered heterocycloalkyl group; the 3- to 8-membered heterocycloalkyl group is unsubstituted or further substituted at any position by 1 to 3 substituents selected from halogen, cyano, $-L_2-OR_a$, $-L_2-OC(O)R_a$, $-L_2-NR_aR_b$, $-L_2-NR_aC(O)OR_b$, $-L_2-NR_aC(O)NR_aR_b$, $-L_2-NR_bC(NR_b)NR_aR_b$, and $-L_2-C(O)OR_b$;

$R_5$ is =O, $=NR_a$, $-OR_a$, or $-NR_aR_b$;

$R_{5'}$ is absent, or $R_{5'}$ is $-R_c$, $-L_2-OR_a$, $-L_2-NR_aR_b$, $-L_2-NR_aC(O)OR_b$, $-L_2-NR_aC(O)NR_aR_b$, $-L_2-NR_bC(NR_b)NR_aR_b$, or $-L_2-C(O)OR_b$;

$R_7$ is phenyl, 5- to 6-membered heteroaryl, 3- to 8-membered heterocycloalkyl, or an 8-to 12-membered fused ring group; the $R_7$ is unsubstituted or optionally substituted at any position by one or more than one substituent selected from $-L_3-W$, $-R_c$, halogen, cyano, nitro, alkyl, alkenyl, alkynyl, alkoxy, haloalkyl, haloalkoxy, and alkyl amino;

$R_8$ and $R_{8'}$ are each independently hydrogen, halogen, or alkyl, and the $R_8$ or $R_{8'}$ is unsubstituted or optionally substituted at any position by one or more than one substituent selected from $-L_3-W$, halogen, cyano, nitro, alkyl, alkenyl, alkynyl, alkoxy, haloalkyl, haloalkoxy, and alkylamino; $R_8$ and $R_{8'}$ are each independent substituents, or, $R_8$ and $R_{8'}$ together with the carbon atom to which they are attached form an oxo, thio, $C_{1-6}$ alkylene, $C_{3-10}$ cycloalkyl, or 3- to 10-membered heterocycloalkyl group; the $C_{1-6}$ alkylene, $C_{3-10}$ cycloalkyl, or 3- to 10-membered heterocycloalkyl group is unsubstituted or optionally substituted at any position by one or more than one substituent selected from deuterium, halogen, hydroxyl, amino, cyano, oxo, alkyl, haloalkyl, $-L_2-OR_a$, and $-L_2-NR_aR_b$;

$R_9$ is hydrogen, alkyl, haloalkyl, $-L_2-OR_a$, or $-L_2-NR_aR_b$;

W is $Cy^1$, $-SR_d$, $-OR_d$, $-OC(O)R_e$, $-OC(O)NR_eR_{e'}$, $-C(O)OR_e$, $-C(O)R_e$, $-C(O)NR_eR_{e'}$, $-C(O)NR_eS(O)_2R_e$, $-NR_dR_e$, $-NR_dC(O)R_e$, $-N(R_d)C(O)OR_e$, $-N(R_d)C(O)NR_eR_e$ , $-NR_dS(O)_2R_e$, $-NR_dS(O)_2NR_eR_{e'}$, $-S(O)_{1-2}R_e$, $-S(O)_2NR_eR_{e'}$, $-S(O)(=NR_d)R_e$, $-S(O)_2N(R_e)C(O)R_{e'}$, $-P(O)(OR_e)_2$, $-P(O)(OR_e)R_{e'}$, $-OP(O)(OR_e)_2$, or $-B(OR_e)_2$;

$Cy^1$ is cycloalkyl, heterocycloalkyl, aryl, or heteroaryl; the $Cy^1$ is unsubstituted or optionally substituted at any position by one or more than one substituent selected from halogen, alkyl, haloalkyl, haloalkoxy, alkenyl, alkynyl, cyano, $-R_c$, $-L_4-SR_d$, $-L_4-OC(O)R_e$, $-L_4-C(O)OR_e$, $-L_4-C(O)R_e$, $-L_4-C(O)NR_eR_{e'}$, $-L_4-NR_dC(O)R_e$, $-L_4-NR_dS(O)_2R_e$, $-L_4-S(O)_{1-2}R_e$, $-L_4-S(O)_2NR_eR_e$ , $-L_4-OR_d$, and $-L_4-NR_eR_{e'}$;

$L_1$, $L_2$, $L_3$, and $L_4$ are each independently a linkage bond, $C_{1-6}$ alkylene, $C_{2-6}$ alkenylene, or $C_{2-6}$ alkynylene; the $L_1$, $L_2$, $L_3$, or $L_4$ is unsubstituted or optionally substituted at any position by one or more than one substituent selected from oxo, hydroxyl, amino, halogen, cyano, alkyl, haloalkyl, alkoxy, and haloalkoxy;

$R_a$, $R_b$, $R_d$, $R_e$, and $R_{e'}$ are each independently $-R_c$, amino, $C_{1-6}$ alkyl, $C_{2-6}$ alkenyl, $C_{2-6}$ alkynyl, $C_{3-10}$ cycloalkyl, 3- to 10-membered heterocycloalkyl, $C_{6-10}$ aryl, 5- to 10-membered heteroaryl, $C_{3-10}$ cycloalkyl-$C_{1-6}$ alkyl, 3- to 10-membered heterocycloalkyl-$C_{1-6}$ alkyl, phenyl-$C_{1-6}$ alkyl, or 5- to-10 membered heteroaryl-$C_{1-6}$ alkyl; the $R_a$, $R_b$, $R_d$, $R_e$, or $R_{e'}$ is unsubstituted or optionally substituted at any position by 1 to

3 substituents selected from $-OR_f$, $-OC(O)-L_4-R_f$, $-NR_fR_{f'}$, halogen, cyano, nitro, $C_{1-6}$ alkyl, halo-$C_{1-6}$ alkyl, and halo-$C_{1-6}$ alkoxy;

$R_f$ and $R_{f'}$ are each independently $-R_c$, $-NHR_c$, or $C_{1-6}$ alkyl;

each $R_c$ is independently hydrogen or a linkage bond; at least one $R_c$ in D is a linkage bond;

x is 0, 1, 2, or 3;

u is 0, 1, 2, 3, 4, 5, or 6;

w is 0, 1, 2, 3, 4, 5, or 6;

each $L_5$ is independently

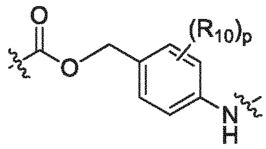 , 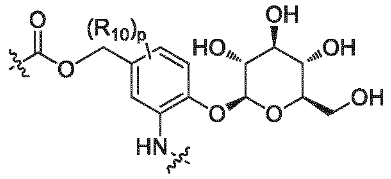 ,

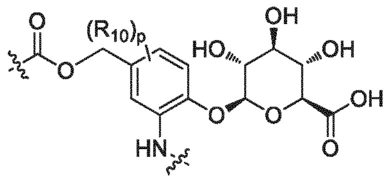 , 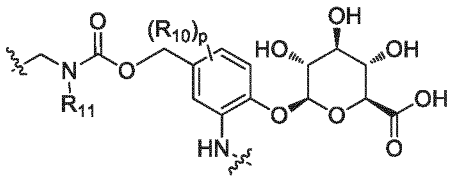 ,

or

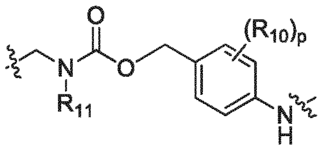 ;

p is 1, 2, or 3; each $R_{10}$ is independently hydrogen, halogen, $C_{1-6}$ alkyl, halo-$C_{1-6}$ alkyl, nitro, cyano, $C_{1-6}$ alkoxy, $C_{1-6}$ alkylamino, $-OC(O)R_{11}$, or $-C(O)N(R_{11})_2$; $R_{11}$ is hydrogen, $C_{1-6}$ alkyl, or $C_{1-6}$ alkylamino-$C_{1-6}$ alkyl;

each Z is independently

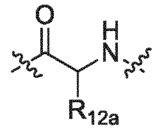 , 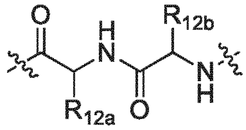 , 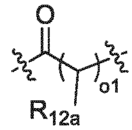 ,

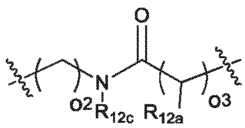 , 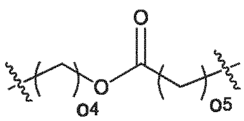 , 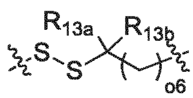 ,

and $-(CH_2CH_2O)_{o7}-$; o1, o2, o3, o4, o5, o6, o7 are each independently 0, 1, 2, 3, 4, 5, 6, 7, or 8; $R_{12a}$, $R_{12b}$, and $R_{12c}$ are each independently hydrogen, methyl, ethyl, n-propyl, isopropyl, $-SO_3H$,

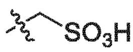 , 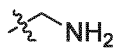 ,

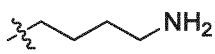, 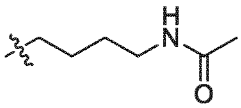, 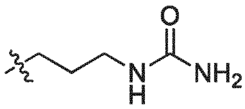, 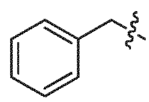, 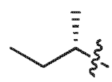,

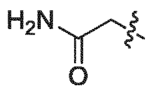, 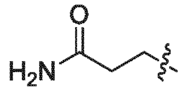, or 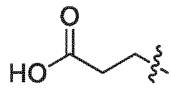;

$R_{13a}$ and $R_{13b}$ are each independently hydrogen or methyl;
each T is independently $-(CH_2)_{v1}-$, $-(CH_2CH_2O)_{v2}-$,

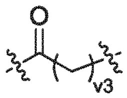, 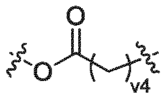,

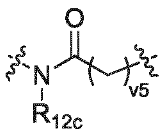, 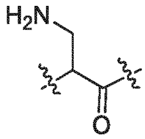, 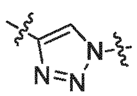, or 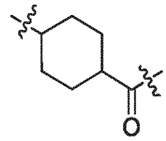;

v1, v2, v3, v4, and v5 are each independently 1, 2, 3, 4, 5, or 6;
M' is

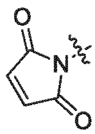, 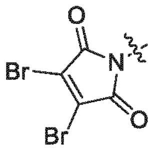, 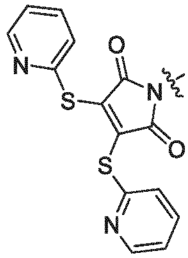, or 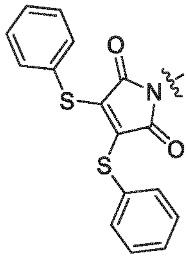;

scheme 19-4:

the compound of formula II' is

$$D-(L_5)_x-(Z)_u-(T)_w-M'$$

(II) ;

wherein D is the compound of formula I,

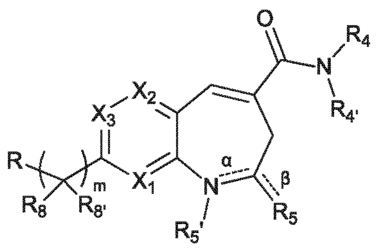

(I) ;

$\alpha$ **and** $\beta$ **are independently a single bond or a double bond**; at least one selected from the group of $\alpha$ **and** $\beta$ is a single bond;

m is 0 or 1;

$X_1$ is N or $CR_2$, $X_2$ is N or $CR_3$, and $X_3$ is N or $CR_1$; when $X_1$ is $CR_2$, $X_2$ is $CR_3$, and $X_3$ is $CR_1$, m is 1;

R is $-C(O)-L_1-R_7$, $-C(O)-NR_9-L_1-R_7$, $-C(S)-NR_9-L_1-R_7$, $-NR_9-L_1-R_7$, $-NR_9-C(O)-L_1-R_7$, $-NR_9-C(O)-NR_9-L_1-R_7$, $-O-L_1-R_7$, $-S(O)_2-NR_9-L_1-R_7$, $-CH=CH-L_1-R_7$, or $-S(O)_2-L_1-R_7$;

$R_1$, $R_2$, and $R_3$ are each independently hydrogen, deuterium, halogen, hydroxyl, amino, cyano, alkyl, haloalkyl, $-L_2-OR_a$, or $-L_2-NR_aR_b$;

$R_4$ and $R_{4'}$ are each independently hydrogen, alkyl, alkenyl, alkynyl, cycloalkyl, heterocycloalkyl, aryl, heteroaryl, cycloalkylalkyl, heterocycloalkylalkyl, arylalkyl, or heteroarylalkyl; the $R_4$ or $R_{4'}$ is unsubstituted or optionally substituted at any position by one or more than one substituent selected from halogen, cyano, $-L_2-OR_a$, $-L_2-NR_aR_b$, $-L_2-NR_aC(O)OR_b$, $-L_2-NR_aC(O)NR_aR_b$, $-L_2-NR_bC(NR_b)NR_aR_b$, and $-L_2-C(O)OR_b$; or, $R_4$ and $R_{4'}$ together with the N atom to which they are attached form a 3- to 8-membered heterocycloalkyl group; the 3- to 8-membered heterocycloalkyl group is unsubstituted or further substituted at any position by 1 to 3 substituents selected from halogen, cyano, $-L_2-OR_a$, $-L_2-NR_aR_b$, $-L_2-NR_aC(O)OR_b$, $-L_2-NR_aC(O)NR_aR_b$, $-L_2-NR_bC(NR_b)NR_aR_b$, and $-L_2-C(O)OR_b$;

$R_5$ is =O, $=NR_a$, $-OR_a$, or $-NR_aR_b$;

$R_{5'}$ is absent, or $R_{5'}$ is $-R_c$, $-L_2-OR_a$, $-L_2-NR_aR_b$, $-L_2-NR_aC(O)OR_b$, $-L_2-NR_aC(O)NR_aR_b$, $-L_2-NR_bC(NR_b)NR_aR_b$, or $-L_2-C(O)OR_b$;

$R_7$ is phenyl, 5- to 6-membered heteroaryl, 3- to 8-membered heterocycloalkyl, or an 8-to 12-membered fused ring group; the $R_7$ is unsubstituted or optionally substituted at any position by one or more than one substituent selected from $-L_3-W$, $-R_c$, halogen, cyano, nitro, alkyl, alkenyl, alkynyl, alkoxy, haloalkyl, haloalkoxy, and alkylamino;

$R_8$ and $R_{8'}$ are each independently hydrogen, halogen, or alkyl, and the $R_8$ or $R_{8'}$ is unsubstituted or optionally substituted at any position by one or more than one substituent selected from $-L_3-W$, halogen, cyano, nitro, alkyl, alkenyl, alkynyl, alkoxy, haloalkyl, haloalkoxy, and alkylamino; $R_8$ and $R_{8'}$ are each independent substituents, or, $R_8$ and $R_{8'}$ together with the carbon atom to which they are attached form an oxo, thio, $C_{1-6}$ alkylene, $C_{3-10}$ cycloalkyl, or 3- to 10-membered heterocycloalkyl group; the $C_{1-6}$ alkylene, $C_{3-10}$ cycloalkyl, or 3- to 10-membered heterocycloalkyl group is unsubstituted or optionally substituted at any position by one or more than one substituent selected from deuterium, halogen, hydroxyl, amino, cyano, oxo, alkyl, haloalkyl, $-L_2-OR_a$, and $-L_2-NR_aR_b$;

$R_9$ is hydrogen, alkyl, haloalkyl, $-L_2-OR_a$, or $-L_2-NR_aR_b$;

W is $Cy^1$, $-SR_d$, $-OR_d$, $-OC(O)R_e$, $-OC(O)NR_eR_{e'}$, $-C(O)OR_e$, $-C(O)R_e$, $-C(O)NR_eR_{e'}$, $-C(O)NR_eS(O)_2R_e$, $-NR_dR_e$, $-NR_dC(O)R_e$, $-N(R_d)C(O)OR_e$, $-N(R_d)C(O)NR_eR_{e'}$, $-NR_dS(O)_2R_e$, $-NR_dS(O)_2NR_eR_{e'}$, $-S(O)_{1-2}R_e$, $-S(O)_2NR_eR_{e'}$, $-S(O)(=NR_d)R_e$, $-S(O)_2N(R_e)C(O)R_{e'}$, $-P(O)(OR_e)_2$, $-P(O)(OR_e)R_{e'}$, $-OP(O)(OR_e)_2$, or $-B(OR_e)_2$;

$Cy^1$ is cycloalkyl, heterocycloalkyl, aryl, or heteroaryl; the $Cy^1$ is unsubstituted or optionally substituted at any position by one or more than one substituent selected from halogen, alkyl, haloalkyl, haloalkoxy, alkenyl, alkynyl, cyano, $-R_c$, $-L_4-SR_d$, $-L_4-OC(O)R_e$, $-L_4-C(O)OR_e$, $-L_4-C(O)R_e$, $-L_4-C(O)NR_eR_{e'}$, $-L_4-NR_dC(O)R_e$, $-L_4-NR_dS(O)_2R_e$, $-L_4-S(O)_{1-2}R_e$, $-L_4-S(O)_2NR_eR_{e'}$, $-L_4-OR_d$, and $-L_4-NR_eR_{e'}$;

$L_1$, $L_2$, $L_3$, and $L_4$ are each independently a linkage bond, $C_{1-6}$ alkylene, $C_{2-6}$ alkenylene, or $C_{2-6}$ alkynylene; the $L_1$, $L_2$, $L_3$, or $L_4$ is unsubstituted or optionally substituted at any position by one or more than one substituent selected from oxo, hydroxyl, amino, halogen, cyano, alkyl, haloalkyl, alkoxy, and haloalkoxy;

$R_a$, $R_b$, $R_d$, $R_e$, and $R_{e'}$ are each independently $-R_c$, amino, $C_{1-6}$ alkyl, $C_{2-6}$ alkenyl, $C_{2-6}$ alkynyl, $C_{3-10}$ cycloalkyl, 3- to 10-membered heterocycloalkyl, $C_{6-10}$ aryl, 5- to 10-membered heteroaryl, $C_{3-10}$ cycloalkyl-$C_{1-6}$ alkyl, 3- to 10-membered heterocycloalkyl-$C_{1-6}$ alkyl, phenyl-$C_{1-6}$ alkyl, or 5- to-10 membered heteroaryl-$C_{1-6}$ alkyl; the $R_a$, $R_b$, $R_d$, $R_e$, or $R_{e'}$ is unsubstituted or optionally substituted at any position by 1 to 3 substituents selected from $-OR_f$, $-NR_fR_{f'}$, halogen, cyano, nitro, $C_{1-6}$ alkyl, halo-$C_{1-6}$ alkyl, and halo-$C_{1-6}$ alkoxy;

$R_f$ and $R_{f'}$ are each independently $-R_c$, $-NHR_c$, or $C_{1-6}$ alkyl;

each $R_c$ is independently hydrogen or a linkage bond; at least one $R_e$ in D is a linkage bond;

x is 0, 1, 2, or 3;

u is 0, 1, 2, 3, 4, 5, or 6;

w is 0, 1, 2, 3, 4, 5, or 6;

each $L_5$ is independently

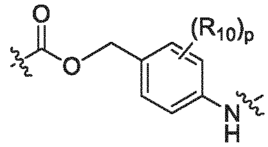, 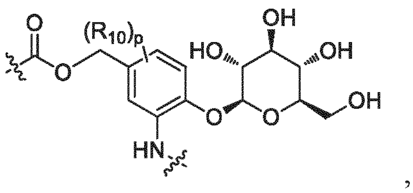,

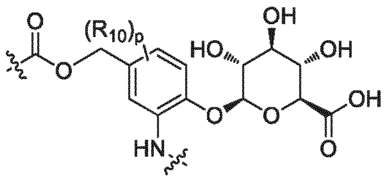, 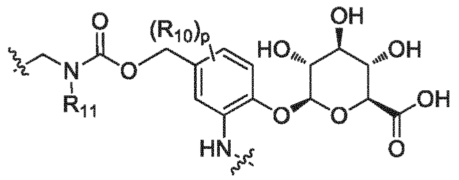,

or

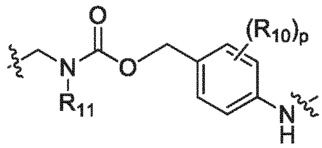;

p is 1, 2, or 3; each $R_{10}$ is independently hydrogen, halogen, $C_{1-6}$ alkyl, halo-$C_{1-6}$ alkyl, nitro, cyano, $C_{1-6}$ alkoxy, $C_{1-6}$ alkylamino, -OC(O)$R_{11}$, or -C(O)N($R_{11}$)$_2$; $R_{11}$ is hydrogen, $C_{1-6}$ alkyl, or $C_{1-6}$ alkylamino-$C_{1-6}$ alkyl;

each Z is independently

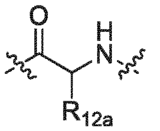, 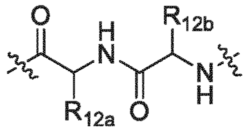, 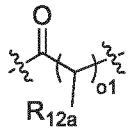,

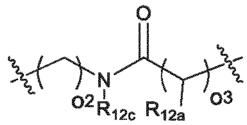, 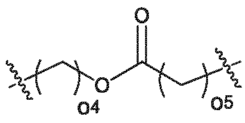, 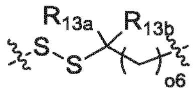,

and -($CH_2CH_2O$)$_{o7}$-; o1, o2, o3, o4, o5, $_o$6, o7 are each independently 0, 1, 2, 3, 4, 5, 6, 7, or 8; $R_{12a}$, $R_{12b}$, and $R_{12c}$ are each independently hydrogen, methyl, ethyl, n-propyl, isopropyl, -$SO_3H$,

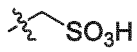, 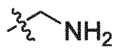,

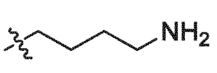, 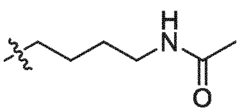, 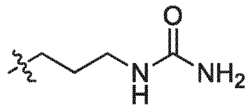, 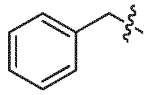, 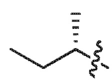,

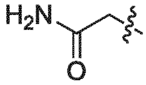, 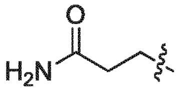, or 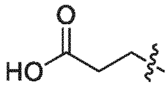;

$R_{13a}$ and $R_{13b}$ are each independently hydrogen or methyl;
each T is independently $-(CH_2)_{v1}-$, $-(CH_2CH_2O)_{v2}-$,

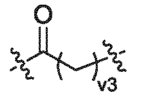, 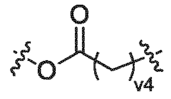,

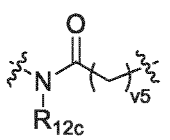, 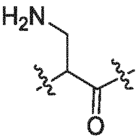, 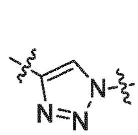, or 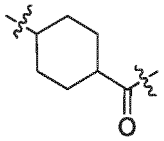;

v1, v2, v3, v4, and v5 are each independently 1, 2, 3, 4, 5, or 6;
M' is

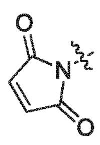, 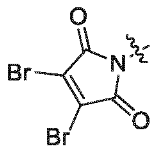, 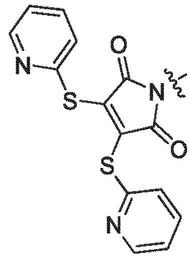, or 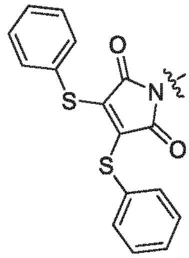.

**20.** The compound of formula II', the solvate thereof, the pharmaceutically acceptable salt thereof, or the solvate of the pharmaceutically acceptable salt thereof according to at least one selected from the group of claims 13 to 18, wherein the compound of formula II' is any one of the following compounds:

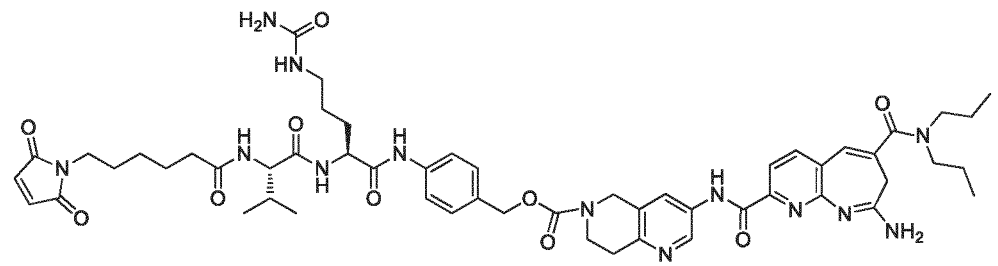,

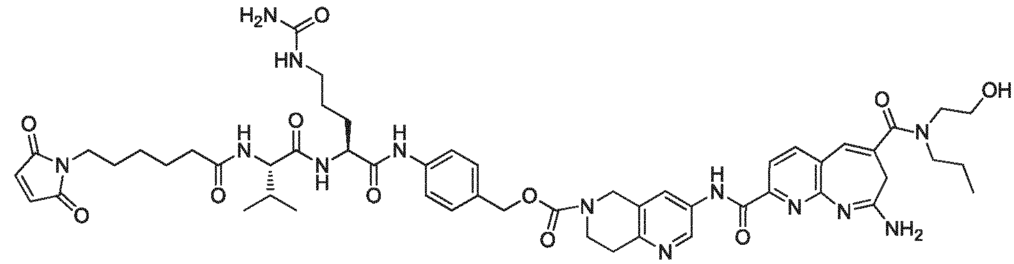,

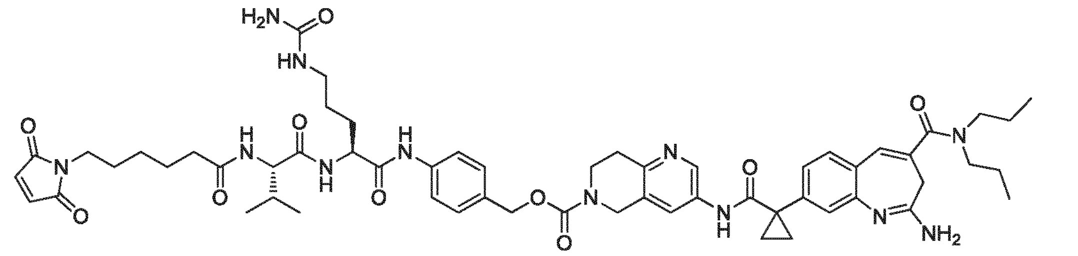

,

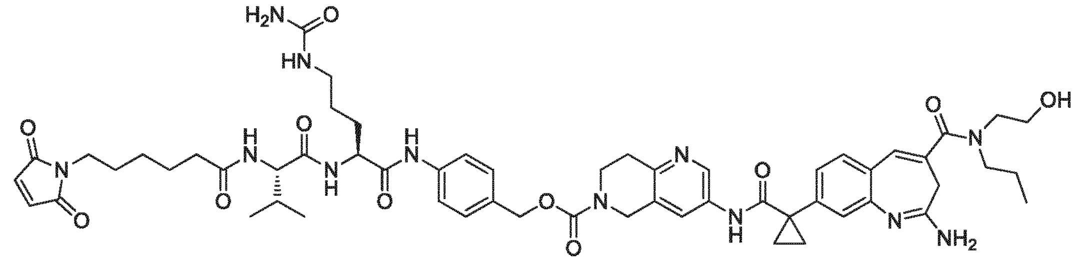

,

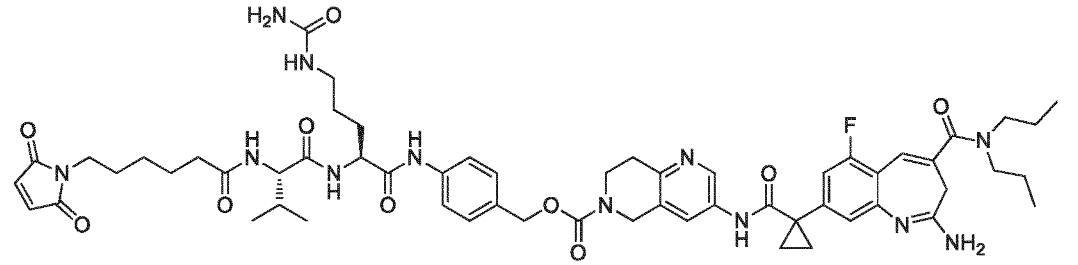

,

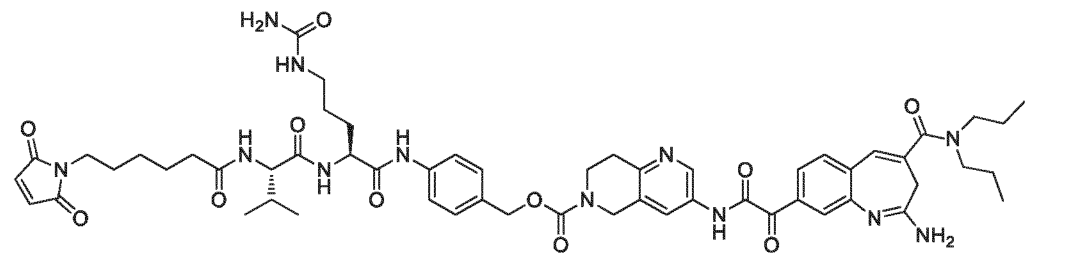

,

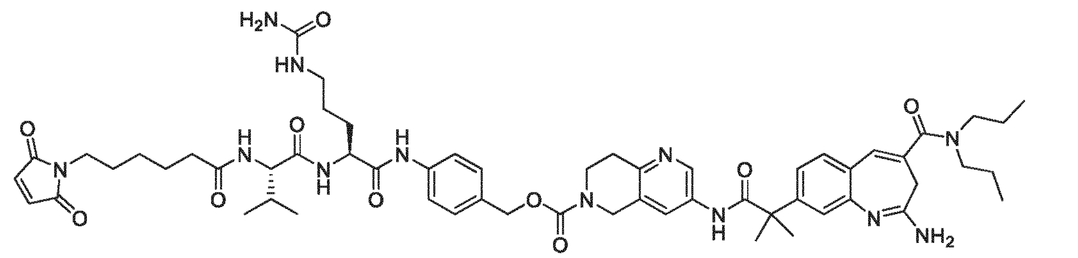

,

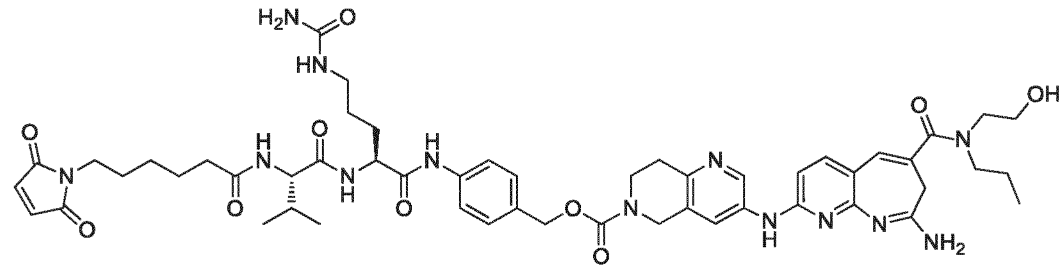

,

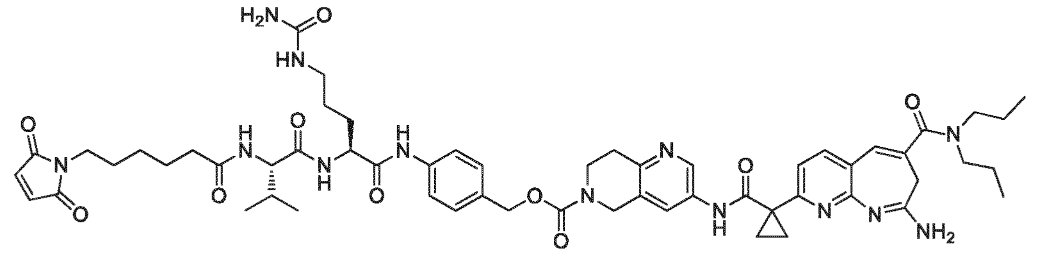

,

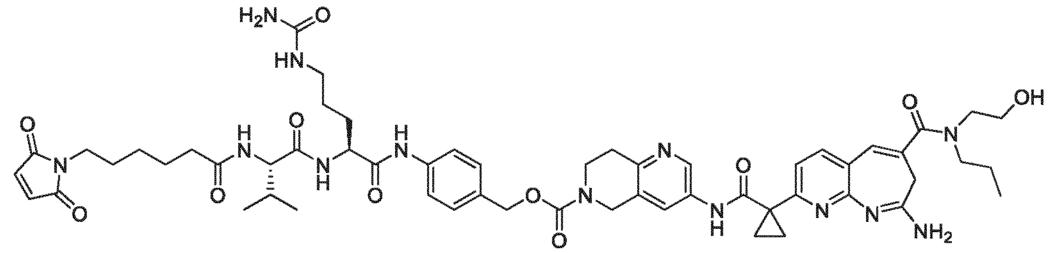

,

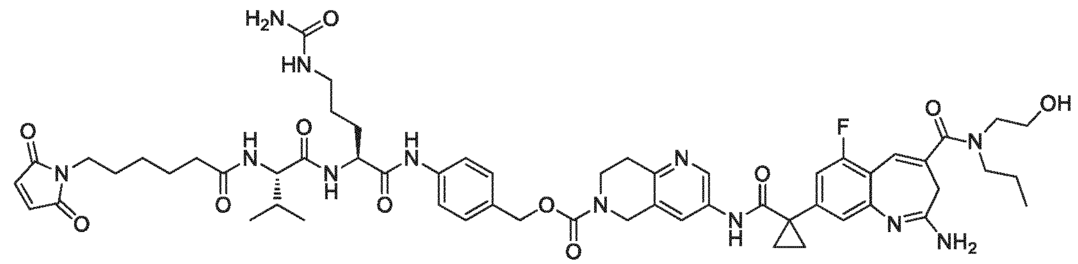

,

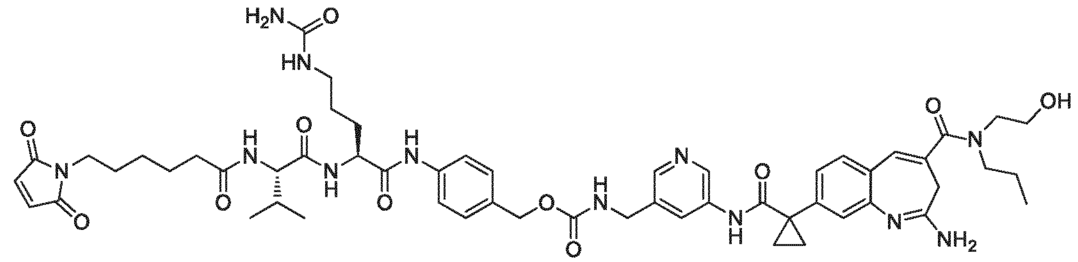

,

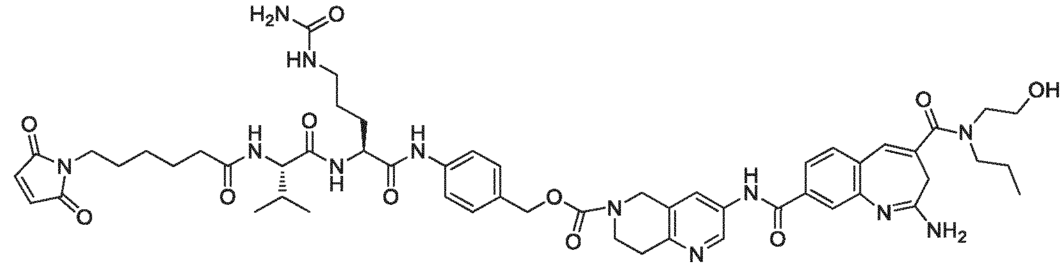

,

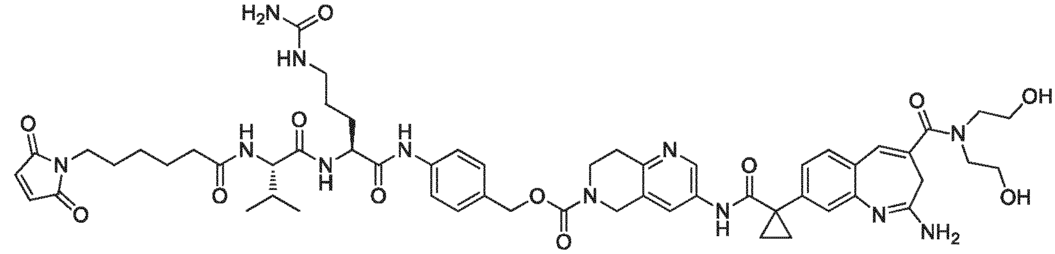

,

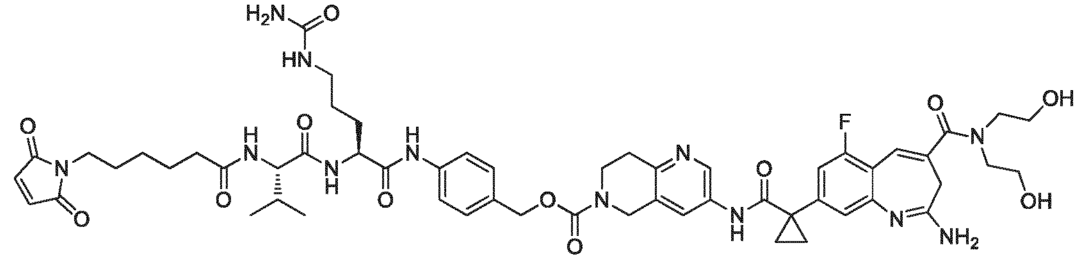

,

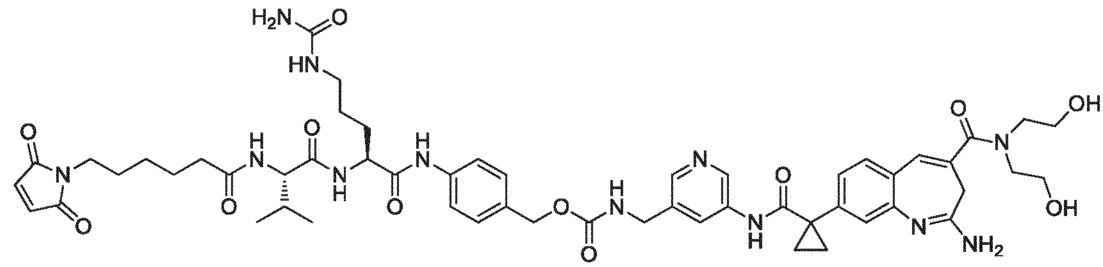

,

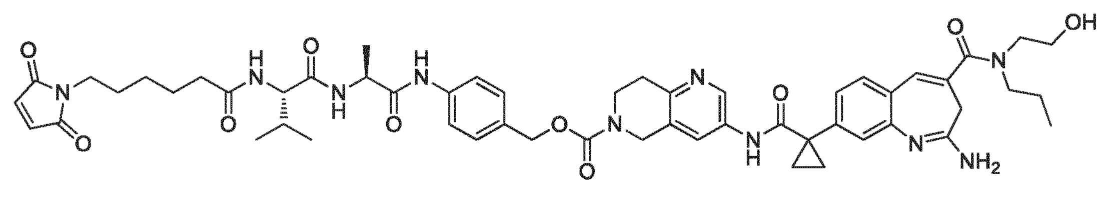

,

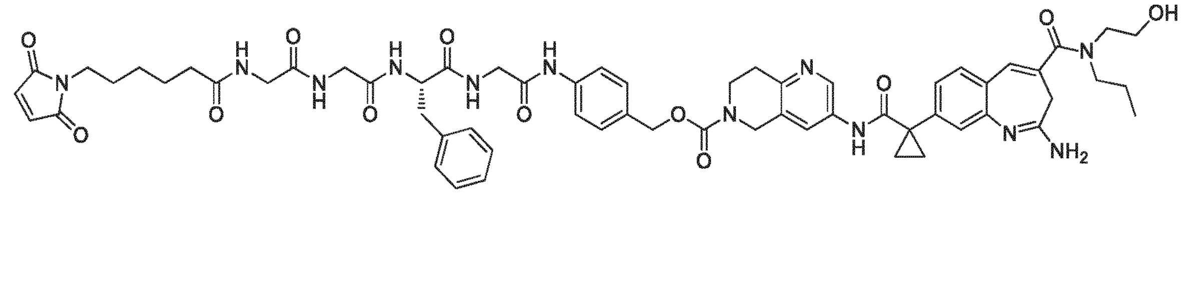

,

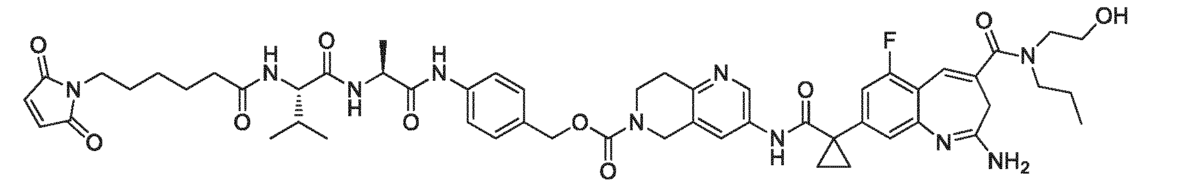

,

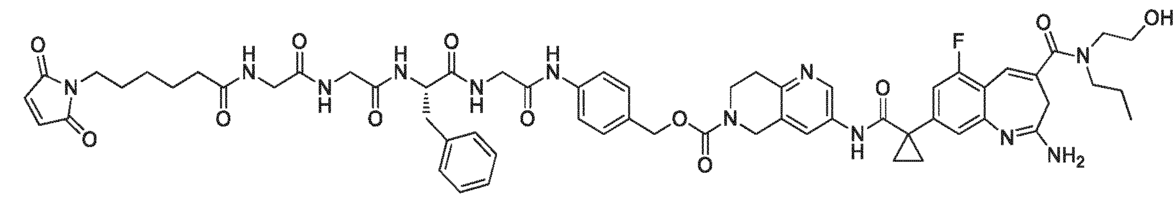

,

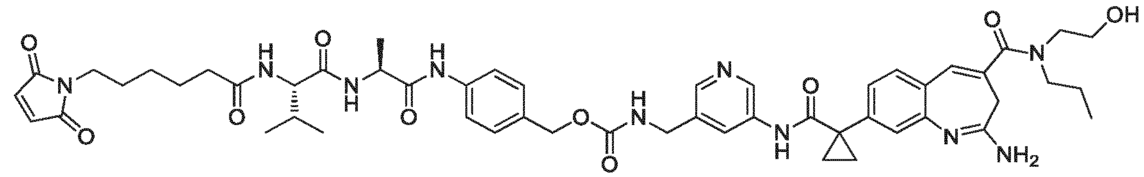

,

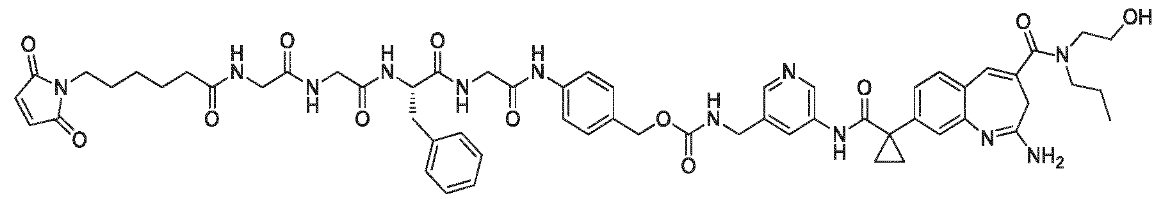

,

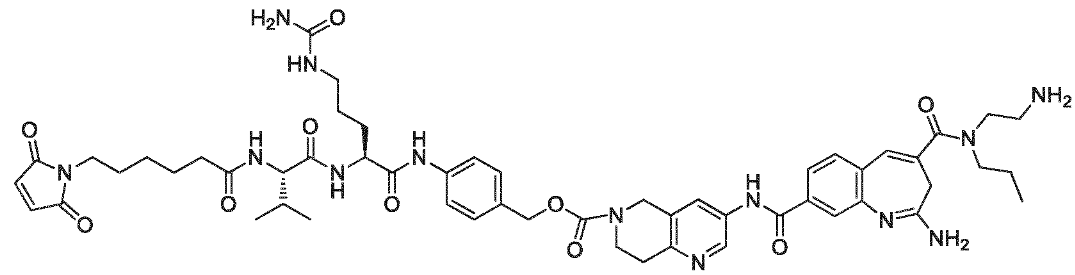

,

or

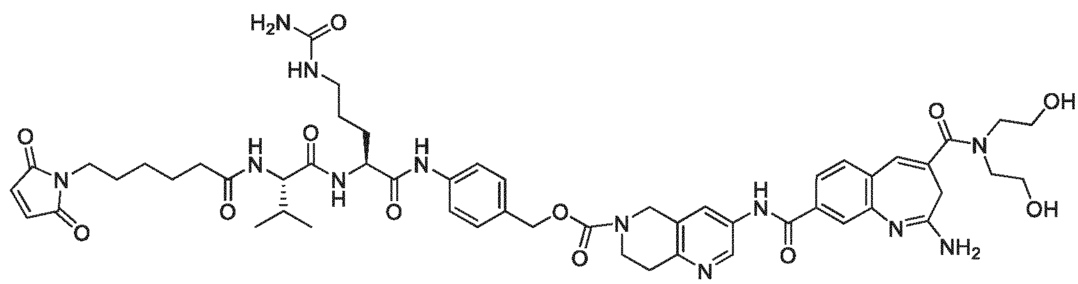

.

**21.** An immune-stimulating antibody conjugate of formula III or a pharmaceutically acceptable salt thereof,

$$Ab\text{—}(L\text{—}D)_t$$

(III)

wherein Ab is an antibody;
L is a linker that attaches Ab to D;
D is a group formed by losing one hydrogen atom in the compound of formula I according to at least one selected from the group of claims 1 to 11;
t is any value from 1 to 8.

**22.** The immune-stimulating antibody conjugate of formula III or the pharmaceutically acceptable salt thereof according to claim 21, wherein the immune-stimulating antibody conjugate of formula III satisfies one or more than one of the following conditions:

zz) the antibody contains one or more than one antigen-binding domain that can bind to an antigen;
aaa) the antibody contains one Fc region;
bbb) the antibody is a monoclonal antibody;
ccc) the D is a group formed by losing one hydrogen atom from $R_7$ of the compound of formula I;
ddd) the L is a degradable linker or a non-degradable linker;
eee) the t is any value from 2 to 5.

**23.** The immune-stimulating antibody conjugate of formula III or the pharmaceutically acceptable salt thereof according to claim 22, wherein the immune-stimulating antibody conjugate of formula III satisfies one or more than one of the following conditions:

fff) the antibody contains one or two antigen-binding domains that can bind to an antigen;
ggg) the D is a group formed by losing one hydrogen atom from the secondary or primary amine of $R_7$ of the compound of formula I;
hhh) the L is a linker that can be degraded by lysosomal enzymes.

**24.** The immune-stimulating antibody conjugate of formula III or the pharmaceutically acceptable salt thereof according to claim 23, wherein the immune-stimulating antibody conjugate of formula III satisfies one or more than one of the following conditions:

iii) the antibody is an anti-HER2 antibody;
jjj) the D is attached to the linker via the a-end of

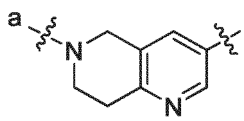

or

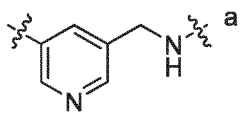

;

kkk) the L is

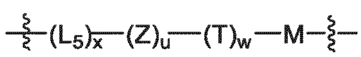

,

x is 0, 1, 2, or 3;
u is 0, 1, 2, 3, 4, 5, or 6;

w is 0, 1, 2, 3, 4, 5, or 6;
each $L_5$ is independently

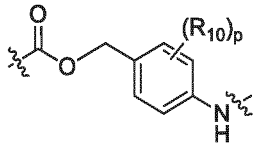, 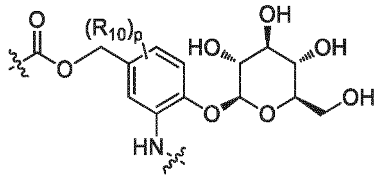,

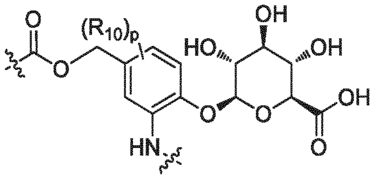, 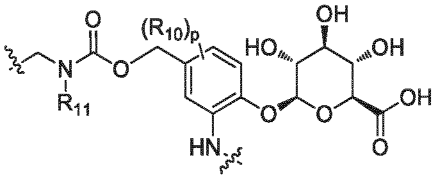, or 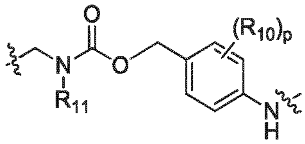;

p is 1, 2, or 3; each $R_{10}$ is independently hydrogen, halogen, $C_{1-6}$ alkyl, halo-$C_{1-6}$ alkyl, nitro, cyano, $C_{1-6}$ alkoxy, $C_{1-6}$ alkylamino, $-OC(O)R_{11}$, or $-C(O)N(R_{11})_2$; $R_{11}$ is hydrogen, $C_{1-6}$ alkyl, or $C_{1-6}$ alkylamino-$C_{1-6}$ alkyl;
each Z is independently

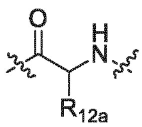, 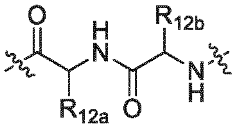, 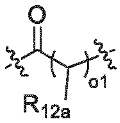, 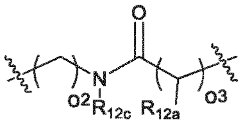,

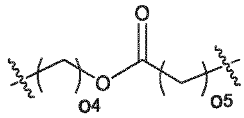, 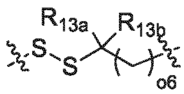,

and $-(CH_2CH_2O)_{o7}-$; o1, o2, o3, o4, o5, o6, o7 are each independently 0, 1, 2, 3, 4, 5, 6, 7, or 8; $R_{12a}$, $R_{12b}$, and $R_{12c}$ are each independently hydrogen, methyl, ethyl, *n*-propyl, isopropyl, $-SO_3H$,

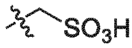, 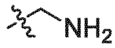, 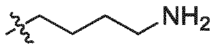,

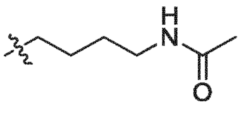, 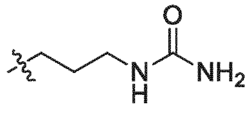, 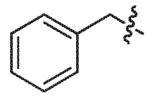, 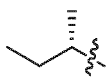, 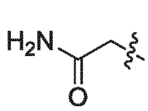,

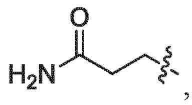, or 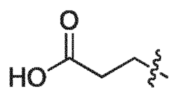;

$R_{13a}$ and $R_{13b}$ are each independently hydrogen or methyl;
each T is independently $-(CH_2)_{v1}-$, $-(CH_2CH_2O)_{V2}-$,

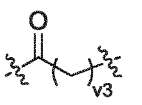, 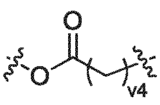,

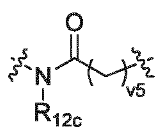, 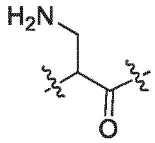, 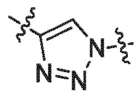, or 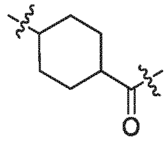;

v1, v2, v3, v4, and v5 are each independently 1, 2, 3, 4, 5, or 6;
M is a linkage bond or a linker head.

**25.** The immune-stimulating antibody conjugate of formula III or the pharmaceutically acceptable salt thereof according to claim 24, wherein the immune-stimulating antibody conjugate of formula III satisfies one or more than one of the following conditions:

lll) the antibody is Trastuzumab;
mmm) the $L_5$ is

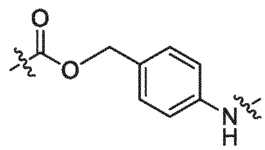;

nnn) the carbonyl end of the $L_5$ is attached to the D;
ooo) the Z is an amino acid;
ppp) the

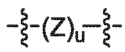

is a peptide chain;
qqq) the carbonyl end of the

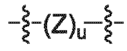

is attached to the amino end of the $L_5$;
rrr) the

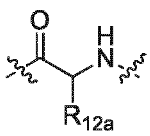 is 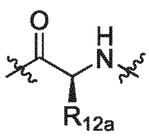 or 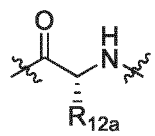;

sss) the carbonyl end of the T is attached to the amino end of the

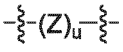;

ttt) the amino end of the M is attached to the non-carbonyl end of the T.

**26.** The immune-stimulating antibody conjugate of formula III or the pharmaceutically acceptable salt thereof according to claim 25, wherein the L is

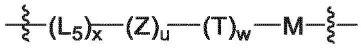,

x is 1;
u is 1, 2, 3, 4, 5, or 6;
w is 1;
Ls is

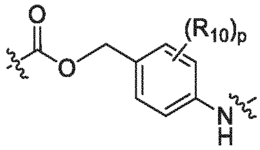;

p is 1; $R_{10}$ is hydrogen; the carbonyl end of the $L_5$ is attached to the D;
each Z is independently

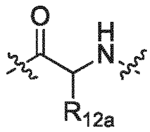;

each $R_{12a}$ is independently hydrogen, methyl, ethyl, n-propyl, isopropyl,

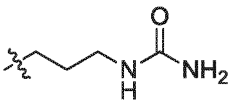, or 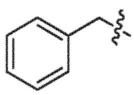;

the carbonyl end of the

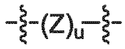

is attached to the amino end of the $L_5$;
Tis

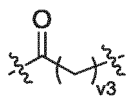;

v3 is 1, 2, 3, 4, 5, or 6; the carbonyl end of the T is attached to the amino end of

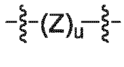;

M is

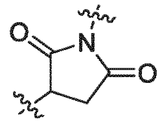,

the amino end of the M is attached to the non-carbonyl end of the T.

**27.** The immune-stimulating antibody conjugate of formula III or the pharmaceutically acceptable salt thereof according to at least one selected from the group of claims 21 to 26, wherein the definition of the immune-stimulating antibody conjugate of formula III is as described in any one of the following schemes:

scheme 27-1:

Ab is an antibody;
L is a linker that attaches Ab to D;
D is a group formed by losing one hydrogen atom in the compound of formula I;
t is any value from 1 to 8;

scheme 27-2:

Ab is an anti-HER2 monoclonal antibody;
Lis

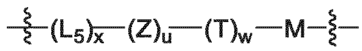

x is 1;
u is 1, 2, 3, 4, 5, or 6;
w is 1;
$L_5$ is

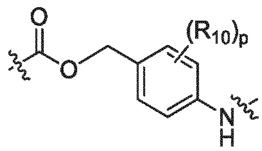;

p is 1; $R_{10}$ is hydrogen; the carbonyl end of the $L_5$ is attached to the D;
each Z is independently

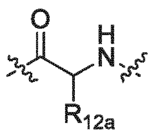;

each $R_{12a}$ is independently hydrogen, methyl, ethyl, n-propyl, isopropyl,

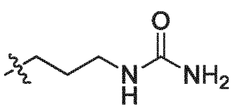, or 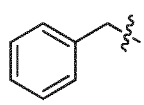;

the carbonyl end of the

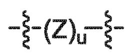

is attached to the amino end of the $L_5$;

Tis

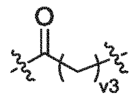

v3 is 1, 2, 3, 4, 5, or 6; the carbonyl end of the T is attached to the amino end of

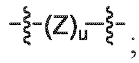

M is

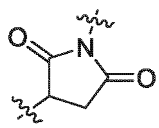

,

the amino end of the M is attached to the non-carbonyl end of the T;

the D is a group formed by losing one hydrogen atom from $R_7$ of the compound of formula I;

t is any value from 1 to 8;

scheme 27-3:

Ab is an antibody;

L is a linker that attaches Ab to D;

t is any value from 1 to 8;

D is the compound of formula I;

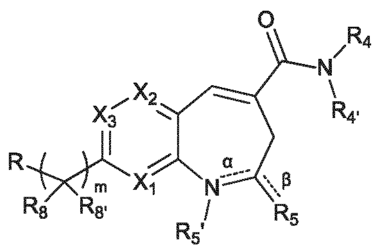

(I) ;

α and β are independently a single bond or a double bond; at least one selected from the group of α and β is a single bond;

m is 0 or 1;

$X_1$ is N or $CR_2$, $X_2$ is N or $CR_3$, and $X_3$ is N or $CR_1$; when $X_1$ is $CR_2$, $X_2$ is $CR_3$, and $X_3$ is $CR_1$, m is 1;

R is $-C(O)-L_1-R_7$, $-C(O)-NR_9-L_1-R_7$, $-C(S)-NR_9-L_1-R_7$, $-NR_9-L_1-R_7$, $-NR_9-C(O)-L_1-R_7$, $-NR_9-C(O)-NR_9-L_1-R_7$, $-O-L_1-R_7$, $-S(O)_2-NR_9-L_1-R_7$, $-CH=CH-L_1-R_7$, or $-S(O)_2-L_1-R_7$;

$R_1$, $R_2$, and $R_3$ are each independently hydrogen, deuterium, halogen, hydroxyl, amino, cyano, alkyl, haloalkyl, $-L_2-OR_a$, or $-L_2-NR_aR_b$;

$R_4$ and $R_{4'}$ are each independently hydrogen, alkyl, alkenyl, alkynyl, cycloalkyl, heterocycloalkyl, aryl, heteroaryl, cycloalkylalkyl, heterocycloalkylalkyl, arylalkyl, or heteroarylalkyl; the $R_4$ or $R_{4'}$ is unsubstituted or optionally substituted at any position by one or more than one substituent selected from halogen, cyano,

$-L_2-OR_a$, $-L_2-OC(O)R_a$, $-L_2-NR_aR_b$, $-L_2-NR_aC(O)OR_b$, $-L_2-NR_aC(O)NR_aR_b$, $-L_2-NR_bC(NR_b)NR_aR_b$, and $-L_2-C(O)OR_b$; or, $R_4$ and $R_{4'}$ together with the N atom to which they are attached form a 3- to 8-membered heterocycloalkyl group; the 3- to 8-membered heterocycloalkyl group is unsubstituted or further substituted at any position by 1 to 3 substituents selected from halogen, cyano, $-L_2-OR_a$, $-L_2-OC(O)R_a$, $-L_2-NR_aR_b$, $-L_2-NR_aC(O)OR_b$, $-L_2-NR_aC(O)NR_aR_b$, $-L_2-NR_bC(NR_b)NR_aR_b$, and $-L_2-C(O)OR_b$;

$R_5$ is =O, $=NR_a$, $-OR_a$, or $-NR_aR_b$;

$R_{5'}$ is absent, or $R_{5'}$ is $-R_c$, $-L_2-OR_a$, $-L_2-NR_aR_b$, $-L_2-NR_aC(O)OR_b$, $-L_2-NR_aC(O)NR_aR_b$, $-L_2-NR_bC(NR_b)NR_aR_b$, or $-L_2-C(O)OR_b$;

$R_7$ is phenyl, 5- to 6-membered heteroaryl, 3- to 8-membered heterocycloalkyl, or an 8-to 12-membered fused ring group; the $R_7$ is unsubstituted or optionally substituted at any position by one or more than one substituent selected from $-L_3-W$, $-R_c$, halogen, cyano, nitro, alkyl, alkenyl, alkynyl, alkoxy, haloalkyl, haloalkoxy, and alkylamino;

$R_8$ and $R_{8'}$ are each independently hydrogen, halogen, or alkyl, and the $R_8$ or $R_{8'}$ is unsubstituted or optionally substituted at any position by one or more than one substituent selected from $-L_3-W$, halogen, cyano, nitro, alkyl, alkenyl, alkynyl, alkoxy, haloalkyl, haloalkoxy, and alkylamino; $R_8$ and $R_{8'}$ are each independent substituents, or, $R_8$ and Rs> together with the carbon atom to which they are attached form an oxo, thio, $C_{1-6}$ alkylene, $C_{3-10}$ cycloalkyl, or 3- to 10-membered heterocycloalkyl group; the $C_{1-6}$ alkylene, $C_{3-10}$ cycloalkyl, or 3- to 10-membered heterocycloalkyl group is unsubstituted or optionally substituted at any position by one or more than one substituent selected from deuterium, halogen, hydroxyl, amino, cyano, oxo, alkyl, haloalkyl, $-L_2-OR_a$, and $-L_2-NR_aR_b$;

$R_9$ is hydrogen, alkyl, haloalkyl, $-L_2-OR_a$, or $-L_2-NR_aR_b$;

W is $Cy^1$, $-SR_d$, $-OR_d$, $-OC(O)R_e$, $-OC(O)NR_eR_{e'}$, $-C(O)OR_e$, $-C(O)R_e$, $-C(O)NR_eR_{e'}$, $-C(O)NR_eS(O)_2R_e$, $-NR_dR_e$, $-NR_dC(O)R_e$, $-N(R_d)C(O)OR_e$, $-N(R_d)C(O)NR_eR_{e'}$, $-NR_dS(O)_2R_e$, $-NR_dS(O)_2NR_eR_{e'}$, $-S(O)_{1-2}R_e$, $-S(O)_2NR_eR_{e'}$, $-S(O)(=NR_d)R_e$, $-S(O)_2N(R_e)C(O)R_{e'}$, $-P(O)(OR_e)_2$, $-P(O)(OR_e)R_{e'}$, $-OP(O)(OR_e)_2$, or $-B(OR_e)_2$;

$Cy^1$ is cycloalkyl, heterocycloalkyl, aryl, or heteroaryl; the $Cy^1$ is unsubstituted or optionally substituted at any position by one or more than one substituent selected from halogen, alkyl, haloalkyl, haloalkoxy, alkenyl, alkynyl, cyano, $-R_c$, $-L_4-SR_d$, $-L_4-OC(O)R_e$, $-L_4-C(O)OR_e$, $-L_4-C(O)R_e$, $-L_4-C(O)NR_eR_{e'}$, $-L_4-NR_dC(O)R_e$, $-L_4-NR_dS(O)_2R_e$, $-L_4-S(O)_{1-2}R_e$, $-L_4-S(O)_2NR_eR_{e'}$, $-L_4-OR_d$, and $-L_4-NR_eR_{e'}$;

$L_1$, $L_2$, $L_3$, and $L_4$ are each independently a linkage bond, $C_{1-6}$ alkylene, $C_{2-6}$ alkenylene, or $C_{2-6}$ alkynylene; the $L_1$, $L_2$, $L_3$, or $L_4$ is unsubstituted or optionally substituted at any position by one or more than one substituent selected from oxo, hydroxyl, amino, halogen, cyano, alkyl, haloalkyl, alkoxy, and haloalkoxy;

$R_a$, $R_b$, $R_d$, $R_e$, and $R_{e'}$ are each independently $-R_c$, amino, $C_{1-6}$ alkyl, $C_{2-6}$ alkenyl, $C_{2-6}$ alkynyl, $C_{3-10}$ cycloalkyl, 3- to 10-membered heterocycloalkyl, $C_{6-10}$ aryl, 5- to 10-membered heteroaryl, $C_{3-10}$ cycloalkyl-$C_{1-6}$ alkyl, 3- to 10-membered heterocycloalkyl-$C_{1-6}$ alkyl, phenyl-$C_{1-6}$ alkyl, or 5- to-10 membered heteroaryl-$C_{1-6}$ alkyl; the $R_a$, $R_b$, $R_d$, $R_e$, or $R_{e'}$ is unsubstituted or optionally substituted at any position by 1 to 3 substituents selected from $-OR_f$, $-OC(O)-L_4-R_f$, $-NR_fR_{f'}$, halogen, cyano, nitro, $C_{1-6}$ alkyl, halo-$C_{1-6}$ alkyl, and halo-$C_{1-6}$ alkoxy;

$R_f$ and $R_{f'}$ are each independently $-R_c$, $-NHR_c$, or $C_{1-6}$ alkyl;

each $R_c$ is independently hydrogen or a linkage bond attached to L; at least one $R_c$ in D is a linkage bond attached to L;

scheme 27-4:

Ab is an antibody;

L is a linker that attaches Ab to D;

t is any value from 1 to 8;

D is the compound of formula I, the stereoisomer, or the pharmaceutically acceptable salt thereof:

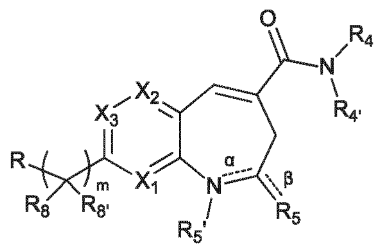

(I) ;

$\alpha$ **and** $\beta$ **are independently a single bond or a double bond;** at least one selected from the group of $\alpha$ **and** $\beta$ is a single bond;

m is 0 or 1;

$X_1$ is N or $CR_2$, $X_2$ is N or $CR_3$, and $X_3$ is N or $CR_1$; when $X_1$ is $CR_2$, $X_2$ is $CR_3$, and $X_3$ is $CR_1$, m is 1;

R is $-C(O)-L_1-R_7$, $-C(O)-NR_9-L_1-R_7$, $-C(S)-NR_9-L_1-R_7$, $-NR_9-L_1-R_7$, $-NR_9-C(O)-L_1-R_7$, $-NR_9-C(O)-NR_9-L_1-R_7$, $-O-L_1-R_7$, $-S(O)_2-NR_9-L_1-R_7$, $-CH=CH-L_1-R_7$, or $-S(O)_2-L_1-R_7$;

$R_1$, $R_2$, and $R_3$ are each independently hydrogen, deuterium, halogen, hydroxyl, amino, cyano, alkyl, haloalkyl, $-L_2-OR_a$, or $-L_2-NR_aR_b$;

$R_4$ and $R_{4'}$ are each independently hydrogen, alkyl, alkenyl, alkynyl, cycloalkyl, heterocycloalkyl, aryl, heteroaryl, cycloalkylalkyl, heterocycloalkylalkyl, arylalkyl, or heteroarylalkyl; the $R_4$ or $R_{4'}$ is unsubstituted or optionally substituted at any position by one or more than one substituent selected from halogen, cyano, $-L_2-OR_a$, $-L_2-NR_aR_b$, $-L_2-NR_aC(O)OR_b$, $-L_2-NR_aC(O)NR_aR_b$, $-L_2-NR_bC(NR_b)NR_aR_b$, and $-L_2-C(O)OR_b$; or, $R_4$ and $R_{4'}$ together with the N atom to which they are attached form a 3- to 8-membered heterocycloalkyl group; the 3- to 8-membered heterocycloalkyl group is unsubstituted or further substituted at any position by 1 to 3 substituents selected from halogen, cyano, $-L_2-OR_a$, $-L_2-NR_aR_b$, $-L_2-NR_aC(O)OR_b$, $-L_2-NR_aC(O)NR_aR_b$, $-L_2-NR_bC(NR_b)NR_aR_b$, and $-L_2-C(O)OR_b$;

$R_5$ is =O, $=NR_a$, $-OR_a$, or $-NR_aR_b$;

$R_{5'}$ is absent, or $R_{5'}$ is $-R_c$, $-L_2-OR_a$, $-L_2-NR_aR_b$, $-L_2-NR_aC(O)OR_b$, $-L_2-NR_aC(O)NR_aR_b$, $-L_2-NR_bC(NR_b)NR_aR_b$, or $-L_2-C(O)OR_b$;

$R_7$ is phenyl, 5- to 6-membered heteroaryl, 3- to 8-membered heterocycloalkyl, or an 8-to 12-membered fused ring group; the $R_7$ is unsubstituted or optionally substituted at any position by one or more than one substituent selected from $-L_3-W$, $-R_c$, halogen, cyano, nitro, alkyl, alkenyl, alkynyl, alkoxy, haloalkyl, haloalkoxy, and alkylamino;

$R_8$ and $R_{8'}$ are each independently hydrogen, halogen, or alkyl, and the $R_8$ or $R_{8'}$ is unsubstituted or optionally substituted at any position by one or more than one substituent selected from $-L_3-W$, halogen, cyano, nitro, alkyl, alkenyl, alkynyl, alkoxy, haloalkyl, haloalkoxy, and alkylamino; $R_8$ and $R_{8'}$ are each independent substituents, or, $R_8$ and Rs> together with the carbon atom to which they are attached form an oxo, thio, $C_{1-6}$ alkylene, $C_{3-10}$ cycloalkyl, or 3- to 10-membered heterocycloalkyl group; the $C_{1-6}$ alkylene, $C_{3-10}$ cycloalkyl, or 3- to 10-membered heterocycloalkyl group is unsubstituted or optionally substituted at any position by one or more than one substituent selected from deuterium, halogen, hydroxyl, amino, cyano, oxo, alkyl, haloalkyl, $-L_2-OR_a$, and $-L_2-NR_aR_b$;

$R_9$ is hydrogen, alkyl, haloalkyl, $-L_2-OR_a$, or $-L_2-NR_aR_b$;

W is $Cy^1$, $-SR_d$, $-OR_d$, $-OC(O)R_e$, $-OC(O)NR_eR_{e'}$, $-C(O)OR_e$, $-C(O)R_e$, $-C(O)NR_eR_{e'}$, $-C(O)NR_eS(O)_2R_e$, $-NR_dR_e$, $-NR_dC(O)R_e$, $-N(R_d)C(O)OR_e$, $-N(R_d)C(O)NR_eR_{e'}$, $-NR_dS(O)_2R_e$, $-NR_dS(O)_2NR_eR_{e'}$, $-S(O)_{1-2}R_e$, $-S(O)_2NR_eR_{e'}$, $-S(O)(=NR_d)R_e$, $-S(O)_2N(R_e)C(O)R_{e'}$, $-P(O)(OR_e)_2$, $-P(O)(OR_e)R_{e'}$, $-OP(O)(OR_e)_2$, or $-B(OR_e)_2$;

$Cy^1$ is cycloalkyl, heterocycloalkyl, aryl, or heteroaryl; the $Cy^1$ is unsubstituted or optionally substituted at any position by one or more than one substituent selected from halogen, alkyl, haloalkyl, haloalkoxy, alkenyl, alkynyl, cyano, $-R_c$, $-L_4-SR_d$, $-L_4-OC(O)R_e$, $-L_4-C(O)OR_e$, $-L_4-C(O)R_e$, $-L_4-C(O)NR_eR_{e'}$, $-L_4-NR_dC(O)R_e$, $-L_4-NR_dS(O)_2R_e$, $-L_4-S(O)_{1-2}R_e$, $-L_4-S(O)_2NR_eR_{e'}$, $-L_4-OR_d$, and $-L_4-NR_eR_{e'}$;

$L_1$, $L_2$, $L_3$, and $L_4$ are each independently a linkage bond, $C_{1-6}$ alkylene, $C_{2-6}$ alkenylene, or $C_{2-6}$ alkynylene; the $L_1$, $L_2$, $L_3$, or $L_4$ is unsubstituted or optionally substituted at any position by one or more than one substituent selected from oxo, hydroxyl, amino, halogen, cyano, alkyl, haloalkyl, alkoxy, and haloalkoxy;

$R_a$, $R_b$, $R_d$, $R_e$, and $R_{e'}$ are each independently $-R_c$, amino, $C_{1-6}$ alkyl, $C_{2-6}$ alkenyl, $C_{2-6}$ alkynyl, $C_{3-10}$ cycloalkyl, 3- to 10-membered heterocycloalkyl, $C_{6-10}$ aryl, 5- to 10-membered heteroaryl, $C_{3-10}$ cycloalkyl-$C_{1-6}$ alkyl, 3- to 10-membered heterocycloalkyl-$C_{1-6}$ alkyl, phenyl-$C_{1-6}$ alkyl, or 5- to-10 membered heteroaryl-$C_{1-6}$ alkyl; the $R_a$, $R_b$, $R_d$, $R_e$, or $R_{e'}$ is unsubstituted or optionally substituted at any position by 1 to

3 substituents selected from $-OR_f$, $-NR_fR_{f'}$,, halogen, cyano, nitro, $C_{1-6}$ alkyl, halo-$C_{1-6}$ alkyl, and halo-$C_{1-6}$ alkoxy;
$R_f$ and $R_{f'}$ are each independently $-R_c$, $-NHR_c$, or $C_{1-6}$ alkyl;
each $R_c$ is independently hydrogen or a linkage bond attached to L; at least one $R_c$ in D is a linkage bond attached to L.

**28.** The immune-stimulating antibody conjugate of formula III or the pharmaceutically acceptable salt thereof according to at least one selected from the group of claims 21 to 26, wherein the linker has any one of the following structures:

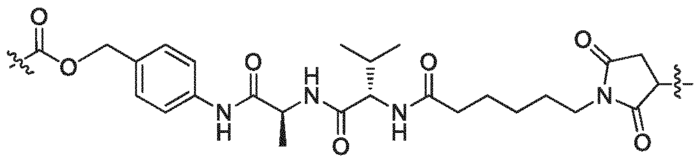

,

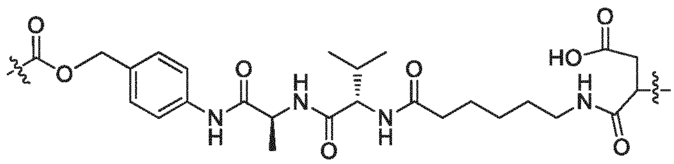

,

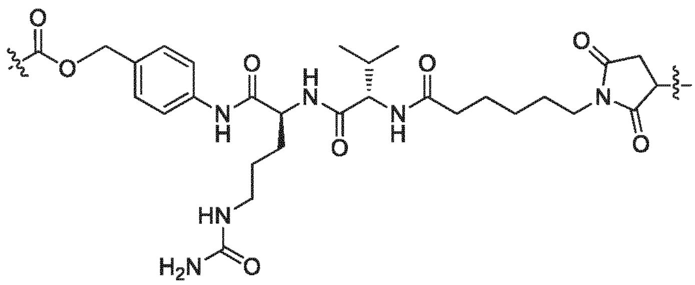

,

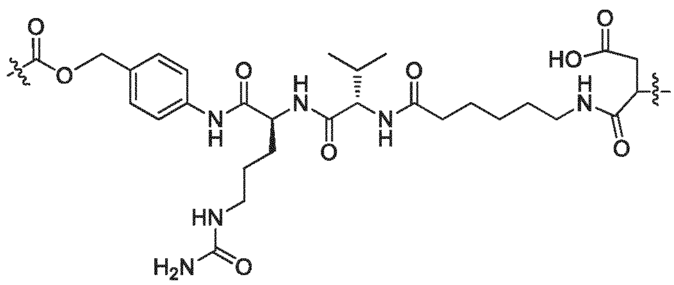

,

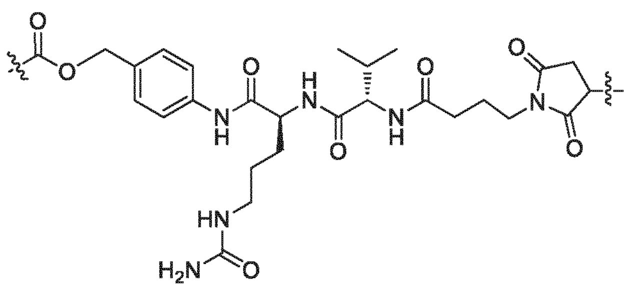

,

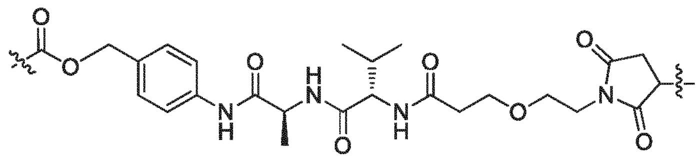

,

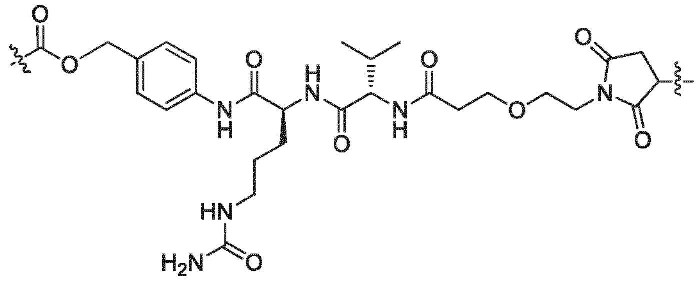

,

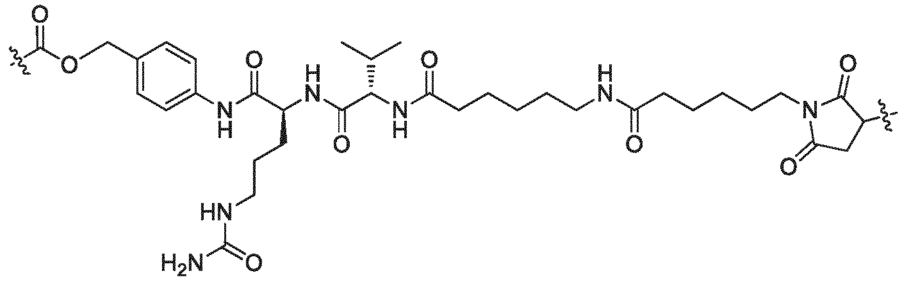

,

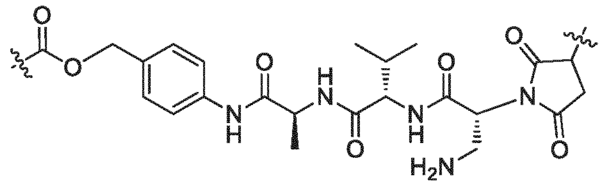

,

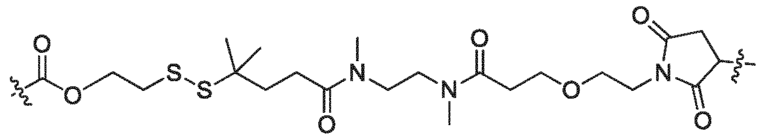

,

or

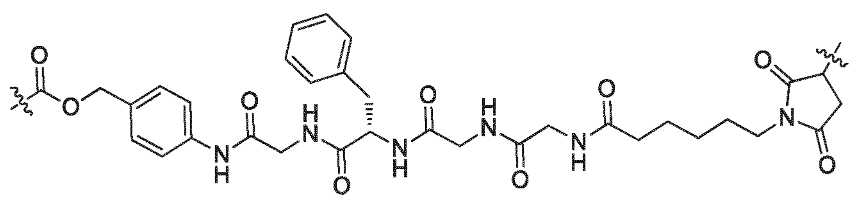

;

the carbonyl end of the linker is attached to the D.

**29.** The immune-stimulating antibody conjugate of formula III or the pharmaceutically acceptable salt thereof according to at least one selected from the group of claims 21 to 26, wherein the immune-stimulating antibody conjugate of formula III has any one of the following structures:

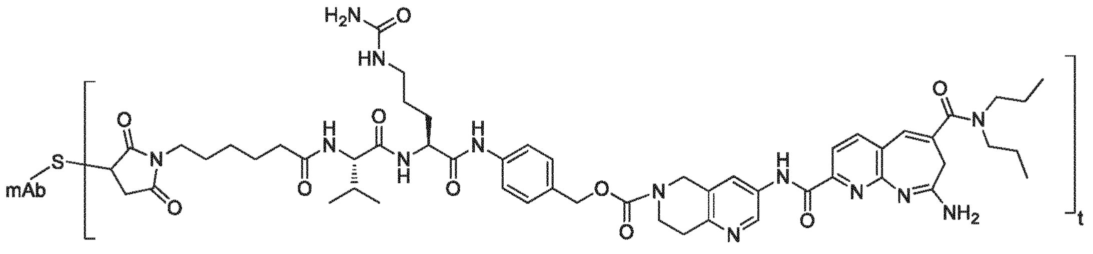
,

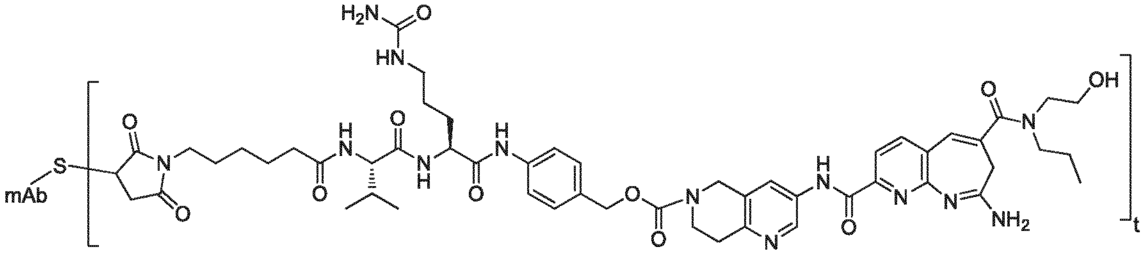
,

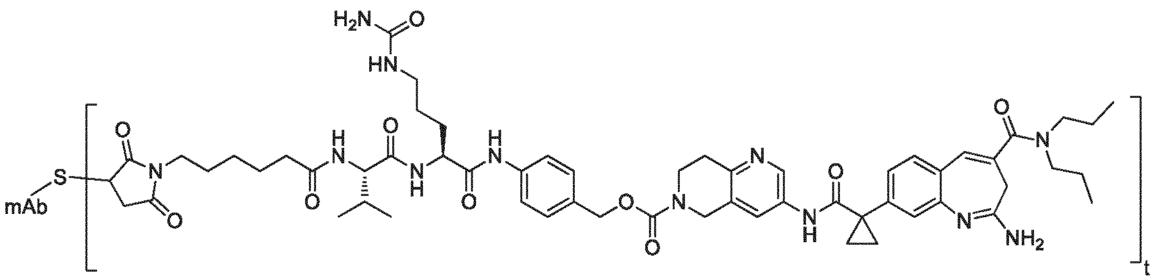
,

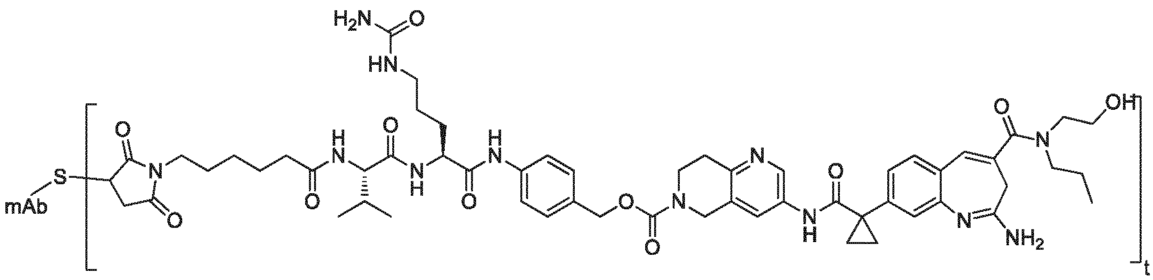
,

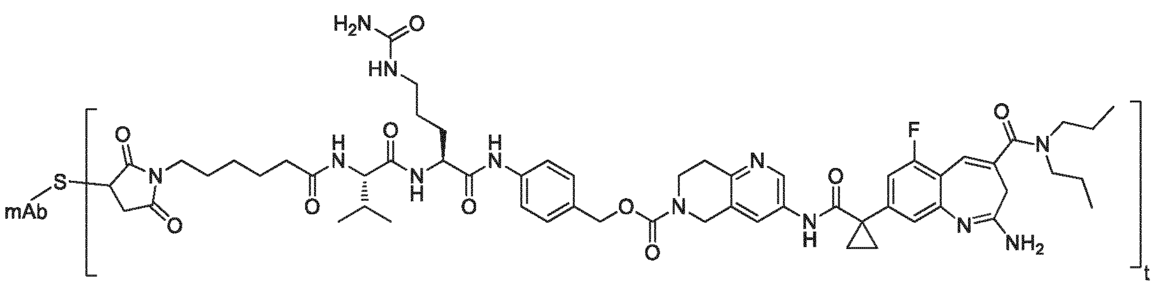
,

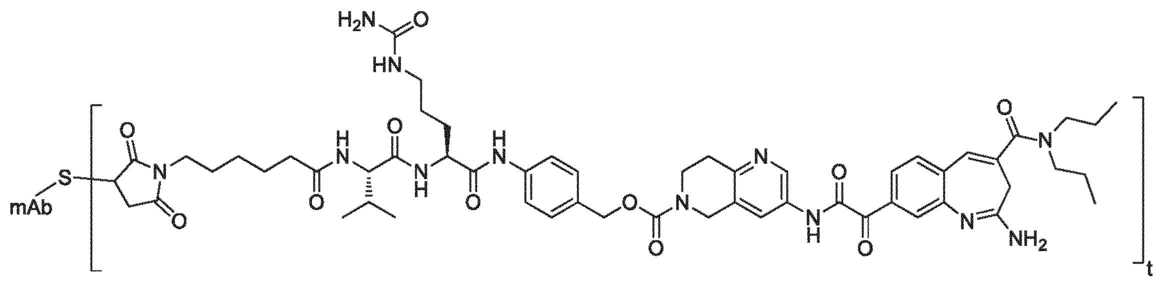

,

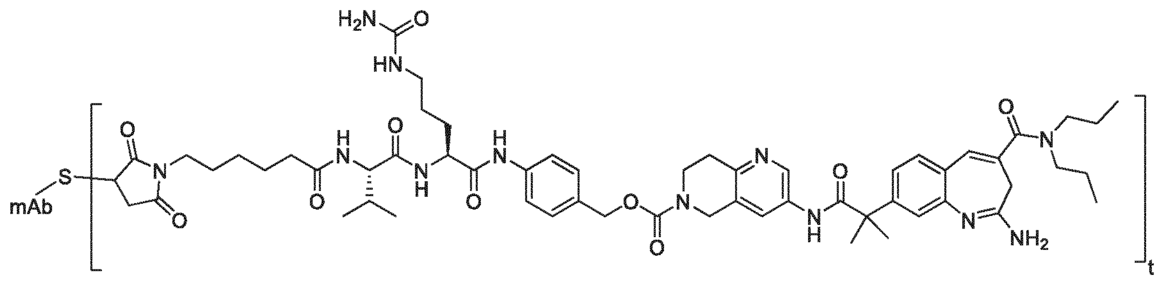

,

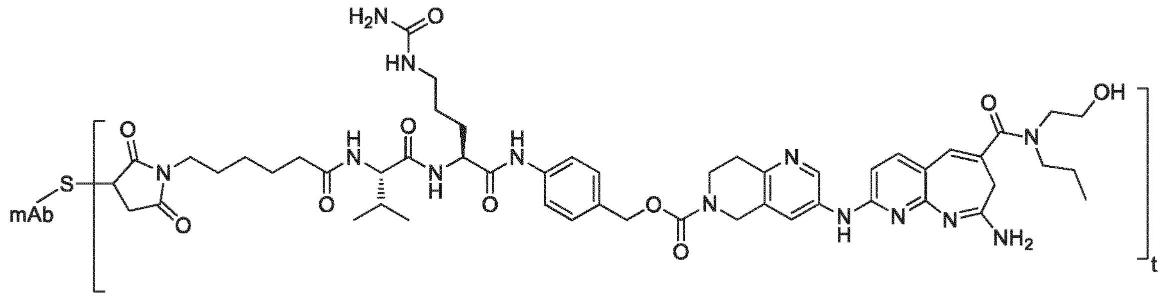

,

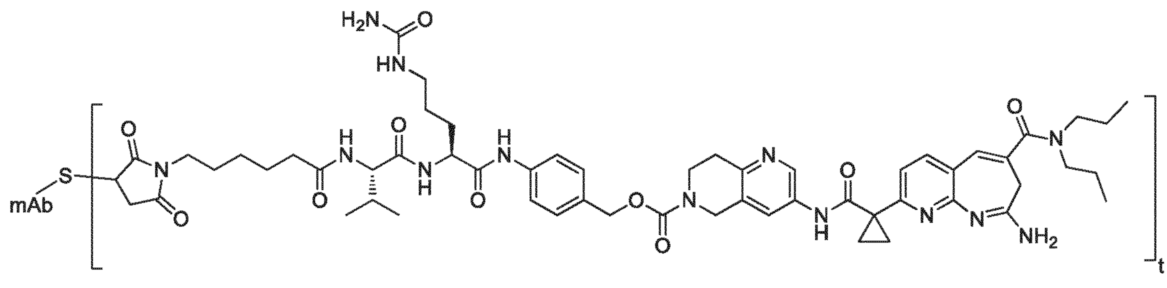

,

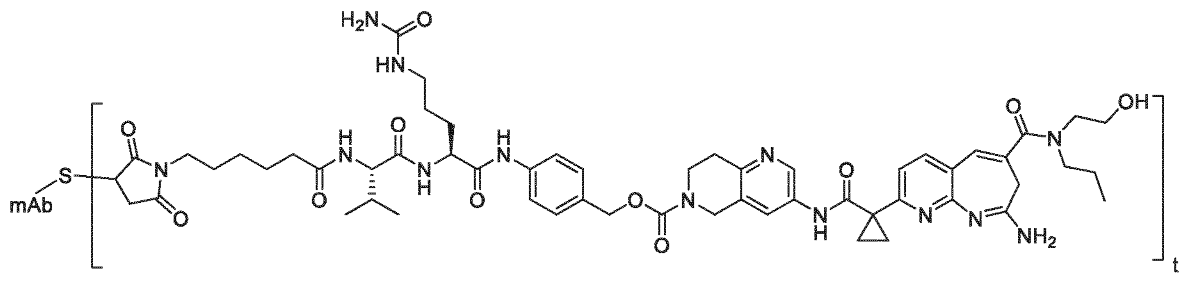

,

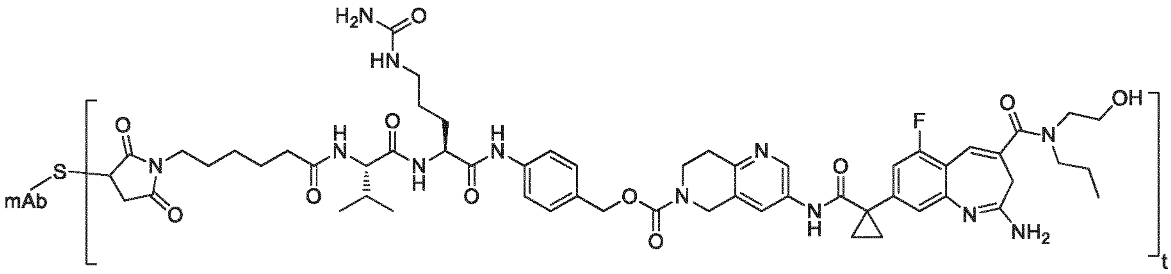

,

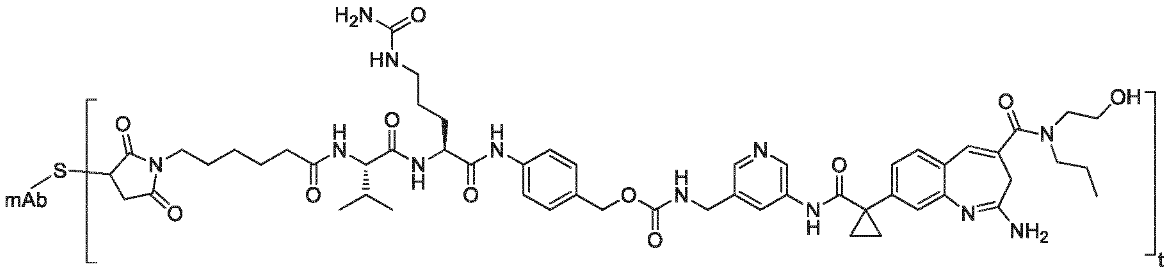

,

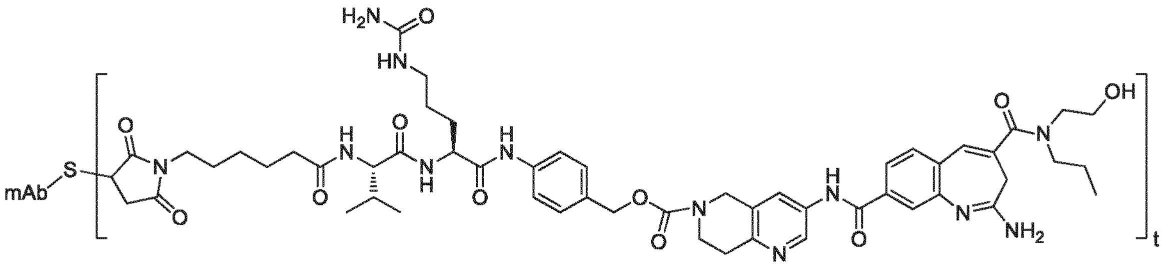

,

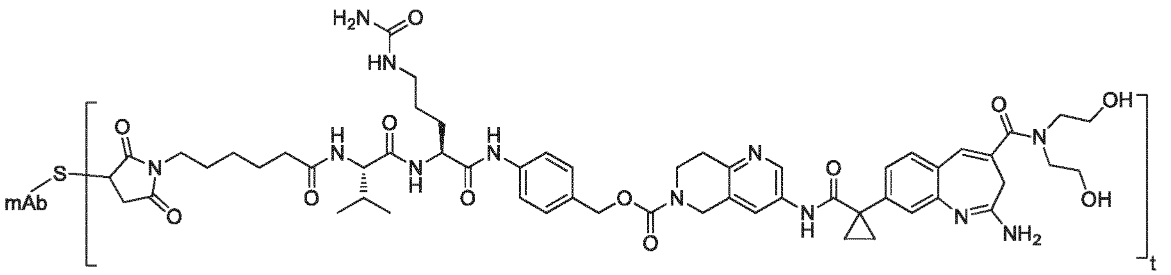

,

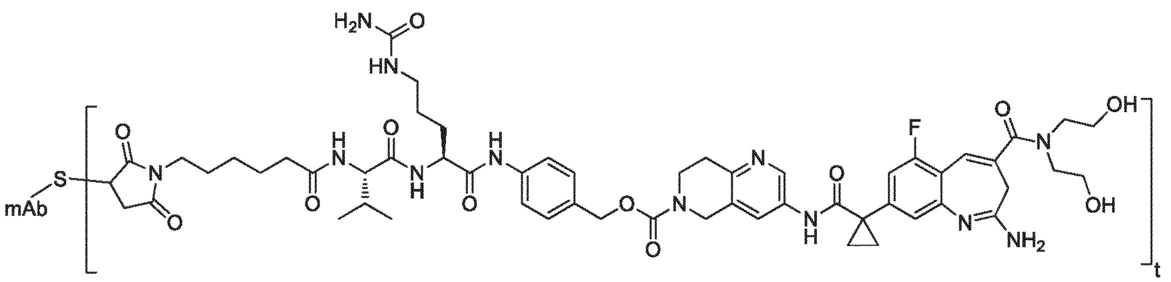

,

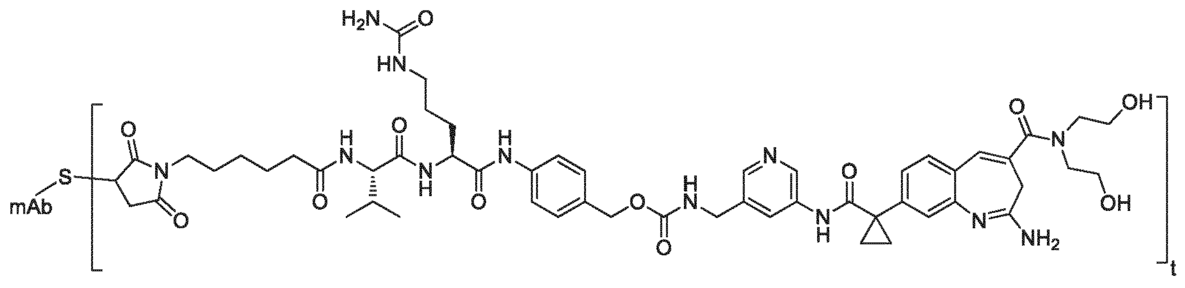

,

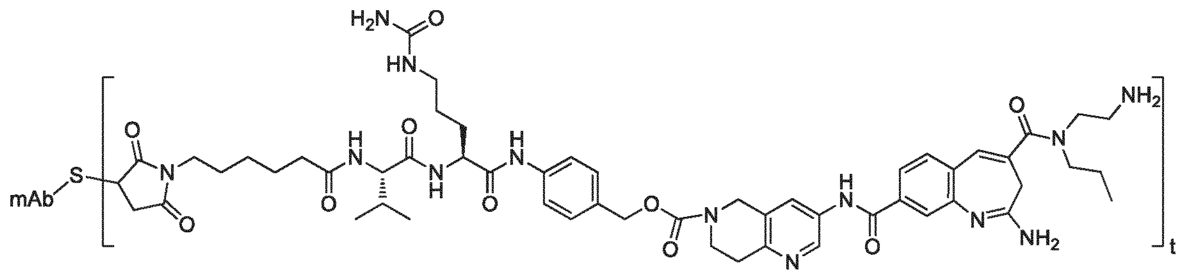

,

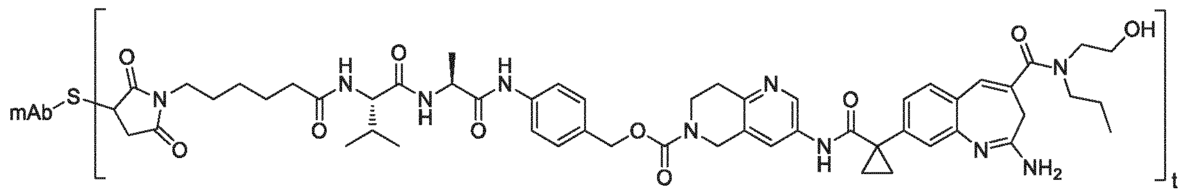

,

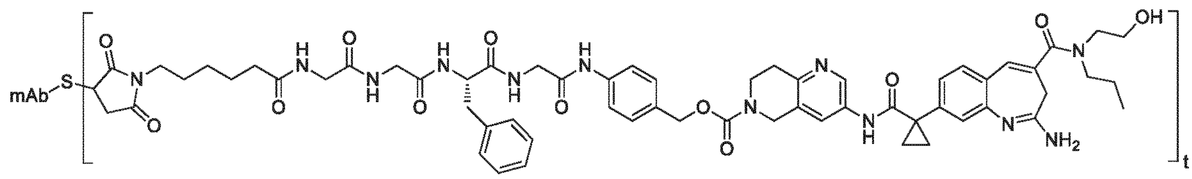

,

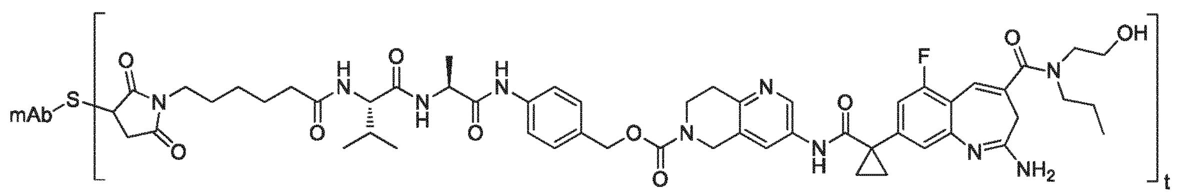

,

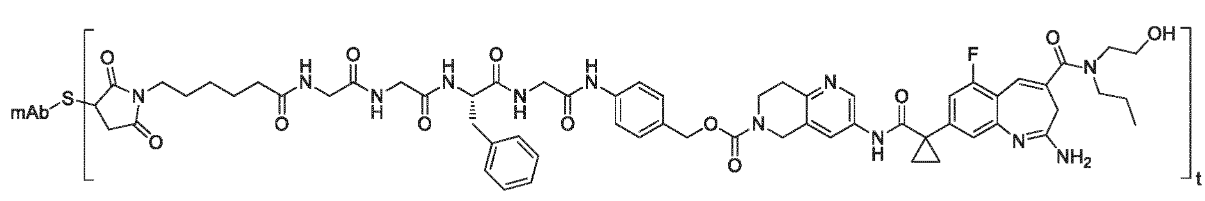

,

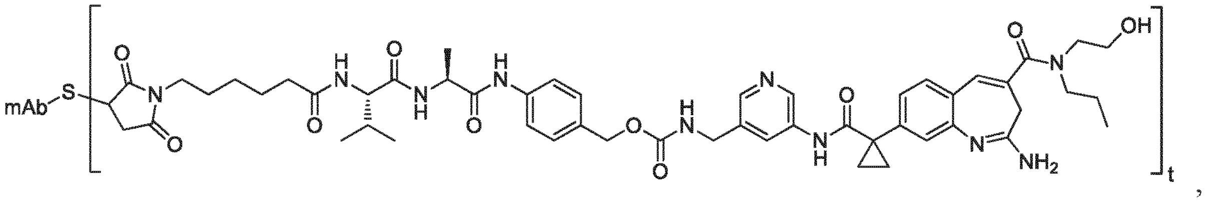

or

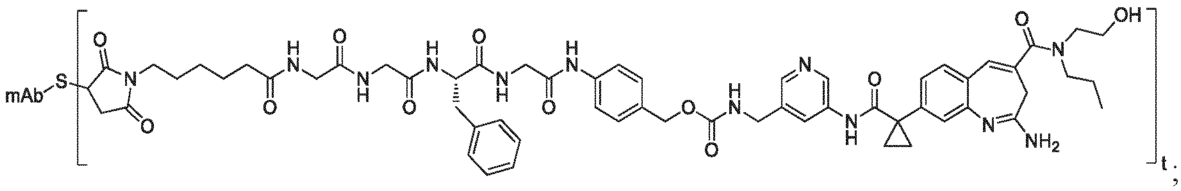

for example,

| Structure | t |
|---|---|
| 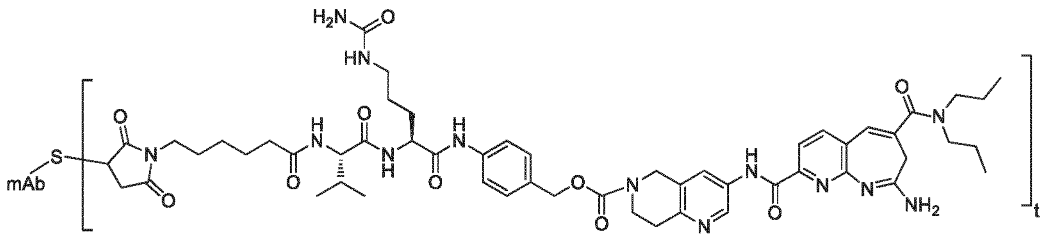 | 4.27 |
| 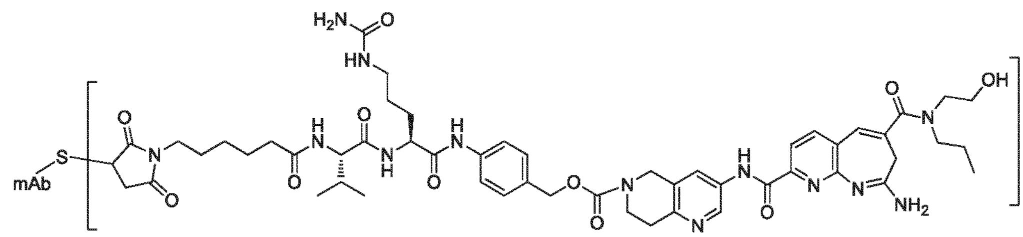 | 4.39 |
| 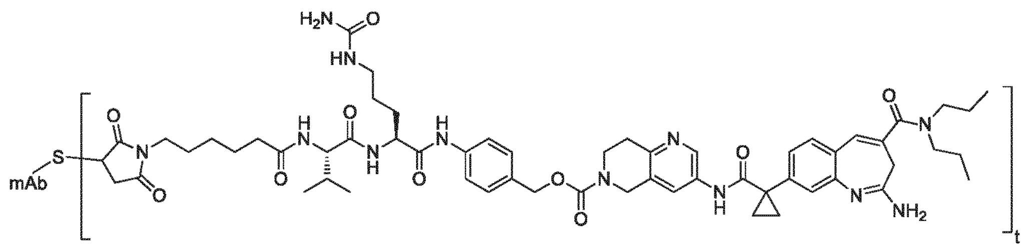 | 4.14 |
| 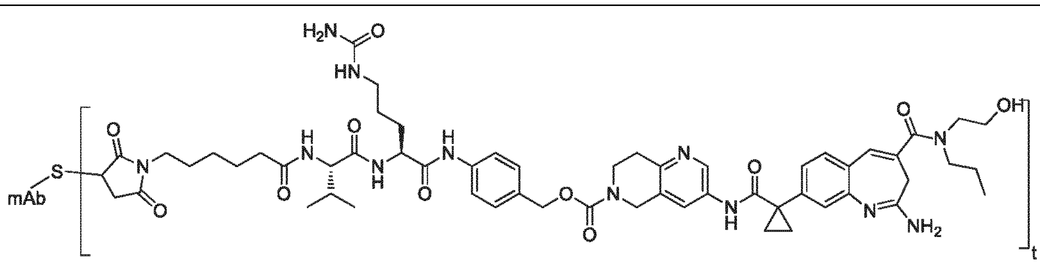 | 4.57 |

(continued)

| Structure | t |
|---|---|
| 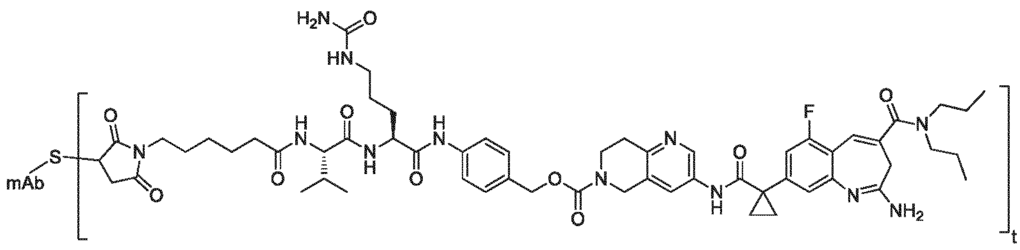 | 4.14 |
| 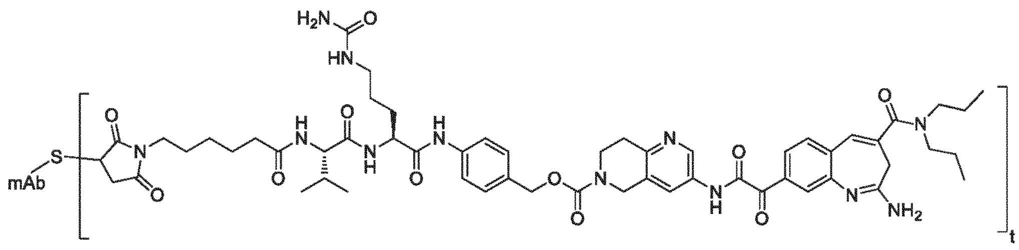 | 4.23 |
| 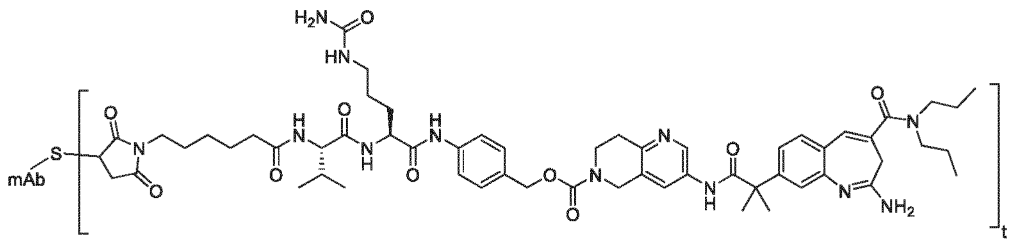 | 4.29 |
| 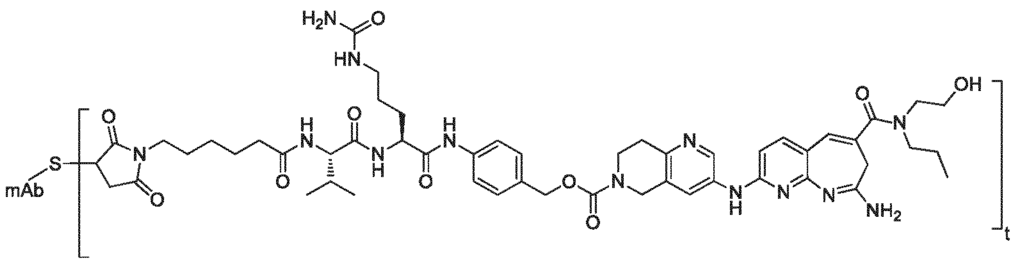 | 4.04 |
| 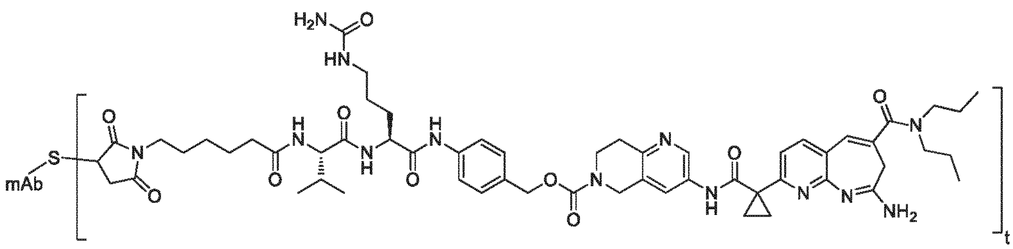 | 4.16 |
| 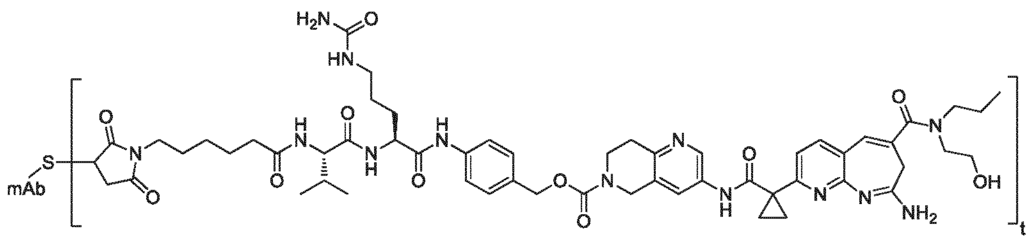 | 4.28 |

(continued)

| Structure | t |
|---|---|
| 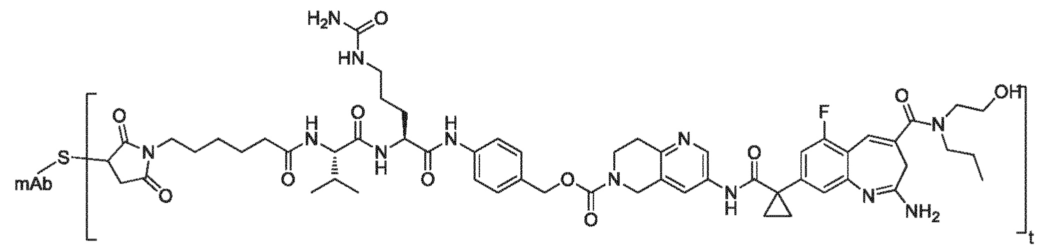 | 3.93 |
| 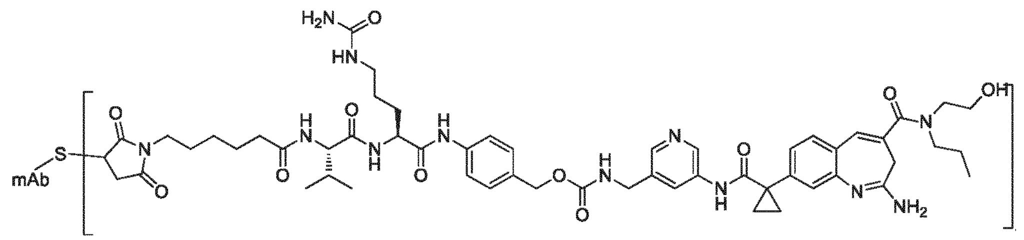 | 4.72 |
| 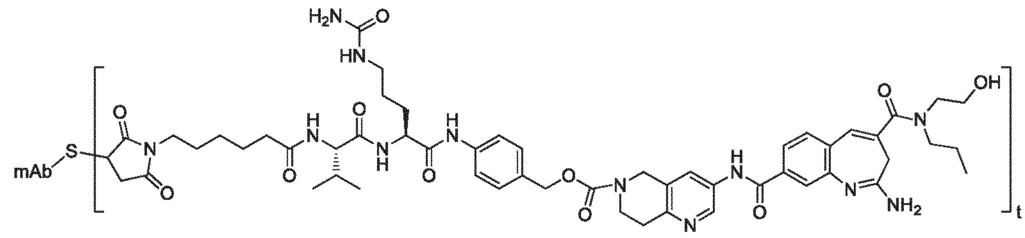 | 4.61 |
| 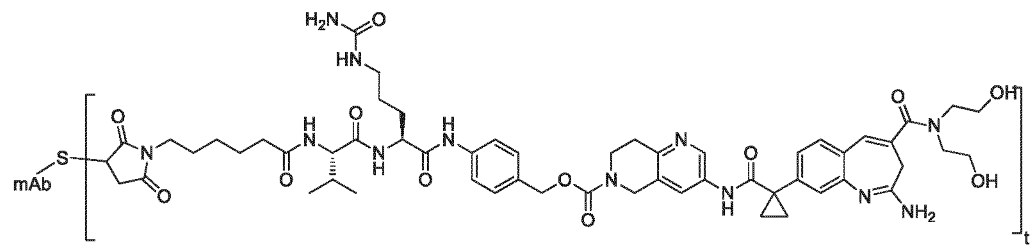 | 4.45 |
| 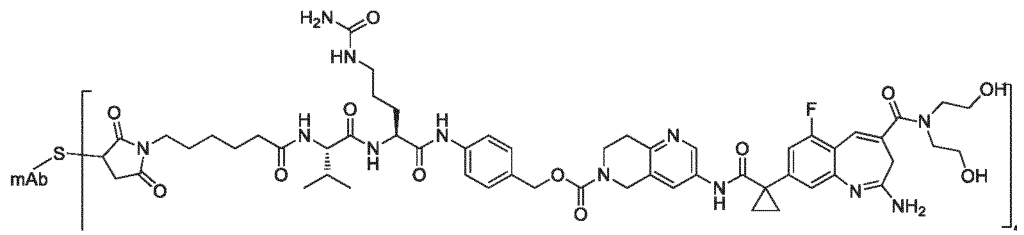 | 4.39 |
| 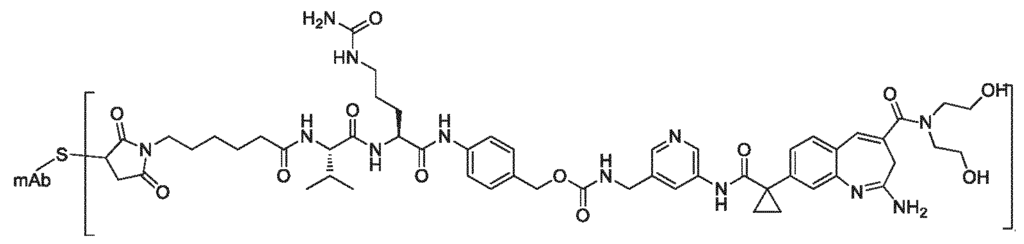 | 4.35 |

(continued)

| Structure | t |
|---|---|
| 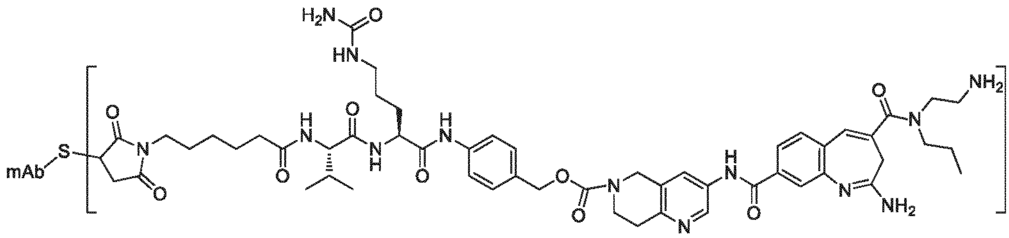 | 3.21 |
| 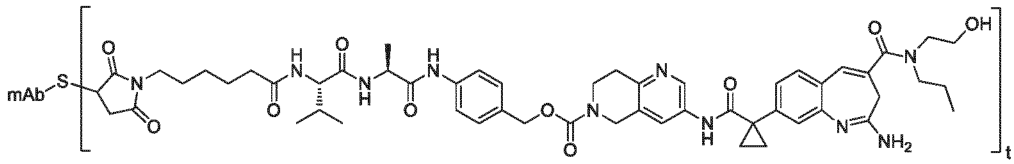 | 4.03 |
| 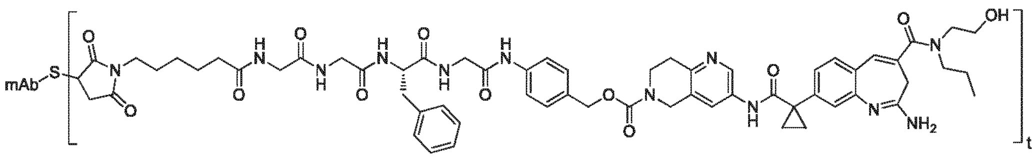 | 3.86 |
| 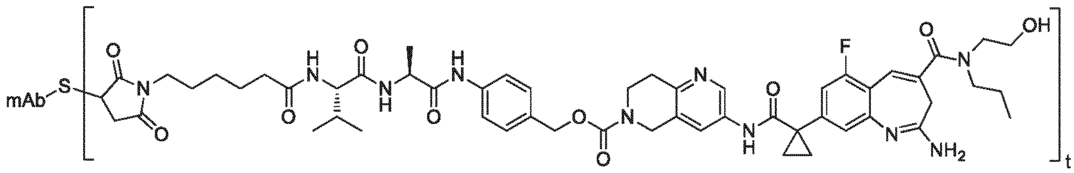 | 3.98 |
| 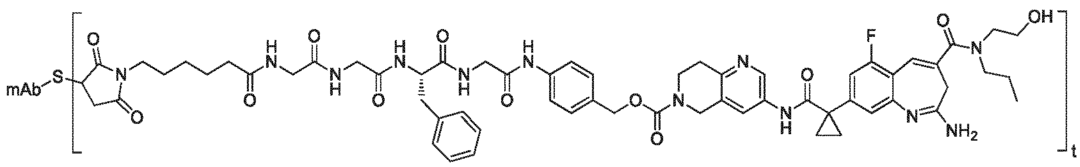 | 4.03 |
| 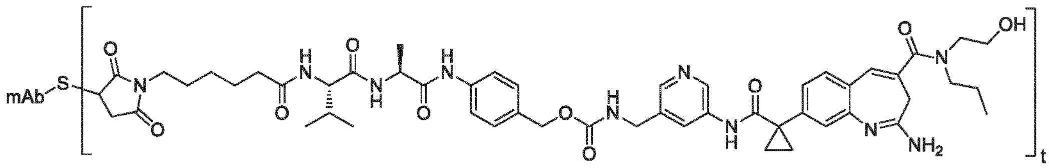 | 4.12 |
| 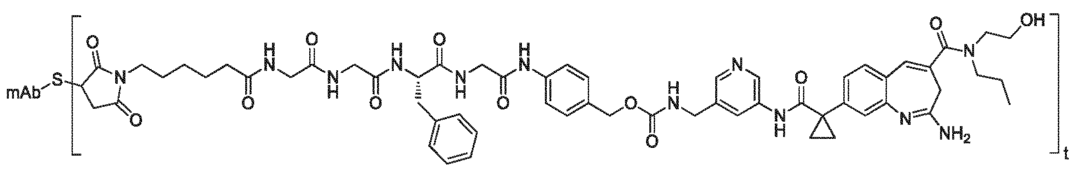 | 4.06 |

**30.** A pharmaceutical composition comprising substance K and a pharmaceutically acceptable excipient;

the substance K is substance K-1, substance K-2, or substance K-3;
the substance K-1 is the compound of formula I, the solvate thereof, the pharmaceutically acceptable salt thereof, or the solvate of the pharmaceutically acceptable salt thereof according to claims 1 to 11;
the substance K-2 is the compound of formula II', the solvate thereof, the pharmaceutically acceptable salt thereof, or the solvate of the pharmaceutically acceptable salt thereof according to claims 13 to 20;
the substance K-3 is the immune-stimulating antibody conjugate of formula III or the pharmaceutically acceptable salt thereof according to claims 21 to 29.

**31.** The pharmaceutical composition according to claim 30, wherein the pharmaceutical composition satisfies one or more than one of the following conditions:

uuu) the substance K is the compound of formula I or the pharmaceutically acceptable salt thereof, the compound of formula II or the pharmaceutically acceptable salt thereof, or the immune-stimulating antibody conjugate of formula III or the pharmaceutically acceptable salt thereof;
vvv) the amount of the substance K is a therapeutically effective amount;
www) the pharmaceutically acceptable excipient comprises a pharmaceutically acceptable carrier, diluent, and/or formulating agent;
xxx) the pharmaceutical composition is administered via the following routes: intramuscular, intraperitoneal, intravenous, subcutaneous, intradermal, or local administration.

**32.** A use of substance K or the pharmaceutical composition according to claim 30 or 31 in manufacture of a medicament;

the substance K is substance K-1, substance K-2, or substance K-3;
the substance K-1 is the compound of formula I, the solvate thereof, the pharmaceutically acceptable salt thereof, or the solvate of the pharmaceutically acceptable salt thereof according to claims 1 to 11;
the substance K-2 is the compound of formula II', the solvate thereof, the pharmaceutically acceptable salt thereof, or the solvate of the pharmaceutically acceptable salt thereof according to claims 13 to 20;
the substance K-3 is the immune-stimulating antibody conjugate of formula III or the pharmaceutically acceptable salt thereof according to claims 21 to 29;
the medicament is 1) a medicament that regulates T cells and other immune cells, 2) a medicament that treats and/or alleviates tumors or viral infectious diseases, or 3) a medicament that treats, alleviates, and/or prevents TLR8-mediated related diseases.

**33.** The use according to claim 32, wherein the use satisfies one or more than one of the following conditions:

yyy) the substance K is the compound of formula I or the pharmaceutically acceptable salt thereof, the compound of formula II or the pharmaceutically acceptable salt thereof, or the immune-stimulating antibody conjugate of formula III or the pharmaceutically acceptable salt;
zzz) the amount of the substance K is a therapeutically effective amount;
aaaa) the TLR8-mediated related diseases refer to tumors or viral infectious diseases;
bbbb) the tumor is a malignant tumor.

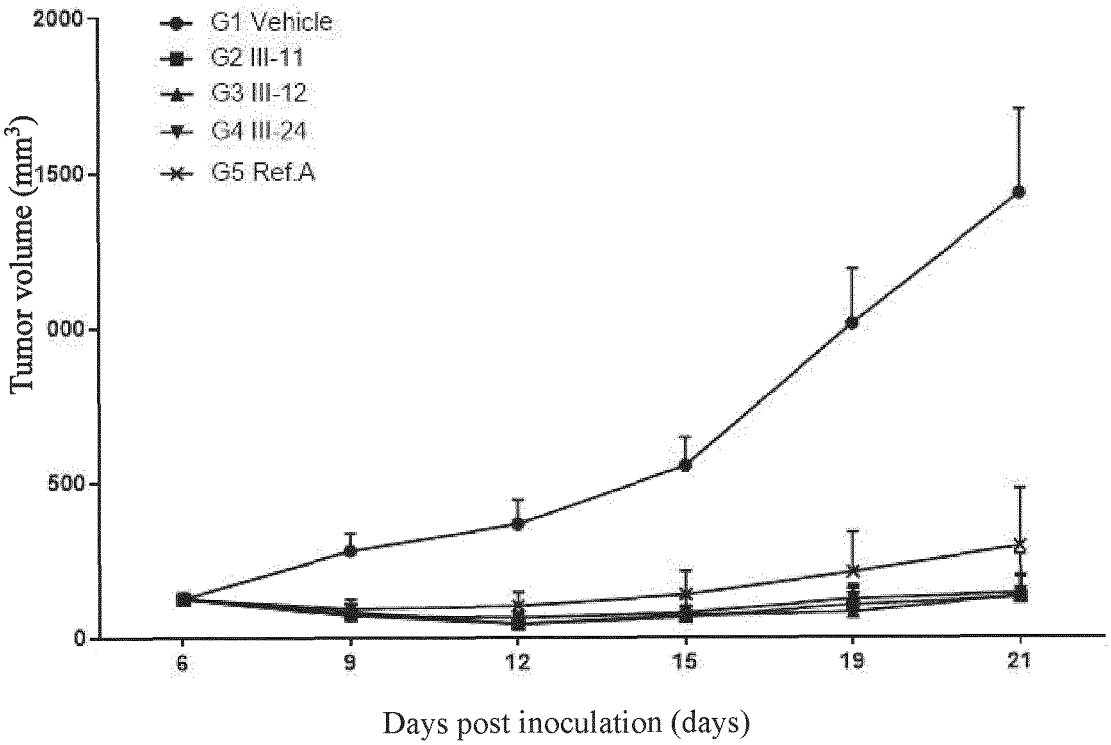
2000
1500
000
500
0
Tumor volume (mm3)
G1 Vehicle
G2 III-11
G3 III-12
G4 III-24
G5 Ref.A
6
9
12
15
19
21
Days post inoculation (days)

Figure 1

## INTERNATIONAL SEARCH REPORT

International application No.
**PCT/CN2022/093953**

**A. CLASSIFICATION OF SUBJECT MATTER**

C07D 223/16(2006.01)i; C07D 403/12(2006.01)i; A61K 31/55(2006.01)i; A61P 35/00(2006.01)i; A61P 31/12(2006.01)i

According to International Patent Classification (IPC) or to both national classification and IPC

**B. FIELDS SEARCHED**

Minimum documentation searched (classification system followed by classification symbols)

C07D A61K A61P

Documentation searched other than minimum documentation to the extent that such documents are included in the fields searched

Electronic data base consulted during the international search (name of data base and, where practicable, search terms used)

EPODOC WPI CNPAT CNKI STN: 上海迪诺医药科技有限公司, TLR8, 偶联物, 癌症, 感染, 免疫, conjugate, cancer, infection, Immunization, STN structural formula search

**C. DOCUMENTS CONSIDERED TO BE RELEVANT**

| Category* | Citation of document, with indication, where appropriate, of the relevant passages | Relevant to claim No. |
|---|---|---|
| X | WO 2021067242 A1 (BOLT BIOTHERAPEUTICS, INC.) 08 April 2021 (2021-04-08) description, pages 32-33, and claims 1-69 | 1-6, 30-33 |
| A | CN 109311851 A (F. HOFFMANN-LA ROCHE AG.) 05 February 2019 (2019-02-05) claims 1-17, and embodiments 1-11 | 1-33 |
| A | CN 109311854 A (F. HOFFMANN-LA ROCHE AG.) 05 February 2019 (2019-02-05) claims 1-21, and embodiments 1-28 | 1-33 |

☐ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **04 August 2022** | **17 August 2022** |

Name and mailing address of the ISA/CN

**China National Intellectual Property Administration (ISA/CN)**
**No. 6, Xitucheng Road, Jimenqiao, Haidian District, Beijing 100088, China**

Facsimile No. **(86-10)62019451**

Authorized officer

Telephone No.

Form PCT/ISA/210 (second sheet) (January 2015)

**INTERNATIONAL SEARCH REPORT**
**Information on patent family members**

International application No.
**PCT/CN2022/093953**

| Patent document cited in search report | Publication date (day/month/year) | Patent family member(s) | Publication date (day/month/year) |
|---|---|---|---|
| WO 2021067242 A1 | 08 April 2021 | AU 2020359446 A1 | 21 April 2022 |
| | | CA 3152601 A1 | 08 April 2021 |
| | | KR 20220077131 A | 08 June 2022 |
| | | IL 291760 A | 01 June 2022 |
| CN 109311851 A | 05 February 2019 | WO 2017216054 A1 | 21 December 2017 |
| | | US 2020172513 A1 | 04 June 2020 |
| | | EP 3468963 A1 | 17 April 2019 |
| | | JP 2019523770 A | 29 August 2019 |
| | | US 2019135788 A1 | 09 May 2019 |
| CN 109311854 A | 05 February 2019 | US 2019077764 A1 | 14 March 2019 |
| | | EP 3464274 A1 | 10 April 2019 |
| | | WO 2017202703 A1 | 30 November 2017 |
| | | JP 2019520330 A | 18 July 2019 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202110547613
- CN 202110825530X
- CN 202210515784
- US 5821337 A
- US 7560111 B
- WO 2018138356 A1
- CN 110612104 A


### Non-patent literature cited in the description


- *Int Immunopharmacol,* 2002, vol. 2, 443-451
- **HUDIS CA.** *N Engl J Med.,* 2007, vol. 357 (1), 39-51
- **CHO et al.** *Nature,* 2003, vol. 421, 756-760
- **FRANKLIN et al.** *Cancer Cell,* 2004, vol. 5, 317-328
- **AGUS et al.** *J Clin Oncol.,* 07 February 2005, vol. 23 (11), 2534-43
- **NORDSTROM J. et al.** *Breast Cancer Research,* 2011, vol. 13, R123
- **BERGSTROM D. A. et al.** *Cancer Res.,* 2015, vol. 75, LB-231
- *Proc. Natl. Acad. Sci. U.S.A.,* 15 May 1969, vol. 63 (1), 78-85
- **BERGE et al.** Pharmaceutically acceptable salts. *J. Pharm. Sci.,* 1977, vol. 66, 1-19